# EUROPÄISCHE PATENTSCHRIFT

(11) **EP 0 513 580 B1**
(45) Veröffentlichungstag und Bekanntmachung des Hinweises auf die Patenterteilung: **23.10.1996**
(21) Anmeldenummer: 92107059.5
(22) Anmeldetag: 24.04.1992
(51) Int. Cl.: C07D 261/20, C07C 69/618, C07C 69/734, C07C 69/65, C07F 9/40, C07D 235/26, C07D 235/28, C07D 239/52, A01N 43/54, C07D 239/46, C07D 239/34, C07D 213/64, C07D 239/88, C07D 317/30

(54) **Alpha-Phenylacrylsäurederivate, Verfahren zu ihrer Herstellung und ihre Verwendung zur Bekämpfung von Schädlingen und Schadpilzen**
Alpha-phenyl-acrylic acid derivatives, process for their preparation and their use as parasiticides and fungicides
Dérivés d'acide alpha-phénylacrylique, procédé pour leur préparation et leur usage comme parasiticides et fongicides

(30) Priorität: 17.05.1991 DE 4116090
(43) Veröffentlichungstag der Anmeldung: 19.11.1992
(73) Patentinhaber: BASF Aktiengesellschaft, 67063 Ludwigshafen (DE)
(72) Erfinder: Grammenos, Wassilios, Dr., W-6700 Ludwigshafen (DE); Kirstgen, Reinhard, Dr., W-6730 Neustadt (DE); Oberdorf, Klaus, Dr., W-6900 Heidelberg (DE); Sauter, Hubert, Dr., W-6800 Mannheim 1 (DE); Roehl, Franz, Dr., W-6700 Ludwigshafen (DE); Otter, Rainer, Dr., W-6947 Laudenbach (DE); Ammermann, Eberhard, Dr., W-6148 Heppenheim (DE); Lorenz, Gisela, Dr., W-6730 Neustadt (DE); Kardorff, Uwe, Dr., W-6800 Mannheim 1 (DE); Kuenast, Christoph, Dr., W-6701 Otterstadt (DE)

(56) Entgegenhaltungen:
- EP-A- 0 278 595
- EP-A- 0 280 185
- EP-A- 0 342 459
- EP-A- 0 350 691

## Beschreibung

Die vorliegende Erfindung betrifft α-Phenylacrylsäurederivate der allgemeinen Formel I, 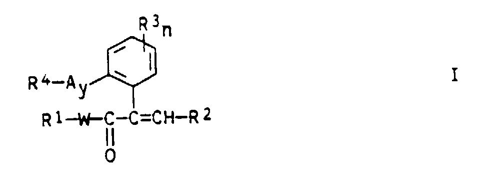 in der die Variablen folgende Bedeutung haben:
n
   0, 1, 2, 3 oder 4, wobei die Reste R³ verschieden sein können, wenn n für
   2, 3 oder 4 steht;
y
   0 oder 1;
R¹
   Wasserstoff;
C₁-C₁₅-Alkyl, C₃-C₁₅-Alkenyl, C₃-C₈-Alkinyl oder C₃-C₈-Cycloalkyl, wobei diese Gruppen ein bis fünf Halogenatome tragen können;
Vinyl oder Ethinyl, wenn W für eine direkte Bindung steht;
R²
   Cyano, C₂-C₄-Alkenyl, C₂-C₄-Alkinyl;
C₃-C₆-Cycloalkyl, wobei der Ring neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
C₁-C₄-Alkyl, welches unsubstituiert oder partiell oder vollständig halogeniert sein kann;
C₁-C₂-Alkyl, welches eine C₁-C₄-Alkoxygruppe, eine C₁-C₄-Alkylthiogruppe oder einen 3- bis 6-gliedrigen cyclischen Rest trägt, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
R³ Wasserstoff, Nitro, Cyano, Halogen;
C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
oder, für den Fall, daß n 2, 3 oder 4 bedeutet, zwei benachbarte Substituenten zusammen eine 1,3-Butadien-1,4-diylgruppe, welche ein bis vier Halogenatome und/oder eine oder zwei der folgenden Gruppen tragen kann: Nitro, Cyano, C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
R⁴ Wasserstoff; CHO;
ggf. subst. Alkyl; ggf. subst. Alkenyl; ggf. subst. Alkinyl;
ein ggf. subst. gesättigter oder ein- oder zweifach ungesättigter Cyclus mit bis zu acht Ringgliedern, welcher neben Kohlenstoffatomen ein bis drei Heteroatome aus der Gruppe Sauerstoff, Schwefel und Stickstoff als Ringglieder enthalten kann;
R'₃P-, R'₂P(=O)- und R"₂P(=O)-, wobei R' Phenyl und R" C₁-C₄-Alkoxy bedeutet;
oder ein ggf. subst. ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann;
W
   eine direkte Bindung, Sauerstoff, Schwefel oder Stickstoff, wobei der Stickstoff ein Wasserstoffatom, eine C₁-C₄-Alkylgruppe oder eine C₁-C₄-Alkoxygruppe tragen kann;
A
   -O-; -C(=O)-; -O-C(=O)-; -C(=O)-O-;
-S-; -S(=O)-; -O-S(=O)-; -S(=O)-O-; -S(=O)₂-; -O-S(=O)₂-; -S(=O)₂-O-;
-NR⁵-; -O-NR⁵-; -NR⁵-C(=O)-; -C(=O)-NR⁵-; -NR⁵-C(=O)-NR⁶-; -S(=O)-NR⁵-; -NR⁵-S(=O)₂-; -S(=O)₂-NR⁵- ;
-N=N-; -NR⁵-NR⁶-; -NR⁵-NR⁶-C(=O)-; -C(=O)-NR⁵-NR⁶-; -NR⁵-NR⁶-S(=O)₂-; -S(=O)₂-NR⁵-NR⁶- ;
-O-(C₂-C₄-alkylen)-O-; -C(=O)-(C₂-C₄-alkylen)-O-; -O-C(=O)-(C₂-C₄-alkylen)-O-; -C(=O)-O-(C₂-C₄-alkylen)-O-;
-S-(C₂-C₄-alkylen)-O-; -S(=O)₂-(C₂-C₄-alkylen)-O-; -O-S(=O)₂-(C₂-C₄-alkylen)-O-; -S(=O)₂-O-(C₂-C₄alkylen)-O-;
-NR⁵-(C₂-C₄-alkylen)-O-; -O-NR⁵-(C₂-C₄-alkylen)-O-; -NR⁵-C(=O)-O-(C₂-C₄-alkylen)-O-; -C(=O)-NR⁵-(C₂-C₄-alkylen)-O-; -NR⁵-C(=O)-NR⁶-(C₂-C₄-alkylen)-O-;
-NR⁵-NR⁶-(C₂-C₄-alkylen)-O-; -NR⁵-NR⁶-C(=O)-(C₂-C₄-alkylen)-O-; -C(=O)-NR⁵-NR⁶-(C₂-C₄-alkylen)-O-;
C₁-C₆-Alkylen, C₂-C₆-Alkenylen oder C₂-C₆-Alkinylen, wobei diese Kohlenstoffketten ein bis vier Reste, ausgewählt aus einer Gruppe bestehend aus C₁-C₄-Alkylgruppen und Halogenatomen, tragen können und wobei diese Kohlenstoffketten durch eine der folgenden Gruppen unterbrochen sein können oder durch eine der folgenden Gruppen an R⁴ oder an den Phenylring gebunden sein können: -O-, -S-, -NR⁵-, -C(=O)-, -C(=O)-O-, -O-C(=O)-, -S(=O)-, -S(=O)₂-, -S(=O)₂-O-, -O-S(=O)₂-, -NR⁵-C(=O)-, -C(=O)-NR⁵-, -NR⁵-C(=O)-NR⁶-, -N=N-, -NR⁵-NR⁶-, -NR⁵-NR⁶-C(=O)- und -C(=O)-NR⁵-NR⁶-;
R⁵ und R⁶ unabhängig voneinander
Wasserstoff, C₁-C₄-Alkyl oder C₁-C₄-Alkoxy;
wobei n nicht 0 bedeutet oder R¹ nicht C₁-C₅-Alkyl bedeutet oder W nicht Sauerstoff bedeutet, wenn R⁴ für ggf. subst. Phenyl steht und die Gruppierung A_{y} eine der folgenden Ketten bezeichnet: -O-, -CH₂O-, -OCH₂-, -CH₂CH₂-, -CH=CH-, -C≡C-, Oxy-C₂-C₆-alkylen oder Oxy-C₂-C₆-alenylen;
oder wobei n nicht 0 bedeutet oder R¹ nicht C₁-C₅-Alkyl bedeutet oder W nicht Sauerstoff bedeutet, wenn die Gruppierung A_{y} eine der folgenden Ketten bezeichnet: -C(=O)-O-CH₂-, -O-C(=O)-O-CH₂-, -C₁-C₁₂-Alkylen-C(=O)-OCH₂-, -Oxy-(C₁-C₁₂)-alkylen-C(=O)-OCH₂-, -C₁-C₁₂-Alkenylen-C(=O)-OCH₂- oder -Oxy-C₁-C₁₂-Alkenylen-C(=O)-OCH₂-, wobei die Alkylen- oder Alkenylengruppen Halogenatome tragen können.

Aus der Literatur sind Phenylcrotonsäurederivate als wirksam gegen Schadpilze bekannt (EP-A 280 185; EP-A 336 211; EP-A 337 211; EP-A 342 459; EP-A 348 766; Deutsche Patentanmeldung P 40 20 384.0; Deutsche Patentanmeldung P 40 20 388.3).

Aufgabe der vorliegenden Erfindung waren neue, zur Bekämpfung von Schadpilzen und Schädlingen geeignete Verbindungen sowie Verfahren zu ihrer Herstellung und ihre Verwendung.

Demgemäß wurden die eingangs definierten Verbindungen I gefunden. Außerdem wurden Verfahren zu ihrer Herstellung, sie enthaltende Mittel zur Bekämpfung von Schadpilzen und Verfahren zu ihrer Verwendung gefunden.

Desweiteren wurde gefunden, daß sich Verbindungen der allgemeinen Formel I' 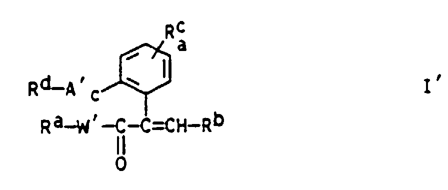 in der die Variablen die folgende Bedeutung haben:
a 0, 1, 2, 3 oder 4, wobei die Reste R^{c} verschieden sein können, wenn a für 2, 3 oder 4 steht;
c
   0 oder 1;
R^{a}
   Wasserstoff;
C₁-C₁₅-Alkyl, C₂-C₁₅-Alkenyl, C₂-C₈-Alkinyl oder C₃-C₈-Cycloalkyl, wobei diese Gruppen ein bis fünf Halogenatome tragen können;
R^{b}
   Cyano, C₂-C₄-Alkenyl, C₂-C₄-Alkinyl;
C₃-C₆-Cycloalkyl, wobei der Ring neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
C₁-C₄-Alkyl, welches unsubstituiert oder partiell oder vollständig halogeniert sein kann;
C₁-C₂-Alkyl, welches eine C₁-C₄-Alkoxygruppe, eine C₁-C₄-Alkylthiogruppe oder einen 3- bis 6-gliedrigen cyclischen Rest trägt, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch ein bis drei der folgenden Reste tragen kann: Halogen und C₁-C₄-Alkyl;
R^{c}
   Wasserstoff, Nitro, Cyano, Halogen;
C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio,
oder, für den Fall, daß n für 2, 3 oder 4 steht, zwei benachbarte Substituenten R³ zusammen eine 1,3-Butadien-1,4-diylgruppe, welche ein bis vier Halogenatome und oder ein oder zwei der folgenden Gruppen tragen kann: Nitro, Cyano, C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
R^{d} Wasserstoff;
ggf. subst. Alkyl; ggf. subst. Alkenyl; ggf. subst. Alkinyl;
ein ggf. subst. gesättigter oder ein- oder zweifach ungesättigter Cyclus mit bis zu acht Ringgliedern, welcher neben Kohlenstoffatomen ein bis drei Heteroatome aus der Gruppe Sauerstoff, Schwefel und Stickstoff als Ringglieder enthalten kann;
R'₃P-, R'₂P(=O)- und R"₂P(=O)-, wobei R' Phenyl und R" C₁-C₄-Alkoxy bedeutet;
oder ein ggf. subst. ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann;
W' eine direkte Bindung, Sauerstoff, Schwefel oder Stickstoff, wobei der Stickstoff ein Wasserstoffatom, eine C₁-C₄-Alkylgruppe oder eine C₁-C₄-Alkoxygruppe tragen kann;
A' -O-; -C(=O)-; -O-C(=O)-; -C(=O)-O-;
-S-; -S(=O)-; -O-S(=O)-; -S(=O)-O-; -S(=O)₂-; -O-S(=O)₂-; -S(=O)₂-O-;
-NR^{e}-; -O-NR^{e}-; -NR^{e}-C(=O)-; -C(=O)-NR^{e}-; -NR^{e}-C(=O)-NR^{e}-; -S(=O)-NR^{e}-; -NR^{e}-S(=O)-; -S(=O)₂-NR^{e}-; -NR^{e}-S(=O)₂-;
-N=N-; -NR^{e}-NR^{f}-; -NR^{e}-NR^{f}-C(=O)-; -C(=O)-NR^{e}-NR^{f}-; -NR^{e}-NR^{f}-S(=O)₂-; -S(=O)₂-NR^{e}-NR^{f}-;
-O-(C₂-C₄-alkylen)-O-; -C (=O)-(C₂-C₄-alkylen)-O-; -O-C(=O)-(C₂-C₄-alkylen)-O-; -C(=O)-O-(C₂-C₄-alkylen)-O-;
-S-(C₂-C₄-alkylen)-O-; -S (=O)-(C₂-C₄-alkylen)-O-; -O-S(=O)₂-(C₂-C₄-alkylen)-O-; -S(=O)₂-O-(C₂-C₄-alkylen)-O-;
-NR^{e}-(C₂-C₄-alkylen)-O-;-ONR^{e}-(C₂-C₄-alkylen)-O-;-NR^{e}-C(=O)-(C₂-C₄-alkylen)-O-; -C(=O)-NR^{e}-(C₂-C₄-alkylen)-O-; -NR^{e}-C(=O)-NR^{f}-(C₂-C₄-alkylen)-O-;
-N=N-(C₂-C₄-alkylen)-O-; -NR^{e}NR^{f}-(C₂-C₄-alkylen)-O-;-NR^{e}-NR^{f}-C(=O)-(C₂-C₄-alkylen)-O-; -C(=O)-NR^{e}-NR^{f}- (C₂-C₄-alkylen)-O-;
C₁-C₆-Alkylen, C₂-C₆-Alkenylen oder C₂-C₆-Alkinylen, wobei diese Kohlenstoffketten ein bis vier Reste, ausgewählt aus einer Gruppe bestehend aus C₁-C₄-Alkylgruppen und Halogenatomen, tragen können und wobei diese Kohlenstoffketten durch eine der folgenden Gruppen unterbrochen sein können oder durch eine der folgenden Gruppen an R⁴ oder an den Phenylring gebunden sein können: -O-, -S-, -NR^{e}-, -C(=O)-, -C(=O)-O-, -O-C(=O)-, -S(=O)-, -S(=O)₂-, -S(=O)₂-O-, -O-S(=O)₂-, -NR^{e}-C(=O)-, -C(=O)-NR^{e}-, -NR^{e}-C(=O)-NR^{f}-, -N=N-, -NR^{e}-NR^{f}-, -NR^{e}-NR^{f}-C(=O)- und -c(=O)-NR^{e}-NR^{f}-;
R^{e} und R^{f} unabhängig voneinander
   Wasserstoff, C₁-C₄-Alkyl oder C₁-C₄-Alkoxy;
zur Bekämpfung von Schädlingen eignen. Eine derartige Wirkung solcher Verbindungen war bislang der Literatur nicht zu entnehmen.

Daneben betrifft die vorliegende Erfindung Verbindungen der Formel I', in denen die Substituenten die folgende Bedeutung haben:
A'_{c} -O-, -S-, -OCH₂-, -SCH₂-, -CH=CH- -CO₂CH₂- oder -N(CH₃)-CH₂;
R^{a} Methyl;
W Sauerstoff;
R^{b} Methyl, Ethyl oder Methoxymethyl;
a 0, und
R^{d} 2,5-Dichlorphenyl, 5-Chlor-2-methylphenyl, 2,4-Dimethylphenyl, 2-Chlor-5-methylphenyl, 2,3,5-Trimethylphenyl oder 2-Methyl-5-isopropylphenyl,
   sowie, für den Fall, daß A' für -OCH₂- und R^{b} für Methyl steht, 2,5-Dimethylphenyl.

Man erhält die a-Phenylacrylsäurederivate der allgemeinen Formel I in Analogie zu den in der eingangs zitierten Literatur beschriebenen Verfahren.

Beispielsweise erhält man die Verbindungen I, in denen W nicht für eine direkte Bindung steht, vorteilhaft dadurch, daß man eine Phenoxalsäure der allgemeinen Formel II in an sich bekannter Weise in einem inerten organischen Lösungsmittel in Gegenwart einer Base mit einem Wittigbzw. Wittig-Horner Reagens der allgemelnen Formeln IIIa bzw. IIIb umsetzt. 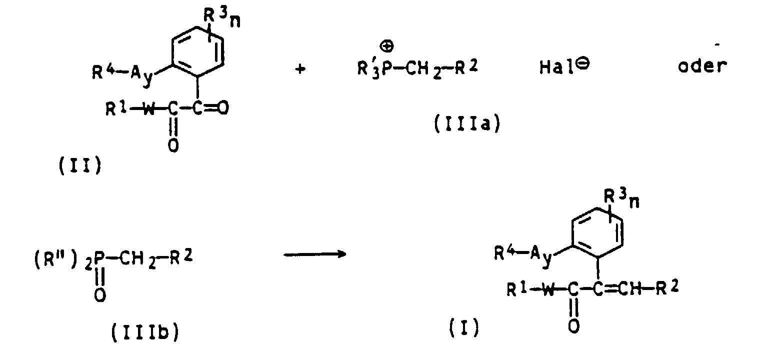 In der Formel IIIa steht R' für Phenyl.

Hal in der Formel IIIa bedeutet ein Halogenatom wie Fluor, Chlor, Brom und Iod.

In der Formel IIIb steht R'' für Alkoxy, vorzugsweise C₁-C₄-Alkoxy, insbesondere C₁-C₂-Alkoxy.

Die Umsetzung wird in der Regel bei Temperaturen von (-20)°C bis 60°C, vorzugsweise (-10)°C bis 30°C, durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran, Dimethylsulfoxid, Dimethylformamid und Diethylether.

Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall-und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriummethanolat, Kalium-t.-butylat, Natriumhydrid, Kaliumcarbonat und n-Butyllithium.

Die Basen werden im allgemeinen in äquimolaren Mengen eingesetzt, sie können aber auch im überschuß oder gegebenenfalls als Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem überschuß von 0,1 bis 10 mol-Äq., vorzugsweise 0,2 bis 1,5 mol-Äq. zu verwenden.

Die Umsetzung erfolgt zweckmäßigerweise bei Atmosphärendruck oder unter dem Eigendruck des jeweiligen Lösungsmittels.

Daneben erhält man die Verbindungen der allgemeinen Formel I dadurch, daß man eine Phenylessigsäure der allgemeinen Formel IV in an sich bekannter Weise in einem inerten organischen Lösungsmittel in Gegenwart einer Base mit einem Aldehyd der allgemeinen Formel V umsetzt.

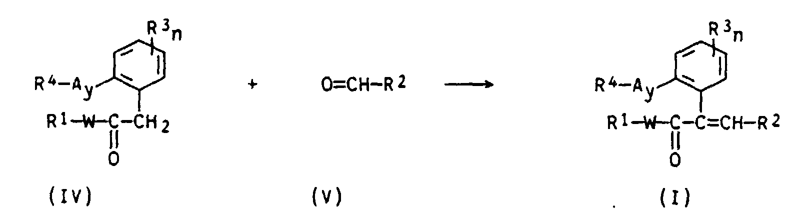

Die Umsetzung wird in der Regel bei Temperaturen von (-80)°C bis 100°C, vorzugsweise (-78)°C bis 80°C, durchgeführt.

Geeignete Lösungsmittel sind die eingesetzten Aldehyde selbst oder aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid und Dimethylformamid, besonders bevorzugt die eingesetzten Aldehyde V, Toluol, Cyclohexan, Tetrahydrofuran, Dimethylformamid und Diethylether.

Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid, Galziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium, Phenyllithium und Lithiumdiisopropylamid, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine, in Betracht.

Besonders bevorzugt werden Natriummethanolat, Kalium-t.-butylat, Lithiumdiisopropylamid und Kaliumcarbonat.

Die Basen werden im allgemeinen in katalytischen Mengen eingesetzt, sie können aber auch äquimolar, im überschuß oder gegebenenfalls als Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem überschuß von 0,1 bis 10 mol-Äq., vorzugsweise 0,2 bis 1,5 mol-Äq. zu verwenden.

Zur Steigerung der Ausbeute kann es von Nutzen sein, die Umsetzung in Gegenwart von Phasentransferkatalysatoren durchzuführen. In diesem Sinn geeignete Phasentransferkatalysatoren sind beispielsweise quartäre Ammonium- bzw. Phosphoniumsalze sowie Kronenether und Cryptaten, vorzugsweise Tetrabutylammonium- bzw. -phosphoniumbromid.

Auch bei dieser Methode arbeitet man vorteilhaft unter Normaldruck oder unter dem Eigendruck des jeweiligen Lösungsmittels.

Daneben erhält man die Verbindungen der allgemeinen Formel I auch dadurch, daß man eine Phosphonyl-phenylessigsäure der allgemeinen Formel VI in an sich bekannter Weise in einem inerten organischen Lösungsmittel in Gegenwart einer Base mit einem Aldehyd der allgemeinen Formel V umsetzt.

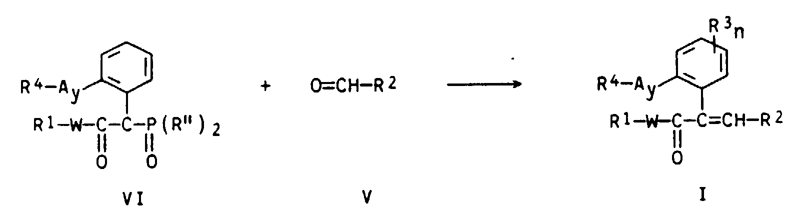

Die Umsetzung wird in der Regel bei Temperaturen von (-78)°C bis 80°C, vorzugsweise (-78)°C bis 60°C, durchgeführt.

Geeignete Lösungsmittel sind beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran, Dimethylformamid und Diethylether.

Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Lithiumdiisopropylamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyliithium und Phenyllithium, Alkylmagnesiumhalogenide wie Methylmagnesiumchlorid sowie Alkalimetall-und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine, in Betracht.

Besonders bevorzugt werden Natriummethanolat, Kalium-tert.-butylat, Lithiumdiisopropylamid, Kaliumcarbonat und n-Butyllithium.

Die Basen werden im allgemeinen im überschuß oder gegebenenfalls als Lösungsmittel verwendet.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem überschuß von 0,1 bis 10 mol-Äq., vorzugsweise 0,2 bis 1,5 mol-Äq. zu verwenden.

Auch bei dieser Methode arbeitet man zweckmäßigerweise bei Atmosphärendruck oder unter dem Eigendruck des jeweiligen Lösungsmittels.

Die für die vorstehend ausgeführten Umsetzungen benötigten Vorprodukte der allgemeinen Formel II in der W nicht für eine direkte Bindung steht (IIa), können, ausgehend von Phenylderivaten der allgemeinen Formel VII, erhalten werden. Die Seitenkette der Substruktur R⁴-A_{y}- wird zur besseren übersichtlichkeit als R∗ bezeichnet:

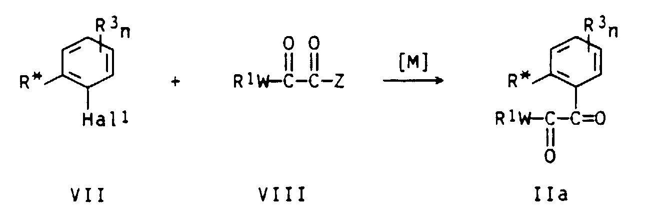

Hal¹ in der Formel VII steht für ein Halogenatom wie Fluor, Chlor, Brom und Jod, vorzugsweise Chlor und Brom, insbesondere Brom.

Der Substituent Z in der Formel VIII bedeutet ein Halogenatom wie für Hal¹ genannt oder eine C₁-C₄-Alkoxygruppe wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy.

Die Umsetzung wird in der Regel bei Temperaturen von (-30)°C bis 80°C, vorzugsweise (-10)°C bis 50°C, durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, sowie Dimethylsulfoxid und Dimethylformamid, besonders bevorzugt Diethylether und Tetrahydrofuran.

Es können auch Gemische der genannten Lösungsmittel verwendet werden.

Als Hilfsreagens [M] finden Metalle wie Magnesium oder Metallorganyle wie Methyl-Lithium, Butyl-Lithium und Phenyl-Lithium Verwendung.

Besonders bevorzugt werden Magnesium und Butyl-Lithium.

Das Hilfsreagens wird im allgemeinen in stöchiometrischen/katalytischen Mengen eingesetzt. Vorzugsweise verwendet man es in Mengen von 1 mol-Äq./mol-% bis 2 mol-Äq./mol-%, insbesondere 1 mol-Äq./mol-% bis 1,3 mol-Äq./mol-%, bezogen auf das Phenylderivat VII.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem überschuß von 0,1 bis 10 mol-Äq., vorzugsweise 0,2 bis 1,5 mol-Äq. zu verwenden.

Auch die Herstellung der Vorprodukte IIa erfolgt zweckmäßig bei Atmosphärendruck oder unter dem Eigendruck des jeweiligen Lösungsmittels.

Die Verbindungen IIa, in denen die Gruppierung R¹-W- Methoxy bedeutet, können durch allgemein bekannte Methoden derivatisiert werden:

### A. Verbindungen der Formel I, in der A für Sauerstoff (-O-), Schwefel (-S-) oder -C≡C- und y für 1 steht, lassen sich wie in Schema 1 beschrieben herstellen.

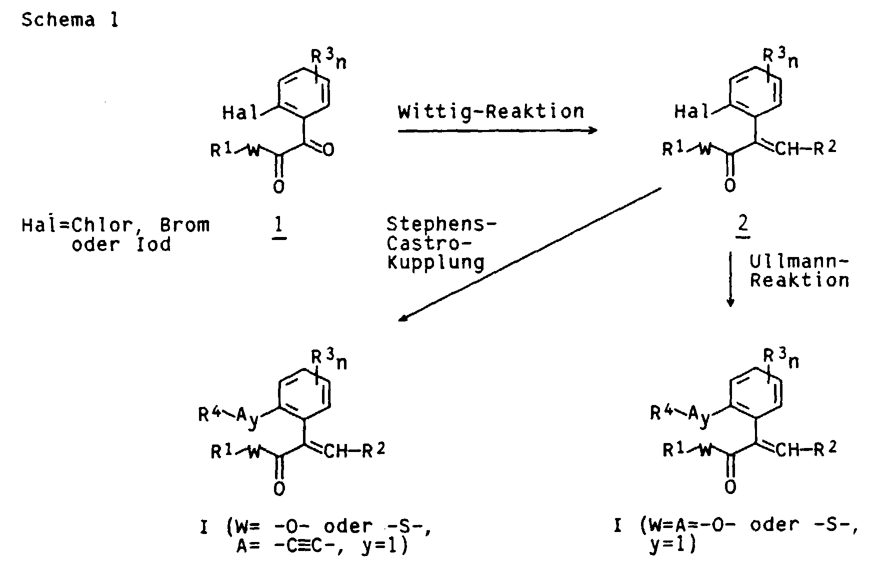

Die bekannten α-Ketocarbonsäure-Derivate der allgemeinen Formel 1 (vgl. EP 207 101 und US 3 622 569) lassen sich über eine Wittig- oder Wittig-Horner-Reaktion [vgl. EP 348 766] in an sich bekannter Weise in die α-Alkylacrylsäure-Derivate der Formel 2 umwandeln, aus den man im Sinne einer "Ullmann Reaktion" [vgl. 1) Russ.Chem.Rev. 43, 679-689 (1974); 2) J.Org.Chem. 29, 977, 3624 (1964)], in an sich bekannter Weise die Verbindungen der allgemeinen Formel I erhält, in der A und W Sauerstoff (-O-) oder Schwefel (-S-) und y 1 bedeuten.

Ausgehend von α-Alkylacrylsäure-Derivaten der allgemeinen Formel 2, in der Hal insbesondere für Iod oder Brom steht, kann man analog, im Sinne einer "Stephens-Castro-Kupplung" [vgl. J.Org.Chem 28, 2163 (1963)], die Verbindungen der allgemeinen Formel I erhalten, in der A für -C≡C-, und y für 1 steht, und W Sauerstoff (-O-) oder Schwefel (-S-) bedeutet.

### B. Verbindungen der Formel I, in denen Ay für -OCH₂-, -SCH₂- und -N(Alkyl)-CH₂- steht, lassen sich wie in Schema 2 beschrieben herstellen.

Zur besseren Übersichtlichkeit wird die Gruppe 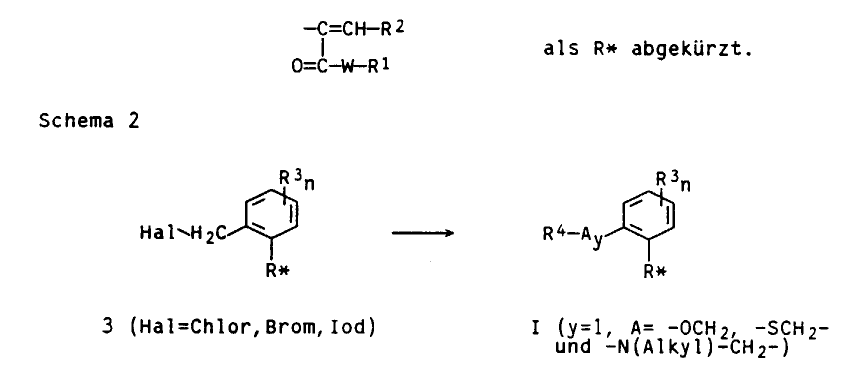 Benzylhalogenide der Formel 3 kann man in die Produkte der Formel I, in der y=1 und A -OCH₂, -SCH₂- oder -N(Alkyl)-CH₂- bedeutet, überführen, indem man sie mit Alkali-, Erdalkali-, Silber- oder Ammoniumsalzen der Formel R⁴-Kation, worin R⁴ die vorgehend angegebene Bedeutung hat, in einem Lösungsmittel wie Aceton, Toluol, Dimethylformamid oder Tetrahydrofuran unter Zusatz eines Katalysators z.B. 0,01 bis 10 Gew.-% Kaliumjodid oder Tetramethyldiamin zur Reaktion bringt.

Die Benzylhalogenide der allgemeinen Formel 3 lassen sich beispielsweise wie im folgenden Reaktionsschema (Schema 3) beschrieben herstellen.

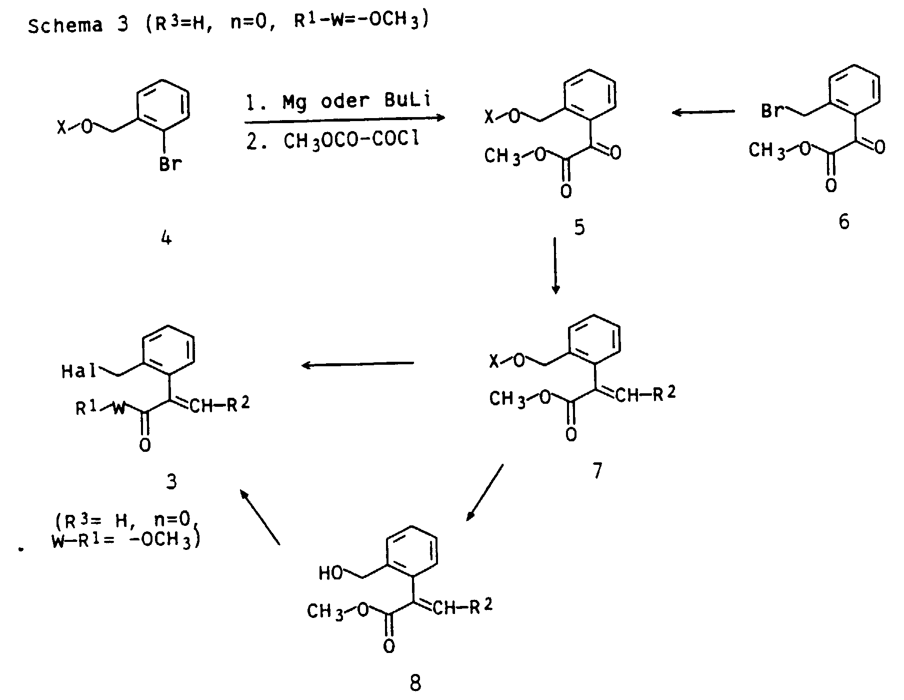

Ausgehend von Brombenzol-Derivaten der Formel 4 oder vom literaturbekannten (EP 342 459) o-Brommethyl-phenylglyoxylsäuremmethylester (6) kann man nach literaturbekannten Methoden [vgl. 1) J.Org.Chem. 46, 211-213 (1981); 2) EP 253 213; 3) Synthetic Comm. 11, 343 (1981); 4) EP 342 459] die Zwischenprodukte der allgemeinen Formel 5, in der X eine Alkoholschutz-Gruppe bedeutet wie z. B. Methyl, Ethyl, Phenyl oder Acetyl (vgl. T.W. Greene "Protective Groups in Organic Synthesis", J. Wiley and Sons, 1981, S. 10-86) erhalten, welche dann, nach Einführung der α-Alkylacryl-Gruppe entweder direkt mit Hilfe von Bortrihalogenid-Reagenzien der Formel B(Hal)₃ (vgl. Synthesis 1983, 249f) oder über die freien Benzylalkohole der Formel 8 zu den Benzylhalogeniden der allgemeinen Formel 3 umgewandelt werden.

Die Benzylhalogenide der allgemeinen Formel 3 lassen sich beispielsweise wie im folgenden Reaktionsschema 4 beschrieben (Schema 4) darstellen.

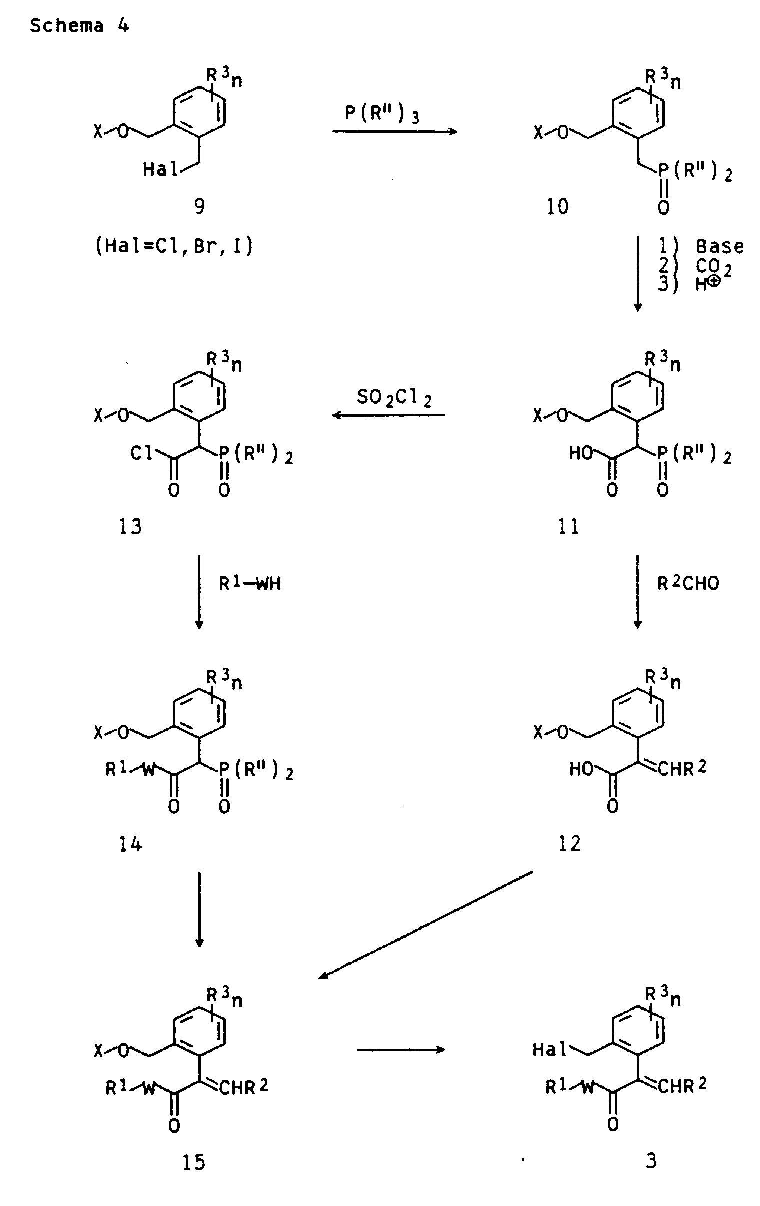

Ausgehend von den Benzylhalogeniden lassen sich z.B. über eine Michaelis-Arbuzov-Reaktion (vgl. Houben-Weyl, 4. Aufl., Bd. XII/1, S. 433ff) die Benzylphosphonate darstellen. Nach Deprotonierung mittels einer starken Base und Anlagerung von Kohlendioxid erhält man Carboxymethanphosphonsäureester (vgl. Synthesis, 661-664 (1986)). Wie im Schema aufgezeigt kann man die Phosphonsäureester direkt oder nach Modifikation des Carboxyterminus (vgl. Schema 7) mit Aldehyden R²CHO zu α,β-ungesättigten Carbonylderivaten umsetzen (vgl. Synthesis, 790-792 (1986)). Deren Umwandlung zu Benzylhalogeniden kann wie im Schema 3 beschrieben erfolgen.

### C. Verbindungen der allgemeinen Formel I, in der Ay für -CH=CH- steht, lassen sich wie in Schema 5 beschrieben herstellen. Zur besseren Übersichtlichkeit wird die Gruppe

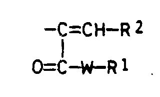

als R∗ abgekürzt.

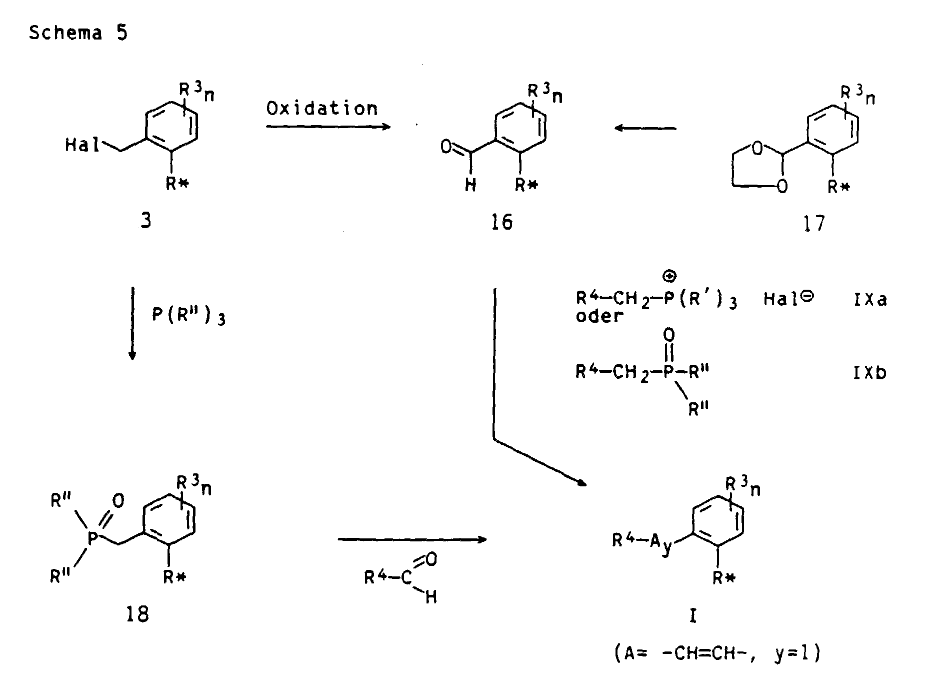

Die Herstellung der Verbindungen der Formel I, in der Ay für -CH=CH- steht, erfolgt durch Umsetzung von 2-Formyl-α-alkylacrylsäure-Derivaten der allgemeinen Formel 16 mit einem Phosphonsäureester der allgemeinen Formel IXb oder mit einem Phosphoniumsalz der Formel IXa Die Durchführung der Reaktion erfolgt in bekannter Weise [vgl. J.Am. Chem.Soc. 83, 1733 (1961)]. Die Ausgangsstoffe werden üblicherweise in einem stöchiometrischen Verhältnis eingesetzt. Ein Überschuß bis zu 10 % (Gew.-%) an einem der beiden Reaktionsteilnehmer über die stöchiometrische Menge hinaus ist möglich. Die Umsetzung wird in einem inerten Lösungs- oder Verdünnungsmittel (z.B. Diethylether, Tetrahydrofuran, Toluol) in Gegenwart einer äquivalenten Menge einer Base (z.B. Natriumhydrid, Natriumamid, Kalium-tert.-butylat, Natriummethylat, Butyllithium, Natrium-bis-trimethylsilylamid) durchgeführt. Die Umsetzungen verlaufen gewöhnlich in einem Temperaturbereich von -70°C bis +30°C. Da sie in einigen Fällen unter Wärmeentwicklung verlaufen, kann es von Vorteil sein, Kühlmöglichkeiten zu verwenden.

Zur Herstellung der neuen Verbindungen der Formel I, in der Ay -CH=CH-bedeutet, steht noch ein zweites Verfahren zur Verfügung. Dabei werden Aldehyde der Formel R⁴-CHO mit dem Benzylphosphonsäureester der Formel 18 umgesetzt [vgl. J. Am. Chem. Soc. 83, 1733 (1964)].

Die Darstellung der 2-Formyl-2-alkylacrylsäure-Verbindungen der Formel 16 gelingt beispielsweise in einfacher Weise durch Oxidation der Benzylhalogenide 3, z.B. mit Methylmorpholin-N-oxidmonohydrat (vgl. EP 393 428) oder mit Dimethylsulfoxid (analog J. Chem. Soc. 1964, 520).

### D. Verbindungen der Formel I in der Ay für -NH- steht lassen sich z.B. wie in Schema 6 beschrieben herstellen.

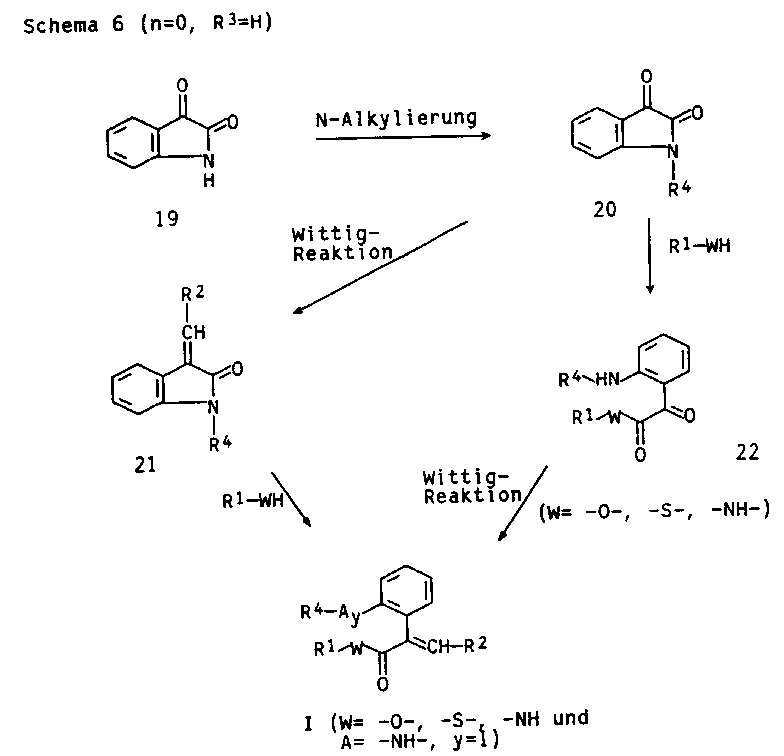

Ausgehend von Isatin (19) wird nach literaturbekannten Methoden das N-alkylierte Isatin 20 hergestellt [vgl. 1) J. Heterocyclic Chem. 24, 1249-1251 (1987); 2) Liebigs. Ann. Chem. 794-804 (1982); 3) Chem.Ber. 3060-3062 (1966)], welches dann entweder direkt oder nach Aufbau der α-Alkylacryl-Gruppe nach ebenso bekannten Methoden im Sinne einer nucleophilen Ringöffnungsreaktion in die Produkte der allgemeinen Formel I (A= -NH- und W= -O-, -S- oder -NH-) überführt wird [vgl. 1) Chem. Ber. 3060-3062 (1966); 2) Zh. Org. Khim. 22 (1986) 11, 2409-20; 3) Liebigs Ann. Chem. 1982, 794-804].

### E. Verbindungen der allgemeinen Formel I, in der R⁴ für eine Gruppe

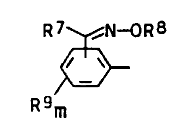

und Ay für -OCH₂- steht, lassen sich beispielsweise so herstellen, indem man einen substituierten Oximether der Formel 23 mit einem Benzylhalogenid der Formel 3 umsetzt.

Zur besseren übersichtlichkeit wird die Gruppe

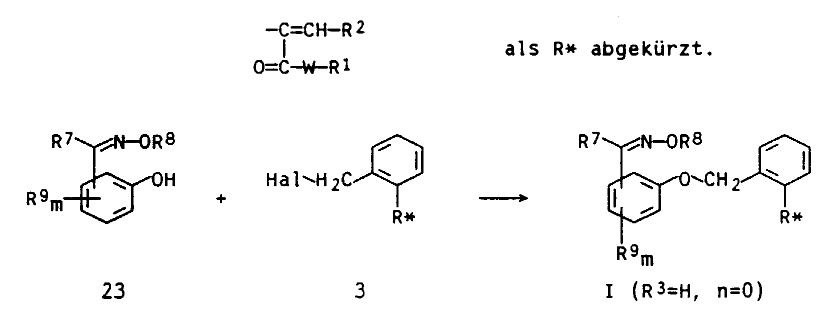

Die beschriebene Umsetzung kann z.B. in einem inerten Lösungs- oder Verdünnungsmittel (z.B. Aceton, Acetonitril, Dimethylsulfoxid, Dimethylformamid, N-Methylpyrrolidon, N,N'-Dimethylpropylenharnstoff oder Pyridin) unter Verwendung einer Base (z.B. Natriumcarbonat, Kaliumcarbonat) durchgeführt werden.

Alternativ kann auch so vorgegangen werden, daß man die Verbindungen der allgemeinen Formel 23 zunächst mit einer Base (z.B. Natriumhydroxid, Kaliumhydroxid, Natriummethanolat) in die entsprechenden Natrium- bzw. Kaliumphenolate überführt und diese dann in einem inerten Lösungs- oder Verdünnungsmittel (z.B. Dimethylformamid) mit den substituierten Benzylverbindungen der allgemeinen Formel 3 zu den erfindungsgemäßen Verbindungen der Formel I umsetzt.

Die Darstellung der neuen Verbindungen der Formel I, in der R⁴, y, A und R∗ die vorgehend genannte Bedeutung besitzen, kann auch so erfolgen, daß man die neuen substituierten Carbonylverbindungen der allgemeinen Formel 25 mit einem Hydroxylamin der allgemeinen Formel 26 oder mit einem Säureadditionssalz (z.B. Hydrochlorid, Hydrobromid) von 26 umsetzt.

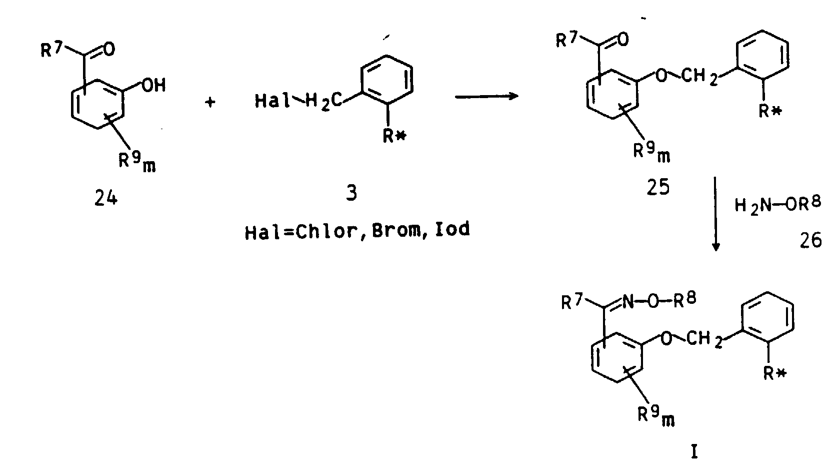

Die neuen substituierten Carbonylverbindungen der Formel 25 erhält man durch Umsetzung der substituierten Benzylverbindungen der allgemeinen Formel 3, in der Hal, Chlor, Brom oder Iod bedeutet, mit substituierten Carbonylverbindungen der Formel 24. Die Verbindungen der Formel 26 sind entweder bekannt, oder können nach allgemein üblichen Verfahren und Methoden hergestellt werden (vgl. EP 386 561).

Die nachfolgende Reaktion mit einem substituierten Hydroxylamin der Formel 26 kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Natriumcarbonat, Kaliumcarbonat, Kaliumhydroxid) zuzusetzen (vgl. EP 386 561).

### F. Verbindungen der allgemeinen Formel I, in der W -S-, -NH-, -N(Alkyl)-, -N(Alkoxy)- oder eine chemische Bindung bedeutet, lassen sich wie in Schema 7 beschrieben herstellen.

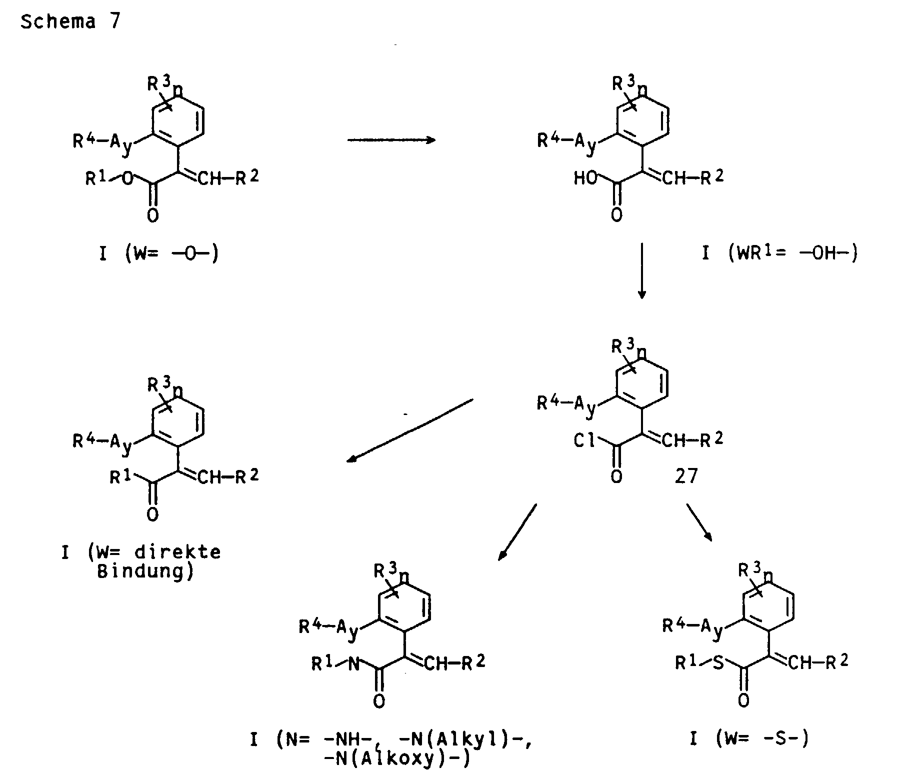

Verbindungen der allgemeinen Formel I, in der W für eine direkte Bindung steht, erhält man aus den Säurechloriden der Formel 27 durch Umsetzung mit Metallorganylen vom Typ [M]Rₓ(Hal)_{z}, wobei x für 1 oder 2 und z für 0 oder 1 steht, und [M] ein Metall wie z.B. Lithium, Magnesium, Zink, Cadmium oder Quecksilber, insbesondere Zink und Magnesium bedeutet. Hal bedeutet in der oben angegebenen Formel Chlor, Brom oder Iod insbesondere aber Chlor oder Brom.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem überschuß von 0,1 bis 10 mol-Äq., vorzugsweise 0,2 bis 1,5 mol-Äq. zu verwenden.

Die Umsetzung wird in der Regel bei Temperaturen von (-30)°C bis 80°C, vorzugsweise (-10)°C bis 60°C, durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, sowie Dimethylsulfoxid und Dimethylformamid, besonders bevorzugt Diethylether und Tetrahydrofuran. Es können auch Gemische der genannten Lösungsmittel verwendet werden (vgl. Houben-Weyl, Bd. VII/2 a, 558 ff (1973)).

Verbindungen der allgemeinen Formel I, in der W für Schwefel (-S-) steht, erhält man beispielsweise aus den Säurechloriden 27 analog Houben-Weyl, Bd. E 5, S. 861ff (1985).

Alternativ können die obengenannten Verbindungen der allgemeinen Formel I auch aus Carbonsäuren der Formel I (WR¹= -OH) hergestellt werden (vgl. Houben-Weyl, Bd. E 5, S. 855ff (1985)).

Verbindungen der allgemeinen Formel I, in der W gleich -NH- oder -N(Alkyl)-, -N(Alkoxy)- ist, lassen sich in an sich bekannter Weise aus den Säurechloriden 27 analog Organikum 16. Auflage S. 412 (1985) herstellen.

Die für die vorstehend ausgeführten Umsetzungen benötigten Vorprodukte der allgemeinen Formel I (WR¹= -OH) können ausgehend von Verbindungen der allgemeinen Formel I (W= -O-) nach literaturbekannten Methoden (vgl. Organikum 16. Auflage, S. 415, 622 (1985)) erhalten werden.

Aus den so erhaltenen Carbonsäuren I (WR¹ -OH) lassen sich in an sich bekannter Weise die Säurechloride 27 herstellen (vgl. Organikum, 16. Auflage, S. 423ff (1985) und Houben-Weyl, Bd. VIII, 464ff).

Im Hinblick auf ihre biologische Wirkung zur Bekämpfung von Schadpilzen kommen Verbindungen I in Betracht, in denen die Indices und die Substituenten die folgende Bedeutung haben:
n
   0, 1, 2, 3 oder 4, wobei die Reste R³ verschieden sein können, wenn n für 2, 3 oder 4 steht; vorzugsweise 0, 1 oder 2, insbesondere 0 oder 1;
y
   0 oder 1;
R¹
   Wasserstoff;
C₁-C₁₅-Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Propyl und 1-Methylethyl, insbesondere Methyl und Ethyl;
C₃-C₁₅-Alkenyl, besonders C₃-C₆-Alkenyl wie 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise 2-Propenyl, 2-Butenyl und 3-Methyl-2-butenyl, insbesondere 2-Propenyl;
C₃-C₈-Alkinyl, besonders C₃-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl und 2-Butinyl, insbesondere 2-Propinyl;
oder C₃-C₈-Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl, insbesondere Cyclopropyl,
wobei diese Kohlenwasserstoff-Gruppen ein bis fünf Halogenatome wie Fluor, Chlor, Brom und Iod, vorzugsweise Fluor, Chlor oder Brom, insbesondere Fluor und Chlor tragen können;
R¹bedeutet außerdem Vinyl oder Ethinyl, wenn W für eine direkte Bindung steht;
R²Cyano;
C₂-C₄-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl, vorzugsweise C₂-C₃-Alkenyl insbesondere Ethenyl und 1-Propenyl;
C₂-C₄-Alkinyl wie Ethinyl, 1-Propinyl, 2-Propinyl, 1-Butinyl, 2-Butinyl, 3-Butinyl und 1-Methyl-2-propinyl, vorzugsweise Ethinyl und 2-Propinyl;
einen 3- bis 6-gliedrigen, gesättigten oder partiell oder vollständig ungesättigten cyclischen Rest, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, wie Cyclopropyl, Cyclobutyl, Cyclopentyl,
Cyclohexyl, Epoxidyl, 2-Tetrahydrofuranyl, 3-Tetrahydrofuranyl, 2-Tetrahydrothienyl, 3-Tetrahydrothienyl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2,5-Dihydrothien-2-yl, 2,5-Dihydrothien-3-yl, 2-Tetrahydropyranyl, 2-Tetrahydrothianyl, 1,3-Dioxolan-2-yl, 1,3-Dithiolan-2-yl, N-Morpholinyl und N-Pyrrolidinyl, vorzugsweise Cyclopropyl und Epoxidyl, insbesondere Cyclopropyl;
   wobei der Cyclus zusätzlich noch ein bis drei der folgenden Reste tragen kann: Halogen wie vorstehend genannt, vorzugsweise Fluor und Chlor und C₁-C₄-Alkyl wie vorstehend im allgemeinen genannt, vorzugsweise C₁-C₂-Alkyl;
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise C₁-C₃-Alkyl, insbesondere Methyl und Ethyl, wobei diese Gruppen durch Halogenatome wie Fluor, Chlor, Brom und Iod, vorzugsweise Fluor und Chlor, partiell oder vollständig halogeniert sein können;
R² bedeutet außerdem
   C₁-C₂-Alkyl, welches eine der folgenden Gruppen trägt:
   - C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise
   - C₁-C₃-Alkoxy, insbesondere Methoxy und Ethoxy;
   - C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise C₁-C₃-Alkylthio, insbesondere Methylthio und Ethylthio;
   - oder einen 3- bis 6-gliedrigen, gesättigten oder partiell oder vollständig ungesättigten cyclischen Rest, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, wie Cyclopropyl, Cyclobutyl, Cyclopentyl, Cyclohexyl, Epoxidyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2,5-Dihydrothien-2-yl, 2,5-Dihydrothlen-3-yl, 2,3-Pyrrolin-2-yl, 2,3-Pyrrolin-3-yl, 2,5-Pyrrolin-2-yl, 2,5-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, -yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, -5-yl, 2-Piperidinyl, 3-Piperidinyl, 4-Piperidinyl, 3-Tetrahydropyridazinyl, 1-Tetrahydropyridazinyl, 2-Tetrahydropyrimidinyl, 4-Tetrahydropyrimidinyl, 1-Tetrahydropyrimidinyl, 2-Tetrahydropyrazinyl, 1,3,5-Tetrahydrotriazin-2-yl und 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, N-Morpholinyl und Dihydrochinazolinyl, insbesondere 2-Oxazolidinyl, 1,3-Dihydrooxazin-2-yl, Oxazol-2-in-2-yl, Oxiranyl, 1,3-Dithian-2-yl, 2-Tetrahydropyranyl und 1,3-Dioxolan-2-yl, vorzugsweise Cyclopropyl, 1,3-Dioxolan-2-yl und 1,3-Dithiolan-2-yl, insbesondere Cyclopropyl;
      wobei der Cyclus zusätzlich noch ein bis drei der folgenden Reste tragen kann: Halogen wie vorstehend genannt, vorzugsweise Fluor und Chlor und C₁-C₄-Alkyl wie vorstehend im allgemeinen genannt, vorzugsweise C₁-C₂-Alkyl;
R³
   Wasserstoff; Nitro; Cyano;
Halogen wie Fluor, Chlor, Brom und Jod, vorzugsweise Fluor, Chlor und Brom, insbesondere Fluor und Chlor;
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 1-Methylethyl und 1,1-Dimethylethyl, insbesondere Methyl und 1,1-Dimethylethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy und 1,1-Dimethylethoxy, insbesondere Methoxy;
   partiell oder vollständig halogeniertes C₁-C₄-Alkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Dichlormethyl, Trichlormethyl und Trifluormethyl, insbesondere Trifluormethyl;
partiell oder vollständig halogeniertes C₁-C₄-Alkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy, 1,1,2,2-Tetrafluorethoxy und Pentafluorethyloxy, vorzugsweise Trichlormethoxy, Diflourmethoxy, Trifluormethoxy, Chlordifluormethoxy und 1,1,2,2-Tetrafluorethoxy insbesondere Difluormethoxy und 1,1,2,2-Tetrafluorethoxy;
oder C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio und Ethylthio, insbesondere Methylthio;
oder, für den Fall, daß n für 2, 3 oder 4 steht, eine 1,3-Butadien-1,4-diylgruppe, welche ein bis vier Halogenatome wie Fluor, Chlor, Brom und Jod, vorzugsweise Fluor und Chlor, insbesondere Chlor und/oder ein oder zwei der folgenden Gruppen tragen kann:
   Nitro, Cyano,
   - C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl und Ethyl, insbesondere Methyl,
   - C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy, insbesondere Methoxy,
   - partiell oder vollständig halogeniertes C₁-C₄-Alkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl,
   - partiell oder vollständig halogeniertes C₁-C₄-Alkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethoxy und 1,1,2,2-Tetrafluorethoxy, insbesondere Difluormethoxy,
   - oder C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio;
R⁴
   Wasserstoff; CHO;
ggf. subst. Alkyl; ggf. subst. Alkenyl; ggf. subst. Alkinyl;
ein ggf. subst. gesättigter oder ein- oder zweifach ungesättigter 3- bis 6-gliedriger Cyclus, welcher neben Kohlenstoffatomen ein bis drei Heteroatome aus der Gruppe Sauerstoff, Schwefel und Stickstoff als Ringglieder enthalten kann;
R'₃P-, R'₂P(=O), R''₂P(=O), wobei R' Phenyl und R'' C₁-C₄-Alkoxy wie vorstehend genannt, insbesondere Methoxy und Ethoxy, bedeutet;
oder ein ggf. subst. ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann;
W
   eine direkte Bindung, Sauerstoff, Schwefel
oder Stickstoff, welcher Wasserstoff, eine C₁-C₄-Alkylgruppe wie Methyl, Ethyl, Propyl, 1-Methylethyl, Butyl, 1-Methylpropyl, 2-Methylpropyl und 1,1-Dimethylethyl, vorzugsweise Methyl, Ethyl und Propyl, insbesondere Methyl und Ethyl,
oder Stickstoff, welcher eine C₁-C₄-Alkoxygruppe wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy und Propyloxy, insbesondere Methoxy, tragen kann;
A
   -O-; -C(=O)-; -O-C(=O)-; -C(=O)-O-; -S-; -S(=O)-; -O-S(=O)-; -S(=O)-O-; -S(=O)₂-; -O-S(=O)₂-; -S(=O)₂-O-; -NR⁵-; -O-NR⁵-; -NR⁵-C(=O)-; -C(=O)-NR⁵-; -NR⁵-C(=O)-NR⁶-; -S(=O)-NR⁵-; -NR⁵-S(=O)₂-; -S(=O)₂-NR⁵-; -N=N-; -NR⁵-NR⁶-; -NR⁵-NR⁶-C(=O)-; -C(=O)-NR⁵-NR⁶-; -NR⁵-NR⁶-S(=O)₂-; -S(=O)₂-NR⁵-NR⁶-; -O-(C₂-C₄-alkylen)-O-; -C(=O)-(C₂-C₄-alkylen)-O-; -O-C(=O)-(C₂-C₄-alkylen)-O-; -C(=O)-O-(C₂-C₄-alkylen)-O-; -S-(C₂-C₄-alkylen)-O-; -S(=O)₂-(C₂-C₄-alkylen)-O-; -O-S(=O)₂-(C₂-C₄-alkylen)-O-; -S(=O)₂-O-(C₂-C₄-alkylen)-O-; -NR⁵-(C₂-C₄-alkylen)-O-; -O-NR⁵-(C₂-C₄-alkylen)-O-; -NR⁵-C(=O)-(C₂-C₄-alkylen)-O-; -C(=O)-NR⁵-(C₂-C₄-alkylen)-O-; -NR⁵-C(=O)-NR⁶-(C₂-C₄-alkylen)-O-; -NR⁵-NR⁶-(C₂-C₄-alkylen)-O-; -NR⁵-NR⁶-C(=O)-(C₂-C₄-alkylen)-O-; -C(=O)-NR⁵-NR⁶-(C₂-C₄-alkylen)-O-;
in den vorstehend für A genannten Gruppen bedeutet
R⁵ und R⁶ unabhängig voneinander Wasserstoff, unverzweigtes oder verzweigtes C₁-C₄-Alkyl wie vorstehend im allgemeinen genannt, vorzugsweise Methyl, Ethyl und 1-Methylethyl, und C₁-C₄-Alkoxy wie vorstehend im allgemeinen genannt, vorzugsweise Methoxy, Ethoxy und 1-Methylethoxy;
und C₂-C₄-Alkylen steht für Ethylen, 1-Methylethylen, 2-Methylethylen, 1,2-Dimethylethylen, Propylen und Butylen;
der Rest A steht ferner für
C₁-C₆-Alkylen wie Methylen, Ethylen, 1-Methyl-methylen, Propylen, 1-Methyl-ethylen, 2-Methyl-ethylen, Butylen, 1-Methyl-propylen, 2-Methyl-propylen, 3-Methyl-propylen, 1-Ethyl-ethylen, 2-Ethyl-ethylen, 1,1-Dimethyl-ethylen, 1,2-Dimethyl-ethylen, 2,2-Dimethyl-ethylen, Pentylen, 1-Methyl-butylen, 2-Methyl-butylen, 3-Methyl-butylen, 4-Methyl-butylen, 1,1-Dimethyl-propylen, 1,2-Dimethyl-propylen, 1,3-Dimethyl-propylen, 2,2-Dimethyl-propylen, 2,3-Dimethyl-propylen, 1-Ethyl-propylen, 2-Ethyl-propylen, 3-Ethyl-propylen, 1-Ethyl-1-methyl-ethylen, 1-Ethyl-2-methyl-ethylen, 2-Ethyl-1-methyl-ethylen, 2-Ethyl-2-methyl-ethylen, 1,1,2-Trimethyl-ethylen, 1,2,2-Trimethyl-ethylen, Hexylen, 1-Methyl-pentylen, 2-Methyl-pentylen, 3-Methyl-pentylen, 4-Methyl-pentylen, 5-Methyl-pentylen, 1,1-Dimethyl-butylen, 1,2-Dimethyl-butylen, 1,3-Dimethyl-butylen, 1,4-Dimethyl-butylen, 2,2-Dimethyl-butylen, 2,3-Dimethyl-butylen,
2,4-Dimethyl-butylen, 3,3-Dimethyl-butylen, 3,4-Dimethyl-butylen, 1-Ethyl-butylen, 2-Ethyl-butylen, 3-Ethyl-butylen, 4-Ethyl-butylen, 1,1,2-Trimethyl-propylen, 1,1,3-Trimethyl-propylen, 1,2,2-Trimethyl-propylen, 1,2,3-Trimethyl-propylen, 2,2,3-Trimethyl-propylen, 2,3,3-Trimethyl-propylen, 1,3,3-Trimethyl-propylen, 1-Ethyl-1-methyl-propylen, 2-Ethyl-1-methyl-propylen, 3-Ethyl-1-methyl-propylen, 1-Ethyl-2-methyl-propylen, 2-Ethyl-2-methyl-propylen, 3-Ethyl-2-methyl-propylen, 1-Ethyl-3-methyl-propylen, 2-Ethyl-3-methyl-propylen, 3-Ethyl-3-methyl-propylen, 1,1-Diethyl-ethylen, 1,2-Diethyl-ethylen, 2,2-Diethyl-ethylen und 1,1,2,2-Tetramethyl-ethylen, vorzugsweise Methylen, Ethylen, Propylen, 1-Methylethylen, 2-Methylethylen und Butylen, insbesondere Methylen und Ethylen;
C₂-C₆-Alkenylen wie Ethenylen, 1-Propenylen, 2-Propenylen, 1-Methyl-ethenylen, 2-Methyl-ethenylen, 1-Butenylen, 2-Butenylen, 3-Butenylen, 1-Methyl-1-propenylen, 2-Methyl-1-propenylen, 3-Methyl-1-propenylen, 1-Methyl-2-propenylen, 2-Methyl-2-propenylen, 3-Methyl-2-propenylen, 1-Ethyl-ethenylen, 2-Ethyl-ethenylen, 1,2-Dimethyl-ethenylen, 1-Pentenylen, 2-Pentenylen, 3-Pentenylen, 4-Pentenylen, 1-Methyl-1-butenylen, 2-Methyl-1-butenylen, 3-Methyl-1-butenylen, 4-Methyl-1-butenylen, 1-Methyl-2-butenylen, 2-Methyl-2-butenylen, 3-Methyl-2-butenylen, 4-Methyl-2-butenylen, 1-Methyl-3-butenylen, 2-Methyl-3-butenylen, 3-Methyl-3-butenylen, 4-Methyl-3-butenylen, 1, 2-Dimethyl-1-propenylen, 1,3-Dimethyl-1-propenylen, 2,3-Dimethyl-1-propenylen, 3,3-Dimethyl-1-propenylen, 1,1-Dimethyl-2-propenylen, 1,2-Dimethyl-2-propenylen, 1,3-Dimethyl-2-propenylen, 2,3-Dimethyl-2-propenylen, 1-Ethyl-1-propenylen, 2-Ethyl-1-propenylen, 3-Ethyl-1-propenylen, 1-Ethyl-2-propenylen, 2-Ethyl-2-propenylen, 3-Ethyl-2-propenylen, 1-Ethyl-2-methyl-ethenylen, 2-Ethyl-1-methyl-ethenylen, 1-Hexenylen, 2-Hexenylen, 3-Hexenylen, 4-Hexenylen, 5-Hexenylen, 1-Methyl-1-pentenylen, 2-Methyl-1-pentenylen, 3-Methyl-1-pentenylen, 4-Methyl-1-pentenylen, 5-Methyl-1-pentenylen, 1-Methyl-2-pentenylen, 2-Methyl-2-pentenylen, 3-Methyl-2-pentenylen, 4-Methyl-2-pentenylen, 5-Methyl-2-pentenylen, 1-Methyl-3-pentenylen, 2-Methyl-3-pentenylen, 3-Methyl-3-pentenylen, 4-Methyl-3-pentenylen, 5-Methyl-3-pentenylen, 1-Methyl-4-pentenylen, 2-Methyl-4-pentenylen, 3-Methyl-4-pentenylen, 4-Methyl-4-pentenylen, 5-Methyl-4-pentenylen, 1-Ethyl-1-butenylen, 2-Ethyl-1-butenylen, 3-Ethyl-1-butenylen, 4-Ethyl-1-butenylen, 1-Ethyl-2-butenylen, 2-Ethyl-2-butenylen, 3-Ethyl-2-butenylen, 4-Ethyl-2-butenylen, 1-Ethyl-3-butenylen, 2-Ethyl-3-butenylen, 3-Ethyl-3-butenylen, 4-Ethyl-3-butenylen, 1,1-Dimethyl-2-butenylen, 1,1-Dimethyl-3-butenylen, 1,2-Dimethyl-1-butenylen, 1,2-Dimethyl-2-butenylen, 1,2-Dimethyl-3-butenylen, 1,3-Dimethyl-1-butenylen, 1,3-Dimethyl-2-butenylen, 1,3-Dimethyl-3-butenylen, 1,4-Dimethyl-1-butenylen, 1,4-Dimethyl-2-butenylen, 1,4-Dimethyl-3-butenylen, 2,2-Dimethyl-3-butenylen, 2,3-Dimethyl-1-butenylen, 2,3-Dimethyl-2-butenylen, 2,3-Dimethyl-3-butenylen, 2,4-Dimethyl-1-butenylen, 2,4-Dimethyl-2-butenylen, 2,4-Dimethyl-3-butenylen, 3,3-Dimethyl-1-butenylen, 3,4-Dimethyl-1-butenylen, 3,4-Dimethyl-2-butenylen, 3,4-Dimethyl-3-butenylen, 3,3-Dimethyl-1-butenylen, 4,4-Dimethyl-1-butenylen, 4,4-Dimethyl-2-butenylen, 1-Ethyl-1-butenylen, 1-Ethyl-2-butenylen, 1-Ethyl-3-butenylen, 2-Ethyl-1-butenylen, 2-Ethyl-2-butenylen, 2-Ethyl-3-butenylen, 3-Ethyl-1-butenylen, 3-Ethyl-2-butenylen, 3-Ethyl-3-butenylen, 4-Ethyl-1-butenylen, 4-Ethyl-2-butenylen, 4-Ethyl-3-butenylen, 1,1,2-Trimethyl-2-propenylen, 1,1,3-Trimethyl-2-propenylen, 1,2,3-Trimethyl-2-propenylen, 1,3,3-Trimethyl-2-propenylen, 1-Ethyl-1-methyl-2-propenylen, 1-Ethyl-2-methyl-1-propenylen, 1-Ethyl-2-methyl-2-propenylen, 1-Ethyl-3-methyl-1-propenylen, 1-Ethyl-3-methyl-2-propenylen, 2-Ethyl-1-methyl-2-propenylen, 2-Ethyl-3-methyl-1-propenylen, 2-Ethyl-3-methyl-2-propenylen, 3-Ethyl-1-methyl-1-propenylen, 3-Ethyl-1-methyl-2-propenylen, 3-Ethyl-2-methyl-1-propenylen, 3-Ethyl-2-methyl-2-propenylen, 3-Ethyl-3-methyl-1-propenylen und 1,2-Diethyl-ethenylen, vorzugsweise Ethenylen, 1-Propenylen, 2-Propenylen und 2-Butenylen, insbesondere Ethenylen;
oder C₂-C₆-Alkinylen wie Ethinylen, 1-Propinylen, 2-Propinylen, 1-Butinylen, 2-Butinylen, 3-Butinylen, 1-Methyl-2-propinylen, 3-Methyl-1-propinylen, 1-Pentinylen, 2-Pentinylen, 3-Pentinylen, 4-Pentinylen, 3-Methyl-1-butinylen, 4-Methyl-1-butinylen, 1-Methyl-2-butinylen, 4-Methyl-2-butinylen, 1-Methyl-3-butinylen, 2-Methyl-3-butinylen, 1,1-Dimethyl-2-propinylen, 3,3-Dimethyl-1-propinylen, 1-Ethyl-2-propinylen, 3-Ethyl-1-propinylen, 1-Hexinylen, 2-Hexinylen, 3-Hexinylen, 4-Hexinylen, 5-Hexinylen, 3-Methyl-1-pentinylen, 4-Methyl-1-pentinylen, 5-Methyl-1-pentinylen, 1-Methyl-2-pentinylen, 4-Methyl-2-pentinylen, 5-Methyl-2-pentinylen, 1-Methyl-3-pentinylen, 2-Methyl-3-pentinylen, 5-Methyl-3-pentinylen, 1-Methyl-4-pentinylen, 2-Methyl-4-pentinylen, 3-Methyl-4-pentinylen, 3,3-Dimethyl-1-butinylen, 3,4-Dimethyl-1-butinylen, 4,4-Dimethyl-1-butinylen, 1,1-Dimethyl-2-butinylen, 1,4-Dimethyl-2-butinylen, 4,4-Dimethyl-2-butinylen, 1,1-Dimethyl-3-butinylen, 1,2-Dimethyl-3-butinylen, 2,2-Dimethyl-3-butinylen, 3-Ethyl-1-butinylen, 4-Ethyl-1-butinylen, 1-Ethyl-2-butinylen, 4-Ethyl-2-butinylen, 1-Ethyl-3-butinylen, 2-Ethyl-3-butinylen, 3-Ethyl-3-methyl-1-propinylen und 1-Ethyl-1-methyl-2-propinylen, vorzugsweise Ethinylen und 2-Propinylen, insbesondere Ethinylen,
wobei diese Kohlenstoffketten in der Bedeutung A ein bis vier Halogenatome wie Fluor, Chlor, Brom und Jod, vorzugsweise Fluor und Chlor tragen können,
und wobei diese Kohlenstoffketten in der Bedeutung A durch eine der folgenden Gruppen unterbrochen sein können oder durch eine der folgenden Gruppen an R⁴ oder an den Phenylring gebunden sein können: -O-, -S-, -NR⁵-, -C(=O)-, -C(=O)-O-, -O-C(=O)-, -S(=O)-, -S(=O)₂-, OS(=O)₂-O-, -O-S(=O)₂-, -NR⁵-C(=O)-, -C(=O)-NR⁵-, -NR⁵-C(=O)-NR⁶-, -N=N-, -NR⁵-NR⁶-, -NR⁵-NR⁶-C(=O)- und -C(=O)-NR⁵-NR⁶-;
wobei n nicht 0 bedeutet oder R¹ nicht C₁-C₅-Alkyl bedeutet oder W nicht Sauerstoff bedeutet, wenn R⁴ für ggf. subst. Phenyl steht und die Gruppierung -A_{y}- eine der folgenden Ketten bezeichnet: -O-, -CH₂O-, -OCH₂-, -CH₂CH₂-, -CH=CH-, -C≡C-, -Oxy-C₂-C₁₂-alkylen-oxy-, -Thio-C₂-C₁₂-alkylen-oxy- oder -Methylenoxy-C₂-C₁₂-alkylen-oxy-,
oder wobei n nicht 0 bedeutet oder R¹ nicht C₁-C₅-Alkyl bedeutet oder W nicht Sauerstoff bedeutet, wenn die Gruppierung -A_{y}- eine der folgenden Ketten bezeichnet: -C(=O)-CH₂-, -O-C(=O)-CH₂-, -C₁-C₁₂-Alkylen-C(=O)-CH₂-, -Oxy-C₁-C₁₂-alkylen-C(=O)-CH₂-, -C₁-C₁₂-Alkenylen-C(=O)-CH₂- oder -Oxy-C₁-C₁₂-alkenylen-C(=O)-CH₂-, wobei die Alkylen- und die Alkenylengruppen Halogenatome oder Hydroxygruppen tragen können.

Im Hinblick auf ihre biologische Wirkung zur Bekämpfung von Schädlingen kommen Verbindungen I' in Betracht, in denen die Indices und die Substituenten die folgende Bedeutung haben:
n
   wie vorstehend im allgemeinen und im besonderen bei n angegeben;
c
   wie vorstehend im allgemeinen und im besonderen bei y angegeben;
R^{a}
   wie vorstehend im allgemeinen und im besonderen bei R¹ angegeben;
R^{b}
   wie vorstehend im allgemeinen und im besonderen bei R² angegeben;
R^{c}
   wie vorstehend im allgemeinen und im besonderen bei R³ angegeben;
R^{d}
   wie vorstehend im allgemeinen und im besonderen bei R⁴ angegeben;
W'
   wie vorstehend im allgemeinen und im besonderen bei W angegeben;
A'
   wie vorstehend im allgemeinen und im besonderen bei A angegeben.

Unter ggf. subst. Alkyl in der Bedeutung R⁴ bzw. R^{d} der allgemeinen Formeln I bzw. I' sind gradkettige oder verzweigte Alkylgruppen mit bis zu 12 Kohlenstoffatomen, besonders C₁-C₆-Alkylgruppen wie vorstehend genannt, und geradkettige C₇-C₁₂-Alkylgruppen insbesondere C₁-C₄-Alkyl, n-Decyl und n-Dodecyl zu verstehen, wobei diese Gruppen partiell oder vollständig halogeniert sein können.

Unter den partiell oder vollständig halogenierten Alkylgruppen sind diejenigen bevorzugt, die ein bis vier Halogenatome wie vorstehend genannt, vorzugsweise Fluor, Chlor und Brom, insbesondere Chlor und Brom, tragen.

Die genannten Alkylgruppen R⁴ bzw. R^{d} können des weiteren einen bis vier der folgenden Reste tragen:
Cyano; Cyanato; Thiocyanato; Nitro; Amino; Hydroxy; Carboxyl;
unverzweigtes oder verzweigtes C₁-C₆-Alkoxy wie Methoxy, Ethoxy, n-Propoxy, 1-Methylethoxy, n-Butoxy, 1-Methylpropoxy, 2-Methylpropoxy, 1,1-Dimethylethyloxy, n-Pentoxy, 1-Methylbutyloxy, 2-Methylbutyloxy, 3-Methylbutyloxy, 1,1-Dimethylpropoxy, 1,2-Dimethylpropoxy, 2,2-Dimethylpropoxy, 1-Ethylpropyloxy, n-Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise C₁-C₄-Alkoxy, insbesondere C₁-C₂-Alkoxy;
unverzweigtes oder verzweigtes C₁-C₆-Alkylthio wie Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 2,2-Di-methylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise C₁-C₂-Alkylthio, insbesondere Methylthio;
C₃-C₈-Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl, insbesondere Cyclopropyl;
unverzweigtes oder verzweigtes C₁-C₆-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino; vorzugsweise Methylamino und Ethylamino, insbesondere Methylamino;
unverzweigtes oder verzweigtes Di-(C₁-C₆)-alkylamino, besonders Di-(C₁-C₄)-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methyl-propyl)amino, N-Butyl-N-(2-methyl-propyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino, vorzugsweise Dimethylamino und Diethylamino, insbesondere Dimethylamino;
unverzweigtes oder verzweigtes C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl und Ethylcarbonyl, insbesondere Methylcarbonyl;
unverzweigtes oder verzweigtes C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl; vorzugsweise C₁-C₄-Alkoxycarbonyl, insbesondere Methoxycarbonyl und 1,1-Dimethylethoxycarbonyl; Phenyl, Phenoxy, Phenylthio und Phenylamino.

Die vorstehend genannten Phenylreste können ihrerseits ein bis drei Substituenten tragen, ausgewählt aus einer Gruppe bestehend aus:
Halogen wie vorstehend genannt, vorzugsweise Fluor, Chlor und Brom, insbesondere Fluor und Chlor;
Cyano, Nitro,
unverzweigtes oder verzweigtes C₁-C₄-Alkyl wie Methyl, Ethyl, Propyl, 1-Methylethyl, Butyl, 1-Methylpropyl, 2-Methylpropyl und 1, 1-Dimethylethyl, insbesondere Methyl und 1,1-Dimethylethyl;
partiell oder vollständig halogeniertes C₁-C₄-Alkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy;
und die Phenylreste können zusätzlich noch so viele Halogenatome wie vorstehend genannt, insbesondere Fluor und Chlor, tragen, daß die Gesamtzahl ihrer Substituenten 4 oder 5 beträgt.

Unter ggf. subst. Alkenyl in der Bedeutung R⁴ bzw. R^{d} der allgemeinen Formeln I bzw. I' sind gradkettige oder verzweigte Alkenylgruppen mit bis zu 12 Kohlenstoffatomen, vorzugsweise C₂-C₆-Alkenyl wie vorstehend genannt sowie n-Alkenylgruppen mit 7 bis 12 C-Atomen zu verstehen,
wobei diese Gruppen ein bis drei Halogenatome wie vorstehend genannt, vorzugsweise Fluor, Chlor und Brom, insbesondere Fluor und Chlor, und/oder ein oder zwei der folgenden Reste tragen können:
Cyano; Cyanato; Thiocyanato; Nitro; Carboxyl;
C₁-C₆-Alkyloxy wie vorstehend im allgemeinen und im besonderen genannt;
C₁-C₆-Alkylthio wie vorstehend im allgemeinen und im besonderen genannt;
C₃-C₈-Cycloalkyl wie vorstehend im allgemeinen und im besonderen genannt;
C₁-C₆-Alkylamino wie vorstehend im allgemeinen und im besonderen genannt;
Di-C₁-C₆-alkylamino wie vorstehend im allgemeinen und im besonderen genannt;
C₁-C₆-Alkylcarbonyl wie vorstehend im allgemeinen und im besonderen genannt;
C₁-C₆-Alkoxycarbonyl wie vorstehend im allgemeinen und im besonderen genannt;
Phenyl, Phenoxy, Phenylthio und Phenylamino.
Die vorstehend genannten Phenylreste können ihrerseits ein bis fünf Halogenatome wie vorstehend genannt, vorzugsweise Fluor, Chlor und Brom, insbesondere Fluor und Chlor, und oder ein bis drei der folgenden Gruppen tragen:
Cyano; Nitro;
unverzweigtes oder verzweigtes C₁-C₄-Alkyl wie vorstehend im allgemeinen und im besonderen genannt;
partiell oder vollständig halogeniertes C₁-C₄-Alkyl wie vorstehend im allgemeinen und im besonderen genannt;
oder C₁-C₄-Alkoxy wie vorstehend im allgemeinen und im besonderen genannt.

Unter ggf. subst. Alkinyl in der Bedeutung R⁴ bzw. R^{d} der allgemeinen Formeln I bzw. I' sind gradkettige oder verzweigte Alkinylgruppen mit bis zu 10 Kohlenstoffatomen, besonders C₂-C₆-Alkinylgruppen wie vorstehend genannt, und gradkettige C₇-C₁₀-Alkinylgruppen zu verstehen,
wobei diese Gruppen ein bis drei Halogenatome wie vorstehend genannt, vorzugsweise Fluor, Chlor und Brom, insbesondere Fluor und Chlor, und/oder ein bis drei der folgenden Reste tragen können:
Cyano; Cyanato; Thiocyanato; Nitro; Carboxyl;
C₁-C₆-Alkyloxy wie vorstehend im allgemeinen und im besonderen genannt;
C₁-C₆-Alkylthio wie vorstehend im allgemeinen und im besonderen genannt;
C₃-C₈-Cycloalkyl wie vorstehend im allgemeinen und im besonderen genannt;
C₁-C₆-Alkylamino wie vorstehend im allgemeinen und im besonderen genannt;
Di-C₁-C₆-alkylamino wie vorstehend im allgemeinen und im besonderen genannt;
C₁-C₆-Alkylcarbonyl wie vorstehend im allgemeinen und im besonderen genannt;
C₁-C₆-Alkoxycarbonyl wie vorstehend im allgemeinen und im besonderen genannt;
Phenyl, Phenoxy, Phenylthio und Phenylamino.

Die vorstehend genannten Phenylreste können ihrerseits ein bis drei Substituenten tragen, ausgewählt aus einer Gruppe bestehend aus Halogen wie vorstehend genannt, vorzugsweise Fluor, Chlor und Brom, insbesondere Fluor und Chlor;
Cyano; Nitro;
unverzweigtes oder verzweigtes C₁-C₄-Alkyl wie vorstehend im allgemeinen und im besonderen genannt;
partiell oder vollständig halogeniertes C₁-C₄-Alkyl wie vorstehend im allgemeinen und im besonderen genannt;
oder C₁-C₄-Alkoxy wie vorstehend im allgemeinen und im besonderen genannt, und die Phenylreste können zusätzlich noch so viele Halogenatome wie vorstehend genannt, vorzugsweise Fluor, Chlor und Brom, insbesondere Fluor und Chlor, tragen, daß die Zahl ihrer Substituenten 4 oder 5 beträgt.

In der Bedeutung R⁴ bzw. R^{d} der allgemeinen Formeln I bzw. I' sind unter ggf. subst. gesättigten oder ein- oder zweifach ungesättigten Cyclen, welche neben Kohlenstoffatomen ein bis drei Heteroatome als Ringglieder enthalten können, ausgewählt aus einer Gruppe bestehend aus zwei Stickstoffatomen und einem Sauerstoff- oder Schwefelatom gesättigte oder ein- oder zweifach ungesättigte, nicht aromatische Ringsysteme mit bis zu acht Ringgliedern zu verstehen, besonders
C₃-C₈-Cycloalkyl wie vorstehend im allgemeinen und im besonderen genannt; außerdem
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl; vorzugsweise Cyclopent-1-enyl, Cyclopent-3-enyl und Cyclohex-1-enyl, insbesondere Cyclopent-3-enyl;
C₅-C₈-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa-1,4-dien-3-yl, Cyclohepta-1,3-dien-1-yl, Cyclohepta-1,3-dien-2-yl, Cyclohepta-1,3-dien-5-yl, Cyclohepta-1,3-dien-6-yl, Cyclohepta-1,4-dien-1-yl, Cyclohepta-1,4-dien-2-yl, Cyclohepta-1,4-dien-3-yl, Cyclohepta-1,4-dien-6-yl, Cycloocta-1,3-dien-1-yl, Cycloocta-1,3-dien-2-yl, Cycloocta-1,3-dien-5-yl, Cycloocta-1,3-dien-6-yl, Cycloocta-1,4-dien-1-yl, Cycloocta-1,4-dien-2-yl, Cycloocta-1,4-dien-3-yl, Cycloocta-1,4-dien-6-yl, Cycloocta-1,4-dien-7-yl, Cycloocta-1,4-dien-1-yl und Cycloocta-1,4-dien-3-yl, vorzugsweise Cyclopenta-1,3-dien-5-yl;
3- bis 6-gliedrige, gesättigte oder partiell ungesättigte Heterocyclen, enthaltend ein bis drei Stickstoffatome oder ein bis drei Heteroatome, ausgewählt aus einer Gruppe bestehend aus zwei Stickstoffatomen, zwei Sauerstoff- und zwei Schwefelatomen oder aus einer Gruppe bestehend aus drei Sauerstoff- und Schwefelatomen wie Epoxidyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2,5-Dihydrothien-2-yl, 2,5-Dihydrothien-3-yl, 2,3-Pyrrolin-2-yl, 2,3-Pyrrolin-3-yl, 2,4-Pyrrolin-2-yl, 2,5-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, N-Morpholinyl und Dihydrochinazolinyl, insbesondere 2-Oxazolidinyl, 1,3-Dihydrooxazin-2-yl, Oxazol-2-in-2-yl, Oxiranyl, 1,3-Dithian-2-yl, 2-Tetrahydropyranyl und 1,3-Dioxolan-2-yl, zu verstehen,
wobei diese cyclischen Gruppen noch ein bis drei Reste tragen können, ausgewählt aus einer Gruppe bestehend aus Halogen wie vorstehend genannt, vorzugsweise Fluor, Chlor und Brom, insbesondere Fluor und Chlor, Cyano; Nitro;
unverzweigtes oder verzweigtes C₁-C₆-Alkyl wie vorstehend im allgemeinen und im besonderen genannt;
C₁-C₆-Alkyloxy wie vorstehend im allgemeinen und im besonderen genannt;
C₁-C₆-Alkylthio wie vorstehend im allgemeinen und im besonderen genannt;
C₃-C₈-Cycloalkyl wie vorstehend im allgemeinen und im besonderen genannt;
C₁-C₆-Alkylamino wie vorstehend im allgemeinen und im besonderen genannt;
Di-(C₁-C₆)-alkylamino wie vorstehend im allgemeinen und im besonderen genannt;
Phenyl, Phenoxy, Phenylthio und Phenylamino,
und wobei die Heterocyclen noch zusätzlich so viele Halogenatome wie vorstehend genannt, vorzugsweise Fluor, Chlor und Brom, insbesondere Fluor und Chlor tragen können, daß die Gesamtzahl der Reste 4 oder 5 beträgt.

Die vorstehend genannten Phenyl-, Phenoxy-, Phenylthio- und Phenylaminoreste können ihrerseits ein bis fünf Halogenatome wie vorstehend genannt, vorzugsweise Fluor, Chlor und Brom, insbesondere Fluor und Chlor, und oder ein bis drei der folgenden Gruppen tragen:
Cyano; Nitro;
C₁-C₄-Alkyl wie vorstehend im allgemeinen und im besonderen genannt;
partiell oder vollständig halogeniertes C₁-C₄-Alkyl wie vorstehend im allgemeinen und im besonderen genannt;
C₁-C₄-Alkoxy wie vorstehend im allgemeinen und im besonderen genannt;
oder C₁-C₄-Alkylthio wie vorstehend im allgemeinen und im besonderen genannt.

Unter ggf. subst. ein- bis dreikernigen aromatischen Systemen, welche neben Kohlenstoffatomen ein bis vier Stickstoffatome oder ein oder zwei Stickstoffatome und ein Sauerstoff- oder Schwefelatom enthalten können, in der Bedeutung R⁴ bzw. R^{d} der allgemeinen Formeln I bzw. I' sind aromatische und heteroaromatische Ringsysteme mit bis zu 14 Ringgliedern, besonders Phenyl, 1-Naphthyl, 2-Naphthyl, 1-Anthranyl, 2-Anthranyl, 9-Anthranyl, 9-Fluorenyl und 2-Fluorenyl, insbesondere Phenyl, fünfring Heteroaromaten enthaltend ein bis drei Heteroatome, ausgewählt aus einer Gruppe bestehend aus drei Stickstoffatomen und einem Sauerstoff- oder Schwefelatom, wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3yl, 1,2,4-Thiadiazol-5-yl, 1,2,4-Triazol-3-yl, 1,3,4-Triazol-2-yl, 1,3,4-Oxadiazol-2-yl, 1,3,4-Thiadiazol-2-yl, 1,3,4-Triazol-2-yl, 1,2,3-Oxadiazol-4-yl, 1,2,3-Oxadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Pyrrolyl, 3-Isoxazolyl, 5-Isoxazolyl, 4-Oxazolyl, 4-Thiazolyl, 4-Pyrazolyl, 5-Pyrazolyl, 1,3,4-Oxadiazol-2-yl und 1,3,4-Thiadiazol-2-yl,
sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 2-Pyrimidinyl, 4-Pyrimidinyl und 1,3,5-Triazin-2-yl,
außerdem andere heteroaromatische Systeme wie 1-Indolyl, 2-Indolyl, 3-Indolyl, 1-Indazolyl, 1H-Indazol-3-yl, 1-Isochinolinyl, 3-Isochinolinyl, 4-Isochinolinyl, 2-Chinoxalinyl, 2-Benzothienyl, 3-Benzothienyl, 1-Benzotriazolyl, 1,2-Benzisoxazol-3-yl, 1,2-Benzisothiazol-3-yl, Benzpyrazol-3-yl, Isoindol-2-yl, Thiazolopyrimidin-2-yl, Benzimidazol-2-yl, Benzoxazol-2-yl, Imidazopyrimidin-2-yl, Oxazolopyrimidin-2-yl, Benzofuran-2-yl, Benzofuran-3-yl, Chinolin-2-yl, Chinolin-3-yl, Chinolin-4-yl, Chinazolin-2-yl, Chinazolin-4-yl, Benzothiazol-2-yl, Cinnolin-3-yl, Cinnolin-4-yl, 1,5-Naphthylidin-2-yl, 1,8-Naphthylidin-2-yl, Phthalazin-1-yl, Pteridin-2-yl, Pteridin-4-yl, Isobenzofuran-1-yl und 7-Purinyl, insbesondere 2-Benzimidazolyl, 2-Benzoxazolyl und 2-Benzothiazolyl zu verstehen,

wobei diese Gruppen ein bis fünf Halogenatome wie vorstehend genannt, vorzugsweise Fluor, Chlor und Brom, insbesondere Fluor und Chlor, und/oder ein bis vier der folgenden Reste tragen können:
Cyano; Cyanato; Thiocyanato; Nitro; Amino; Hydroxy; Carboxyl;
C₁-C₆-Alkyl wie vorstehend im allgemeinen und im besonderen genannt;
partiell oder vollständig halogeniertes C₁-C₄-Alkyl wie vorstehend im allgemeinen und im besonderen genannt;
C₁-C₆-Alkyloxy wie vorstehend im allgemeinen und im besonderen genannt;
partiell oder vollständig halogeniertes C₁-C₄-Alkoxy wie vorstehend im allgemeinen und im besonderen genannt;
C₁-C₆-Alkylthio wie vorstehend im allgemeinen und im besonderen genannt;
C₅-C₁₅-Alkenyloxy wie 3-Methylbut-2-enyl-1-oxy, 3,7-Dimethylocta-2,6-dienyl-1-oxy und 3,7,11-Trimethyldodeca-2,6,10-trienyl-1-oxy;
C₃-C₈-Cycloalkyl wie vorstehend im allgemeinen und im besonderen genannt; C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
C₃-C₈-Cycloalkoxy wie Cyclopropyloxy, Cyclobutyloxy, Cyclopentyloxy, Cyclohexyloxy, Cycloheptyloxy und Cyclooctyloxy;
C₁-C₆-Alkylamino wie vorstehend im allgemeinen und im besonderen genannt;
Di-C₁-C₆-alkylamino wie vorstehend im allgemeinen und im besonderen genannt;
C₁-C₆-Alkylcarbonyl wie vorstehend im allgemeinen genannt, vorzugsweise C₁-C₄-Alkylcarbonyl;
C₁-C₆-Alkoxycarbonyl wie vorstehend im allgemeinen genannt, vorzugsweise C₁-C₄-Alkoxycarbonyl;
C₁-C₆-Alkylaminocarbonyl wie vorstehend im allgemeinen und im besonderen genannt;
Di-(C₁-C₆)-alkylaminocarbonyl, besonders Di-(C₁-C₄)-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propyl-aminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise Di-(C₁-C₄)-alkylaminocarbonyl, insbesondere Di-(C₁-C₂)-alkylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, vorzugsweise C₁-C₄-Alkylcarboxyl, insbesondere C₁-C₂-Alkylcarboxyl;
durch C₁-C₄-Alkoxy wie vorstehend genannt oder durch C₁-C₄-Alkylthio wie vorstehend genannt in 1- oder 2-Position substituiertes Methyl oder Ethyl; Phenyl, Phenoxy, Phenylthio, Phenylamino,
durch Phenyl, Phenoxy, Phenylthio und Phenylamino substituiertes C₁-C₄-Alkyl wie vorstehend genannt, vorzugsweise Methyl oder Ethyl;
durch Phenyl, Phenoxy, Phenylthio und Phenylamino substituiertes C₁-C₄-Alkoxy wie vorstehend genannt, vorzugsweise Methoxy oder Ethoxy.

Die vorstehend genannten Phenylreste können ihrerseits ein bis drei Substituenten tragen, ausgewählt aus einer Gruppe bestehend aus Halogen wie vorstehend im allgemeinen und im besonderen genannt,
Cyano; Nitro;
C₁-C₄-Alkyl wie vorstehend im allgemeinen und im besonderen genannt; partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt; partiell oder vollständig halogeniertem C₁-C₄-Alkoxy wie vorstehend im allgemeinen und im besonderen genannt;
oder C₁-C₄-Alkylthio wie vorstehend im allgemeinen und im besonderen genannt,
und die vorstehend genannten Phenylreste können zusätzlich noch so viele Halogenatome wie vorstehend im allgemeinen und im besonderen genannt, tragen, daß die Gesamtzahl ihrer Reste 4 oder 5 beträgt.

Die vorstehend genannten aromatischen oder heteroaromatischen Systeme in der Bedeutung R⁴ bzw. R^{d} der allgemeinen Formeln I bzw. I' können neben den vorstehend genannten Resten auch einen Rest der Struktur -C(R⁷)=NO-R⁸ tragen, worin die Substituenten die folgende Bedeutung haben:
R7
   Wasserstoff;
unverzweigtes oder verzweigtes C₁-C₆-Alkyl wie vorstehend im allgemeinen und im besonderen genannt;
oder Aryl wie Phenyl, 1-Naphthyl, 2-Naphthyl, 1-Anthranyl und 2-Anthranyl, vorzugsweise Phenyl,
wobei die aromatischen Reste noch einen bis drei Substituenten tragen können, ausgewählt aus einer Gruppe bestehend aus Halogen wie vorstehend im allgemeinen und im besonderen genannt,
Cyano; Cyanato; Thiocyanato; Nitro; Amino; Hydroxy; Carboxyl;
unverzweigtem oder verzweigtem C₁-C₄-Alkyl wie vorstehend im allgemeinen und im besonderen genannt;
partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt;
C₁-C₄-Alkylthio wie vorstehend im allgemeinen und im besonderen genannt;
und partiell oder vollständig halogeniertem C₁-C₄-Alkylthio wie vorstehend im allgemeinen und im besonderen genannt,
und wobei die aromatischen Reste zusätzlich noch so viele Halogenatome wie vorstehend im allgemeinen und im besonderen genannt, tragen können, daß die Gesamtzahl ihrer Substituenten 4 oder 5 beträgt.
R⁸
   unverzweigtes oder verzweigtes C₁-C₆-Alkyl wie vorstehend im allgemeinen und im besonderen genannt, C₂-C₆-Alkenyl und C₂-C₆-Alkinyl wie vorstehend im allgemeinen genannt,
   wobei diese Gruppen noch einen bis drei der Substituenten tragen können, ausgewählt aus einer Gruppe bestehend aus Halogen wie im allgemeinen und im besonderen vorstehend genannt,
   Cyano; Nitro;
   unverzweigtem oder verzweigtem C₁-C₆-Alkoxy wie vorstehend im allgemeinen und im besonderen genannt;
   partiell oder vollständig halogeniertem C₁-C₆-Alkoxy wie vorstehend im allgemeinen und im besonderen genannt;
   C₁-C₆-Alkylthio wie vorstehend im allgemeinen und im besonderen genannt;
   partiell oder vollständig halogeniertem C₁-C₆-Alkylthio wie vorstehend im allgemeinen und im besonderen genannt;
   C₃-C₆-Cycloalkyl wie vorstehend im allgemeinen und im besonderen genannt;
R⁸ bedeutet außerdem 5- oder 6-gliedrige aromatische oder heteroaromatische Systeme wie vorstehend genannt, vorzugsweise Phenyl, wobei die cyclischen Reste ihrerseits ein bis fünf Halogenatome wie vorstehend im allgemeinen und im besonderen genannt, und/oder ein bis drei der folgenden Gruppen tragen können: Cyano; Nitro; unverzweigtes oder verzweigtes C₁-C₄-Alkyl wie vorstehend im allgemeinen und im besonderen genannt; partiell oder vollständig halogeniertes C₁-C₄-Alkyl wie vorstehend im allgemeinen und im besonderen genannt; C₁-C₄-Alkoxy wie vorstehend im allgemeinen und im besonderen genannt; partiell oder vollständig halogeniertes C₁-C₄-Alkoxy wie vorstehend im allgemeinen und im besonderen genannt; oder C₁-C₄-Alkylthio wie vorstehend im allgemeinen und im besonderen genannt.

Im Hinblick auf ihre biologische Wirksamkeit gegen Schädlinge sind besonders solche Verbindungen I' bevorzugt, in denen die Substruktur -A'_{c}- für eine der folgenden Gruppen steht:

| | | | | | |
|---|---|---|---|---|---|
| A∗.1 | -O- | A∗.2 | -S- | A∗.3 | -NH- |
| A∗.4 | -CH₂O- | A∗.5 | -CH₂S- | A∗.6 | -CH₂NH- |
| A∗.7 | -OCH₂- | A∗.8 | -SCH₂- | A∗.9 | -NHCH₂- |
| A∗.10 | -CH₂CH₂- | A∗.11 | -CH=CH- | A∗.12 | -C≡C- |
| A∗.13 | -C(=O)OCH₂- | A∗.14 | -OCH₂CH₂O- | A∗.15 | -NHCH₂CH₂O |
| A∗.16 | -C(=O)NHCH₂CH₂O- | A∗.17 | -O-CH=CH- | A∗.18 | -S-CH=CH- |
| A∗.19 | -O-CH₂CH=CH- | A∗.20 | -S-CH₂CH=CH- | A∗.21 | -C(=O)NH-. |

Im Hinblick auf ihre biologische Wirksamkeit gegen Schädlinge sind auperdem solche Verbindungen I' bevorzugt, in denen der Index a den Wert 0 oder 1 hat.

Im Hinblick auf ihre biologische Wirksamkeit gegen Schädlinge sind auperdem solche Verbindungen I' bevorzugt, in denen die Substruktur R^{a}-w'-für eine der folgenden Gruppen steht:

| | | | | | |
|---|---|---|---|---|---|
| B∗. 1 | OCH₃ | B∗. 2 | OCH₂CH₃ | B∗. 3 | NHCH₃ |
| B∗. 4 | NHCH₂CH₃ | B∗. 5 | CH₃ | B∗. 6 | CH₂CH₃ |

Im Hinblick auf ihre biologische Wirksamkeit gegen Schädlinge sind auperdem solche Verbindungen I' bevorzugt, in denen R^{b} für eine der folgenden Gruppen steht:

| | | | | | |
|---|---|---|---|---|---|
| C∗. 1 | CH₃ | C∗. 2 | CH₂CH₃ | C∗. 3 | (CH₂)₂CH₃ |
| C∗. 4 | CH₂Cl | C∗. 5 | CH₂Br | C∗. 6 | CH₂CN |
| C∗. 7 | CH(CH₃)₂ | C∗. 8 | CH₂OCH₃ | C∗. 9 | CH₂SCH₃ |
| C∗.10 | Cyclopropyl | C∗.11 | CN | C∗.12 | CH=CH₂ |
| C∗.13 | CF₃ | C∗.14 | C≡CH | | |

Im Hinblick auf ihre biologische Wirksamkeit gegen Schädlinge sind auperdem solche Verbindungen I' bevorzugt, in denen R^{c} für eine der folgenden Gruppen steht:

Fluor, Chlor, Methyl, 1,1-Dimethylethyl, Methoxy, Nitro und Trifluormethyl.

Im Hinblick auf die biologische Wirkung gegen Schädlinge sind solche Verbindungen I' bevorzugt, in denen die Substruktur R^{a}-W'- für eine der Gruppen B∗.1, B∗.3 oder B∗.5 und R^{b} für eine der Gruppen C∗.1, C∗.2, C∗.4, C∗.8, C∗.9, C∗.10 oder C∗.ll steht.

Daneben werden Verbindungen der Formel I' bevorzugt, in denen die Substruktur R^{a}-W'- für eine der Gruppen B∗.1 oder B∗.3, R^{b} für eine der Gruppen C∗.1, C∗.2, C∗.4, C∗.8, C∗.10 oder C∗.11 und A'c für eine der Gruppen A∗.7, A∗.8, A∗.11, A∗.13 oder A∗.14 steht.

Im Hinblick auf ihre biologische Wirksamkeit gegen Schädlinge sind auperdem solche Verbindungen I' bevorzugt, in denen Rd für eine der vorstehend definierten, ggf. substituerten ein- bis dreikernigen Aromaten oder Heteroaromaten steht.

Im Hinblick auf ihre biologische Wirksamkeit gegen Schadpilze sind besonders solche Verbindungen I bevorzugt, in denen die Substruktur -Ay-für eine der folgenden Gruppen steht:

| | | | | | |
|---|---|---|---|---|---|
| A∗.1 | -O- | A∗.2 | -S- | A∗.3 | -NH- |
| A∗.4 | -CH₂O- | A∗.5 | -CH₂S- | A∗.6 | -CH₂NH- |
| A∗.7 | -OCH₂- | A∗.8 | -SCH₂- | A∗.9 | -NHCH₂- |
| A∗.10 | -CH₂CH₂- | A∗.11 | -CH=CH- | A∗.12 | -C≡C- |
| A∗.17 | -O-CH=CH- | A∗.18 | -S-CH=CH- | A∗.19 | -OCH₂CH=CH- |
| A∗.20 | -S-CH₂CH=CH- | A∗.21 | -CH₂CH=CH- | | |
| A∗.22 | -CH=CH-O- | A∗.23 | -CH=CH-S- | | |

Im Hinblick auf ihre biologische Wirksamkeit gegen Schadpilze sind außerdem solche Verbindungen I bevorzugt, in denen der Index n den Wert 0 oder 1 hat.

Im Hinblick auf ihre biologische Wirksamkeit gegen Schadpilze sind außerdem solche Verbindungen I bevorzugt, in denen die Substruktur R¹-W- für eine der folgenden Gruppen steht:

| | | | | | |
|---|---|---|---|---|---|
| B∗. 1 | OCH₃ | B∗. 2 | OCH₂CH₃ | B∗. 3 | NHCH₃ |
| B∗. 4 | NHCH₂CH₃ | B∗. 5 | CH₃ | B∗. 6 | CH₂CH₃ |
| B∗. 7 | CH(CH₃)₂ | | | | |

Im Hinblick auf ihre biologische Wirksamkeit gegen Schadpilze sind außerdem solche Verbindungen I bevorzugt, in denen R² für eine der folgenden Gruppen steht:

| | | | | | |
|---|---|---|---|---|---|
| C∗. 1 | CH₃ | C∗. 2 | CH₂CH₃ | C∗. 3 | (CH₂)₂CH₃ |
| C∗. 4 | CH₂Cl | C∗. 5 | CH₂Br | C∗. 6 | CH₂CN |
| C∗. 7 | CH(CH₃)₂ | C∗. 8 | CH₂OCH₃ | C∗. 9 | CH₂SCH₃ |
| C∗.13 | CF₃ | | | | |

Im Hinblick auf ihre biologische Wirksamkeit gegen Schadpilze sind außerdem solche Verbindungen I bevorzugt, in denen R³ für eine der folgenden Gruppen steht:

Wasserstoff, Fluor, Chlor, Brom, Methyl, 1-Methylethyl, 1,1-Dimethylethyl, Cyano, Nitro, Methoxy und Trifluormethyl.

Im Hinblick auf ihre biologische Wirksamkeit gegen Schadpilze sind außerdem solche Verbindungen I bevorzugt, in denen R⁴ für einen ggf. subst. gesättigten oder ein- oder zweifach ungesättigten Cyclus, welcher neben Kohlenstoffatomen ein bis drei Heteroatome aus der Gruppe Sauerstoff, Schwefel und Stickstoff als Ringglied enthalten kann wie vorstehend im allgemeinen und im besonderen ausgeführt, oder für ein ggf. subst. ein- oder zweikerniges aromatisches System, welches neben Kohlenstoffatomen ein bis vier Stickstoffatome oder ein oder zwei Stickstoffatome und/oder ein Sauerstoff- oder Schwefelatom enthalten kann steht.

Hierbei sind insbesondere auch solche Verbindungen I bevorzugt, in denen die aromatischen bzw. heteroaromatischen Reste in der Bedeutung R⁴ mindestens einen Rest der Substruktur -C(R⁷)=NO-R⁸ wie vorstehend im allgemeinen und im besonderen ausgeführt, tragen.

Im Hinblick auf die fungizide Wirkung sind Verbindungen der allgemeinen Formel Ia bevorzugt,

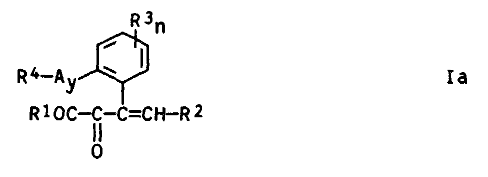

in der der Index n 0 oder 1 bedeutet und
-Ay- für
eine der Gruppen A∗.1, A∗.2, A∗.7, A∗.8, A∗.10 oder A∗.11 steht.

Im Hinblick auf die fungizide Wirkung sind außerdem Verbindungen der allgemeinen Formel Ib bevorzugt,

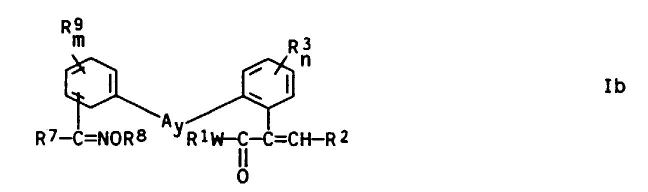

in der die Indices und die Substituenten die folgende Bedeutung haben:
n
   0 oder 1,
m
   0, 1 oder 2, wobei die Reste R⁹ verschieden sein können, wenn m den Wert 2 hat;
-A_{y}-
   eine der Gruppen A∗.1, A∗.2, A∗.7 oder A∗.8.
R⁷
   Wasserstoff;
C₁-C₆-Alkyl wie vorstehend im allgemeinen und im besonderen genannt;
oder Aryl wie Phenyl, 1-Naphthyl, 2-Naphthyl, 1-Anthranyl und 2-Anthranyl, vorzugsweise Phenyl,
wobei die aromatischen Reste noch einen bis drei Substituenten tragen können, ausgewählt aus einer Gruppe bestehend aus Halogen wie vorstehend im allgemeinen und im besonderen genannt;
Cyano; Cyanato; Thiocyanato; Nitro; Amino; Hydroxy; Carboxyl;
unverzweigtem oder verzweigtem C₁-C₄-Alkyl wie vorstehend im allgemeinen und im besonderen genannt;
partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt;
C₁-C₄-Alkylthio wie vorstehend im allgemeinen und im besonderen genannt;
oder partiell oder vollständig halogeniertem C₁-C₄-Alkylthio wie vorstehend im allgemeinen und im besonderen genannt,
und wobei die aromatischen Reste zusätzlich noch so viele Halogenatome wie vorstehend im allgemeinen und im besonderen genannt, tragen können, daß die Gesamtzahl ihrer Substituenten 4 oder 5 beträgt.
R⁸
   C₁-C₆-Alkyl,
C₂-C₆-Alkyl und C₂-C₆-Alkinyl wie vorstehend im allgemeinen und im besonderen genannt,
wobei diese Gruppen ein bis fünf Halogenatome wie im allgemeinen und im besonderen vorstehend genannt, und/oder einen bis drei der folgenden Reste tragen können:
Cyano; Nitro;
C₁-C₆-Alkoxy wie vorstehend im allgemeinen und im besonderen genannt;
partiell oder vollständig halogeniertes C₁-C₆-Alkoxy wie vorstehend im allgemeinen und im besonderen genannt;
C₁-C₆-Alkylthio wie vorstehend im allgemeinen und im besonderen genannt;
partiell oder vollständig halogeniertes C₁-C₆-Alkylthio wie vorstehend im allgemeinen und im besonderen genannt;
C₃-C₆-Cycloalkyl wie vorstehend im allgemeinen und im besonderen genannt;
R⁸ bedeutet außerdem 5- oder 6-gliedriges aromatisches oder heteroaromatisches System wie vorstehend genannt, vorzugsweise Phenyl,
wobei die cyclischen Reste ihrerseits einen bis drei Substituenten tragen können, ausgewählt aus einer Gruppe bestehend aus Halogen wie vorstehend im allgemeinen und im besonderen genannt; Cyano; Nitro; unverzweigtem oder verzweigtem C₁-C₄-Alkyl wie vorstehend im allgemeinen und im besonderen genannt; partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt; oder C₁-C₄-Alkylthio wie vorstehend im allgemeinen und im besonderen genannt, und wobei die cyclischen Reste ihrerseits zusätzlich noch so viele Halogenatome wie vorstehend im allgemeinen und im besonderen genannt, tragen können, daß die Gesamtzahl ihrer Substituenten 4 oder 5 beträgt.
R⁹
   Cyano, Halogen, C₁-C₄-Alkyl, C₁-C₄-Halogenalkyl und C₁-C₄-Alkoxy.

Im Hinblick auf die fungizide Wirkung sind auch Verbindungen der allgemeinen Formel Ic bevorzugt, 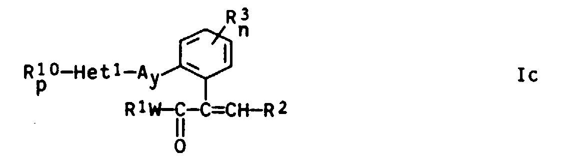 in der die Indices und die Substituenten die folgende Bedeutung haben:
n
   0 oder 1,
p
   0, 1, 2 oder 3, wobei die Reste R¹⁰ verschieden sein können, wenn der Wert von p gleich oder größer als 2 ist;
und -A_{y}-
   eine der Gruppen A∗.1, A∗.2, A∗.7, A∗.8, A∗.10 oder A∗.11.
Het¹
   5- bis 6-gliedrige, gesättigte oder partiell ungesättigte Heterocyclen, welche ein bis drei Stickstoffatome oder ein oder zwei Stickstoffatome und ein oder zwei Sauerstoff- und/oder Schwefelatom oder ein bis zwei Sauerstoff- und/oder Schwefelatom enthalten;
   ein- oder zweikernige heteroaromatische Systeme, welche neben Kohlenstoffatomen ein bis vier Stickstoffatome oder ein oder zwei Stickstoffatome und ein Sauerstoff- oder Schwefelatom enthalten;
R¹⁰
   Cyano, Nitro, Amino, Hydroxy, Carboxyl, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio, partiell oder vollständig halogeniertes C₁-C₄-Alkylthio, C₃-C₆-Cycloalkyl oder ein direkt oder über eine Sauerstoff- oder eine Schwefelbrücke an den Heterocyclus Het¹ gebundenes einkerniges aromatisches oder heteroaromatisches Ringsystem, welches neben Kohlenstoffringgliedern ein oder zwei Stickstoffatome oder ein oder zwei Stickstoffatome und ein Sauerstoff- oder Schwefelatom als Ringglied enthalten kann, und wobei dieses Ringsystem noch einen bis drei Reste tragen kann, ausgewählt aus einer Gruppe bestehend aus Halogen, Cyano, Nitro, C₁-C₄-Alkyl, partiell oder vollständig halogeniertem C₁-C₄-Alkyl, C₁-C₄-Alkoxy und C₁-C₄-Alkylthio, und wobei das Ringsystem zusätzlich noch so viele Halogenatome tragen kann, daß die Gesamtzahl der Reste 4 beträgt.

Im Hinblick auf die fungizide Wirkung sind ebenfalls Verbindungen der allgemeinen Formel Id bevorzugt, 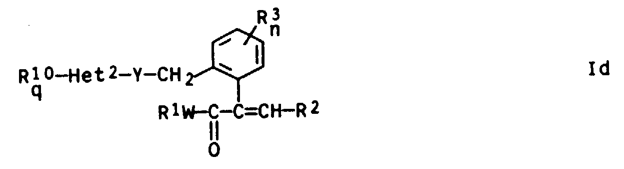 in der die Indices und die Substituenten die folgende Bedeutung haben:
n
   0 oder 1,
q
   0, 1 oder 2, wobei die Reste R¹⁰ verschieden sein können, wenn q den Wert 2 hat;
Y
   Sauerstoff oder Schwefel;
Het²
   6-gliedrige heteroaromatische Systeme, welche neben Kohlenstoffringgliedern ein bis drei Stickstoffatome als Ringglieder enthalten.

Im Hinblick auf die fungizide Wirkung sind außerdem Verbindungen der allgemeinen Formel Id bevorzugt, in denen R¹⁰ für eine der folgenden Gruppen steht:
C₁-C₆-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio, C₃-C₈-Cycloalkyl oder durch C₁-C₄-Alkoxy oder C₁-C₄-Alkylthio substituiertes Methyl oder Ethyl.

Im Hinblick auf die fungizide Wirkung sind außerdem Verbindungen der allgemeinen Formel Ie bevorzugt, 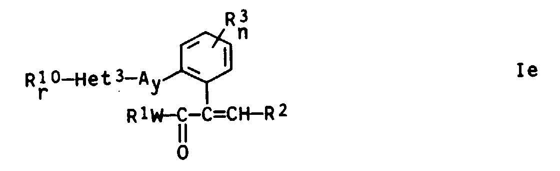 in der die Indices und die Substituenten die folgende Bedeutung haben:
n
   0 oder 1,
r
   0, 1 oder 2, wobei die Reste R¹⁰ verschieden sein können, wenn r den Wert 2 hat;
-A_{y}-
   eine der Gruppen A∗.1, A∗.2, A∗.7, A∗.8, A∗.10 oder A∗.11.
Het3
   5-gliedrige heteroaromatische Systeme, welche neben Kohlenstoffringgliedern ein bis drei Stickstoffatome oder ein oder zwei Stickstoffatome und ein Sauerstoff- oder ein Schwefelatom als Ringglieder enthalten.

Im Hinblick auf die fungizide Wirkung sind außerdem auch Verbindungen der allgemeinen Formel If bevorzugt, 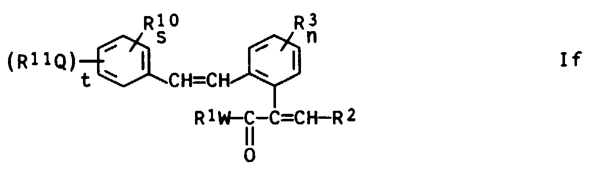 in der die Indices und die Substituenten die folgende Bedeutung haben:
n
   0 oder 1,
s
   0, 1 oder 2, wobei die Reste R¹⁰ verschieden sein können, wenn s den Wert 2 hat;
t
   0, 1 oder 2, wobei die Reste R¹¹Q verschieden sein können, wenn t den Wert 2 hat;
Q
   eine direkte Bindung, Sauerstoff, Schwefel oder eine -NH-Gruppe;
R¹¹
   ein- oder zweikernige heteroaromatische Systeme, welche neben Kohlenstoffatomen ein bis drei Stickstoffatome oder ein oder zwei Stickstoffatome und ein Sauerstoff- oder Schwefelatom enthalten, wobei die (hetero)-aromatischen Systeme einen bis drei Reste tragen können, ausgewählt aus einer Gruppe bestehend aus Halogen, Cyano, Nitro, Amino, Hydroxy, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio und partiell oder vollständig halogeniertem C₁-C₄-Alkylthio,
   und wobei die (hetero)aromatischen Systeme zusätzlich noch so viele Halogenatome tragen können, daß die Gesamtzahl der Reste 4 beträgt.

Im Hinblick auf die fungizide Wirkung sind daneben auch Verbindungen der allgemeinen Formel Ig bevorzugt, 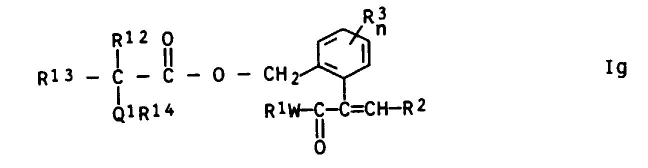 in der die Indices und die Substituenten die folgende Bedeutung haben:
n
   0 oder 1,
Q¹
   eine direkte Bindung, C₁-C₆-Alkylen, C₁-C₆-Alkylenoxy, C₁-C₆-Alkylenthio, Oxy-C₁-C₆-alkylen, Thio-C₁-C₆-alkylen, C₂-C₆-Alkenylen, Sauerstoff oder Schwefel;
R¹² und R¹³
   unabhängig voneinander Wasserstoff, C₁-C₄-Alkyl, welches ein bis drei Halogenatome tragen kann;
   oder gemeinsam mit dem C-Atom an das sie gebunden sind C₃-C₈-Cycloalkyl, welches ein bis drei der folgenden Reste tragen kann: Halogen, C₁-C₄-Alkyl, partiell oder vollständig halogeniertes C₁-C₄-Alkyl und
      C₁-C₄-Alkoxy;
R¹⁴
   ein- oder zweikernige aromatische oder heteroaromatische Systeme, welche neben Kohlenstoffatomen ein bis vier Stickstoffatome oder ein oder zwei Stickstoffatome und ein Sauerstoff- oder Schwefelatom enthalten können, wobei die (hetero)aromatischen Systeme noch einen bis drei der Reste tragen können, ausgewählt aus einer Gruppe, bestehend aus Halogen, Cyano, Nitro, Amino, Hydroxy, Carboxyl, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio,
   und wobei die (hetero)aromatischen System noch so viele Halogenatome tragen können, daß die Gesamtzahl der Reste 4 oder 5 beträgt.

Im Hinblick auf die Wirkung gegen Schädlinge sind Verbindungen der allgemeinen Formel I'a bevorzugt, 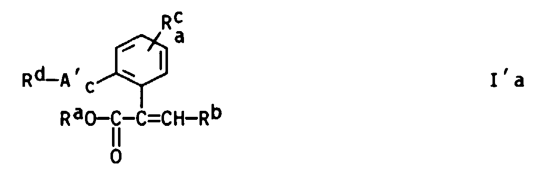 in der die Indices und die Substituenten die folgende Bedeutung haben:
a
   0 oder 1,
und -A'_{c}-
   eine der Gruppen A∗.1, A∗.2, A∗.7, A∗.8, A∗.10, A∗.11, A∗.12, A∗.13 und A∗.14.

Im Hinblick auf die Wirkung gegen Schädlinge sind außerdem Verbindungen der allgemeinen Formel I'b bevorzugt, 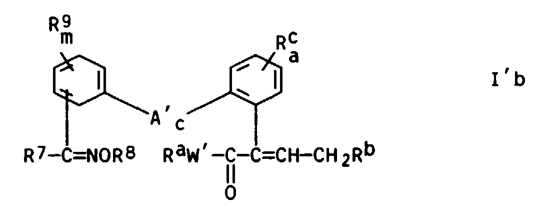 in der die Indices und die Substituenten die folgende Bedeutung haben:
a
   0 oder 1,
m
   0, 1 oder 2, wobei die Reste R⁹ verschieden sein können, wenn m den Wert 2 hat;
-A'_{c}-
   eine der Gruppen A∗.1, A∗.7, A∗.8, A∗.10, A∗.11 und A∗.14.
R⁷
   Wasserstoff;
   C₁-C₆-Alkyl wie vorstehend im allgemeinen und im besonderen genannt;
   Aryl wie Phenyl, 1-Naphthyl, 2-Naphthyl, 1-Anthranyl und 2-Anthranyl, vorzugsweise Phenyl,
   wobei die aromatischen Reste noch einen bis drei Substituenten tragen können, ausgewählt aus einer Gruppe bestehend aus
   Halogen wie vorstehend im allgemeinen und im besonderen genannt;
   Cyano; Cyanato; Thiocyanato; Nitro; Amino; Hydroxy; Carboxyl;
   unverzweigtem oder verzweigtem C₁-C₄-Alkyl wie vorstehend im allgemeinen und im besonderen genannt;
   partiell oder vollständig halogeniertem C₁-C₄-Alkyl wie vorstehend im allgemeinen und im besonderen genannt;
   C₁-C₄-Alkoxy wie vorstehend im allgemeinen und im besonderen genannt;
   partiell oder vollständig halogeniertem C₁-C₄-Alkoxy wie vorstehend im allgemeinen und im besonderen genannt;
   C₁-C₄-Alkylthio wie vorstehend im allgemeinen und im besonderen genannt;
   oder partiell oder vollständig halogeniertem C₁-C₄-Alkylthio wie vorstehend im allgemeinen und im besonderen genannt,
   und wobei die aromatischen Reste noch so viele Halogenatome wie vorstehend im allgemeinen und im besonderen genannt, tragen können, daß die Gesamtzahl ihrer Substituenten 4 oder 5 beträgt.
R⁸
   unverzweigtes oder verzweigtes C₁-C₆-Alkyl,
   C₂-C₆-Alkenyl und C₂-C₆-Alkinyl wie vorstehend im allgemeinen und im besonderen genannt,
   wobei diese Gruppen noch einen bis drei Substituenten tragen können, ausgewählt aus einer Gruppe bestehend aus
   Halogen wie vorstehend im allgemeinen und im besonderen genannt;
   Cyano; Nitro;
   C₁-C₆-Alkoxy wie vorstehend im allgemeinen und im besonderen genannt;
   partiell oder vollständig halogeniertem C₁-C₆-Alkoxy wie vorstehend im allgemeinen und im besonderen genannt;
   C₁-C₆-Alkylthio wie vorstehend im allgemeinen und im besonderen genannt;
   partiell oder vollständig halogeniertem C₁-C₆-Alkylthio wie vorstehend im allgemeinen und im besonderen genannt;
   C₃-C₆-Cycloalkyl wie vorstehend im allgemeinen und im besonderen genannt, tragen können,
   und wobei die Alkyl-, Alkenyl- und Alkinylgruppen noch so viele Halogenatome wie vorstehend im allgemeinen und im besonderen genannt, tragen können, daß die Gesamtzahl ihrer Substituenten 4 oder 5 beträgt.
R⁸ bedeutet außerdem 5- oder 6-gliedriges aromatisches oder heteroaromatisches System wie vorstehend genannt, vorzugsweise Phenyl, wobei die cyclischen Reste ihrerseits noch einen bis drei Substituenten tragen können, ausgewählt aus einer Gruppe bestehend aus
   Halogen wie vorstehend im allgemeinen und im besonderen genannt;
   Cyano; Nitro;
   unverzweigtem oder verzweigtem C₁-C₄-Alkyl wie vorstehend im allgemeinen und im besonderen genannt;
   partiell oder vollständig halogeniertem C₁-C₄-Alkyl wie vorstehend im allgemeinen und im besonderen genannt;
   C₁-C₄-Alkoxy wie vorstehend im allgemeinen und im besonderen genannt;
   partiell oder vollständig halogeniertem C₁-C₄-Alkoxy wie vorstehend im allgemeinen und im besonderen genannt;
   oder C₁-C₄-Alkylthio wie vorstehend im allgemeinen und im besonderen genannt,
   und wobei die cyclischen Reste zusätzlich noch so viele Halogenatome wie vorstehend im allgemeinen und im besonderen genannt, tragen können, daß die Gesamtzahl ihrer Substituenten 4 oder 5 beträgt.
R⁹
   Cyano, Halogen, C₁-C₄-Alkyl, partiell oder vollständig halogeniertes C₁-C₄-Alkyl und C₁-C₄-Alkoxy.

Insbesondere sind solche Verbindungen I'b bevorzugt, in denen die Substruktur R^{a}-W'- für eine der Gruppen B∗.1 oder B∗.3 steht.

Im Hinblick auf die Wirkung gegen Schädlinge sind auch Verbindungen der allgemeinen Formel I'c bevorzugt, 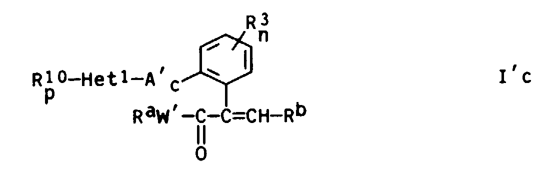 in der die Indices und die Substituenten die folgende Bedeutung haben:
a
   0 oder 1,
p
   0, 1, 2 oder 3, wobei die Reste R¹⁰ verschieden sein können, wenn der Wert von p gleich oder größer als 2 ist;
und -A'c-
   eine der Gruppen A∗.1, A∗.2, A∗.4, A∗.7, A∗.8, A∗.10, A∗.11, A∗.12, A∗.13. und A∗.14.
Het¹
   5- bis 6-gliedrige, gesättigte oder partiell ungesättigte Heterocyclen, welche ein bis drei Stickstoffatome oder ein oder zwei Stickstoffatome und ein oder zwei Sauerstoff- und/oder Schwefelatome oder ein bis zwei Sauerstoff- und/oder Schwefelatome enthalten, wobei jeweils zwei Sauerstoff-und/oder Schwefelatome nicht benachbart sein dürfen;
ein- oder zweikernige heteroaromatische Systeme, welche neben Kohlenstoffatomen ein bis vier Stickstoffatome oder ein oder zwei Stickstoffatome und ein Sauerstoff- oder Schwefelatom enthalten;
R¹⁰
Cyano, Cyanato, Thiocyanato, Nitro, Amino, Hydroxy, Carboxyl, Halogen, C₁-C₆-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio, partiell oder vollständig halogeniertes C₁-C₄-Alkylthio, C₁-C₄-Alkoxycarbonyl, C₁-C₄-Alkylamino, Di-(C₁-C₄)-alkylamino, durch C₁-C₄-Alkoxy oder C₁-C₄-Alkylthio substituiertes Methyl oder Ethyl,
ein direkt oder über eine Methylenbrücke gebundenes einkerniges aromatisches oder heteroaromatisches Ringsystem, welches neben Kohlenstoffringgliedern ein bis drei Stickstoffatome oder ein Sauerstoff- oder ein Schwefelatom oder ein oder zwei Stickstoffatome und ein Sauerstoff- oder ein Schwefelatom enthalten kann, und welches noch einen bis drei Substituenten tragen kann, ausgewählt aus einer Gruppe bestehend aus Halogen, Cyano, Nitro, C₁-C₄-Alkyl, partiell oder vollständig halogeniertem C₁-C₄-Alkyl, C₁-C₄-Alkoxy und C₁-C₄-Alkylthio, Di-(C₁-C₄)-alkylamino, und wobei das (hetero)aromatische Ringsystem noch zusätzlich so viele Halogenatome tragen kann, daß die Gesamtzahl seiner Substituenten 4 beträgt.

Im Hinblick auf die Wirkung gegen Schädlinge sind ebenfalls Verbindungen der allgemeinen Formel I'd bevorzugt, 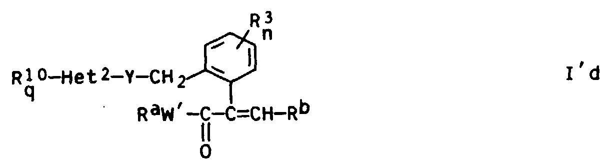 in der die Indices und die Substituenten die folgende Bedeutung haben:
a
   0 oder 1,
q
   0, 1, 2 oder 3, wobei die Reste R¹⁰ verschieden sein können, wenn q den Wert 2 oder 3 hat;
Y
   Sauerstoff oder Schwefel;
Het2
   6-gliedrige heteroaromatische Systeme, welche neben Kohlenstoffringgliedern ein bis drei Stickstoffatome als Ringglieder enthalten.

Im Hinblick auf ihre Wirksamkeit gegen Schädlinge sind insbesondere solche Verbindungen der Formel I'd bevorzugt, in denen R¹⁰ für eine der folgenden Gruppen steht:
C₁-C₆-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio, C₃-C₈-Cycloalkyl oder durch C₁-C₄-Alkoxy oder C₁-C₄-Alkylthio substituiertes Methyl oder Ethyl.

Im Hinblick auf die Wirkung gegen Schädlinge sind außerdem Verbindungen der allgemeinen Formel I'e bevorzugt, 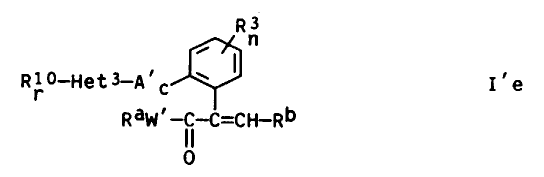 in der die Indices und die Substituenten die folgende Bedeutung haben:
a
   0 oder 1,
r
   0, 1 oder 2, wobei die Reste R¹⁰ verschieden sein können, wenn r den Wert 2 hat;
-A'_{c}-
   eine der Gruppen A∗.1, A∗.7, A∗.8, A∗.10, A∗.11 oder A∗.12;
Het3
   5-gliedrige heteroaromatische Systeme, welche neben Kohlenstoffringgliedern ein bis drei Stickstoffatome oder ein oder zwei Stickstoffatome und ein Sauerstoff- oder ein Schwefelatom oder ein O- oder S-Atom als Ringglieder enthalten.

Im Hinblick auf ihre Wirksamkeit gegen Schädlinge sind insbesondere solche Verbindungen der Formel I'e bevorzugt, in denen R¹⁰ die Bedeutung eines Phenylrestes hat, der mit ein bis drei der folgenden Gruppen substituiert sein kann:
Halogen, Cyano, Nitro, Dimethylamino, C₁-C₄-Alkyl, partiell oder vollständig halogeniertes C₁-C₄-Alkyl und C₁-C₄-Alkoxy.

Im Hinblick auf die Wirkung gegen Schädlinge sind auperdem auch Verbindungen der allgemeinen Formel I'f bevorzugt, 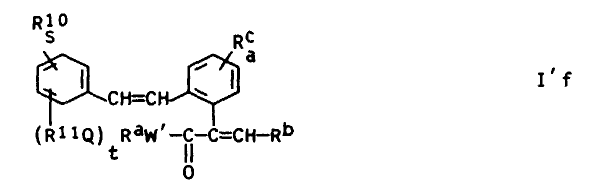 in der die Indices und die Substituenten die folgende Bedeutung haben:
a
   0 oder 1,
s
   0, 1 oder 2, wobei die Reste R¹⁰ verschieden sein können, wenn s den Wert 2 hat;
t
   0, 1 oder 2, wobei die Reste R¹¹Q verschieden sein können, wenn t den Wert 2 hat;
Q
   eine direkte Bindung, Sauerstoff, Schwefel oder eine -NH-Gruppe;
R¹¹
   einkernige aromatische Systeme, welche neben Kohlenstoffatomen ein bis drei Stickstoffatome oder ein oder zwei Stickstoffatome und ein Sauerstoff- oder Schwefelatom enthalten, wobei diese aromatischen Systeme noch einen bis drei Reste tragen können, ausgewählt aus einer Gruppe bestehend aus Halogen, Cyano, Nitro, Amino, Hydroxy, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio und partiell oder vollständig halogeniertem C₁-C₄-Alkylthio, und wobei die aromatischen Systeme zusätzlich noch so viele Halogenatome tragen können, daß die Gesamtzahl ihrer Reste 4 beträgt.

Im Hinblick auf die Wirkung gegen Schädlinge sind insbesondere solche Verbindungen der Formel I'f bevorzugt, in denen R¹⁰ für eine der folgenden Gruppen steht: Wasserstoff, Halogen, Cyano, Nitro, C₁-C₄-Alkyl, partiell oder vollständig halogeniertes C₁-C₄-Alkyl und C₁-C₄-Alkoxy.

Im Hinblick auf die Wirkung gegen Schädlinge sind daneben auch Verbindungen der allgemeinen Formel I'g bevorzugt, 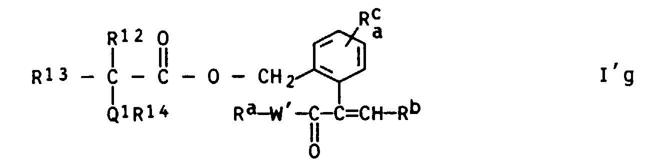 in der die Indices und die Substituenten die folgende Bedeutung haben:
a
   0 oder 1,
Q¹
   eine direkte Bindung, C₁-C₆-Alkylen, C₁-C₆-Alkylenoxy, C₁-C₆-Alkylenthio, Oxy-C₁-C₆-alkylen, Thio-C₁-C₆-alkylen, C₂-C₆-Alkenylen, Sauerstoff oder Schwefel;
R¹² und R¹³
   unabhängig voneinander Wasserstoff, C₁-C₄-Alkyl, welches ein bis drei der folgenden Reste tragen kann: Halogen und C₁-C₄-Alkoxy;
   oder gemeinsam mit dem C-Atom an das sie gebunden sind C₃-C₈-Cycloalkyl, welches ein bis drei der folgenden Reste tragen kann: Halogen, C₁-C₄-Alkyl und partiell oder vollständig halogeniertes C₁-C₄-Alkyl;
R¹⁴
   ein- oder zweikernige aromatische oder heteroaromatische Systeme, welche neben Kohlenstoffatomen ein bis vier Stickstoffatome oder ein oder zwei Stickstoffatome und ein Sauerstoff- oder Schwefelatom enthalten können, wobei die (hetero)aromatischen Systeme noch einen bis drei Reste tragen können, ausgewählt aus einer Gruppe bestehend aus Halogen, Cyano, Nitro, Amino, Hydroxy, Carboxyl, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio, und wobei die (hetero)aromatischen Systeme zusätzlich noch so viele Halogenatome tragen können, daß die Gesamtzahl der Reste 4 oder 5 beträgt.
   Die Indices m (in den Formeln Ib und I'b), q (in den Formeln Id und I'd), r (in den Formeln Ie und I'e) sowie s und t (in den Formeln If und I'f), bedeuten unabhängig voneinander 0, 1 oder 2, vorzugsweise 1 oder 2.
   Der Index p (in den Formeln Ic und I'c) bedeutet 0, 1, 2 oder 3, vorzugsweise 1 oder 2.
   Der Rest R⁹ in den Formeln Ib und I'b steht für die folgenden Gruppen:
   Cyano, Nitro,
   Halogen wie vorstehend im allgemeinen und im besonderen genannt;
   unverzweigtes oder verzweigtes C₁-C₄-Alkyl wie vorstehend im allgemeinen und im besonderen genannt;
   partiell oder vollständig halogeniertes C₁-C₄-Alkyl wie vorstehend im allgemeinen und im besonderen genannt;
   und C₁-C₄-Alkoxy wie vorstehend im allgemeinen und im besonderen genannt.

Der Rest R¹⁰ in den Formeln Ic, Id, Ie, If, I'c, I'd, I'e und I'f steht für die folgenden Gruppen:
Cyano, Cyanato, Thiocyanato, Nitro, Amino, Hydroxy,
Halogen wie vorstehend genannt, vorzugsweise Fluor, Chlor und Brom, insbesondere Fluor und Brom;
unverzweigtes oder verzweigtes C₁-C₆-Alkyl wie vorstehend genannt, vorzugsweise C₁-C₄-Alkyl;
partiell oder vollständig halogeniertes C₁-C₄-Alkyl wie vorstehend genannt, vorzugsweise Chlormethyl und Trifluormethyl, insbesondere Trifluormethyl;
C₁-C₄-Alkoxy wie vorstehend genannt, insbesondere Methoxy und Ethoxy;
partiell oder vollständig halogeniertes C₁-C₄-Alkoxy wie vorstehend genannt, insbesondere Difluormethoxy und 1,1,2,2-Tetrafluorethoxy;
C₁-C₄-Alkylthio wie vorstehend genannt, insbesondere Methylthio;
und partiell oder vollständig halogeniertes C₁-C₄-Alkylthio wie vorstehend im allgemeinen und im besonderen genannt.

R¹⁰ bedeutet außerdem C₃-C₆-Cycloalkyl wie vorstehend im allgemeinen und im besonderen genannt;
oder durch C₁-C₄-Alkoxy oder C₁-C₄-Alkylthio substituiertes Methyl oder Ethyl wie vorstehend im allgemeinen und im besonderen genannt.

Der Rest R¹¹ in den Formeln If und I'f steht für die folgenden Gruppen:
einkernige aromatische Systeme, welche neben Kohlenstoffatomen ein bis drei Stickstoffatome oder ein oder zwei Stickstoffatome und ein Sauerstoff- oder Schwefelatom enthalten, wie vorstehend genannt, vorzugsweise Phenyl, 2-Pyridyl, 3-Pyridyl, 4-Pyridyl, 2-Pyrimidyl, 2-Thienyl, 1-Pyrrolyl, 1-Pyrazolyl und 3-Isoxazolyl;
wobei diese aromatischen Systeme noch einen bis drei Reste tragen können, ausgewählt aus einer Gruppe bestehend aus
Halogen wie vorstehend im allgemeinen und im besonderen genannt;
Cyano, Nitro, Amino, Hydroxy,
unverzweigtem oder verzweigtem C₁-C₄-Alkyl wie vorstehend im allgemeinen und im besonderen genannt;
partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt;
C₁-C₄-Alkylthio wie vorstehend im allgemeinen und im besonderen genannt;
und partiell oder vollständig halogeniertem C₁-C₄-Alkylthio wie vorstehend im allgemeinen und im besonderen genannt,
und wobei die aromatischen Systeme zusätzlich noch so viele Halogenatome wie vorstehend im allgemeinen und im besonderen genannt, tragen können, daß die Gesamtzahl der Reste 4 beträgt.

Die Reste R¹² und R¹³ in den Formeln If, Ig, I'f und I'g stehen unabhängig voneinander für Wasserstoff oder für eine unverzweigte oder verzweigte C₁-C₄-Alkylgruppe wie vorstehend im allgemeinen und im besonderen genannt, die noch einen bis drei der folgenden Reste tragen kann:
Halogen wie vorstehend im allgemeinen und im besonderen genannt;
C₁-C₄-Alkoxy wie vorstehend im allgemeinen und im besonderen genannt;
oder R¹² und R¹³ bedeuten gemeinsam mit dem C-Atom, an das sie gebunden sind, eine C₃-C₈-Cycloalkylgruppe wie vorstehend im allgemeinen und im besonderen genannt, die noch einen bis drei der folgenden Reste tragen kann:
Halogen wie vorstehend im allgemeinen und im besonderen genannt;
unverzweigtes oder verzweigtes C₁-C₄-Alkyl wie vorstehend im allgemeinen und im besonderen genannt;
und partiell oder vollständig halogeniertes C₁-C₄-Alkyl wie vorstehend im allgemeinen und im besonderen genannt.

Der Rest R¹⁴ in den Formeln Ig und I'g steht für die folgenden Gruppen: ein- oder zweikernige aromatische oder heteroaromatische Systeme, welche neben Kohlenstoffatomen ein bis vier Stickstoffatome oder ein oder zwei Stickstoffatome und ein Sauerstoff- oder Schwefelatom enthalten können, wie vorstehend genannt, vorzugsweise Phenyl, 1-Naphtyl, 2-Naphtyl und 3-Pyridinyl, insbesondere Phenyl,
wobei diese (hetero)aromatischen Systeme einen bis drei Reste tragen können, ausgewählt aus einer Gruppe bestehend aus
Halogen wie vorstehend im allgemeinen und im besonderen genannt;
Cyano, Cyanato, Thiocyanato, Nitro, Amino, Hydroxy, Carboxyl,
unverzweigtem oder verzweigtem C₁-C₄-Alkyl wie vorstehend im allgemeinen und im besonderen genannt;
partiell oder vollständig halogeniertem C₁-C₄-Alkyl wie vorstehend im allgemeinen und im besonderen genannt;
C₁-C₄-Alkoxy wie vorstehend im allgemeinen und im besonderen genannt;
partiell oder vollständig halogeniertem C₁-C₄-Alkoxy wie vorstehend im allgemeinen und im besonderen genannt;
und C₁-C₄-Alkylthio wie vorstehend im allgemeinen und im besonderen genannt,
und wobei die (hetero)aromatischen Systeme R14 zusätzlich noch so viele Halogenatome wie vorstehend im allgemeinen und im besonderen genannt,
tragen können, daß die Gesamtzahl der Reste 4 oder 5 beträgt.

Der Rest Het¹ in den Formeln Ic und I'c steht für die folgenden Gruppen:
5- bis 6-gliedrige, gesättigte oder partiell ungesättigte Heterocyclen, welche ein bis drei Stickstoffatome oder ein oder zwei Stickstoffatome und ein oder zwei Sauerstoff- und/oder Schwefelatome oder ein bis drei Sauerstoff- und/oder Schwefelatom enthalten, wie vorstehend im allgemeinen und im besonderen genannt;
ein- oder zweikernige heteroaromatische Systeme, welche neben Kohlenstoffatomen ein bis vier Stickstoffatome oder ein oder zwei Stickstoffatome und/oder ein Sauerstoff- oder Schwefelatom enthalten, wie vorstehend im allgemeinen und im besonderen genannt.

Der Rest Het² in den Formeln Id und I'd steht für die folgenden Gruppen:
6-gliedrige heteroaromatische Systeme, welche neben Kohlenstoffringgliedern ein bis drei Stickstoffatome als Ringglied enthalten wie vorstehend genannt, vorzugsweise 2-Pyridyl, 3-Pyridyl, 2-Pyrimidyl und 4-Pyrimidyl, insbesondere 2-Pyridyl und 4-Pyrimidyl.

Der Rest Het³ in den Formeln Ie und I'e steht für die folgenden Gruppen: 5-gliedrige heteroaromatische Systeme, welche neben Kohlenstoffringgliedern ein bis drei Stickstoffatome oder ein oder zwei Stickstoffatome und ein Sauerstoff- oder ein Schwefelatom als Ringglied enthalten wie vorstehend im allgemeinen und im besonderen genannt.

Der Rest Q in den Formeln If und I'f steht für die folgenden Gruppen:
eine direkte Bindung, Sauerstoff, Schwefel oder eine -NH-Gruppe, vorzugsweise eine direkte Bindung oder Sauerstoff.

Der Rest Q¹ in den Formeln Ig und I'g steht für die folgenden Gruppen:
eine direkte Bindung;
C₁-C₆-Alkylen wie vorstehend genannt, vorzugsweise Methylen, Ethylen und Propylen, insbesondere Methylen;
C₁-C₆-Alkylenoxy wie Methylenoxy, Ethylenoxy, 1-Methyl-methylenoxy, Propylenoxy, 1-Methyl-ethylenoxy, 2-Methyl-ethylenoxy, Butylenoxy, 1-Methyl-propylenoxy, 2-Methyl-propylenoxy, 3-Methyl-propylenoxy, 1-Ethyl-ethylenoxy, 2-Ethyl-ethylenoxy, 1,1-Dimethyl-ethylenoxy, 1,2-Dimethyl-ethylenoxy, 2,2-Dimethyl-ethylenoxy, Pentylenoxy, 1-Methyl-butylenoxy, 2-Methyl-butylenoxy, 3-Methyl-butylenoxy, 4-Methyl-butylenoxy, 1,1-Dimethyl-propylenoxy, 1,2-Dimethyl-propylenoxy, 1,3-Dimethyl-propylenoxy, 2,2-Dimethyl-propylenoxy, 2,3-Dimethyl-propylenoxy, 1-Ethyl-propylenoxy, 2-Ethyl-propylenoxy, 3-Ethyl-propylenoxy, 1-Ethyl-1-methyl-ethylenoxy, 1-Ethyl-2-methyl-ethylenoxy, 2-Ethyl-1-methyl-ethylenoxy, 2-Ethyl-2-methyl-ethylenoxy, 1,1,2-Trimethyl-ethylenoxy, 1,2,2-Trimethyl-ethylenoxy, Hexylenoxy, 1-Methyl-pentylenoxy, 2-Methyl-pentylenoxy, 3-Methyl-pentylenoxy, 4-Methyl-pentylenoxy, 5-Methyl-pentylenoxy, 1,1-Dimethyl-butylenoxy, 1,2-Dimethyl-butylenoxy, 1,3-Dimethyl-butylenoxy, 1,4-Dimethyl-butylenoxy, 2,2-Dimethyl-butylenoxy, 2,3-Dimethyl-butylenoxy, 2,4-Dimethyl-butylenoxy, 3,3-Dimethyl-butylenoxy, 3,4-Dimethyl-butylenoxy, 1-Ethyl-butylenoxy, 2-Ethyl-butylenoxy, 3-Ethyl-butylenoxy, 4-Ethyl-butylenoxy, 1,1,2-Trimethyl-propylenoxy, 1,1,3-Trimethyl-propylenoxy, 1,2,2-Trimethyl-propylenoxy, 1,2,3-Trimethyl-propylenoxy, 2,2,3-Trimethyl-propylenoxy, 2,3,3-Trimethyl-propylenoxy, 1,3,3-Trimethyl-propylenoxy, 1-Ethyl-1-methyl-propylenoxy, 2-Ethyl-1-methyl-propylenoxy, 3-Ethyl-1-methyl-propylenoxy, 1-Ethyl-2-methyl-propylenoxy, 2-Ethyl-2-methyl-propylenoxy, 3-Ethyl-2-methyl-propylenoxy, 1-Ethyl-3-methyl-propylenoxy, 2-Ethyl-3-methyl-propylenoxy, 3-Ethyl-3-methyl-propylenoxy, 1,1-Diethyl-ethylenoxy, 1,2-Diethyl-ethylenoxy, 2,2-Diethyl-ethylenoxy und 1,1,2,2-Tetramethyl-ethylenoxy, vorzugsweise Methylenoxy;
C₁-C₆-Alkylenthio wie Methylenthio, Ethylenthio, 1-Methyl-methylenthio, Propylenthio, 1-Methyl-ethylenthio, 2-Methyl-ethylenthio, Butylenthio, 1-Methyl-propylenthio, 2-Methyl-propylenthio, 3-Methyl-propylenthio, 1-Ethyl-ethylenthio, 2-Ethyl-ethylenthio, 1,1-Dimethyl-ethylenthio, 1,2-Dimethyl-ethylenthio, 2,2-Dimethyl-ethylenthio, Pentylenthio, 1-Methyl-butylenthio, 2-Methyl-butylenthio, 3-Methyl-butylenthio, 4-Methyl-butylenthio, 1,1-Dimethyl-propylenthio, 1,2-Dimethyl-propylenthio, 1,3-Dimethyl-propylenthio, 2,2-Dimethyl-propylenthio, 2,3-Dimethyl-propylenthio, 1-Ethyl-propylenthio, 2-Ethyl-propylenthio, 3-Ethyl-propylenthio, 1-Ethyl-1-methyl-ethylenthio, 1-Ethyl-2-methyl-ethylenthio, 2-Ethyl-1-methyl-ethylenthio, 2-Ethyl-2-methyl-ethylenthio, 1,1,2-Trimethyl-ethylenthio, 1,2,2-Trimethyl-ethylenthio, Hexylenthio, 1-Methyl-pentylenthio, 2-Methyl-pentylenthio, 3-Methyl-pentylenthio, 4-Methyl-pentylenthio, 5-Methyl-pentylenthio, 1,1-Dimethyl-butylenthio, 1,2-Dimethyl-butylenthio, 1,3-Dimethyl-butylenthio, 1,4-Dimethyl-butylenthio, 2,2-Dimethyl-butylenthio, 2,3-Dimethyl-butylenthio, 2,4-Dimethyl-butylenthio, 3,3-Dimethyl-butylenthio, 3,4-Dimethyl-butylenthio, 1-Ethyl-butylenthio, 2-Ethyl-butylenthio, 3-Ethyl-butylenthio, 4-Ethyl-butylenthio, 1,1,2-Trimethyl-propylenthio, 1,1,3-Trimethyl-propylenthio, 1,2,2-Trimethyl-propylenthio, 1,2,3-Trimethyl-propylenthio, 2,2,3-Trimethyl-propylenthio, 2,3,3-Trimethyl-propylenthio, 1,3,3-Trimethyl-propylenthio, 1-Ethyl-1-methyl-propylenthio, 2-Ethyl-1-methyl-propylenthio, 3-Ethyl-1-methyl-propylenthio, 1-Ethyl-2-methyl-propylenthio, 2-Ethyl-2-methyl-propylenthio, 3-Ethyl-2-methyl-propylenthio, 1-Ethyl-3-methyl-propylenthio, 2-Ethyl-3-methyl-propylenthio, 3-Ethyl-3-methyl-propylenthio, 1,1-diethyl-ethylenthio, 1,2-diethyl-ethylenthio, 2,2-diethyl-ethylenthio und 1,1,2,2-Tetramethyl-ethylenthio, vorzugsweise Methylenthio;
Oxy-C₁-C₆-alkylen wie Oxy-methylen, Oxy-ethylen, Oxy-1-methyl-methylen, Oxy-propylen, Oxy-1-methyl-ethylen, Oxy-2-methyl-ethylen, Oxy-butylen, Oxy-1-methyl-propylen, Oxy-2-methyl-propylen, Oxy-3-methyl-propylen, Oxy-1-ethyl-ethylen, Oxy-2-ethyl-ethylen, Oxy-1,1-dimethyl-ethylen, Oxy-1,2-dimethyl-ethylen, Oxy-2,2-dimethyl-ethylen, Oxy-pentylen, Oxy-1-methyl-butylen, Oxy-2-methyl-butylen, Oxy-3-methyl-butylen, Oxy-4-methyl-butylen, Oxy-1,1-dimethyl-propylen, Oxy-1,2-dimethyl-propylen, Oxy-1,3-dimethyl-propylen, Oxy-2,2-dimethyl-propylen, Oxy-2,3-dimethyl-propylen, Oxy-1-ethyl-propylen, Oxy-2-ethyl-propylen, Oxy-3-ethyl-propylen, Oxy-1-ethyl-1-methyl-ethylen, Oxy-1-ethyl-2-methyl-ethylen, Oxy-2-ethyl-1-methyl-ethylen, Oxy-2-ethyl-2-methyl-ethylen, Oxy-1,1,2-trimethyl-ethylen, Oxy-1,2,2-trimethyl-ethylen, Oxy-hexylen, Oxy-1-methyl-pentylen, Oxy-2-methyl-pentylen, Oxy-3-methyl-pentylen, Oxy-4-methyl-pentylen, Oxy-5-methyl-pentylen, Oxy-1,1-dimethyl-butylen, Oxy-1,2-dimethyl-butylen, Oxy-1,3-dimethyl-butylen, Oxy-1,4-dimethyl-butylen, Oxy-2,2-dimethyl-butylen, Oxy-2,3-dimethyl-butylen, Oxy-2,4-dimethyl-butylen, Oxy-3,3-dimethyl-butylen, Oxy-3,4-dimethyl-butylen, Oxy-1-ethyl-butylen, Oxy-2-ethyl-butylen, Oxy-3-ethyl-butylen, Oxy-4-ethyl-butylen, Oxy-1,1,2-trimethyl-propylen, Oxy-1,1,3-trimethyl-propylen, Oxy-1,2,2-trimethyl-propylen, Oxy-1,2,3-trimethyl-propylen, Oxy-2,2,3-trimethyl-propylen, Oxy-2,3,3-trimethyl-propylen, Oxy-1,3,3-trimethyl-propylen, Oxy-1-ethyl-1-methyl-propylen, Oxy-2-ethyl-1-methyl-propylen, Oxy-3-ethyl-1-methyl-propylen, Oxy-1-ethyl-2-methyl-propylen, Oxy-2-ethyl-2-methyl-propylen, Oxy-3-ethyl-2-methyl-propylen, Oxy-1-ethyl-3-methyl-propylen, Oxy-2-ethyl-3-methyl-propylen, Oxy-3-ethyl-3-methyl-propylen, Oxy-1,1-diethyl-ethylen, Oxy-1,2-diethyl-ethylen, Oxy-2,2-diethyl-ethylen und Oxy-1,1,2,2-tetramethyl-ethylen, vorzugsweise Oxy-methylen;
Thio-C₁-C₆-Alkylen wie Thio-methylen, Thio-ethylen, Thio-1-methyl-methylen, Thio-propylen, Thio-1-methyl-ethylen, Thio-2-methyl-ethylen, Thio-butylen, Thio-1-methyl-propylen, Thio-2-methyl-propylen, Thio-3-methyl-propylen, Thio-1-ethyl-ethylen, Thio-2-ethyl-ethylen, Thio-1,1-dimethyl-ethylen, Thio-1,2-dimethyl-ethylen, Thio-2,2-dimethyl-ethylen, Thio-pentylen, Thio-1-methyl-butylen, Thio-2-methyl-butylen, Thio-3-methyl-butylen, Thio-4-methyl-butylen, Thio-1,1-dimethyl-propylen, Thio-1,2-dimethyl-propylen, Thio-1,3-dimethyl-propylen, Thio-2,2-dimethyl-propylen, Thio-2,3-dimethyl-propylen, Thio-1-ethyl-propylen, Thio-2-ethyl-propylen, Thio-3-ethyl-propylen, Thio-1-ethyl-1-methyl-ethylen, Thio-1-ethyl-2-methyl-ethylen, Thio-2-ethyl-1-methyl-ethylen, Thio-2-ethyl-2-methyl-ethylen, Thio-1,1,2-trimethyl-ethylen, Thio-1,2,2-trimethyl-ethylen, Thio-hexylen, Thio-1-methyl-pentylen, Thio-2-methyl-pentylen, Thio-3-methyl-pentylen, Thio-4-methyl-pentylen, Thio-5-methyl-pentylen, Thio-1,1-dimethyl-butylen, Thio-1,2-dimethyl-butylen, Thio-1,3-dimethyl-butylen, Thio-1,4-dimethyl-butylen, Thio-2,2-dimethyl-butylen, Thio-2,3-dimethyl-butylen, Thio-2,4-dimethyl-butylen, Thio-3,3-dimethyl-butylen, Thio-3,4-dimethyl-butylen, Thio-1-ethyl-butylen, Thio-2-ethyl-butylen, Thio-3-ethyl-butylen, Thio-4-ethyl-butylen, Thio-1,1,2-trimethyl-propylen, Thio-1,1,3-trimethyl-propylen, Thio-1,2,2-trimethyl-propylen, Thio-1,2,3-trimethyl-propylen, Thio-2,2,3-trimethyl-propylen, Thio-2,3,3-trimethyl-propylen, Thio-1,3,3-trimethyl-propylen, Thio-1-ethyl-1-methyl-propylen, Thio-2-ethyl-1-methyl-propylen, Thio-3-ethyl-1-methyl-propylen, Thio-1-ethyl-2-methyl-propylen, Thio-2-ethyl-2-methyl-propylen, Thio-3-ethyl-2-methyl-propylen, Thio-1-ethyl-3-methyl-propylen, Thio-2-ethyl-3-methyl-propylen, Thio-3-ethyl-3-methyl-propylen, Thio-1,1-diethyl-ethylen, Thio-1,2-diethyl-ethylen, Thio-2,2-diethyl-ethylen und Thio-1,1,2,2-tetramethyl-ethylen, vorzugsweise Thiomethylen;
C₂-C₆-Alkenylen wie vorstehend genannt, vorzugsweise Ethenylen und Propenylen;
Sauerstoff oder Schwefel.

Beispiele für, hinsichtlich ihrer biologischen Wirkung gegen Schadpilze und Schädlinge, besonders bevorzugte α-Phenylacrylsäurederivate I und I' sind in den folgenden Tabellen 1 bis 6 zusammengestellt.

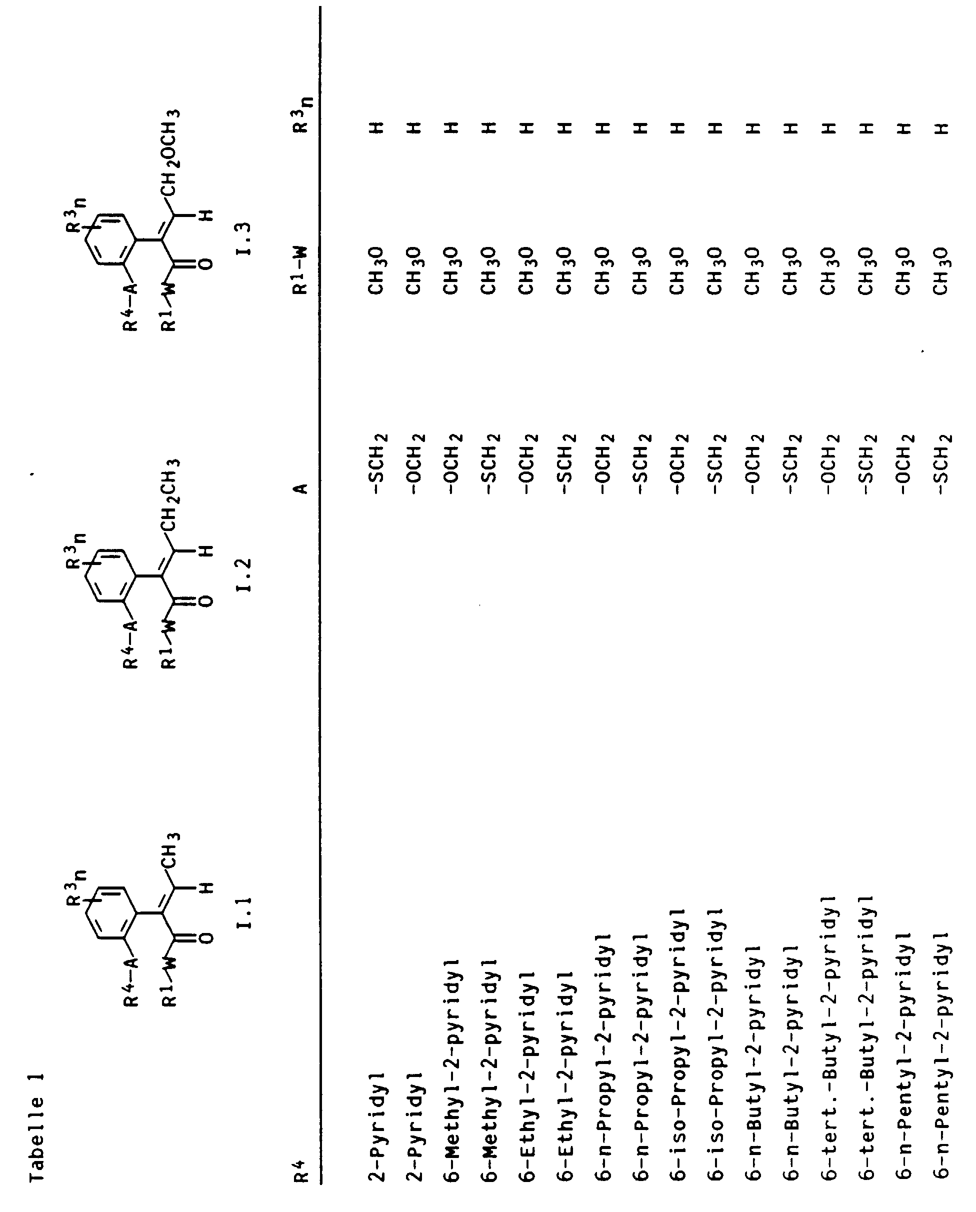

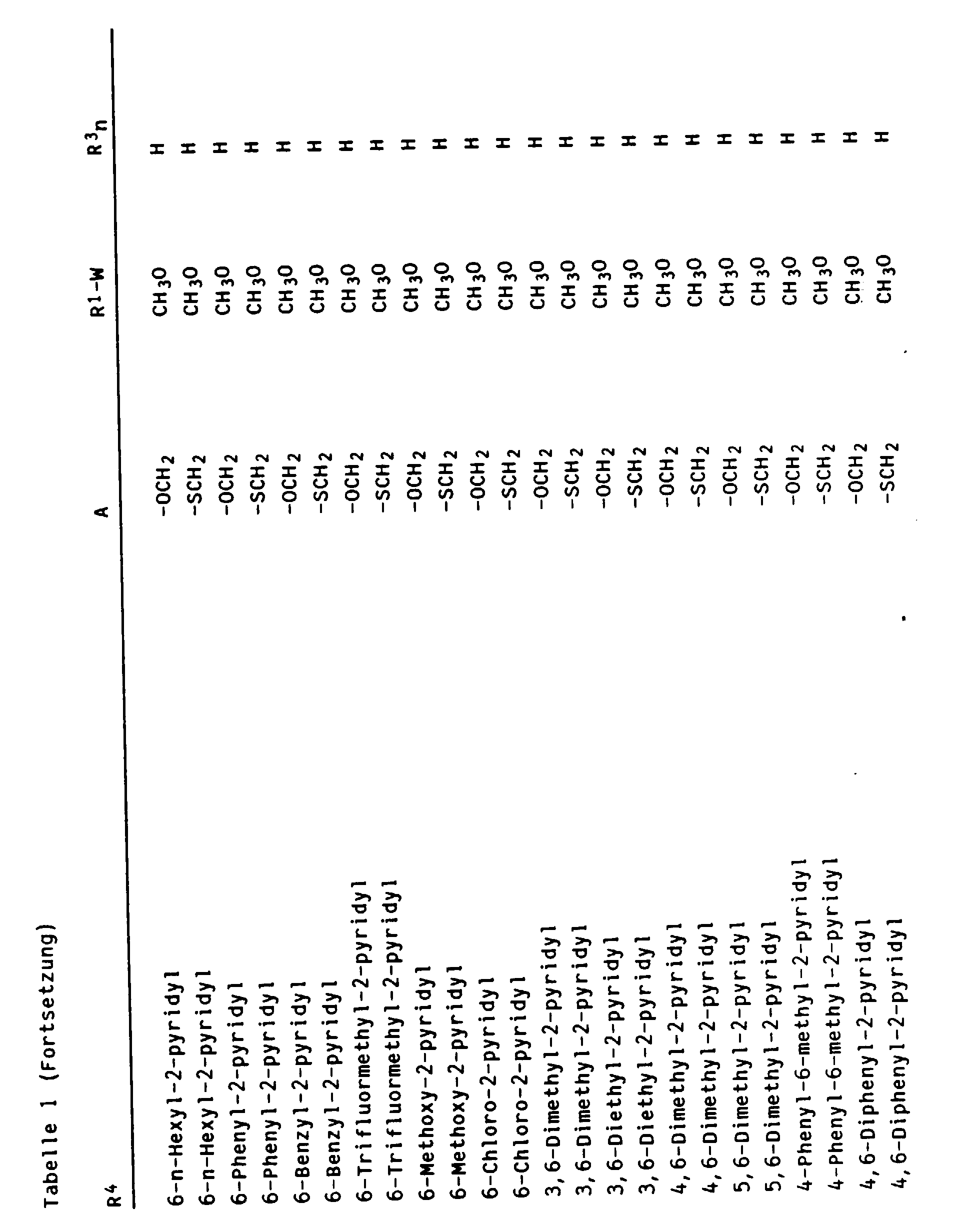

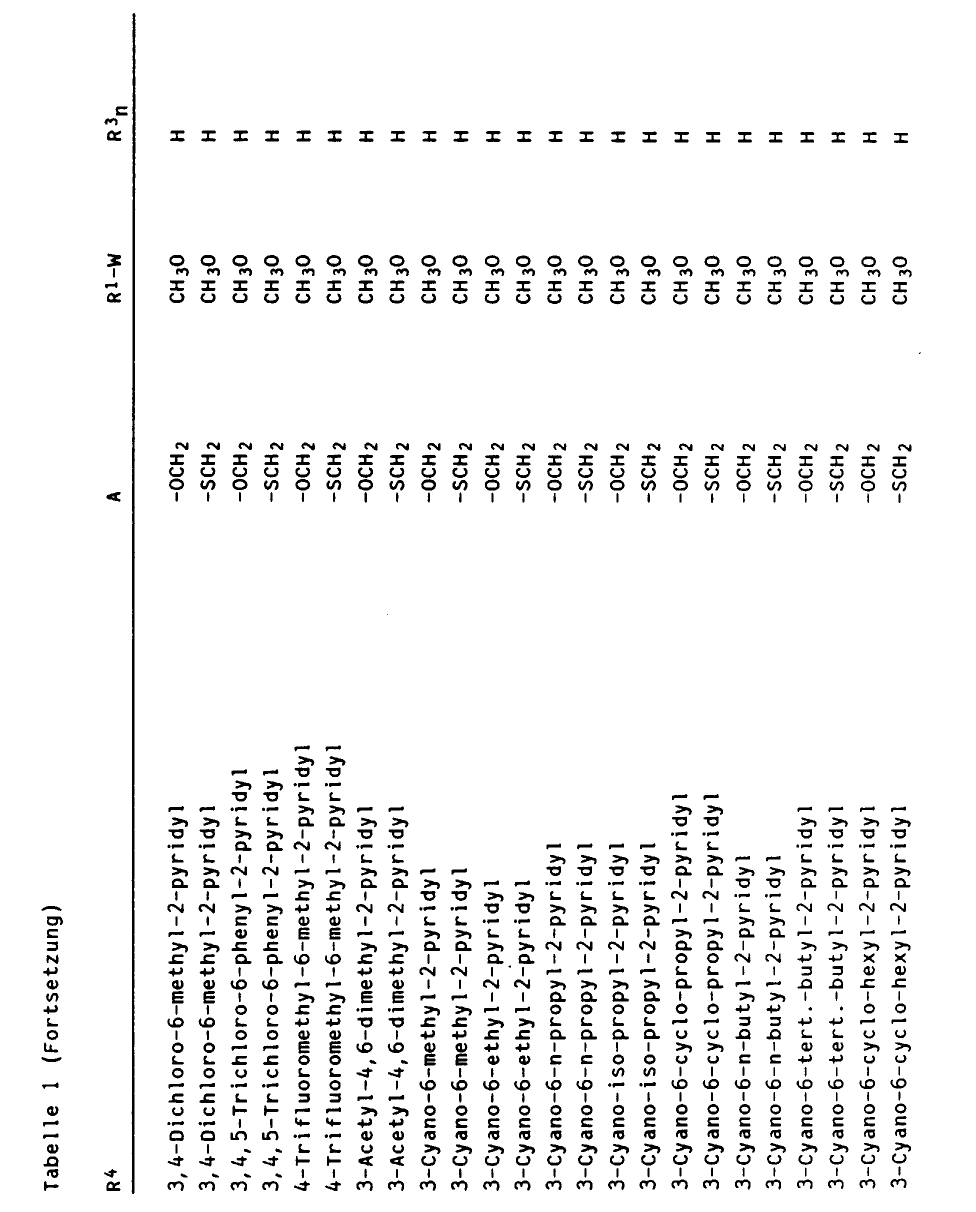

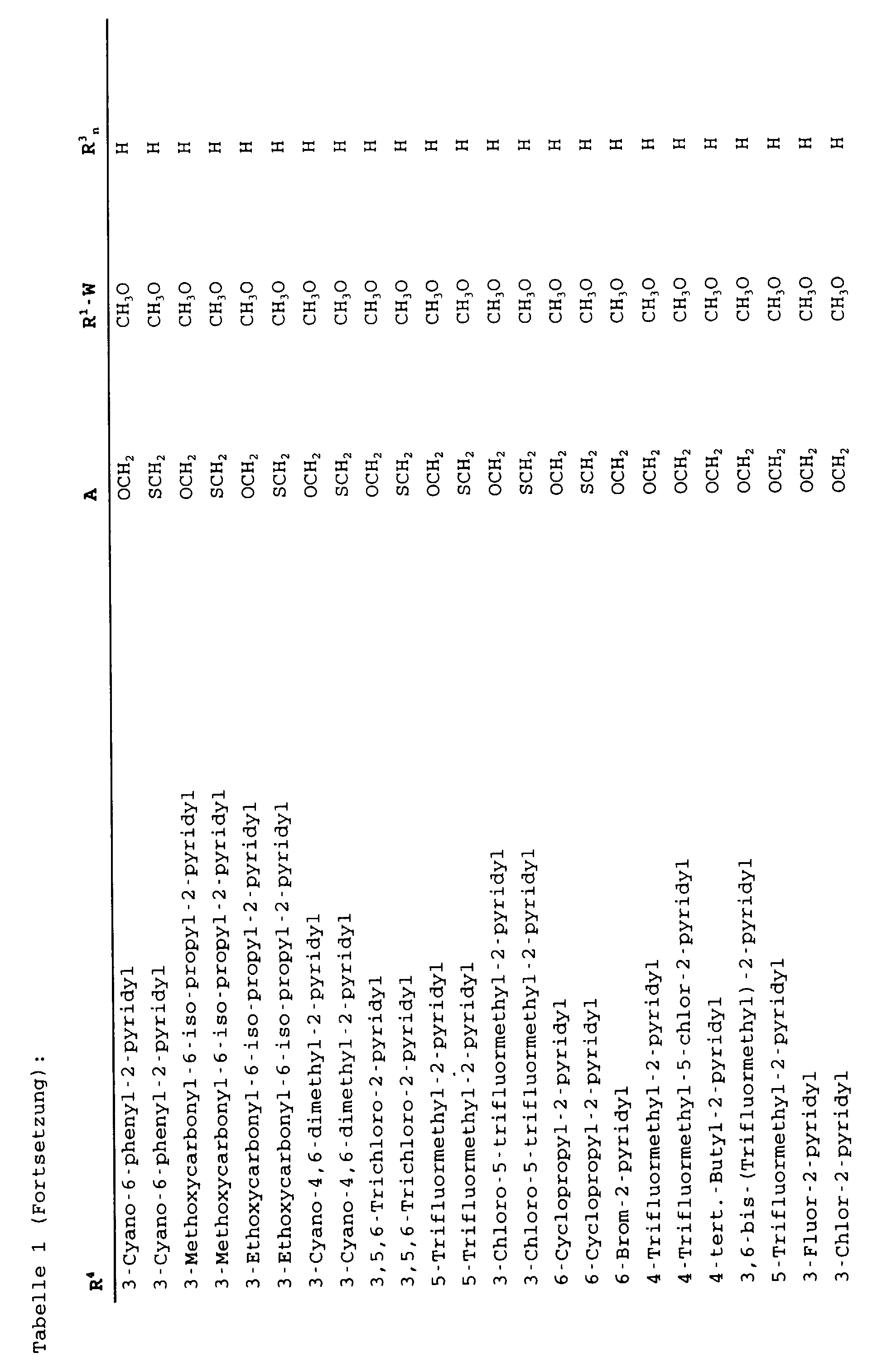

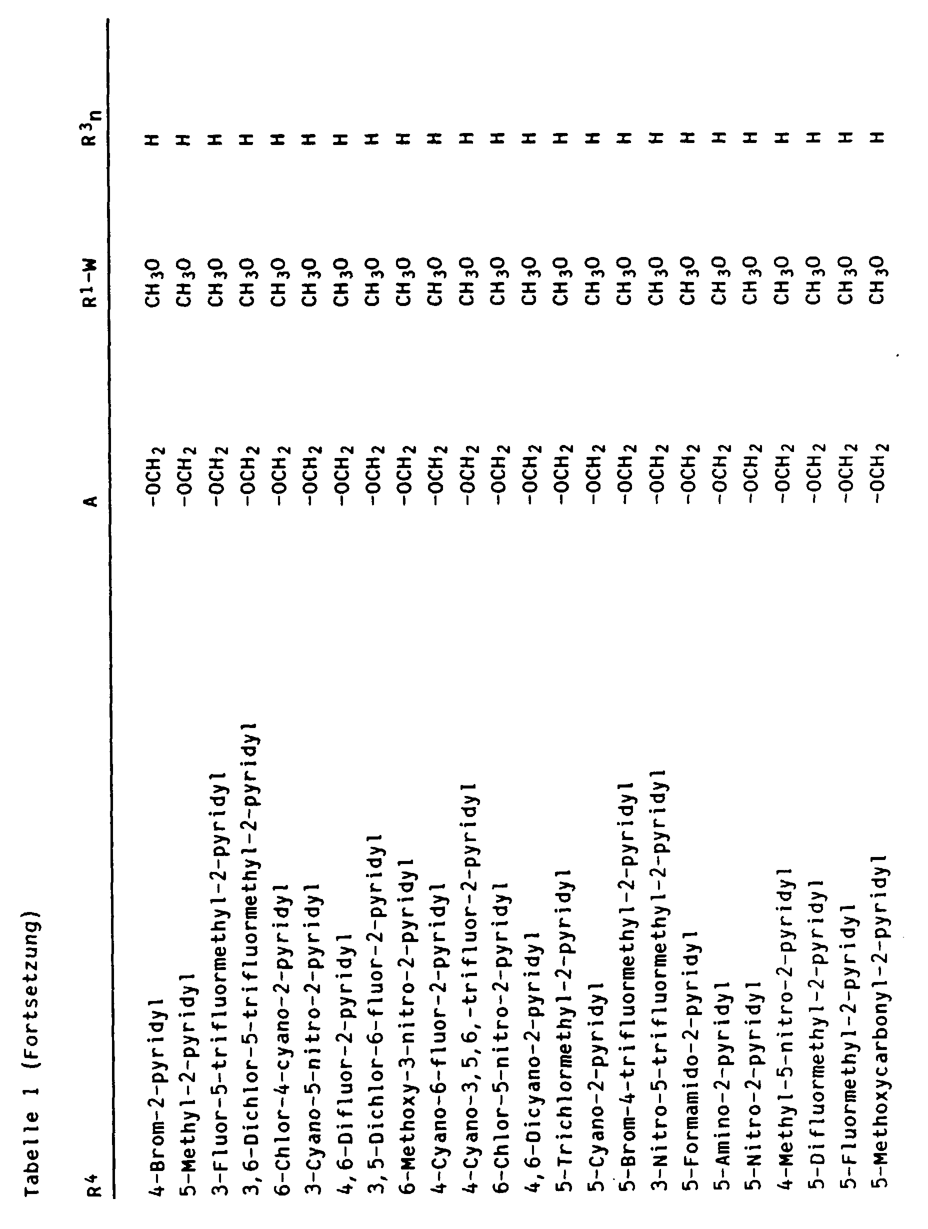

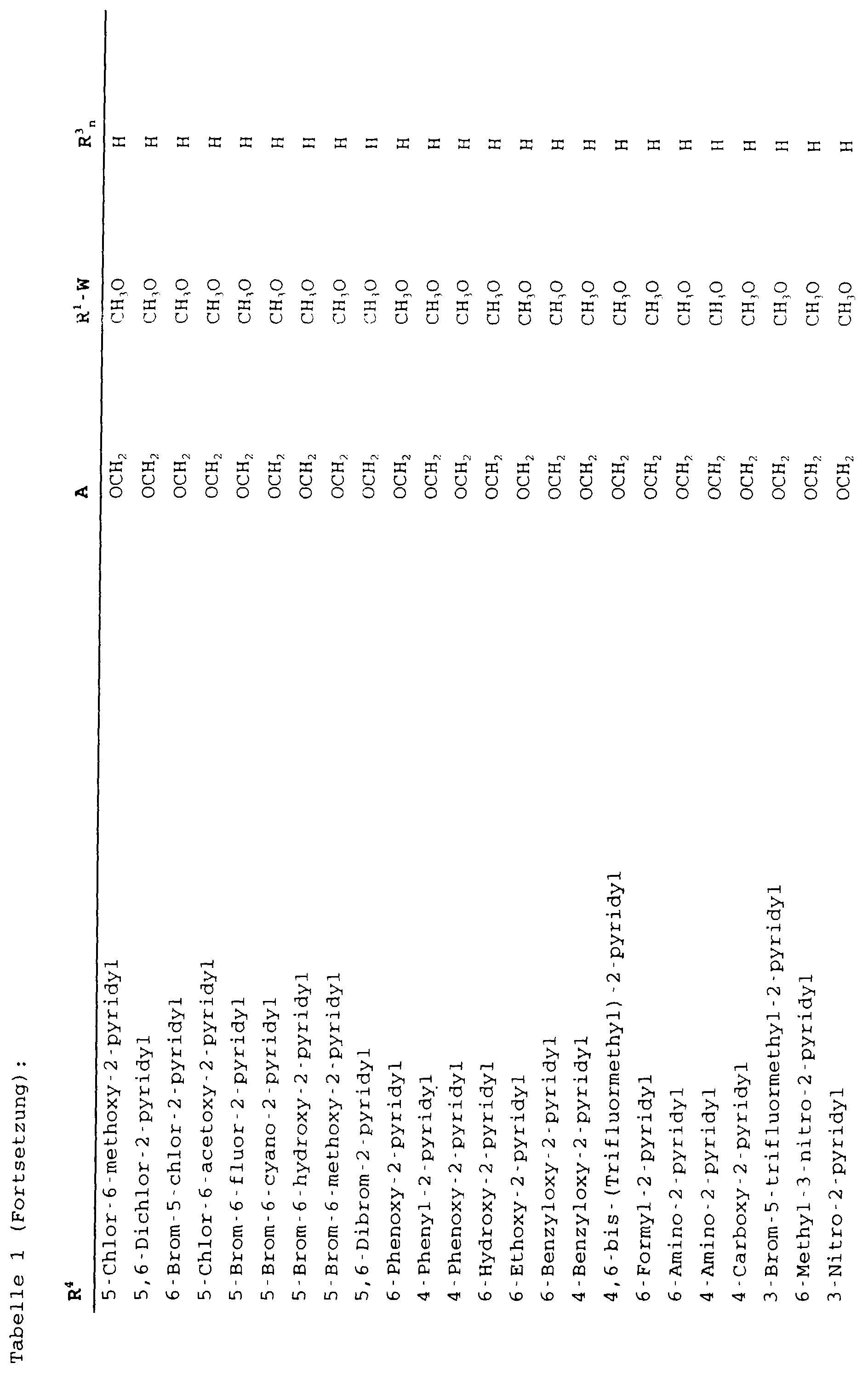

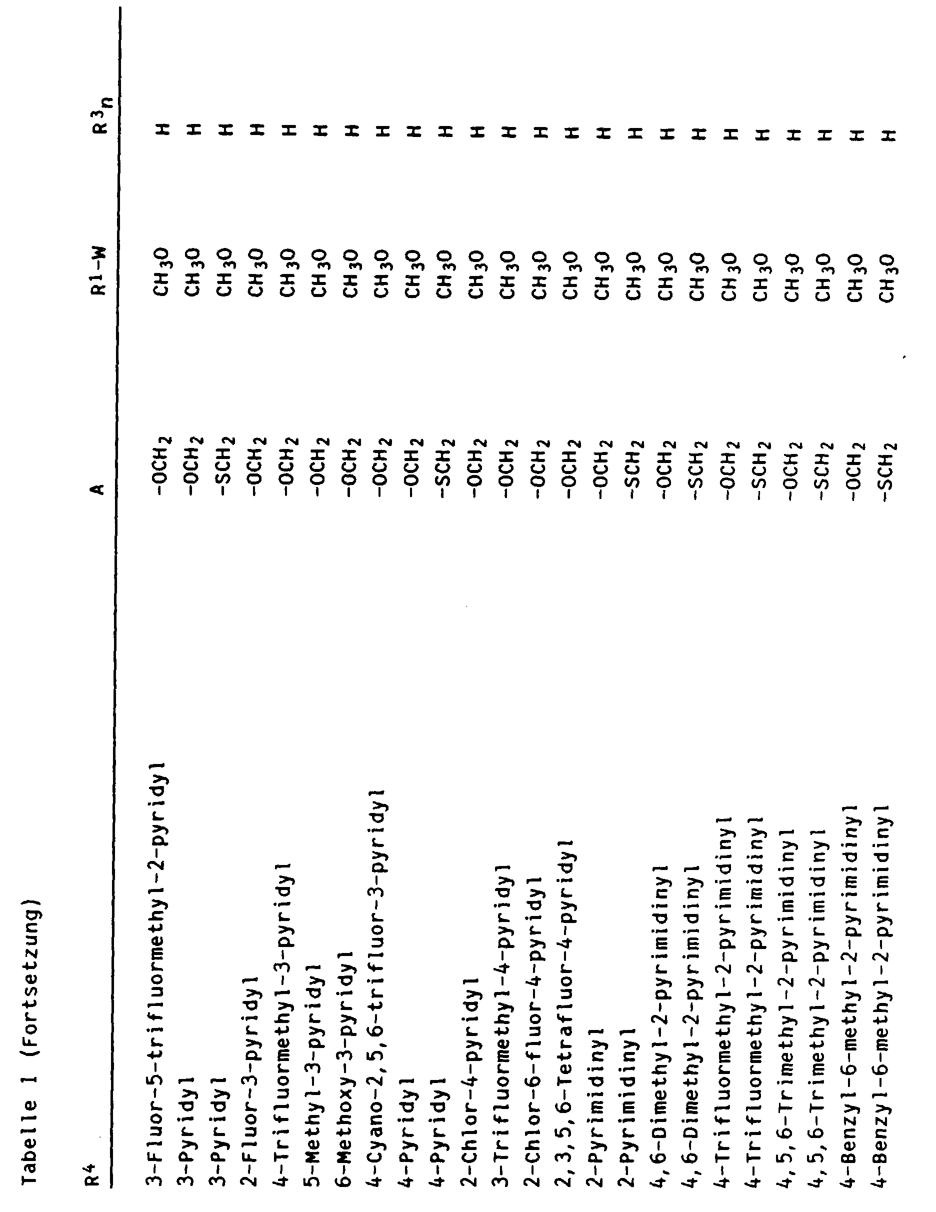

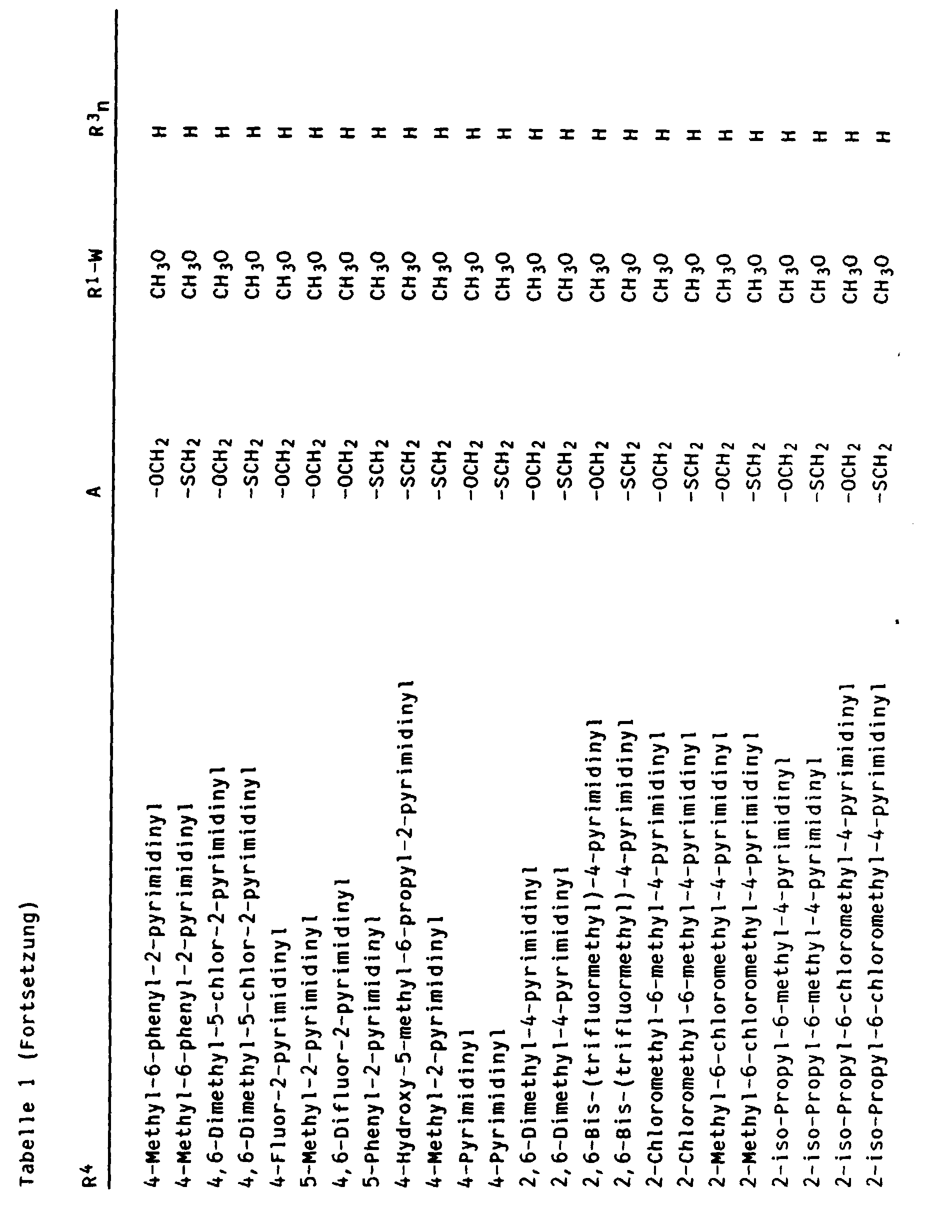

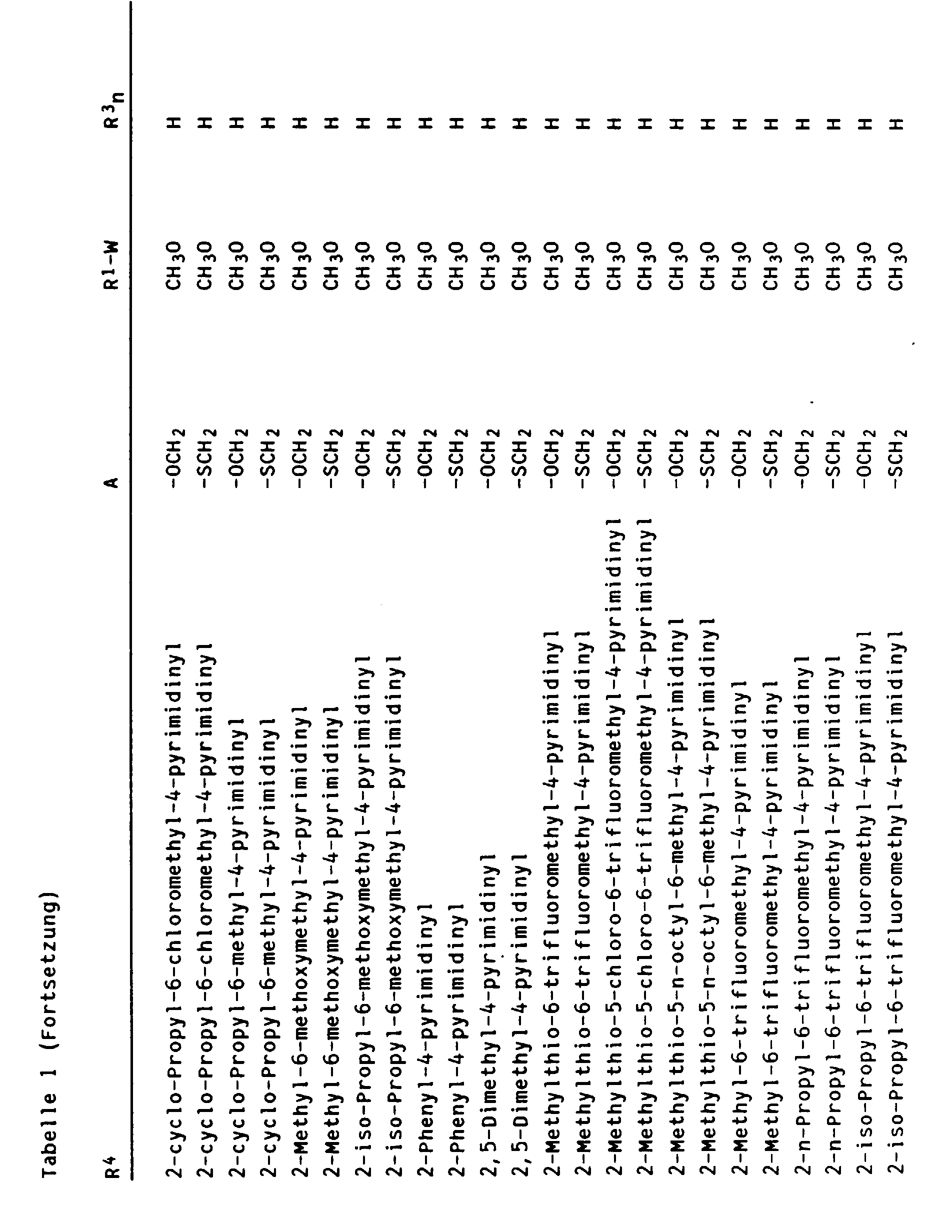

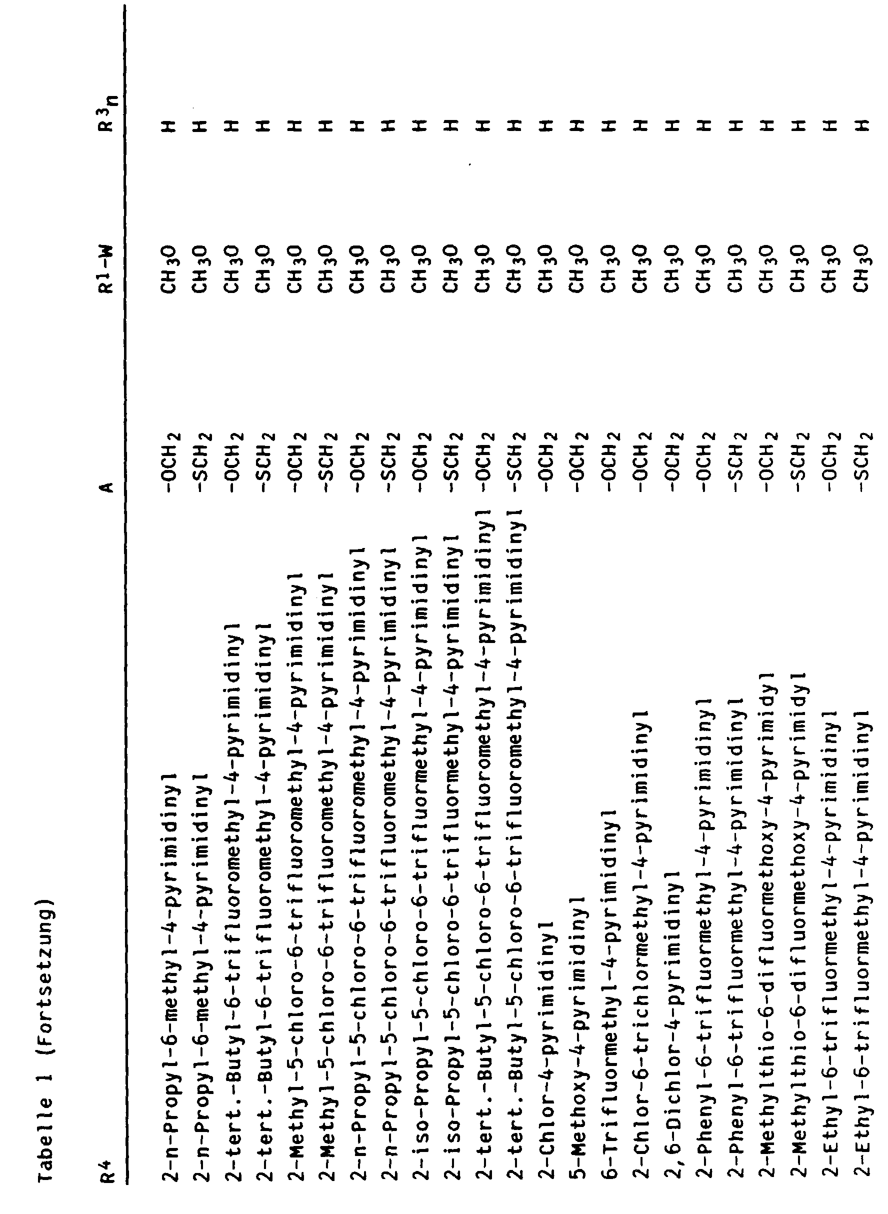

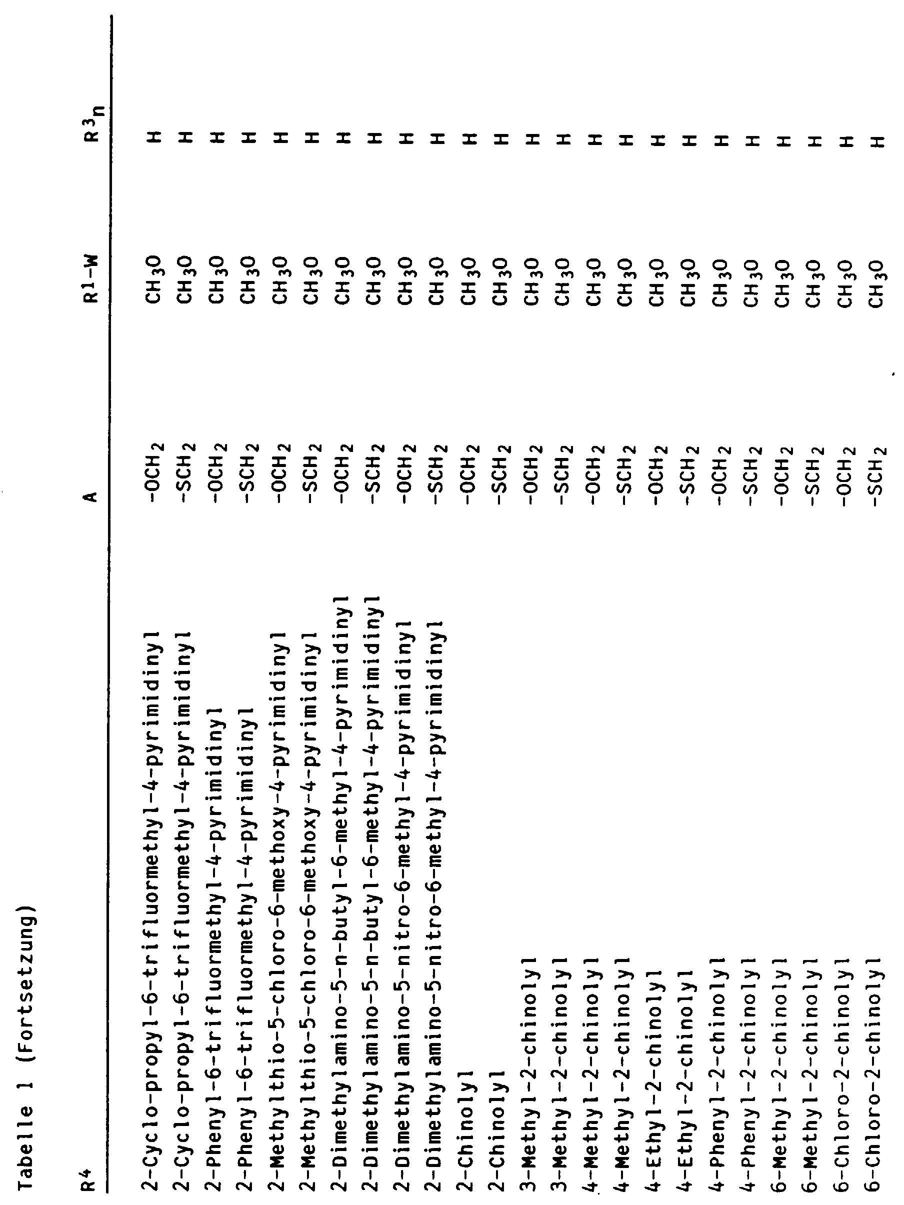

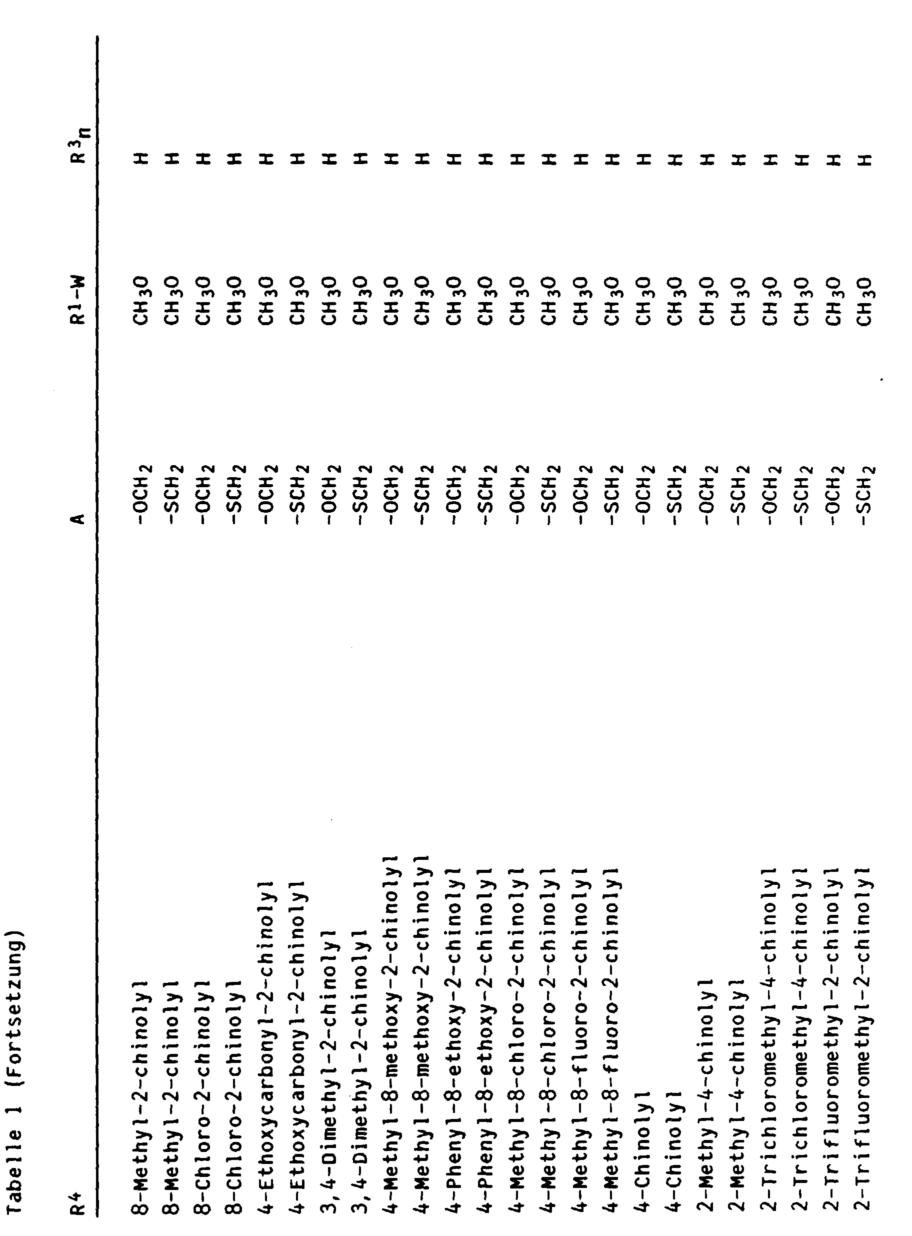

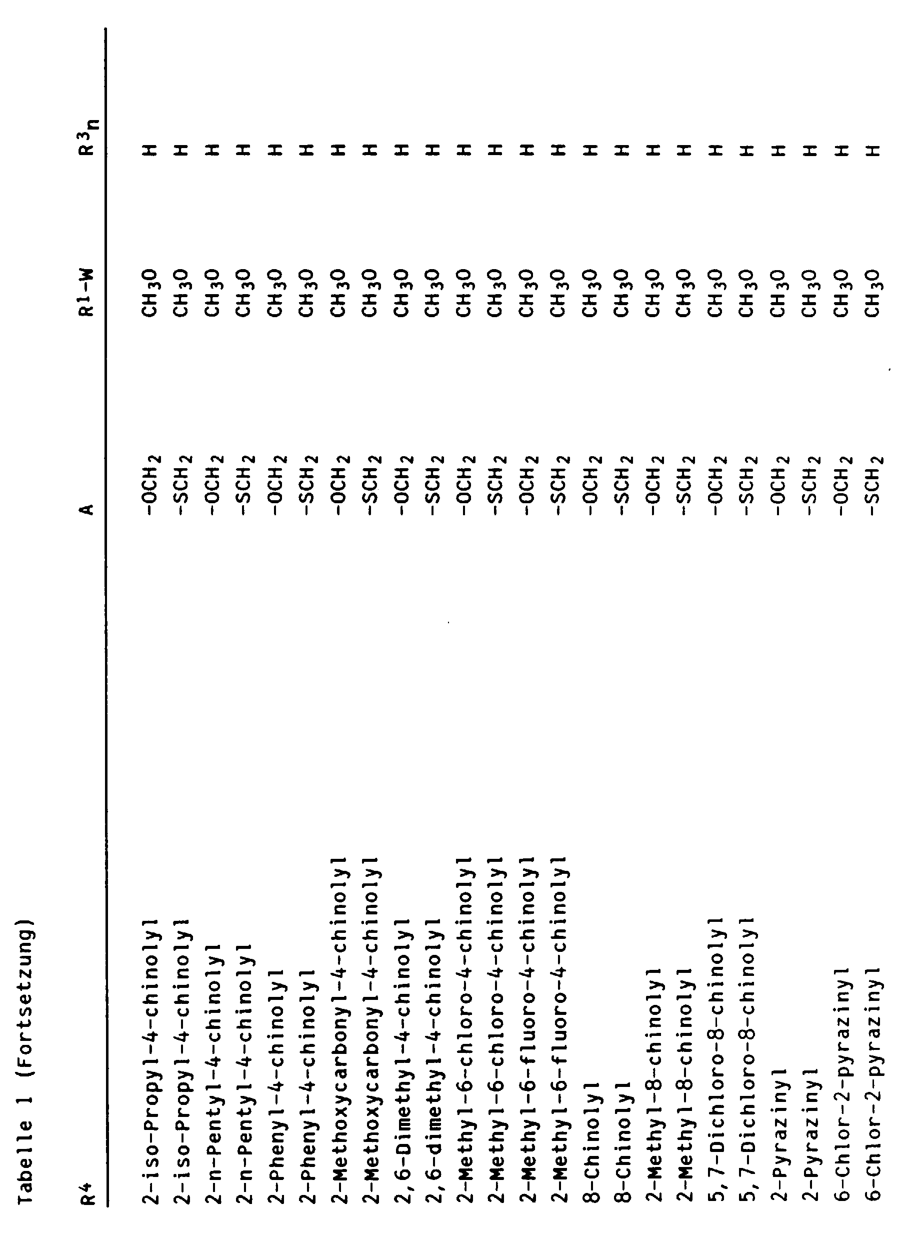

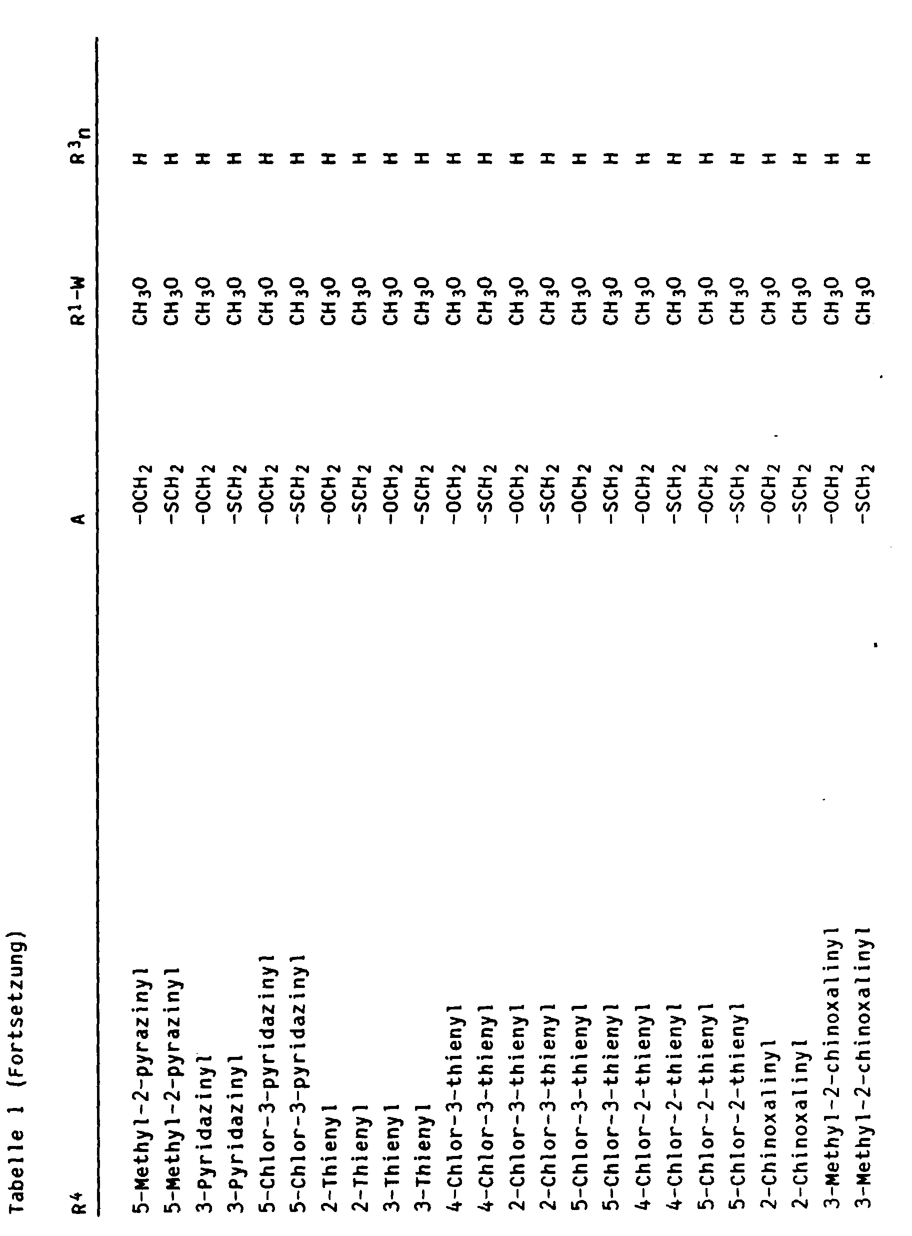

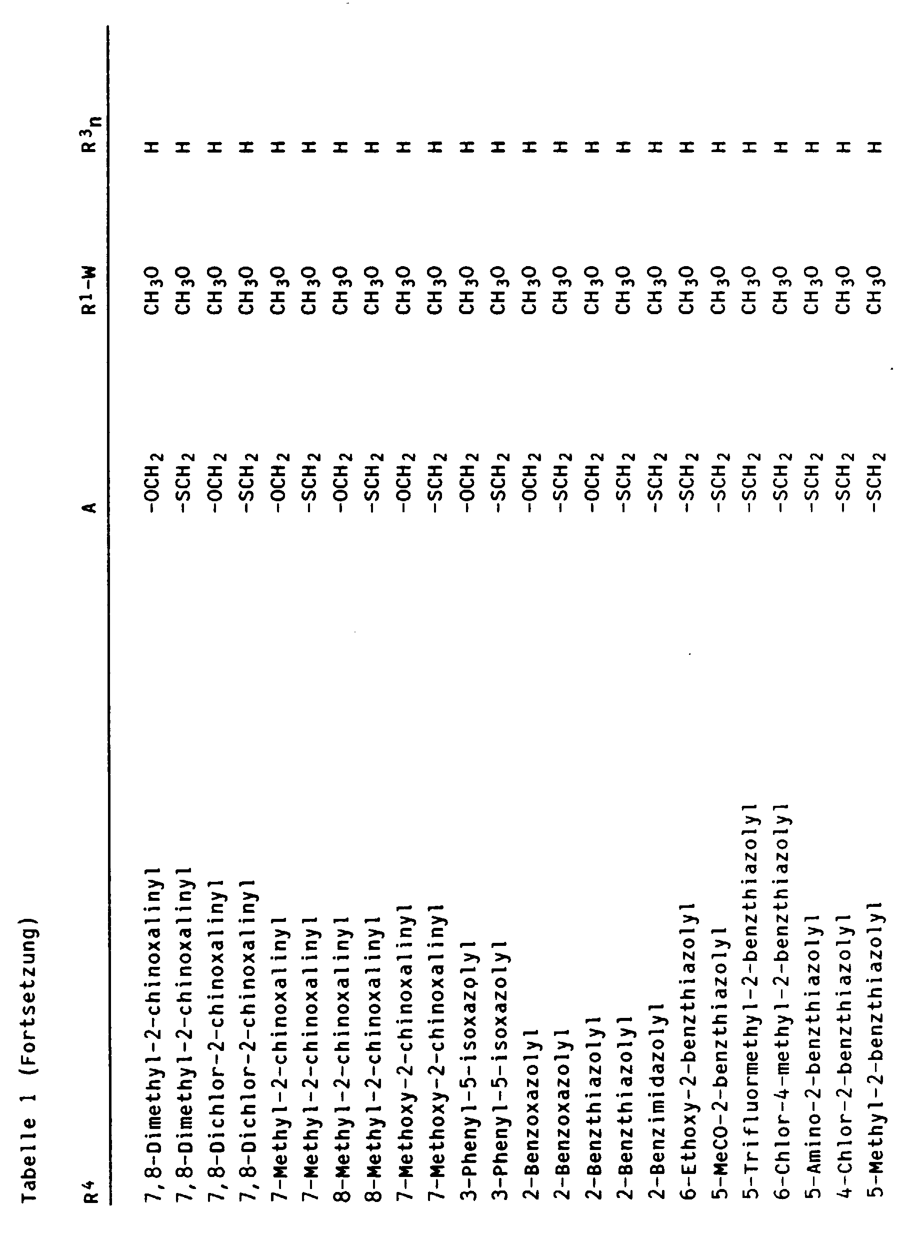

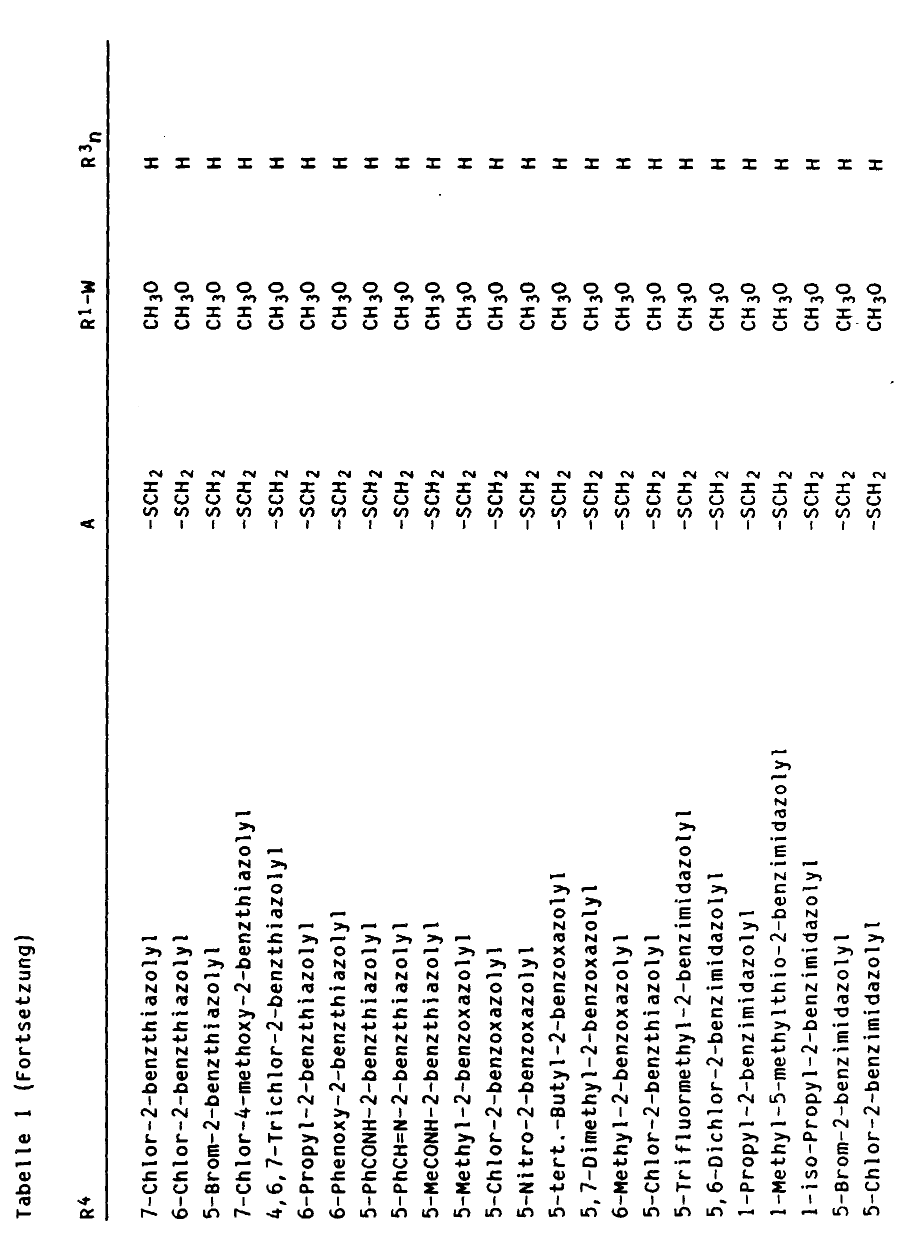

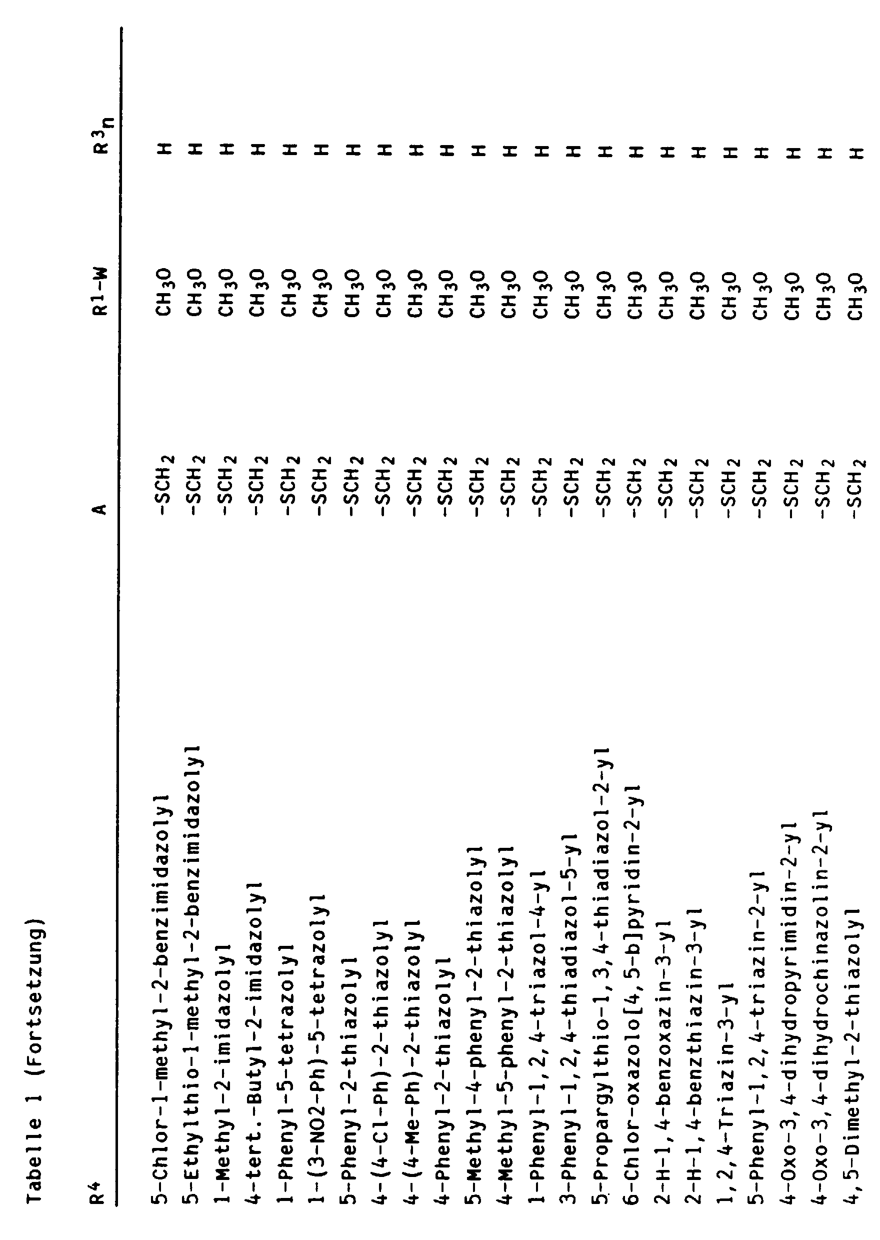

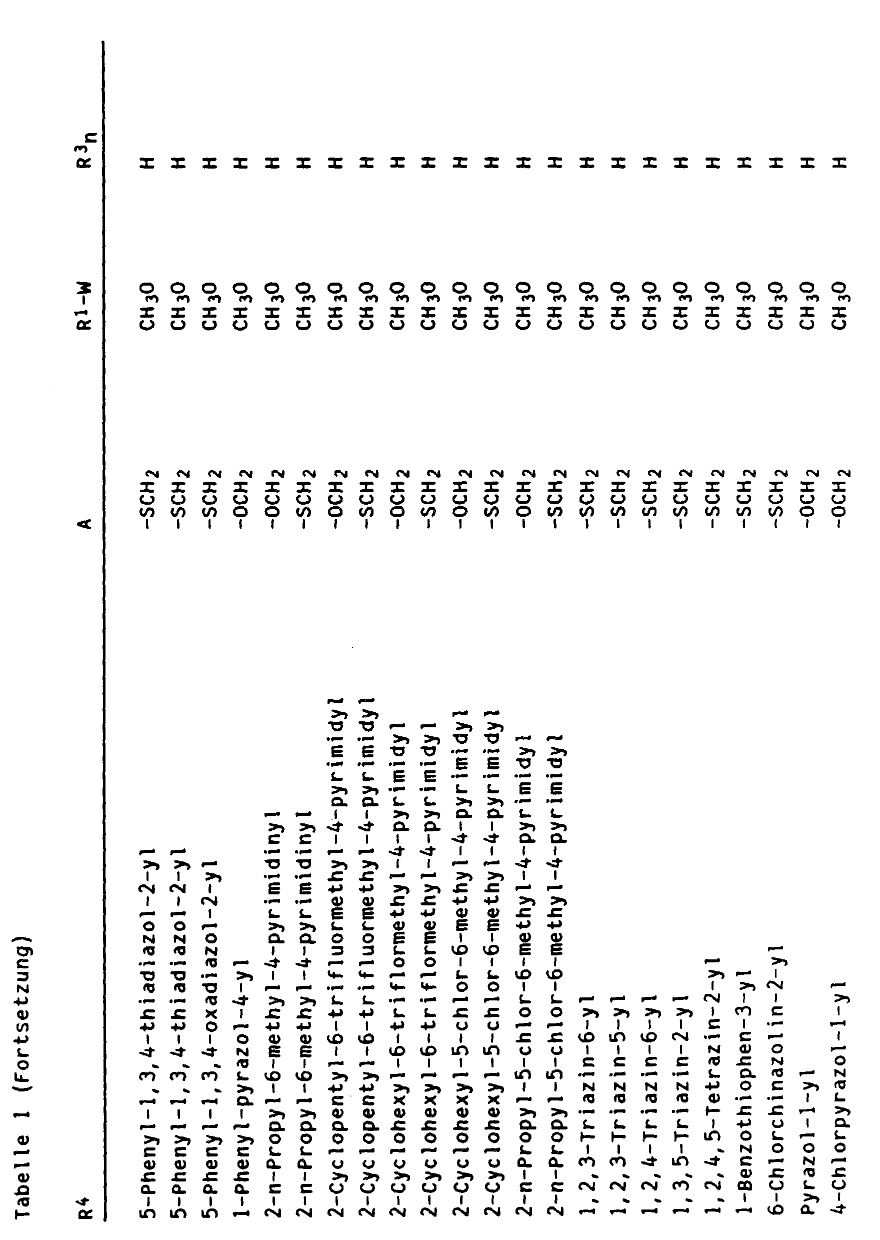

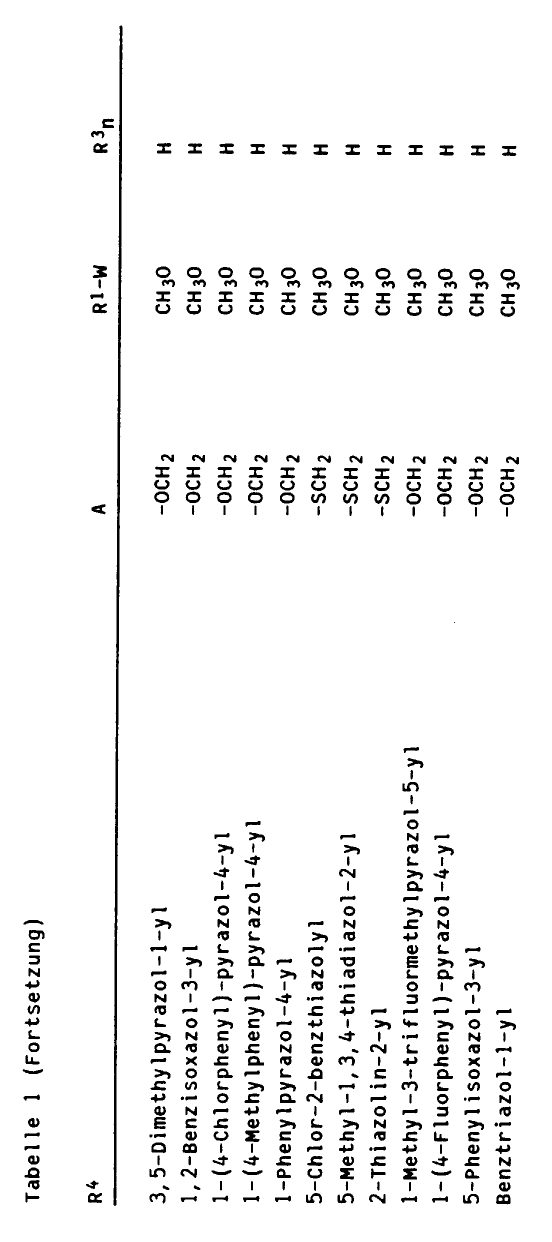

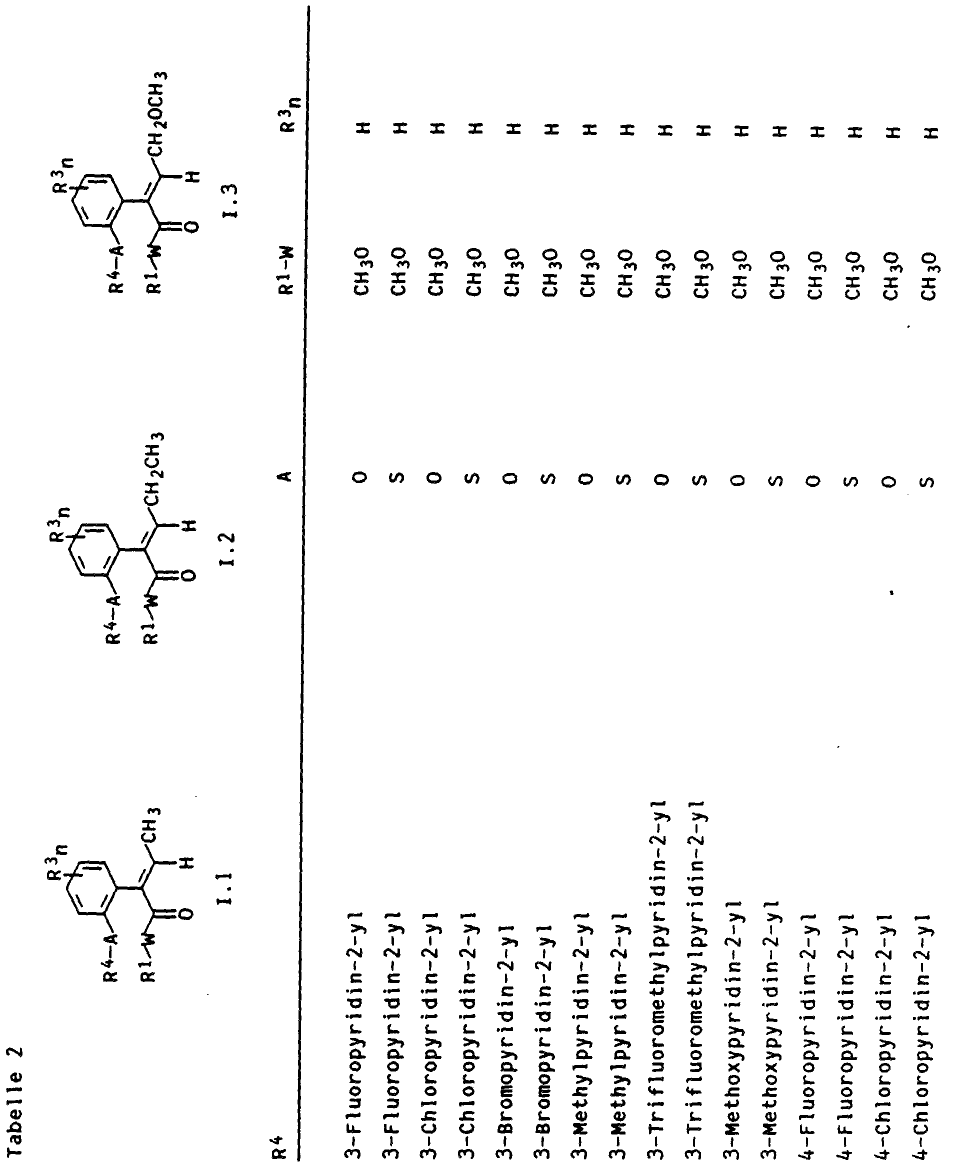

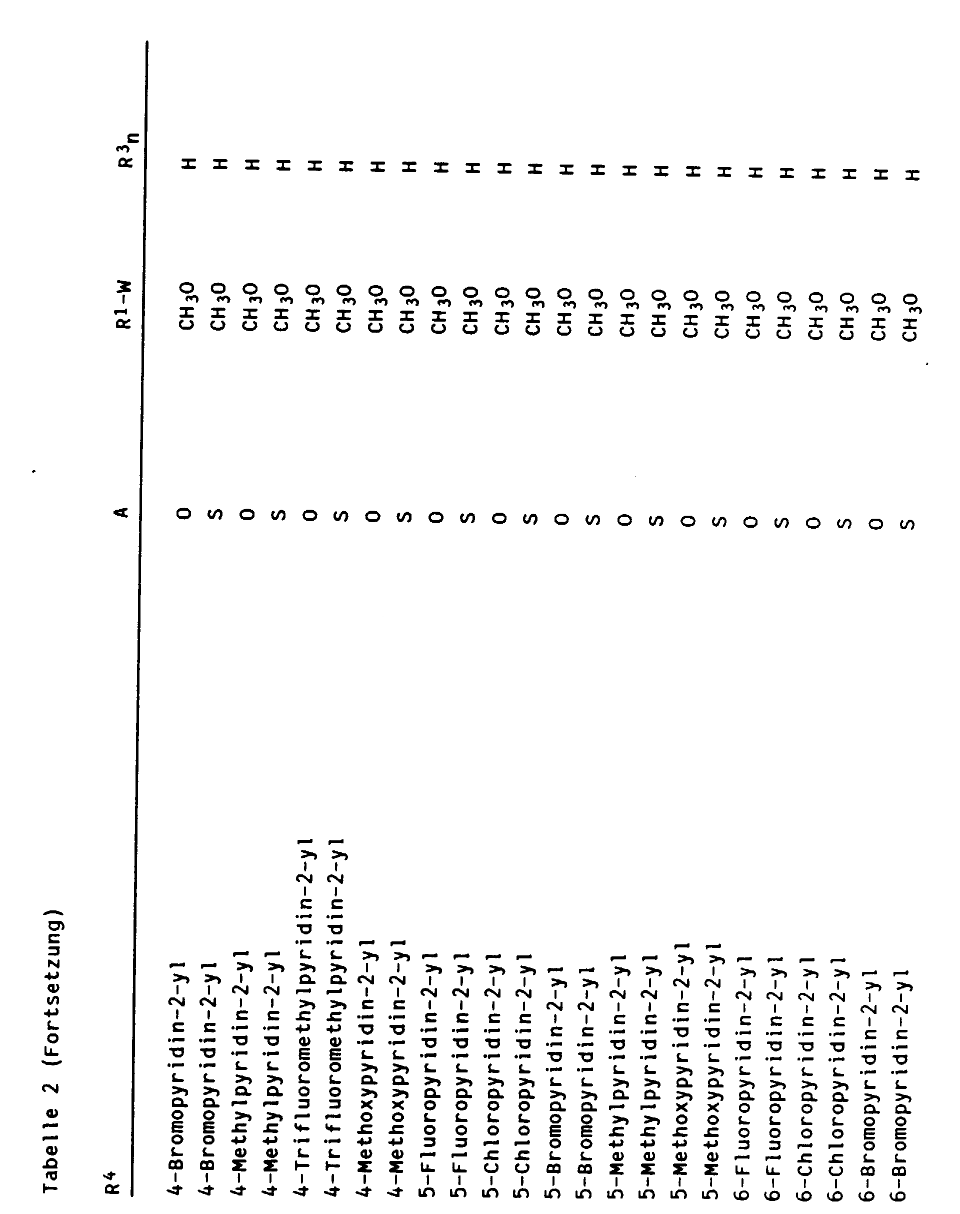

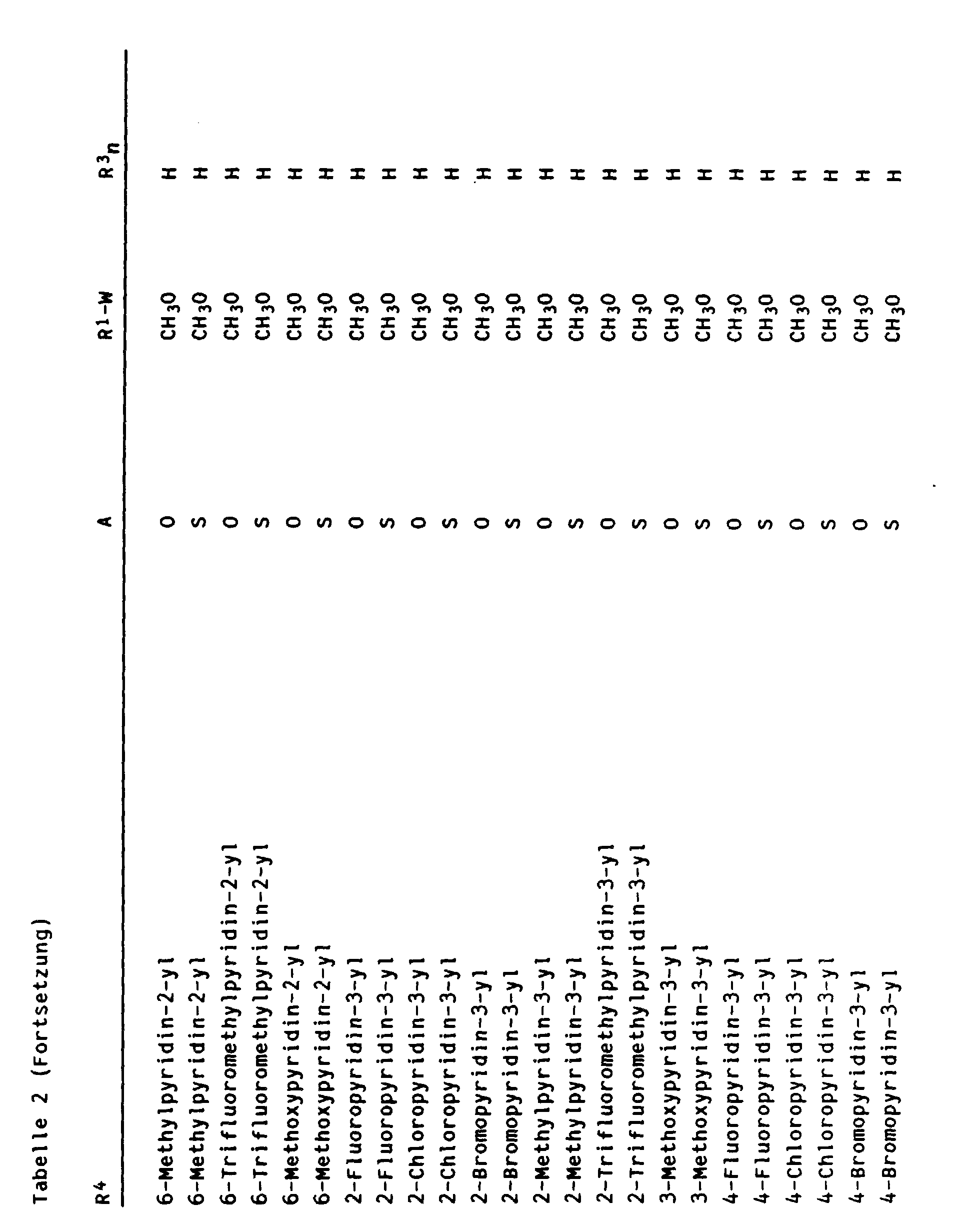

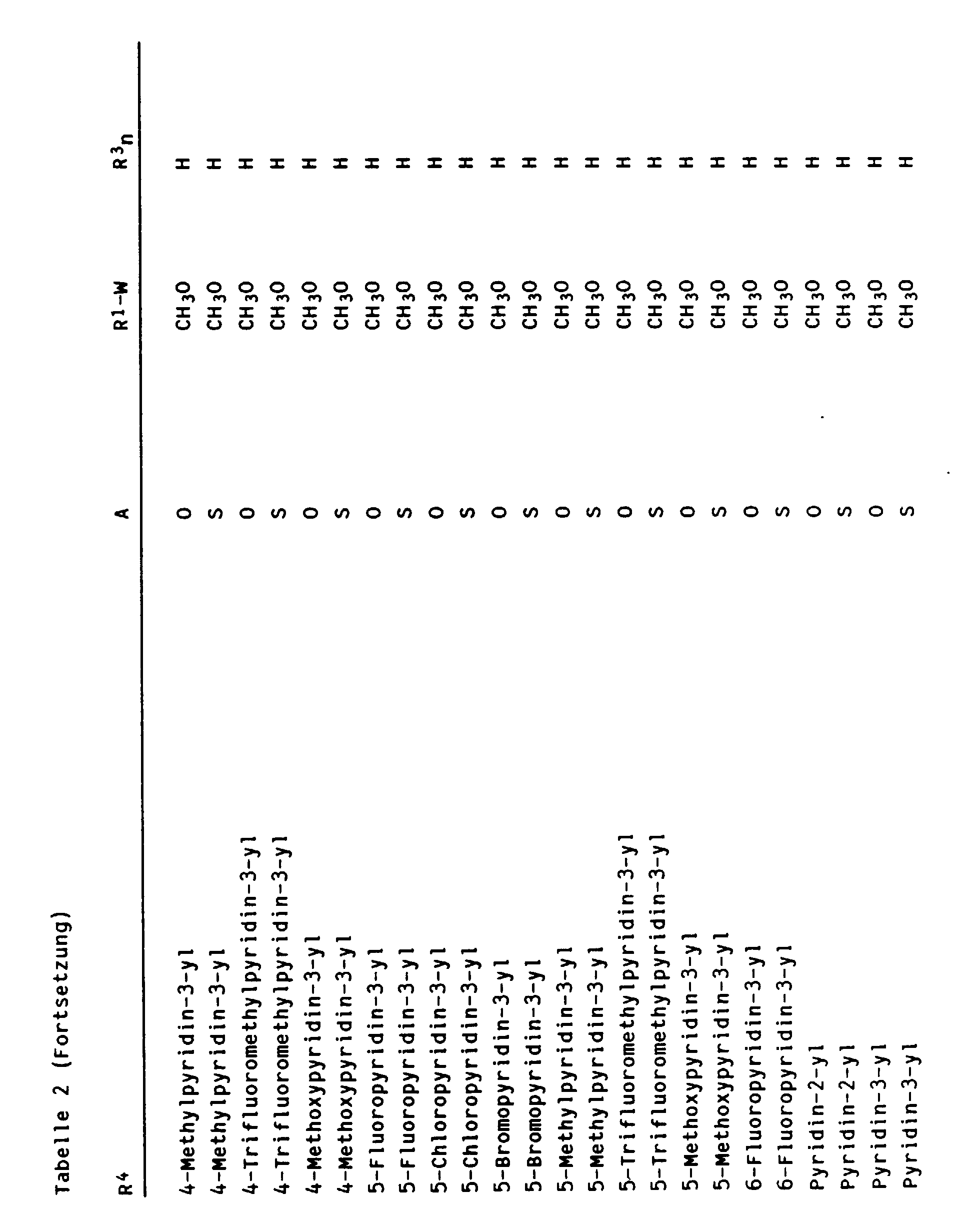

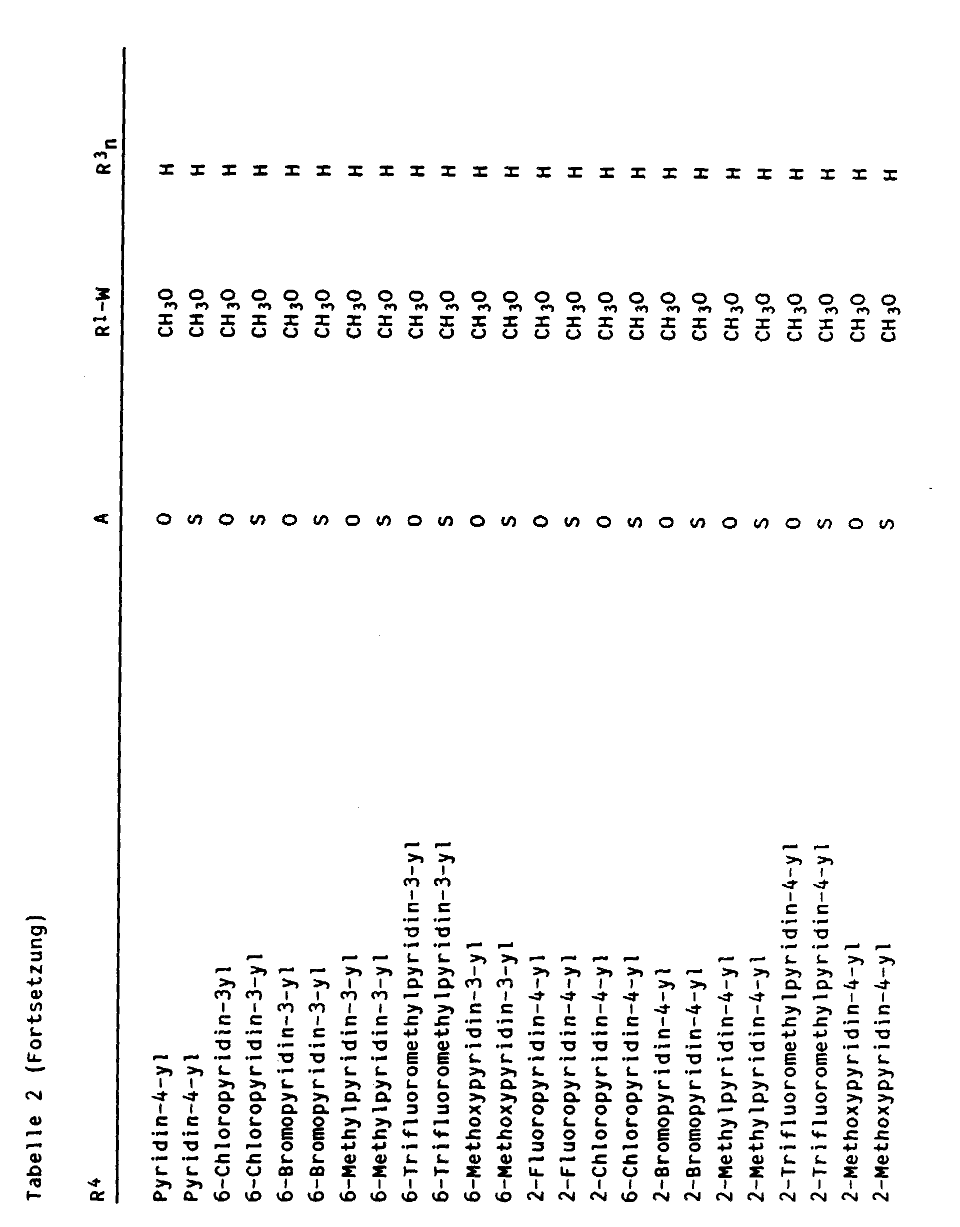

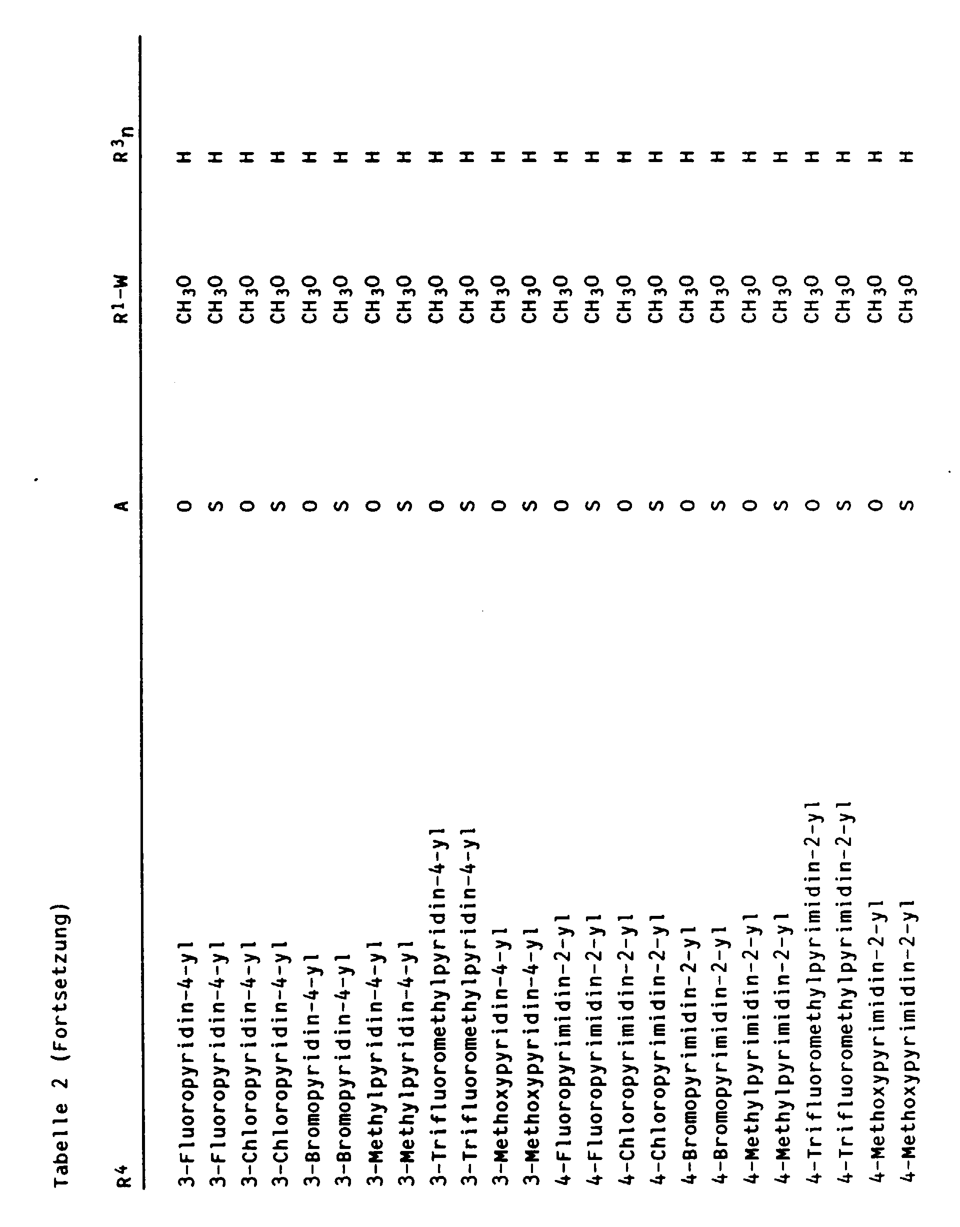

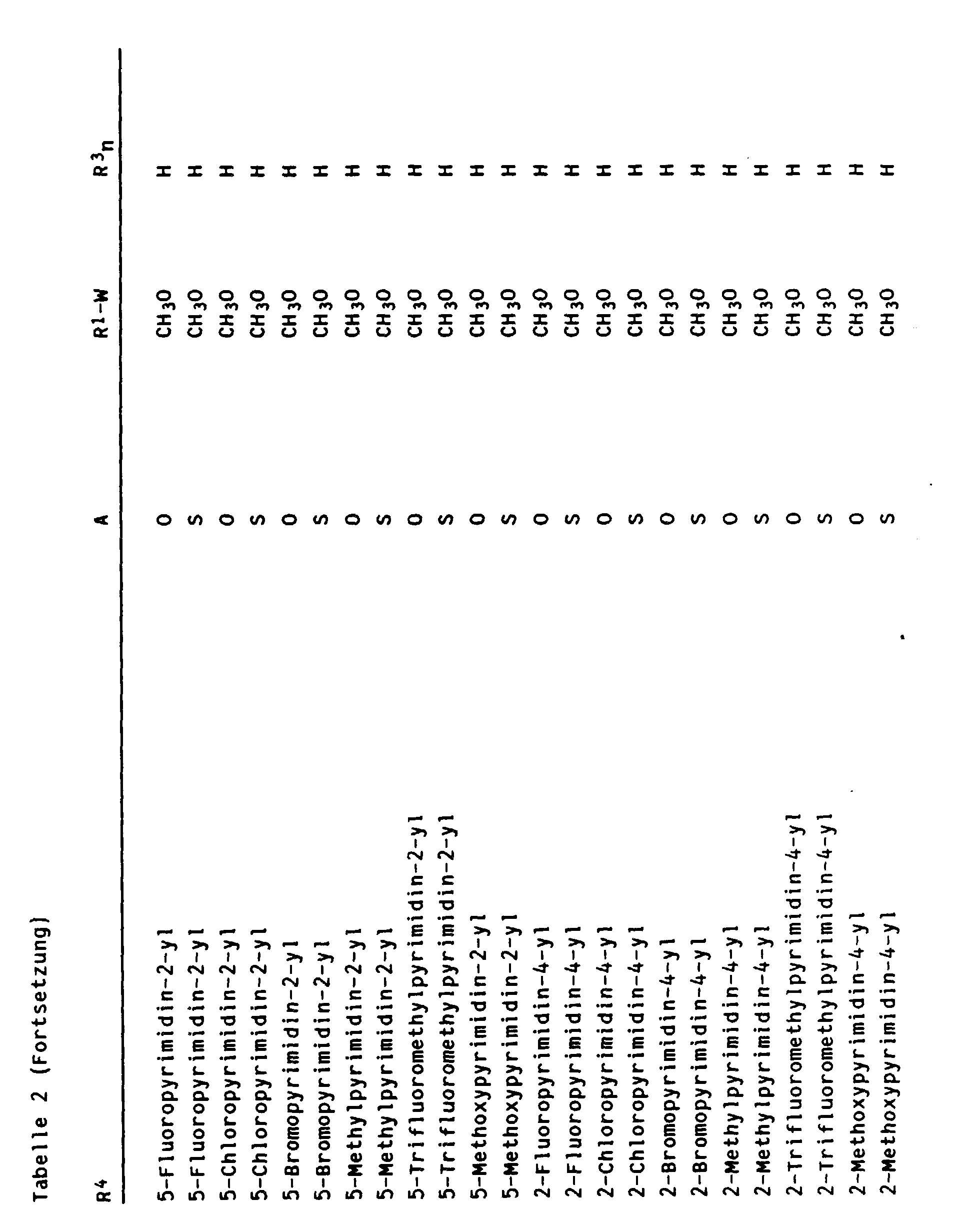

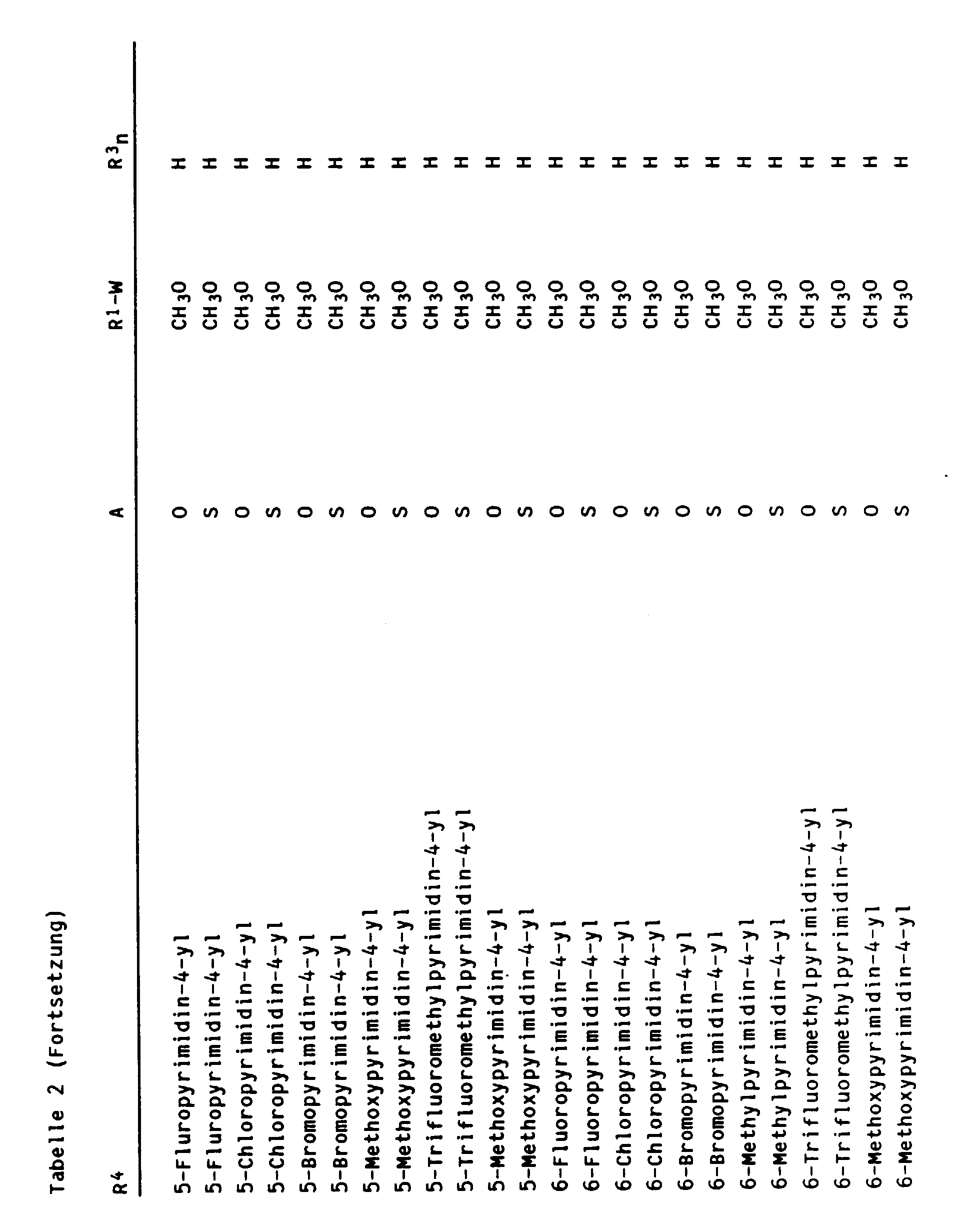

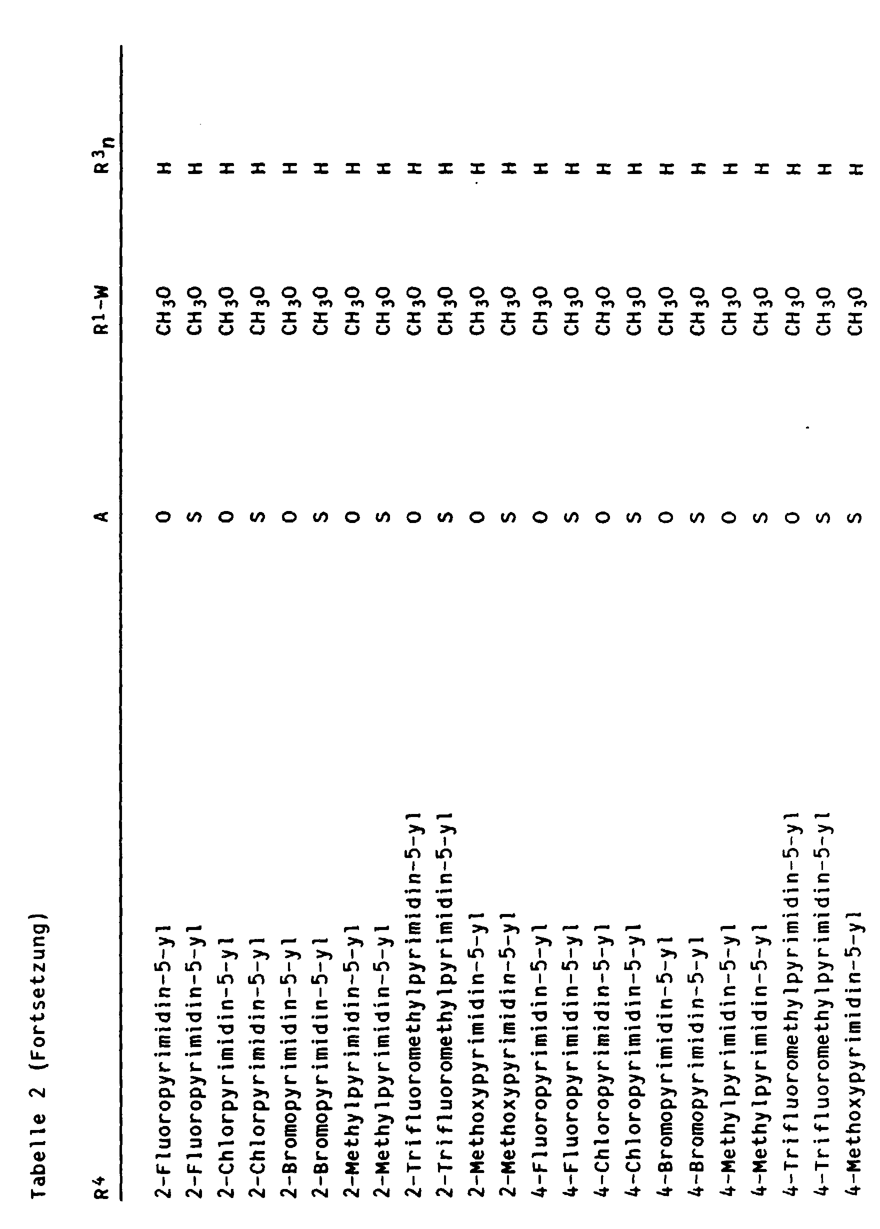

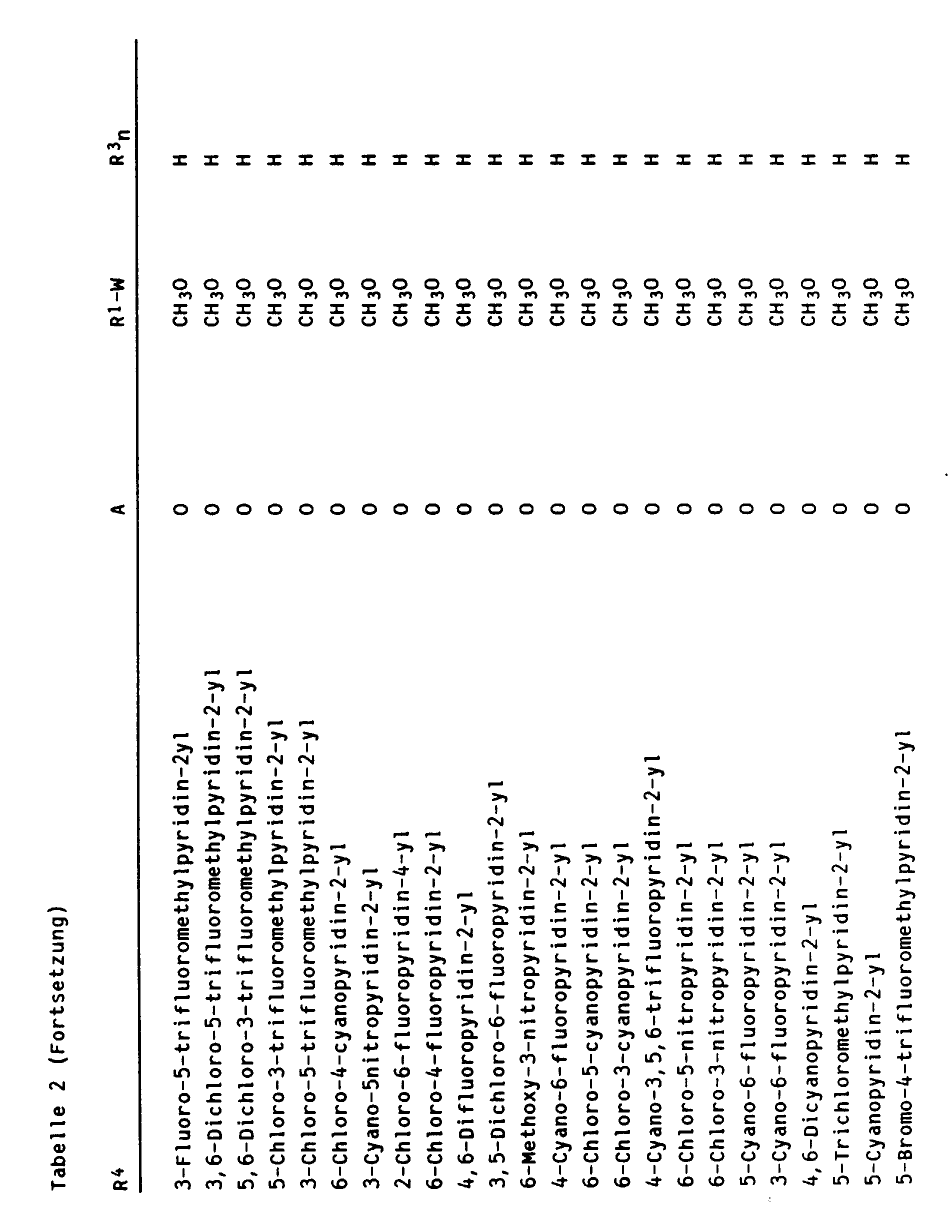

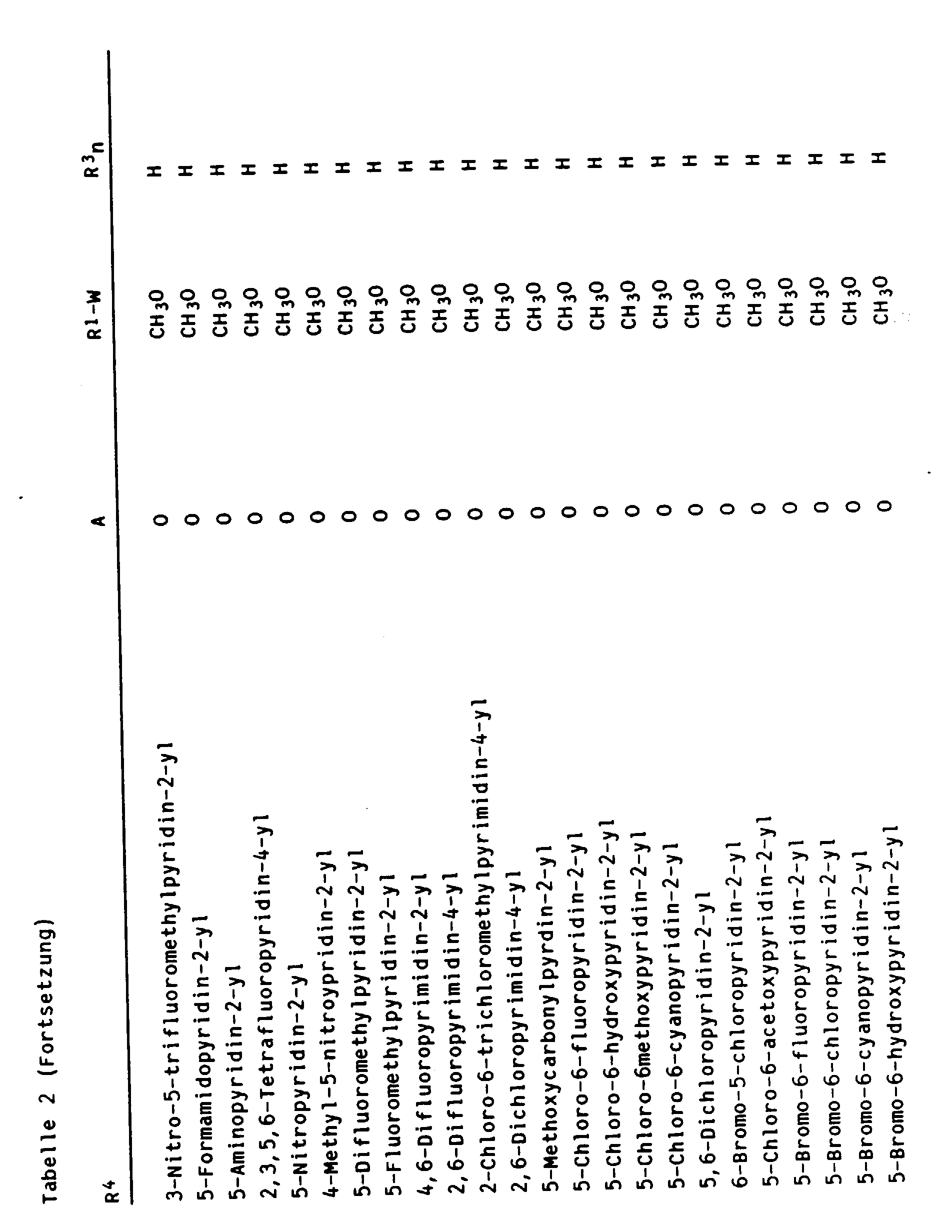

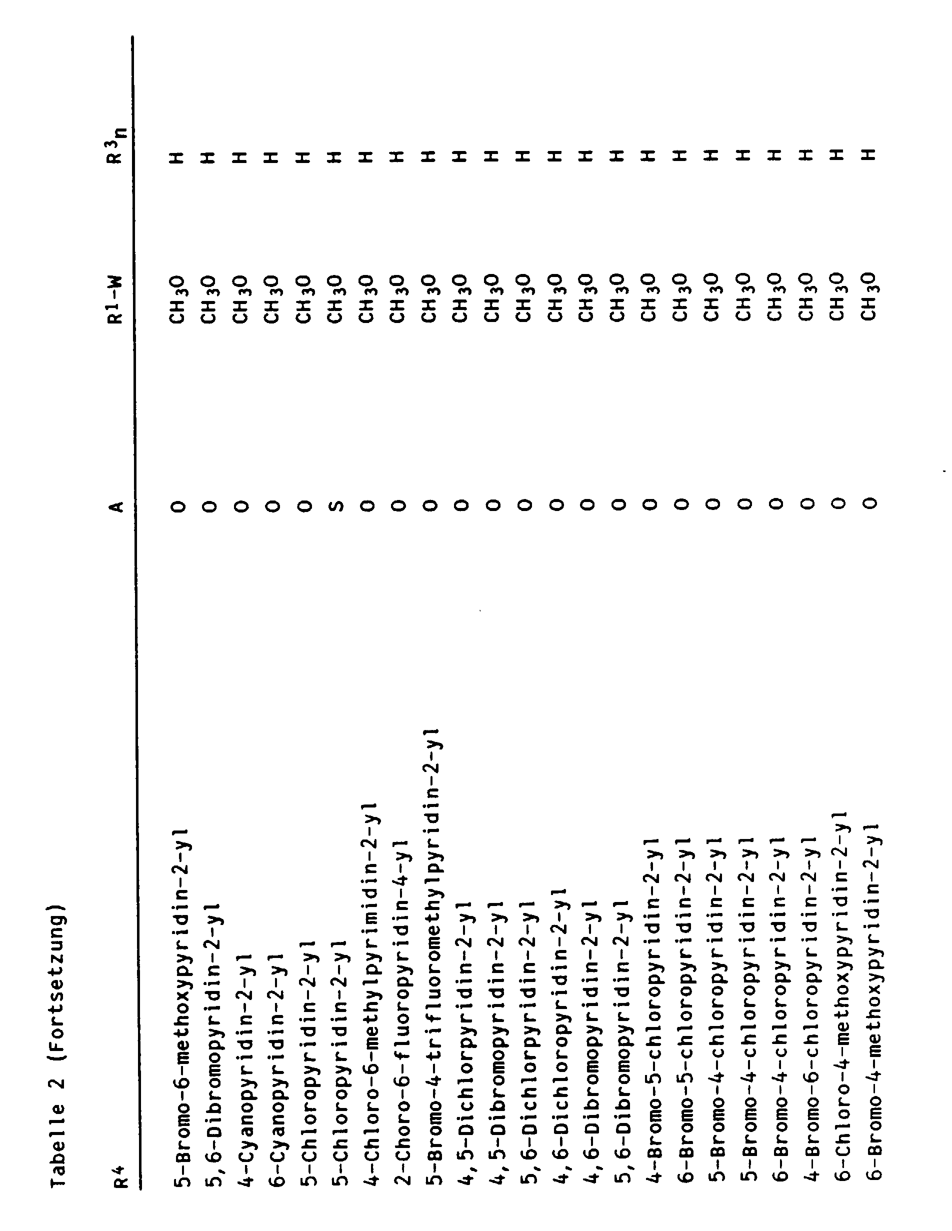

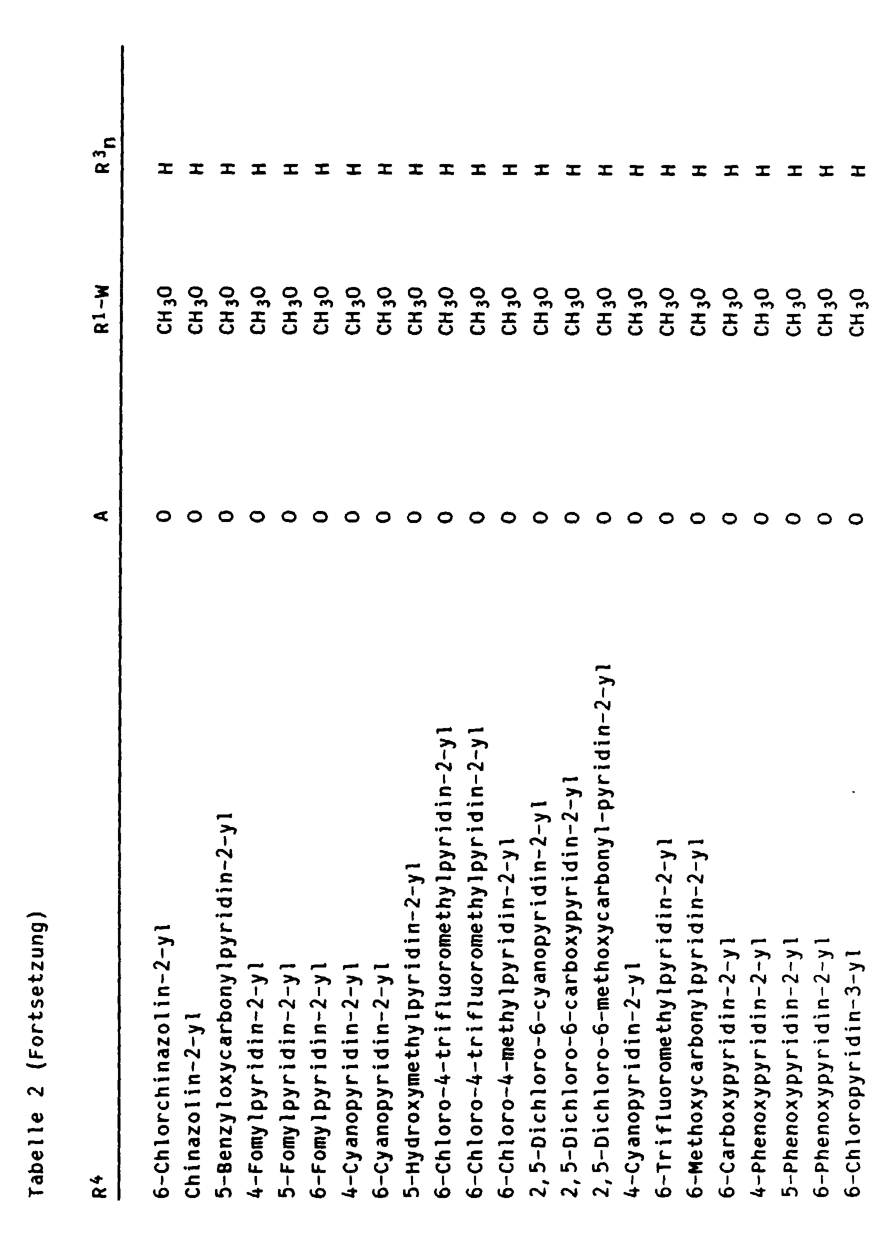

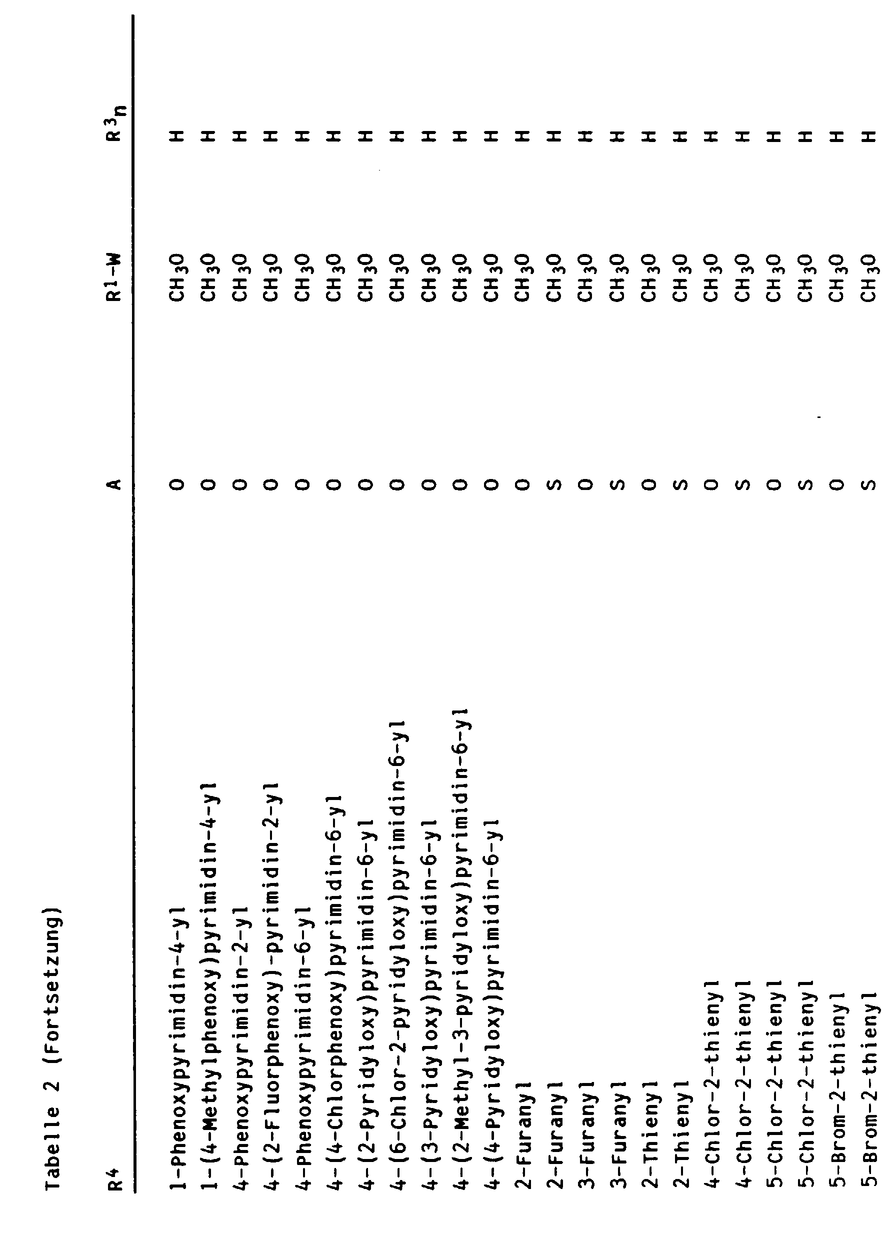

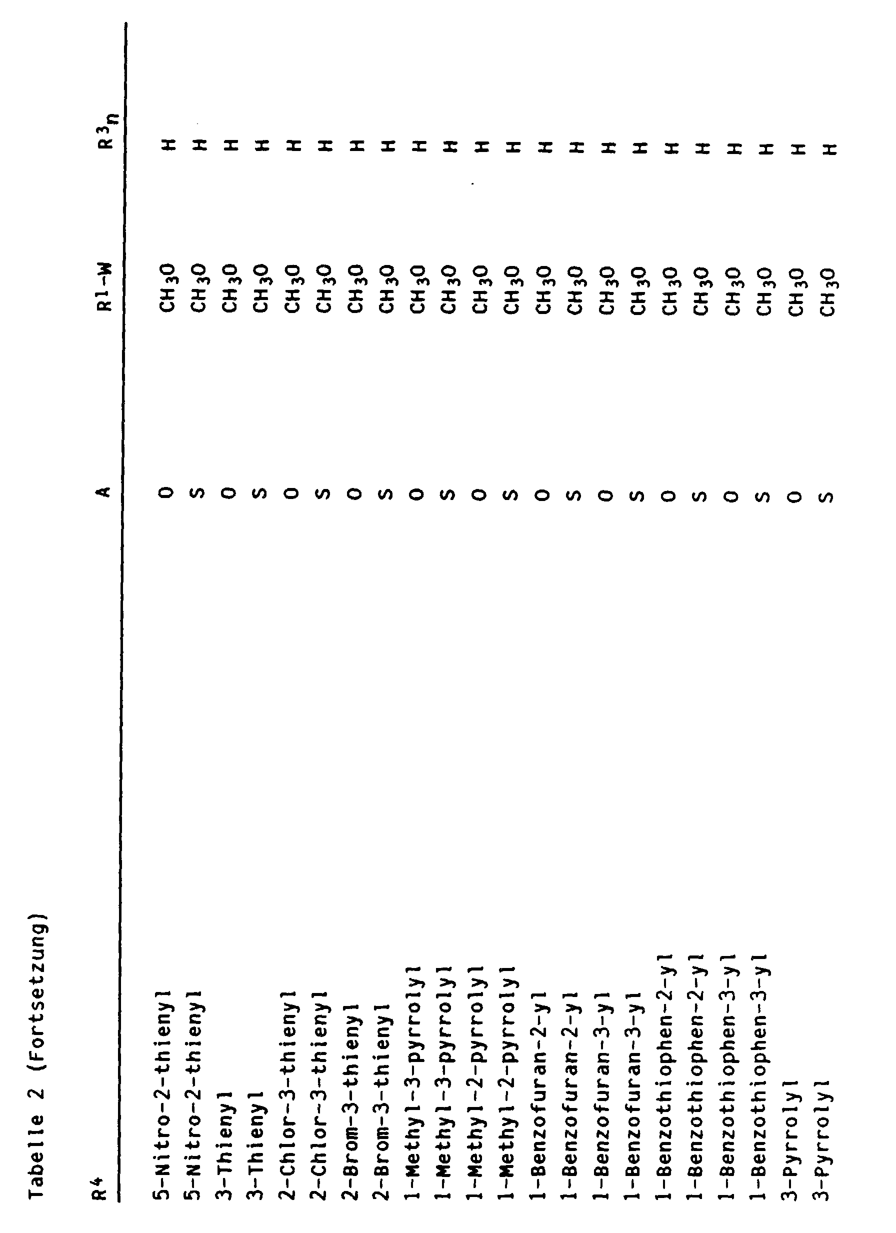

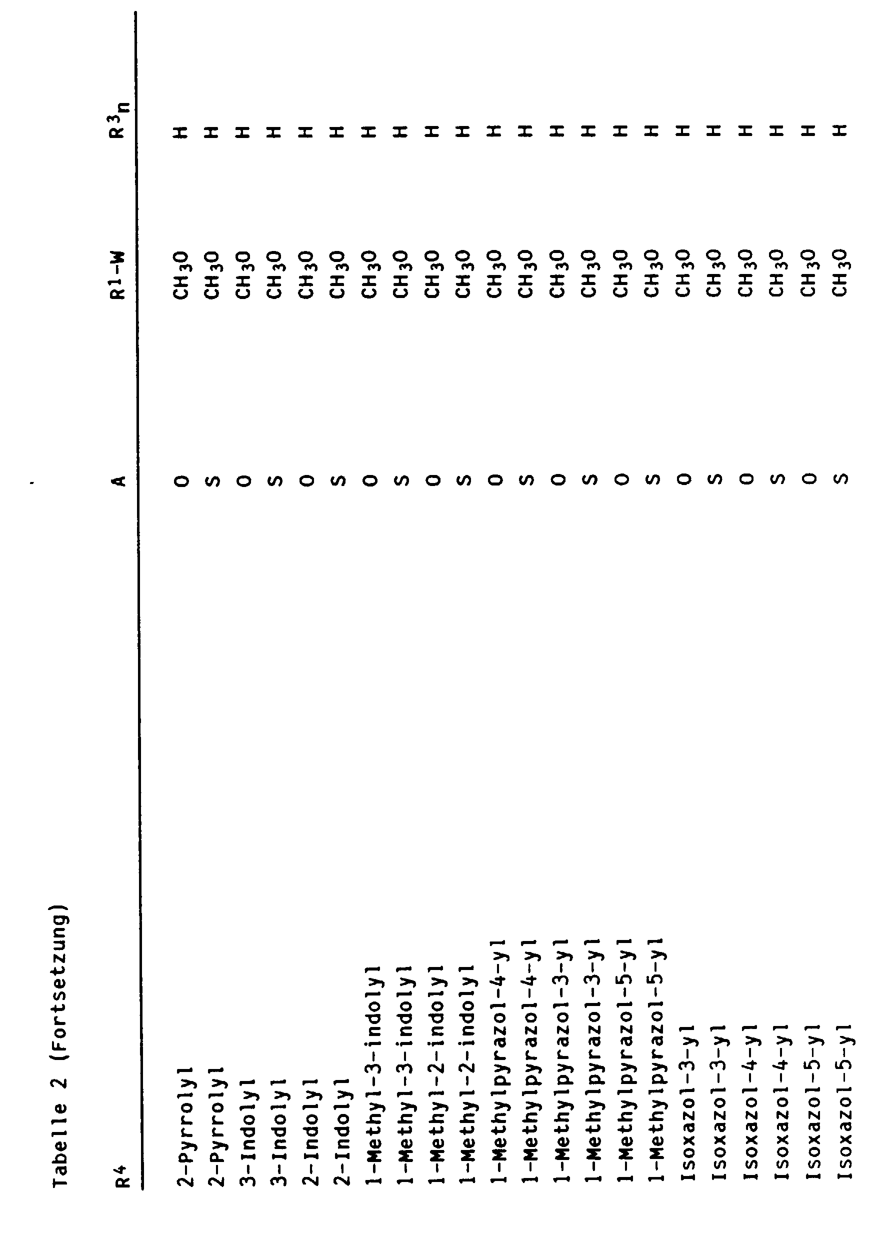

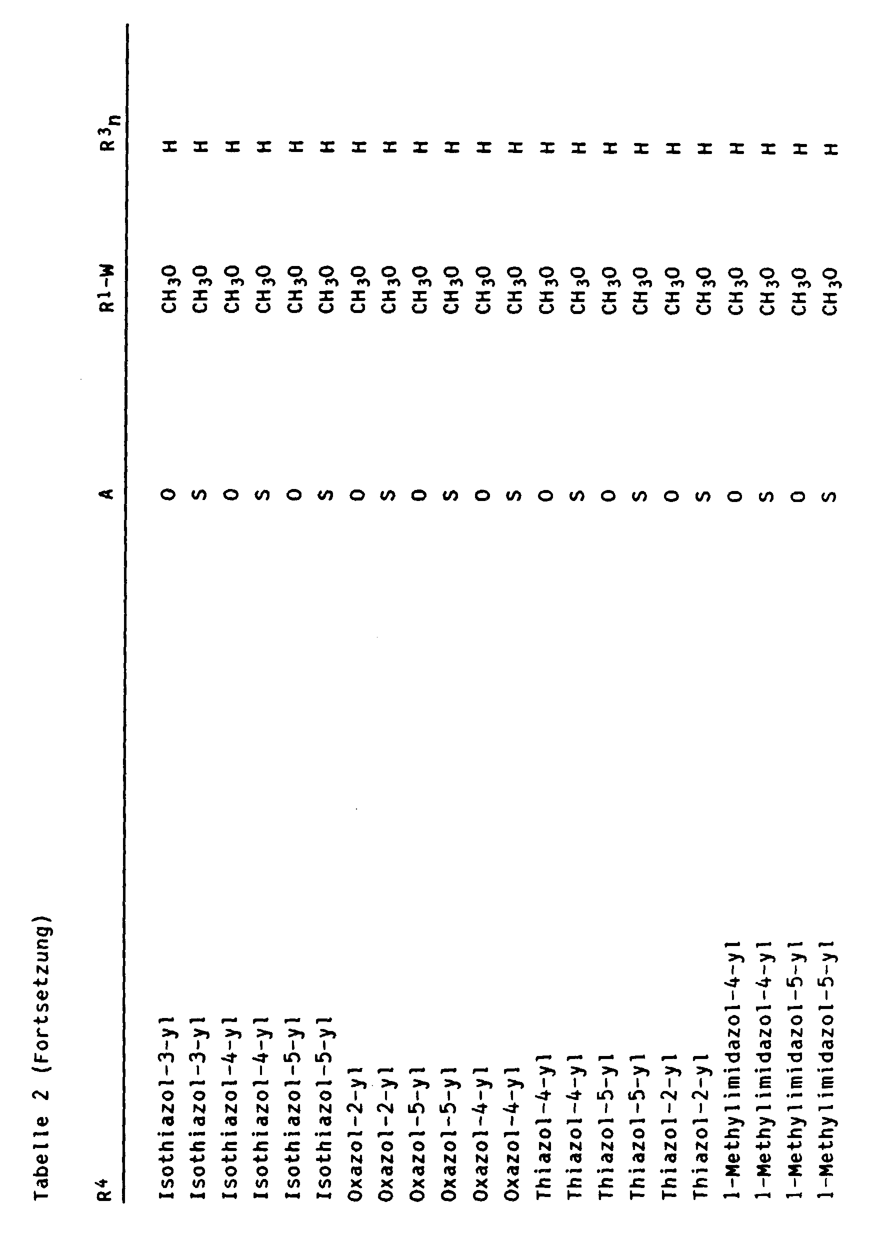

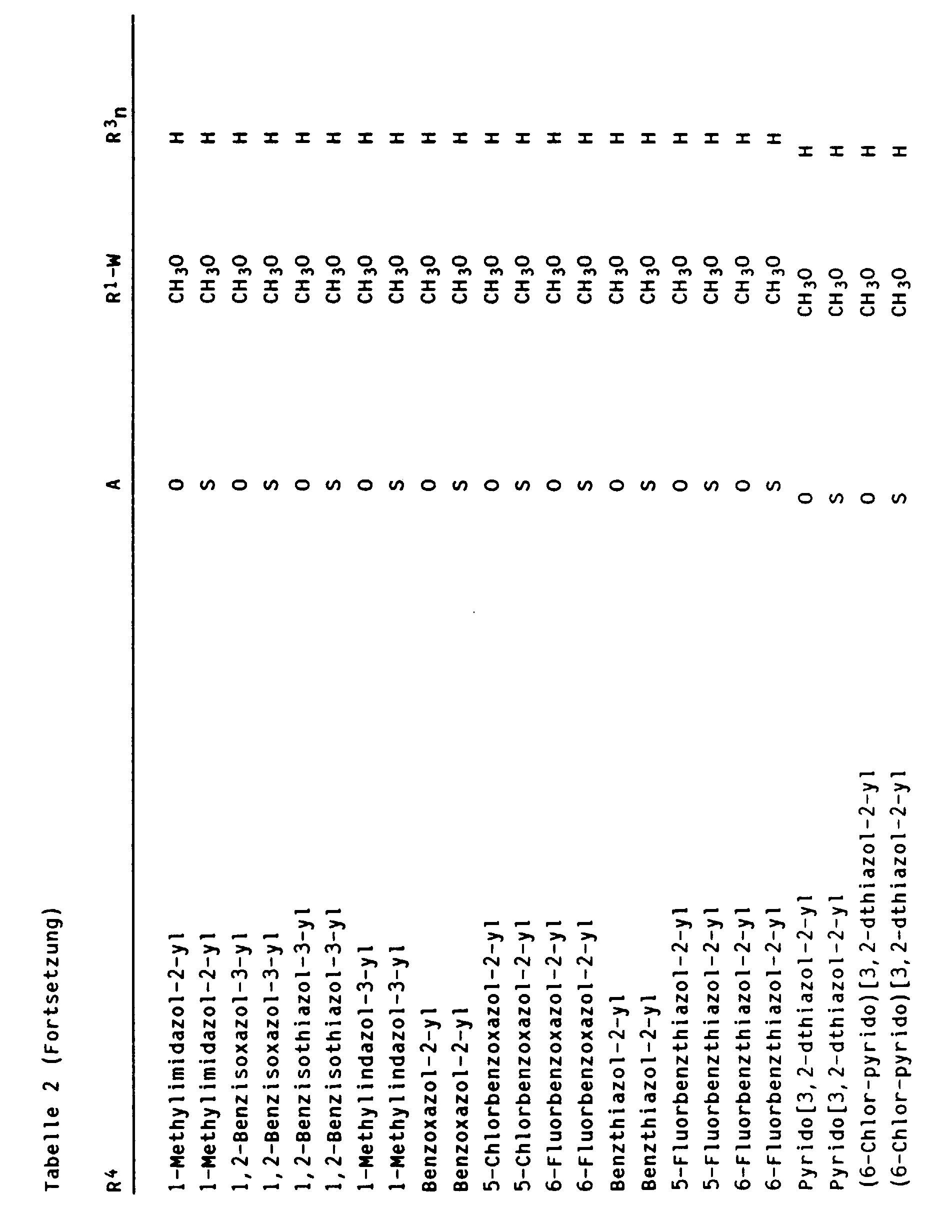

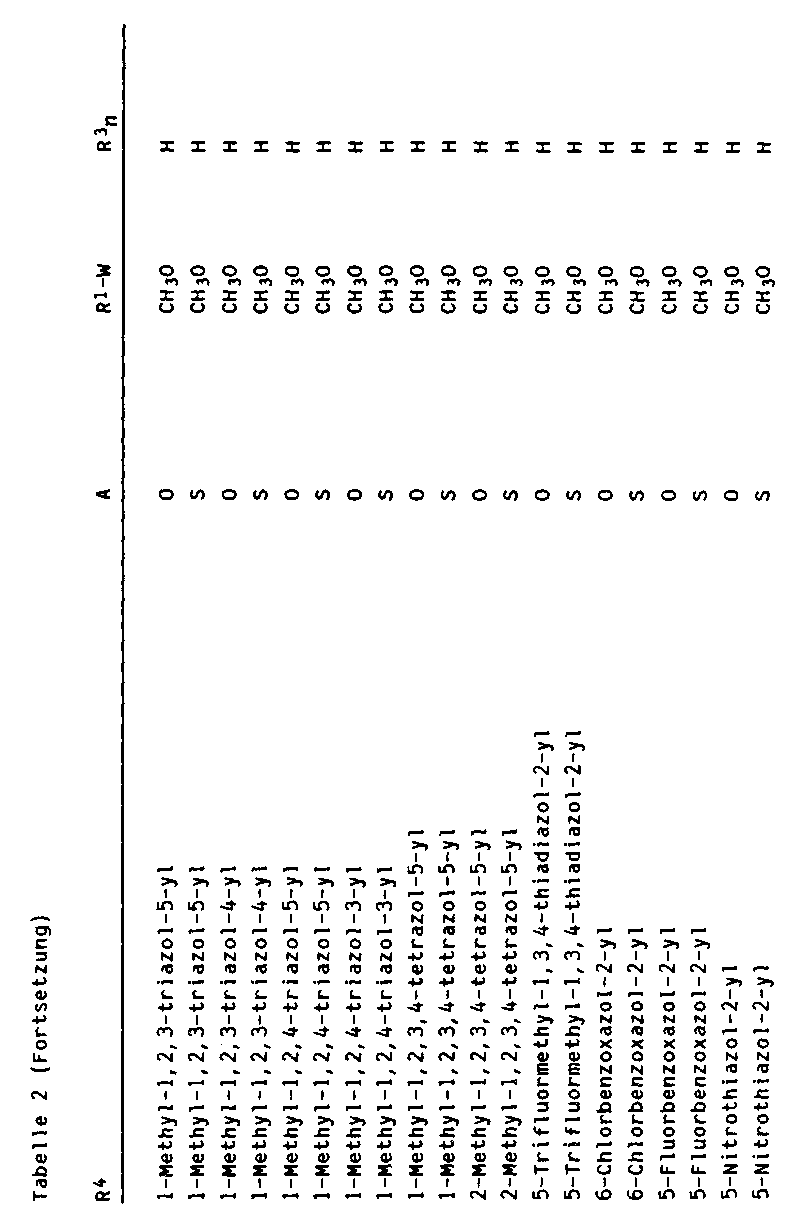

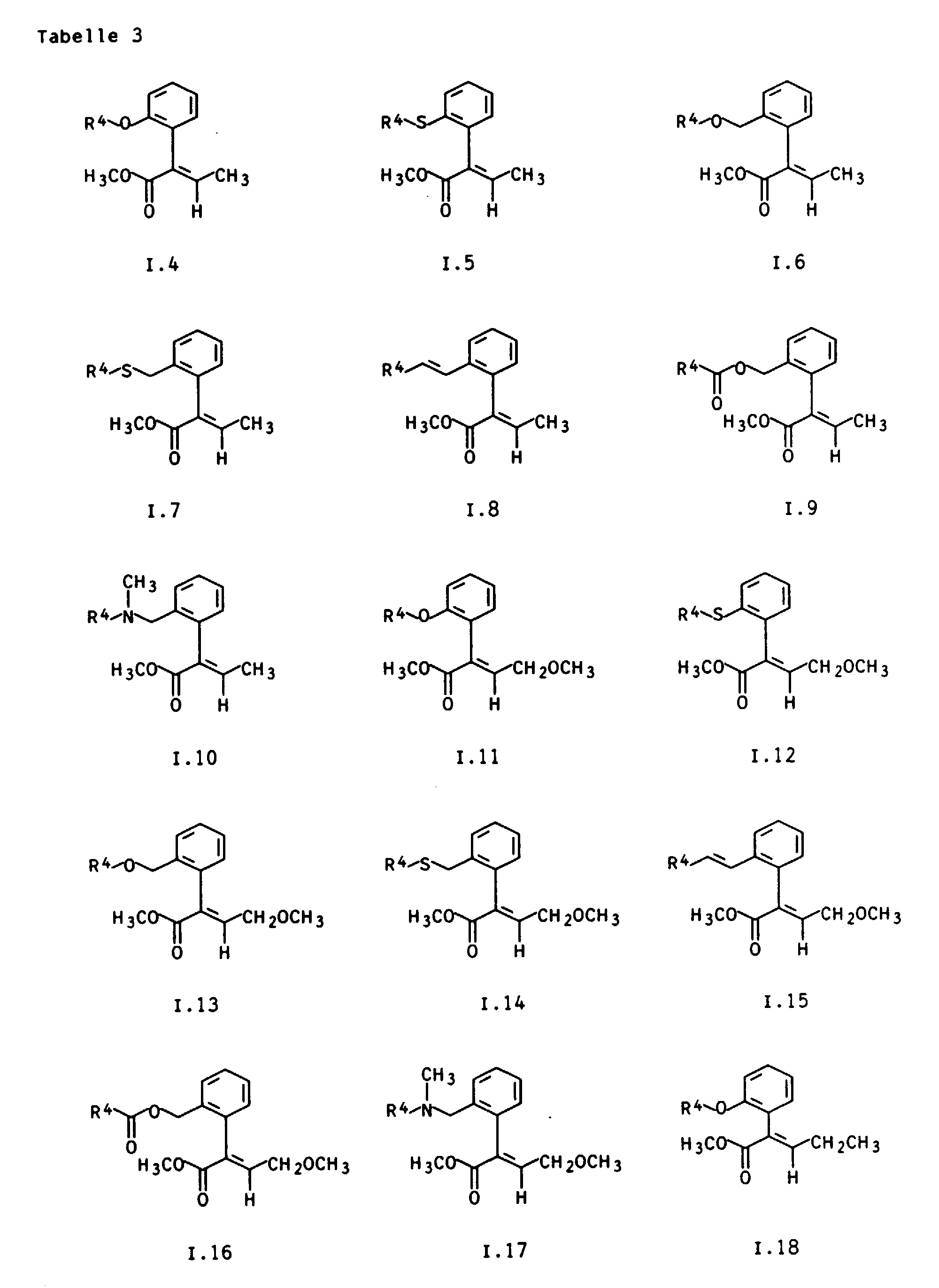

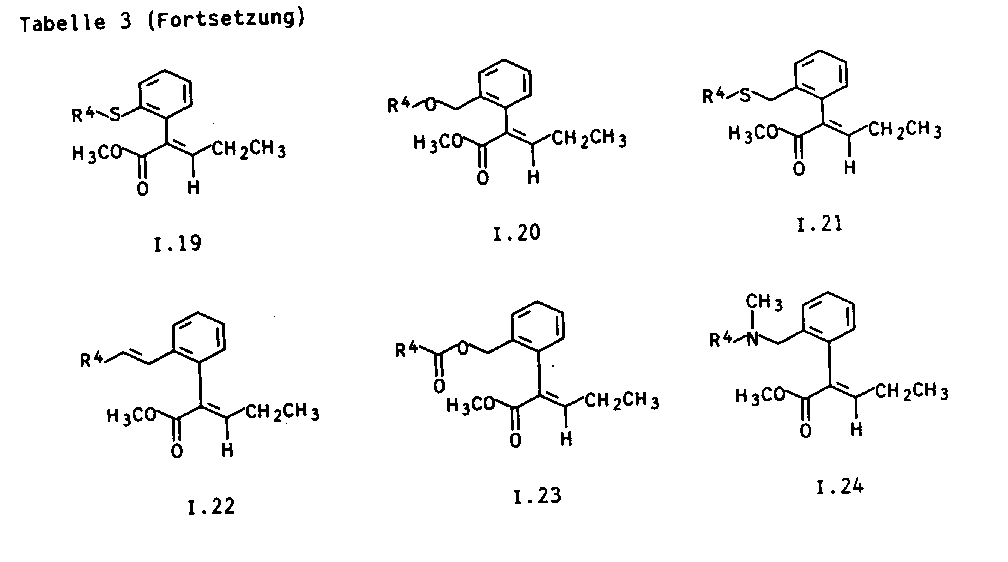

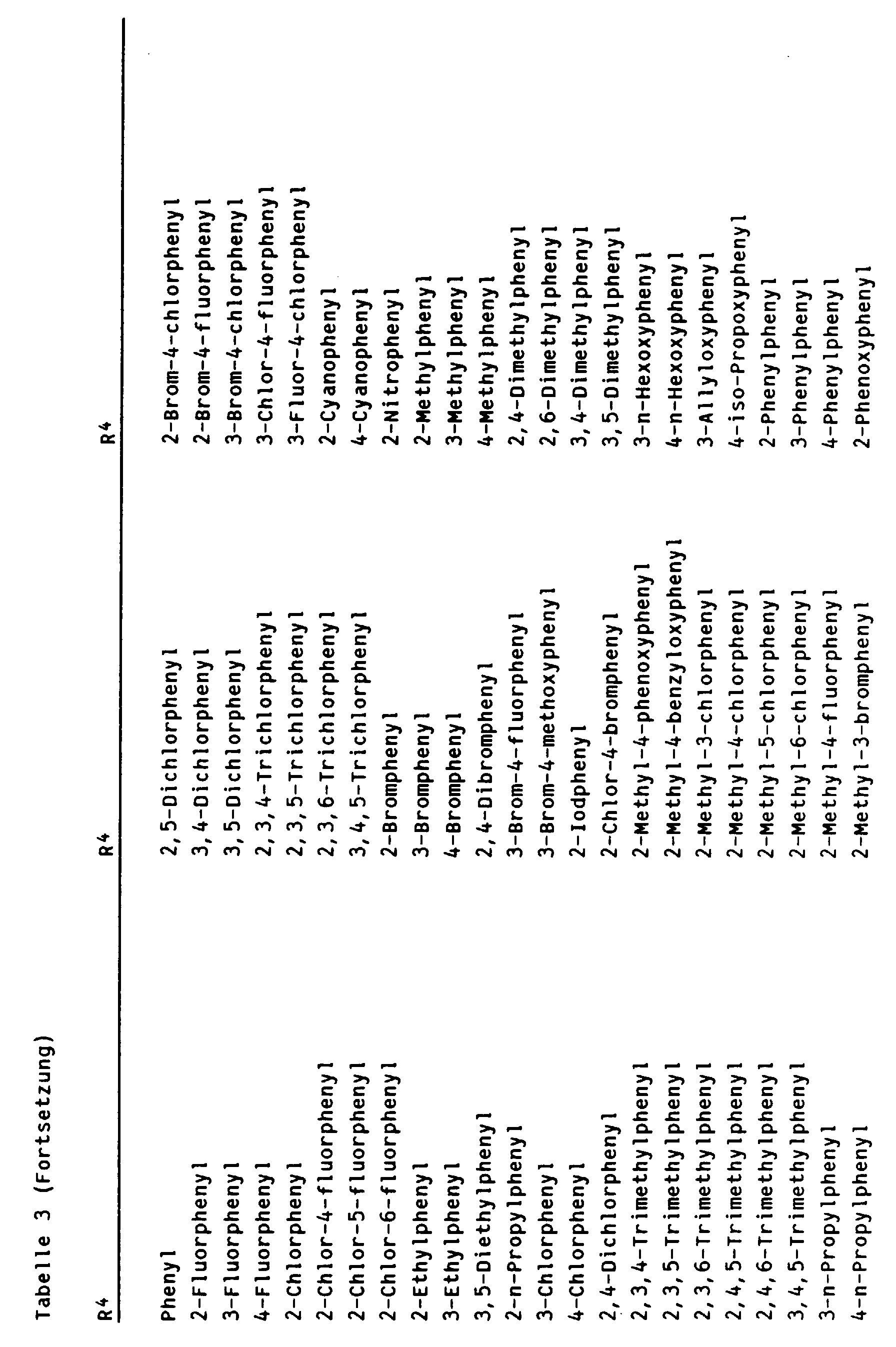

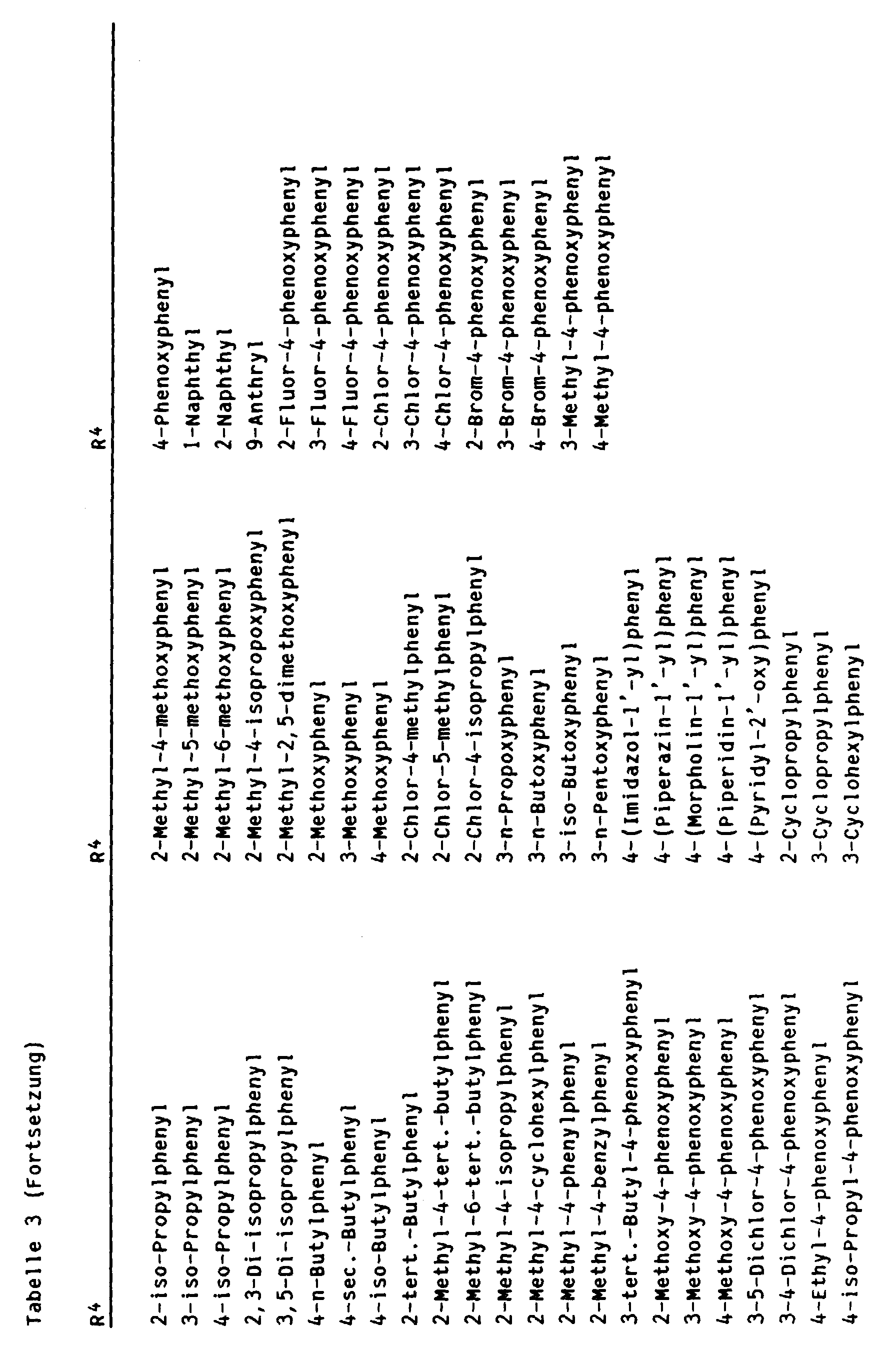

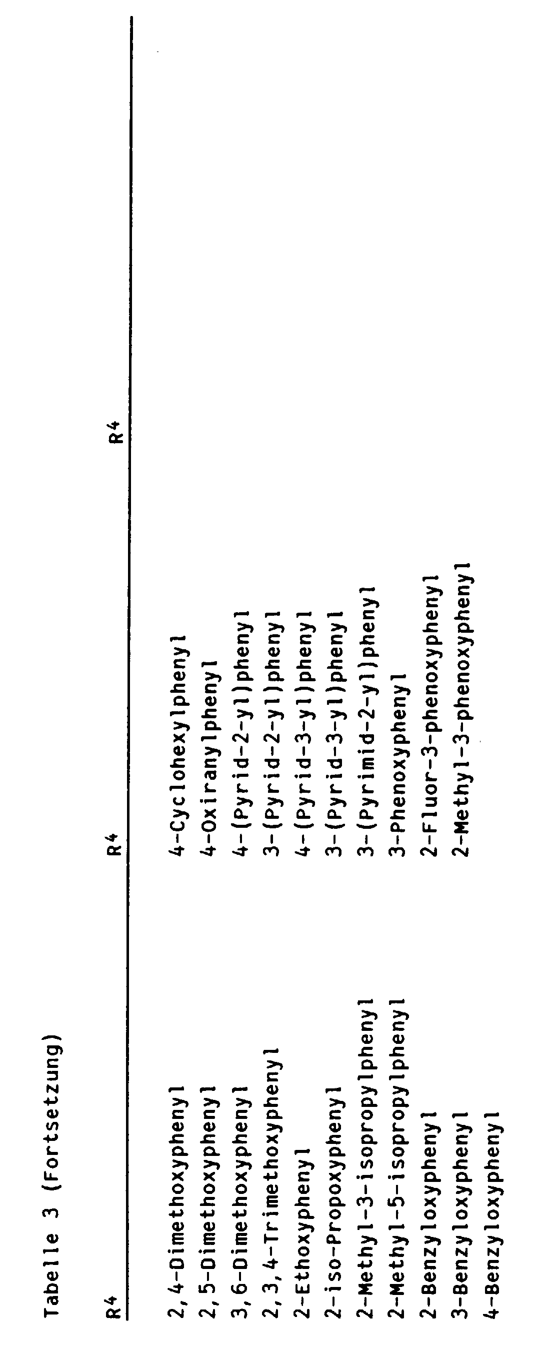

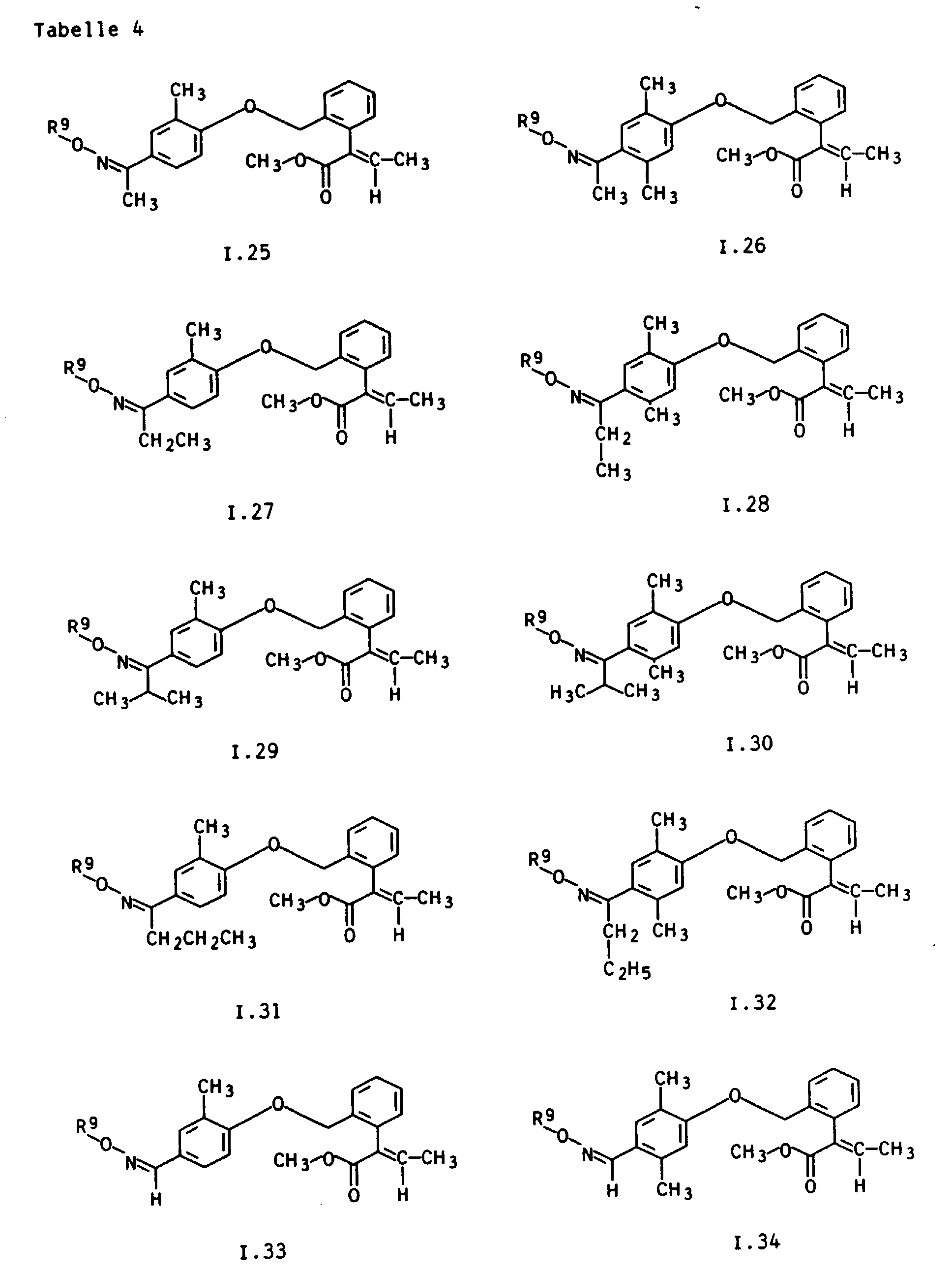

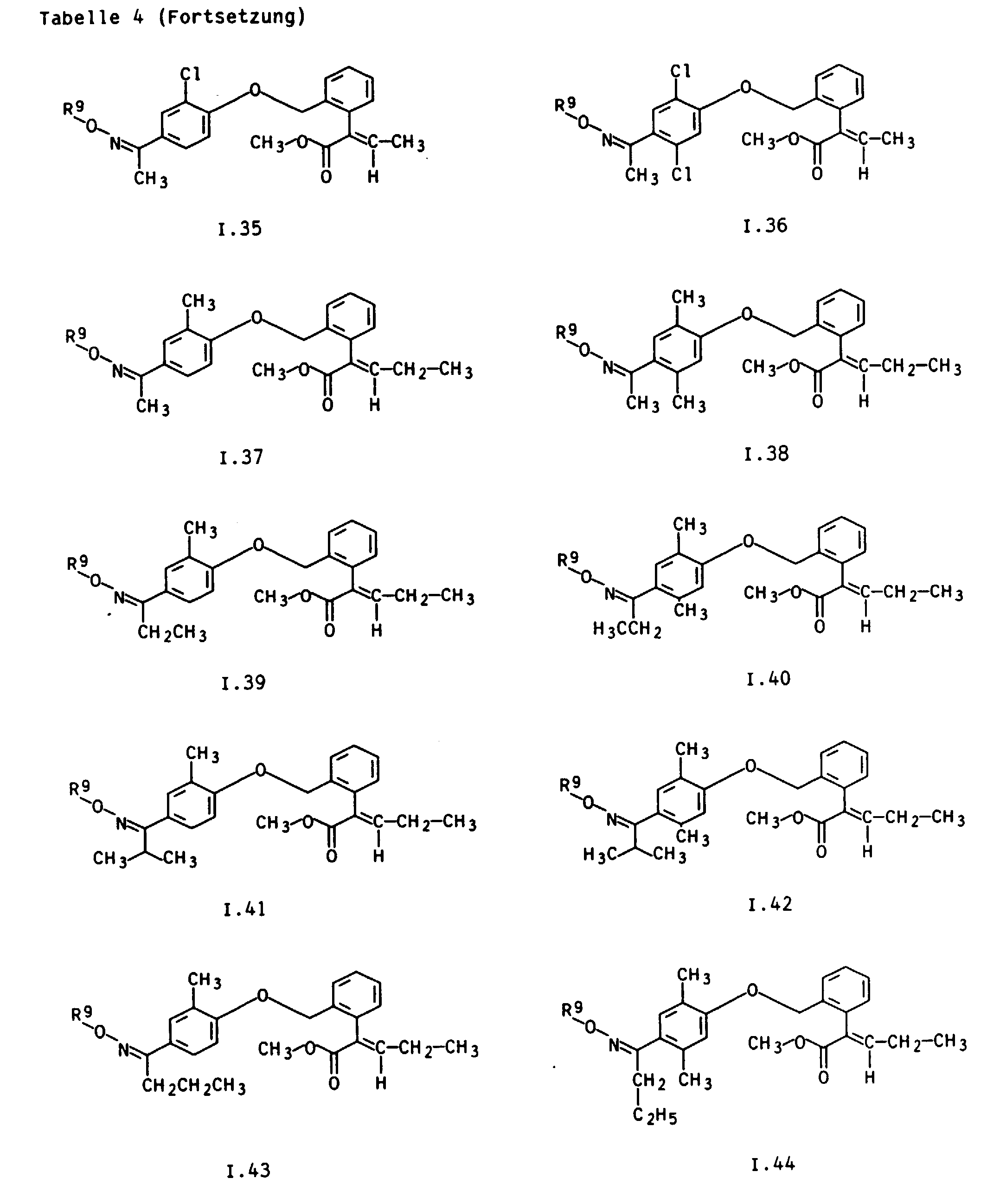

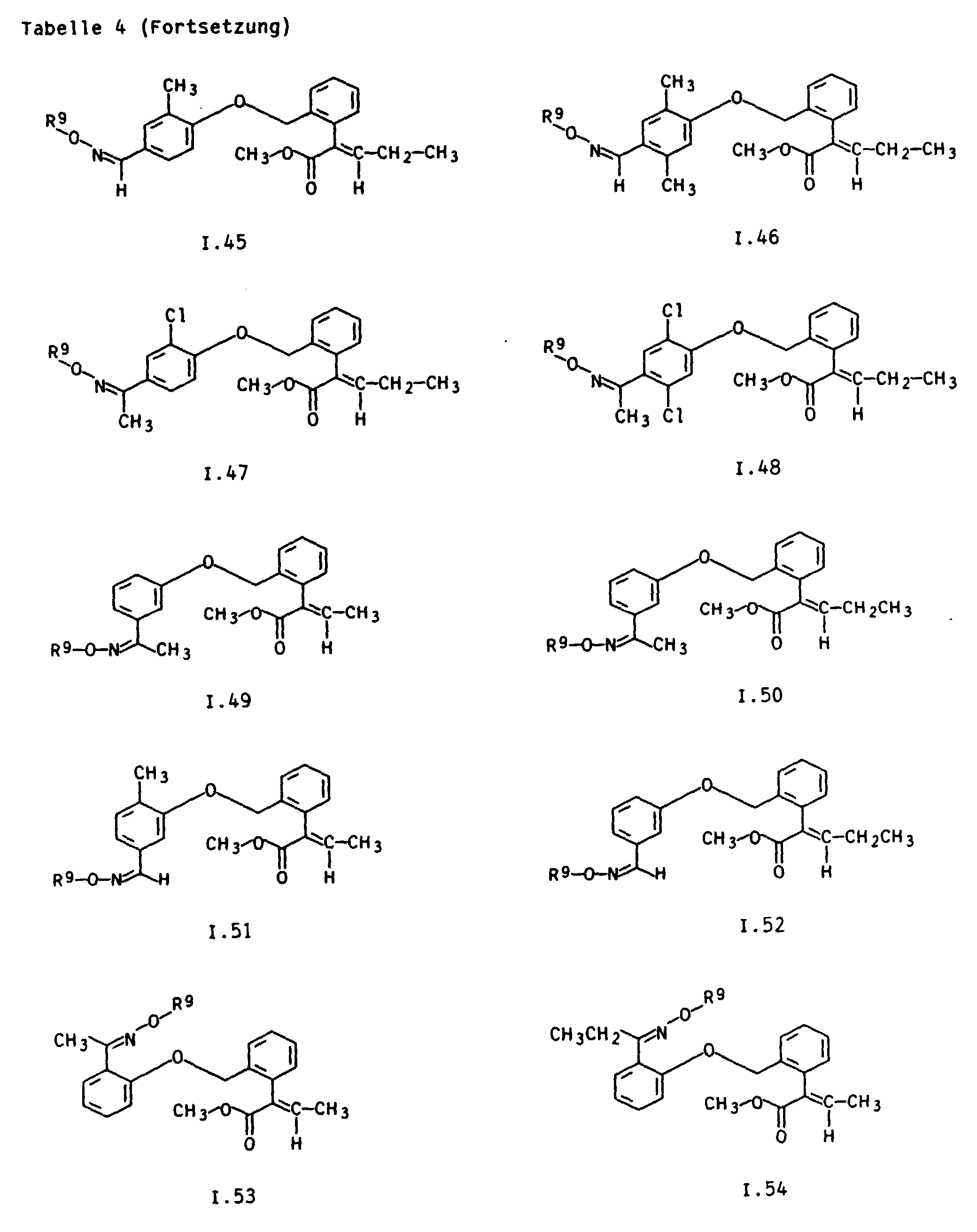

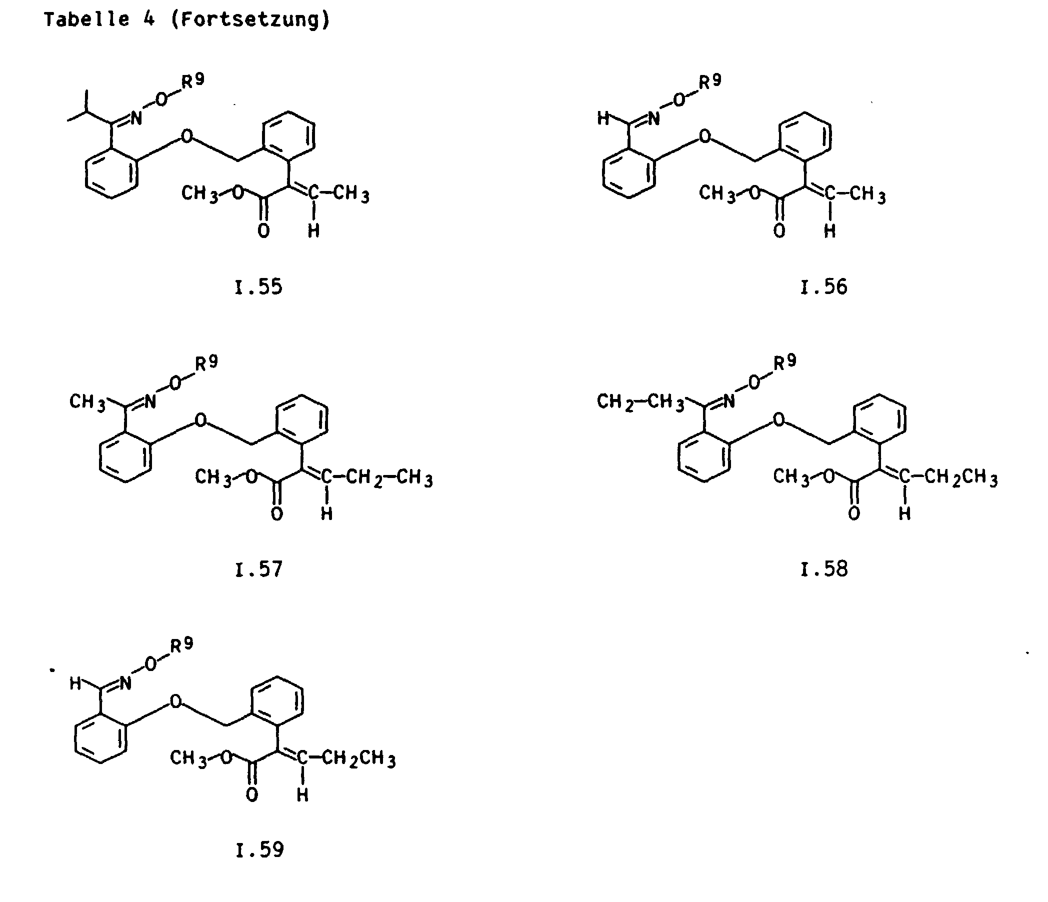

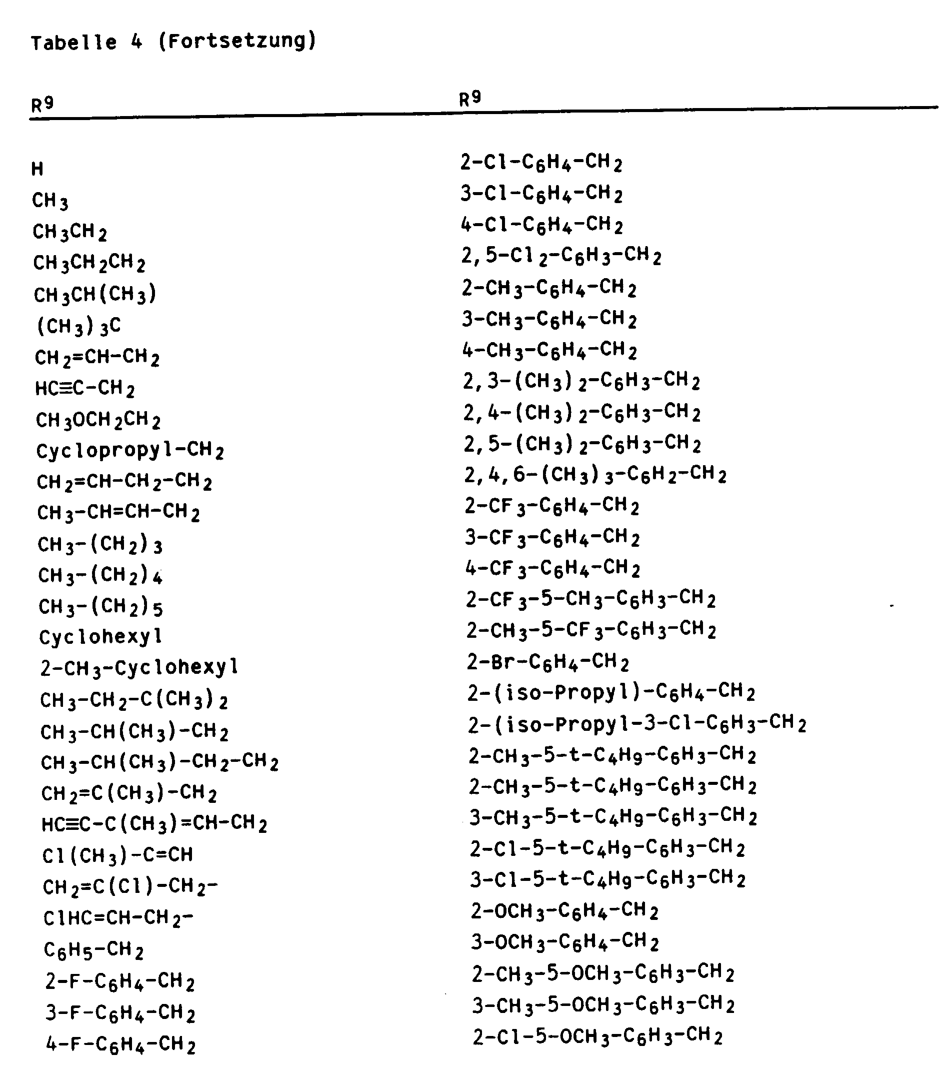

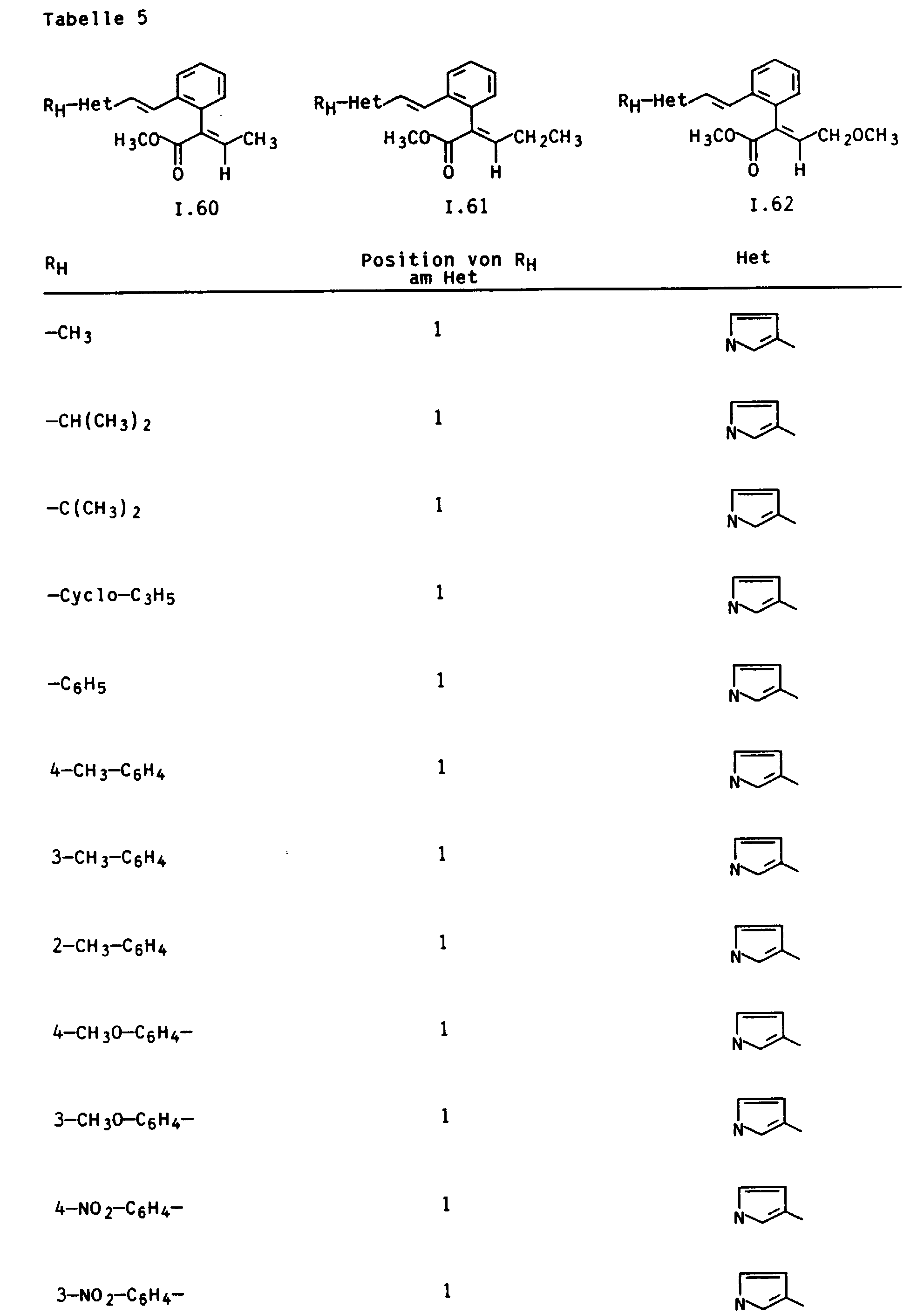

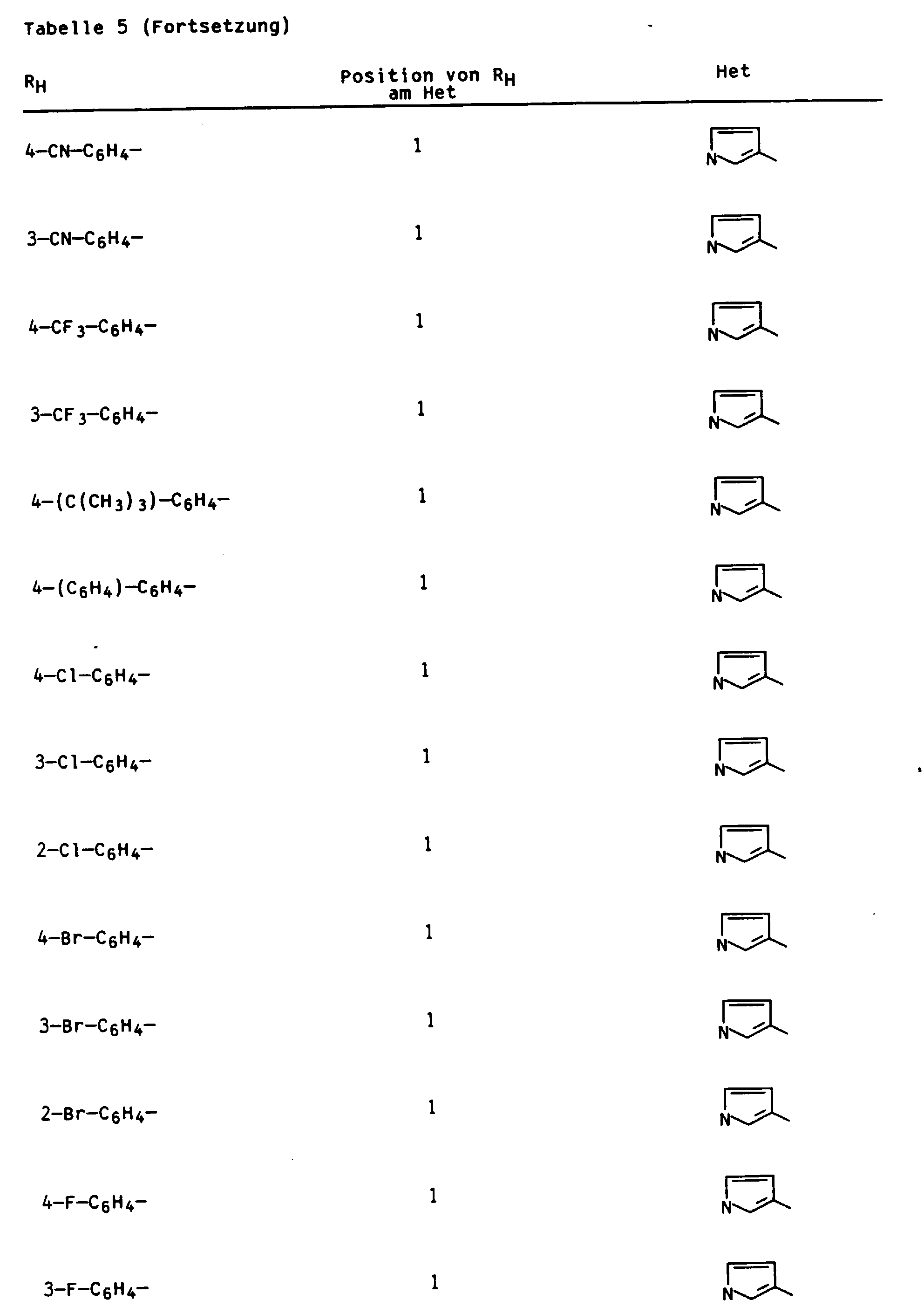

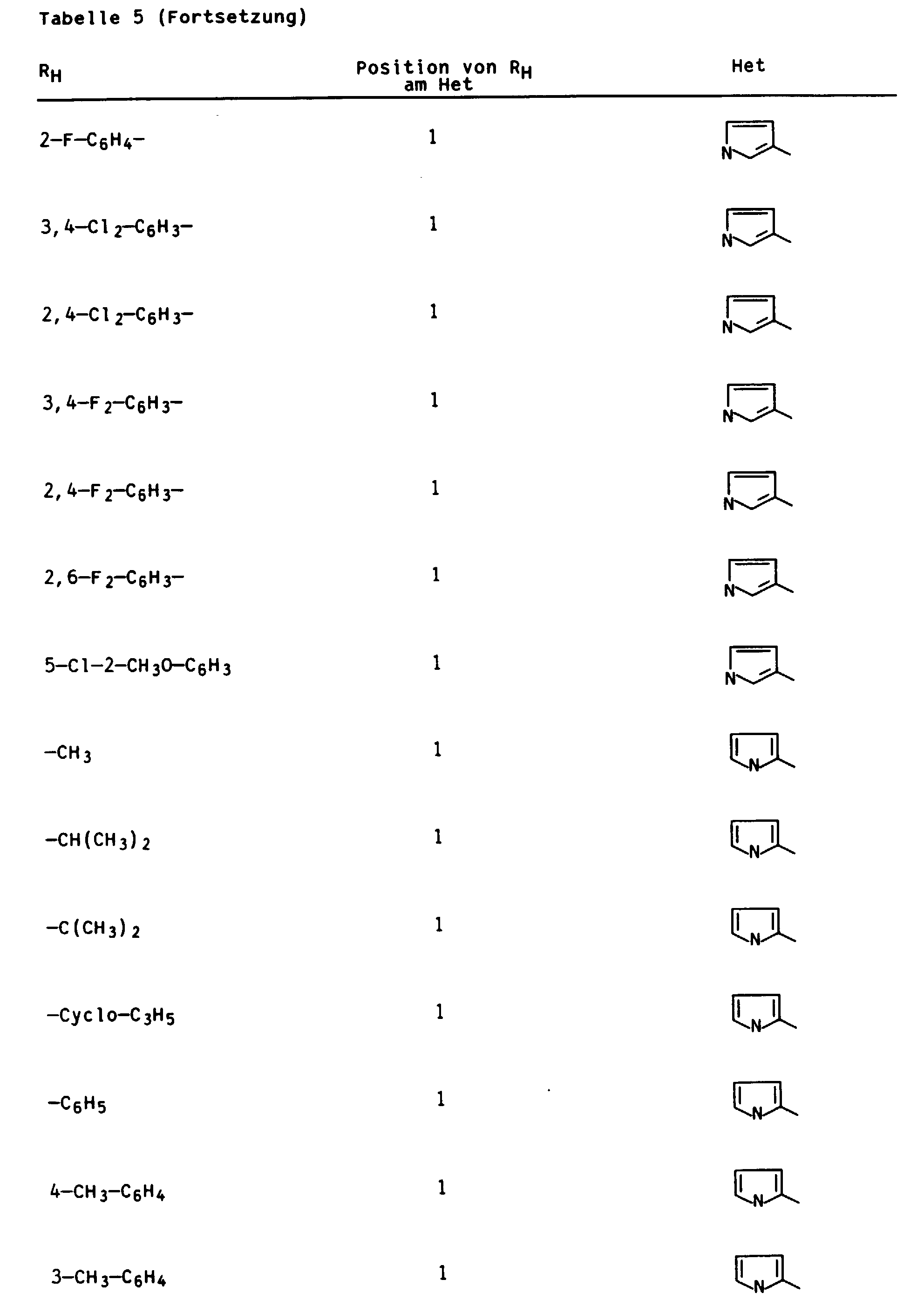

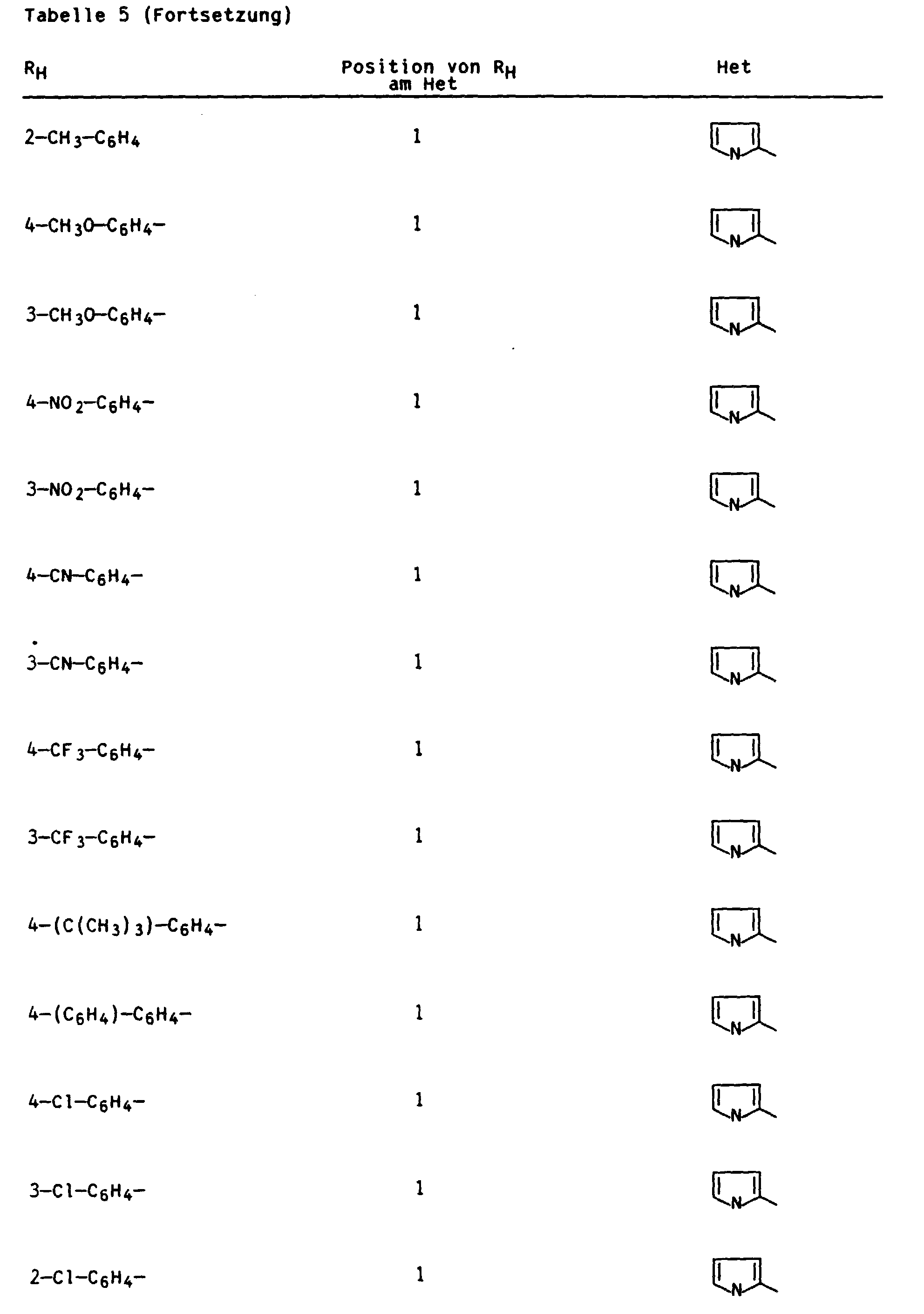

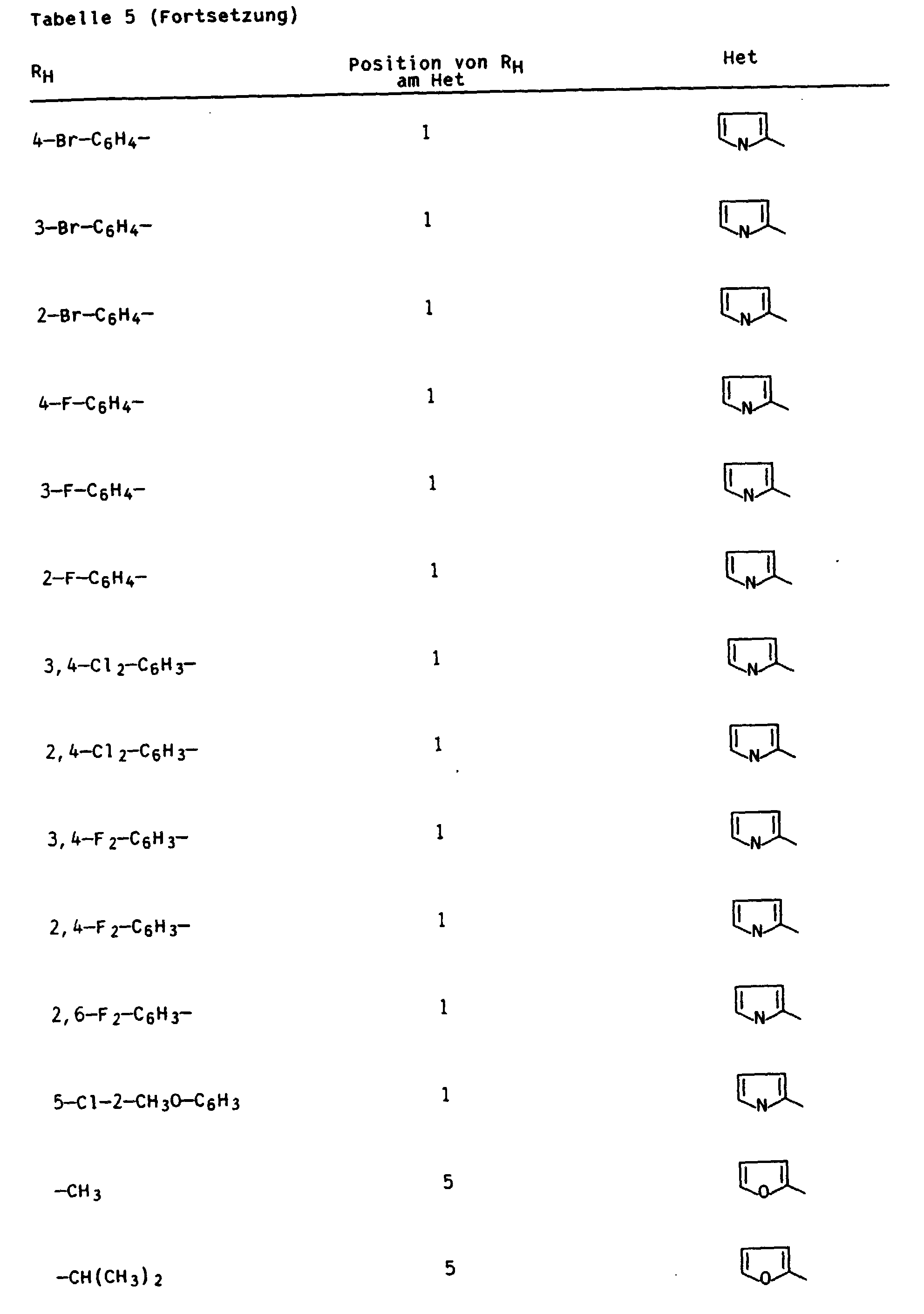

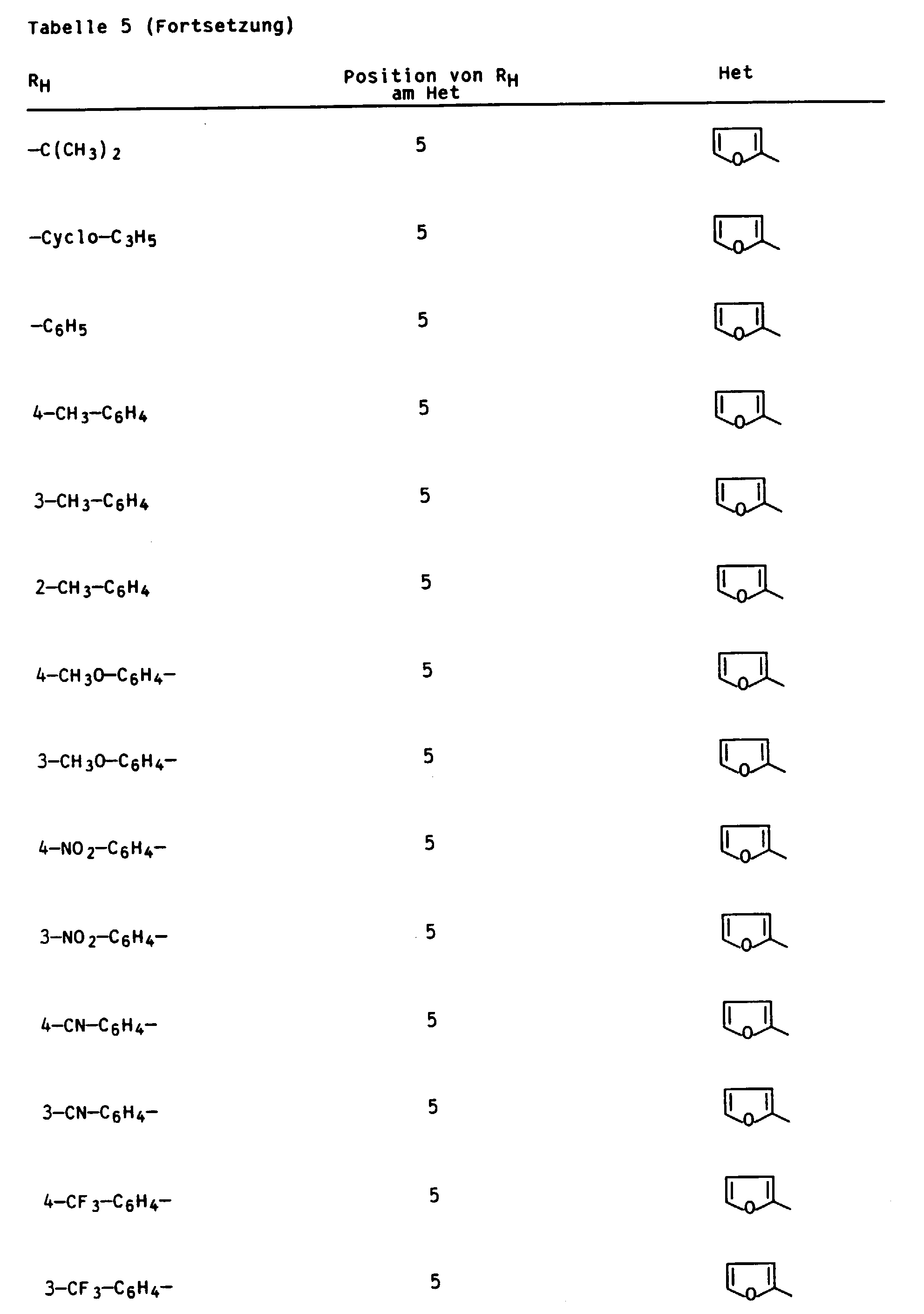

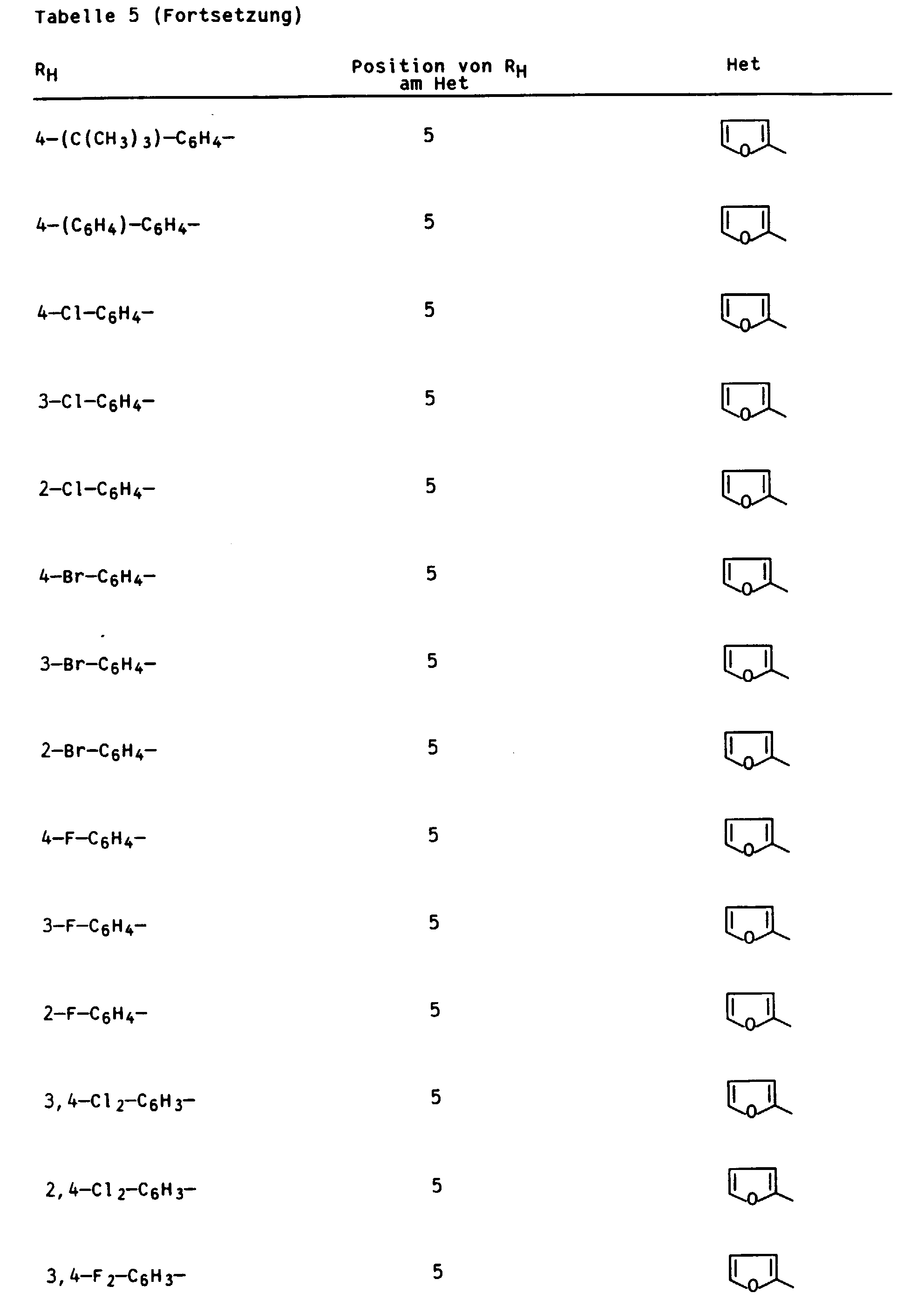

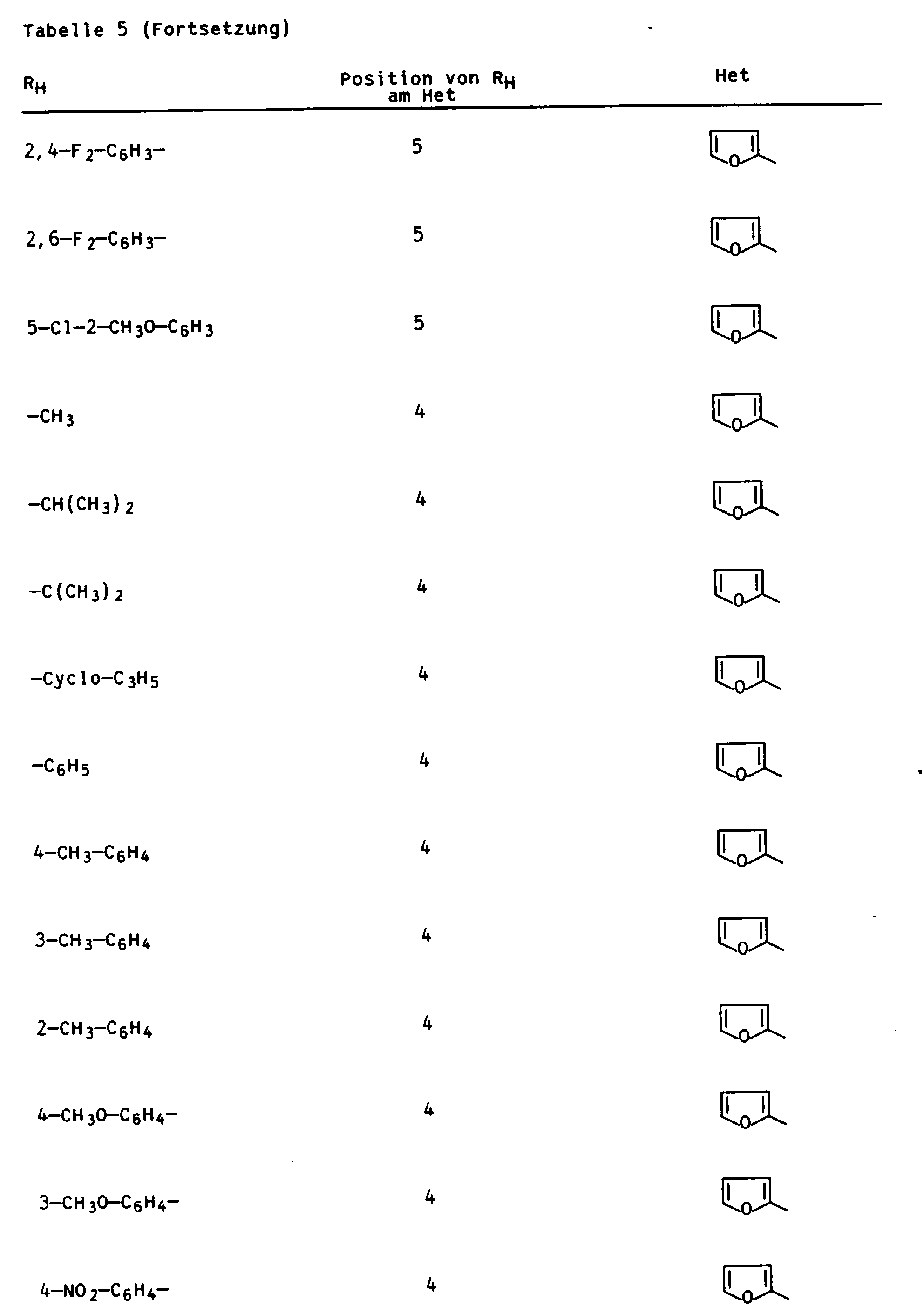

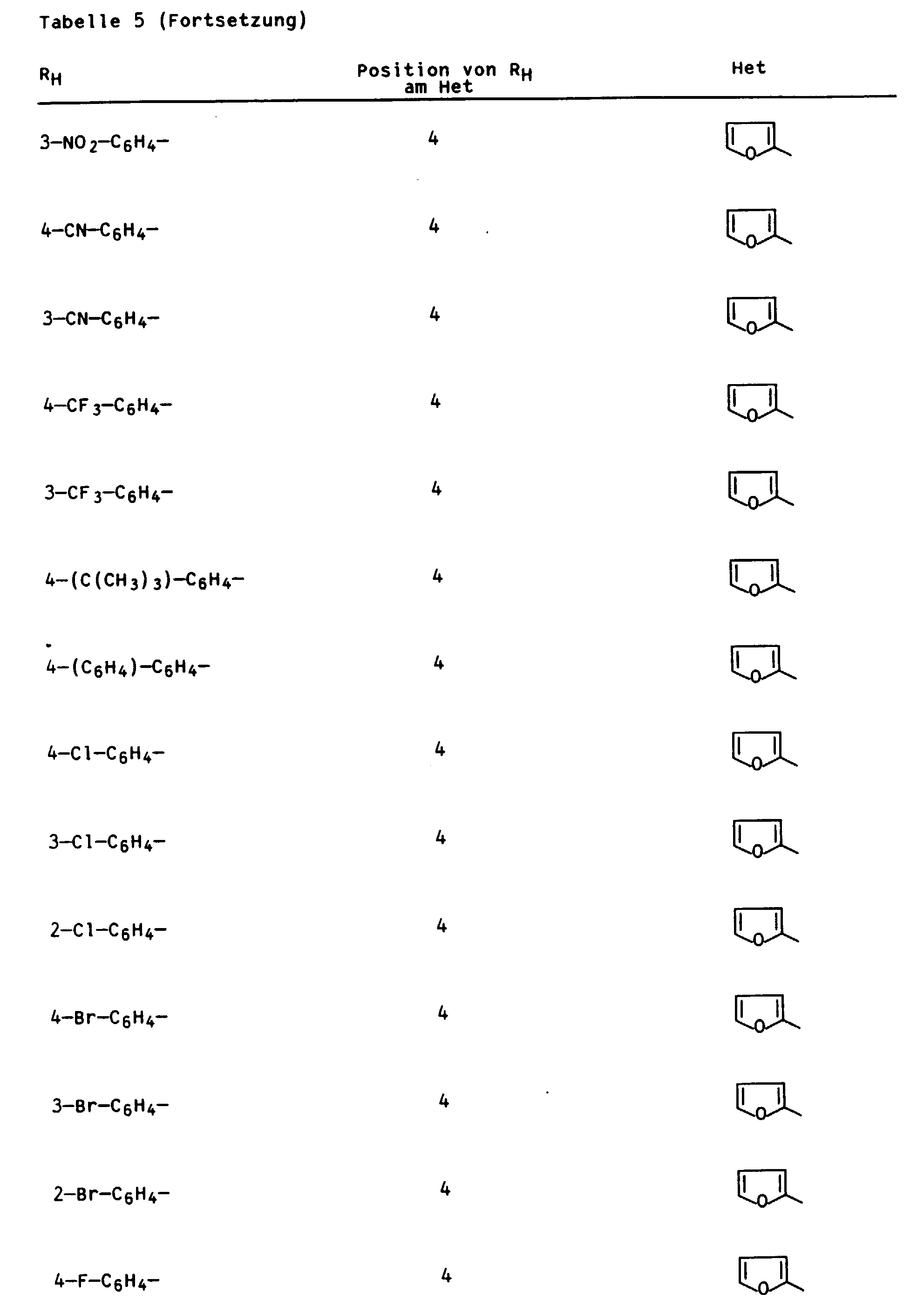

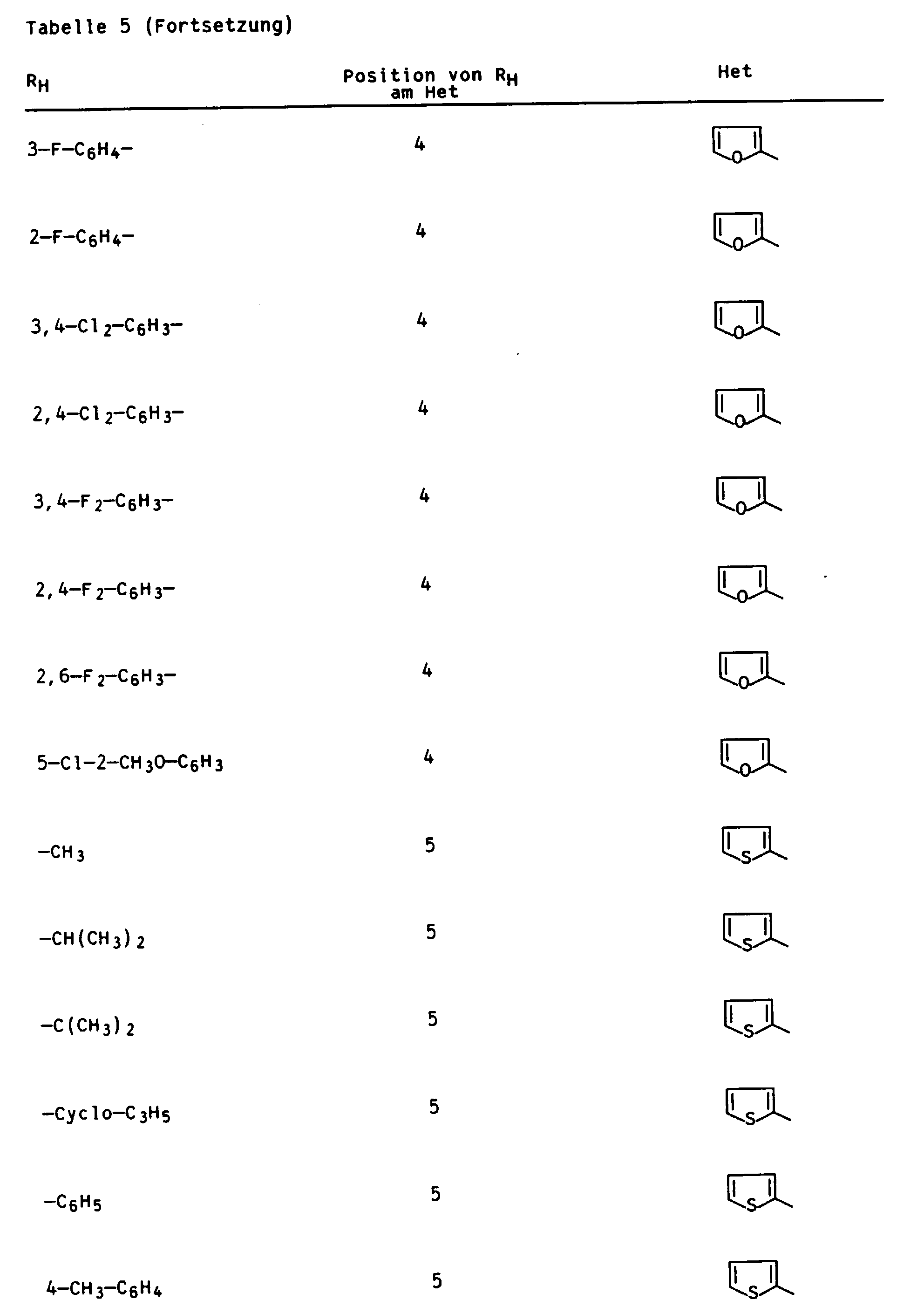

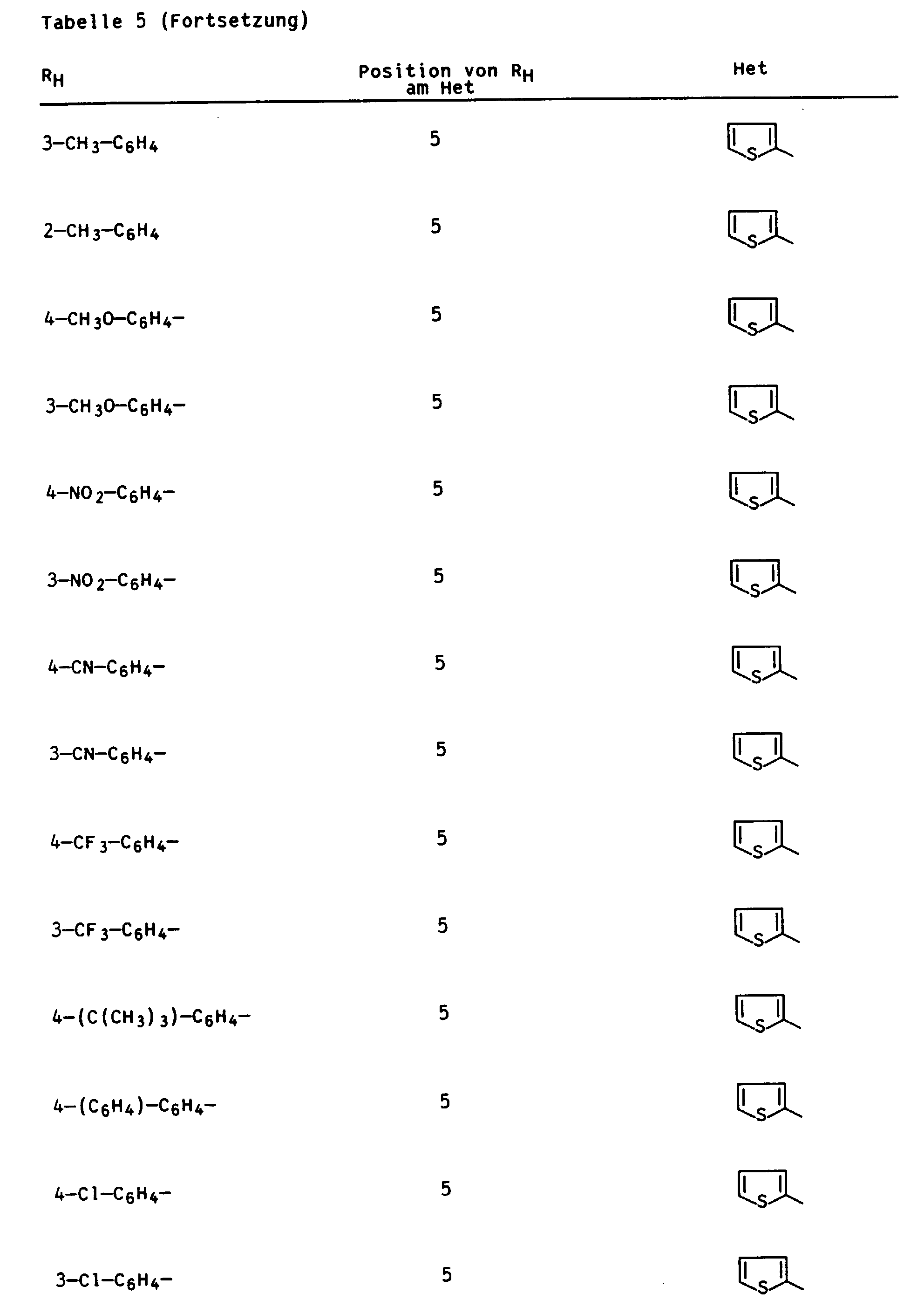

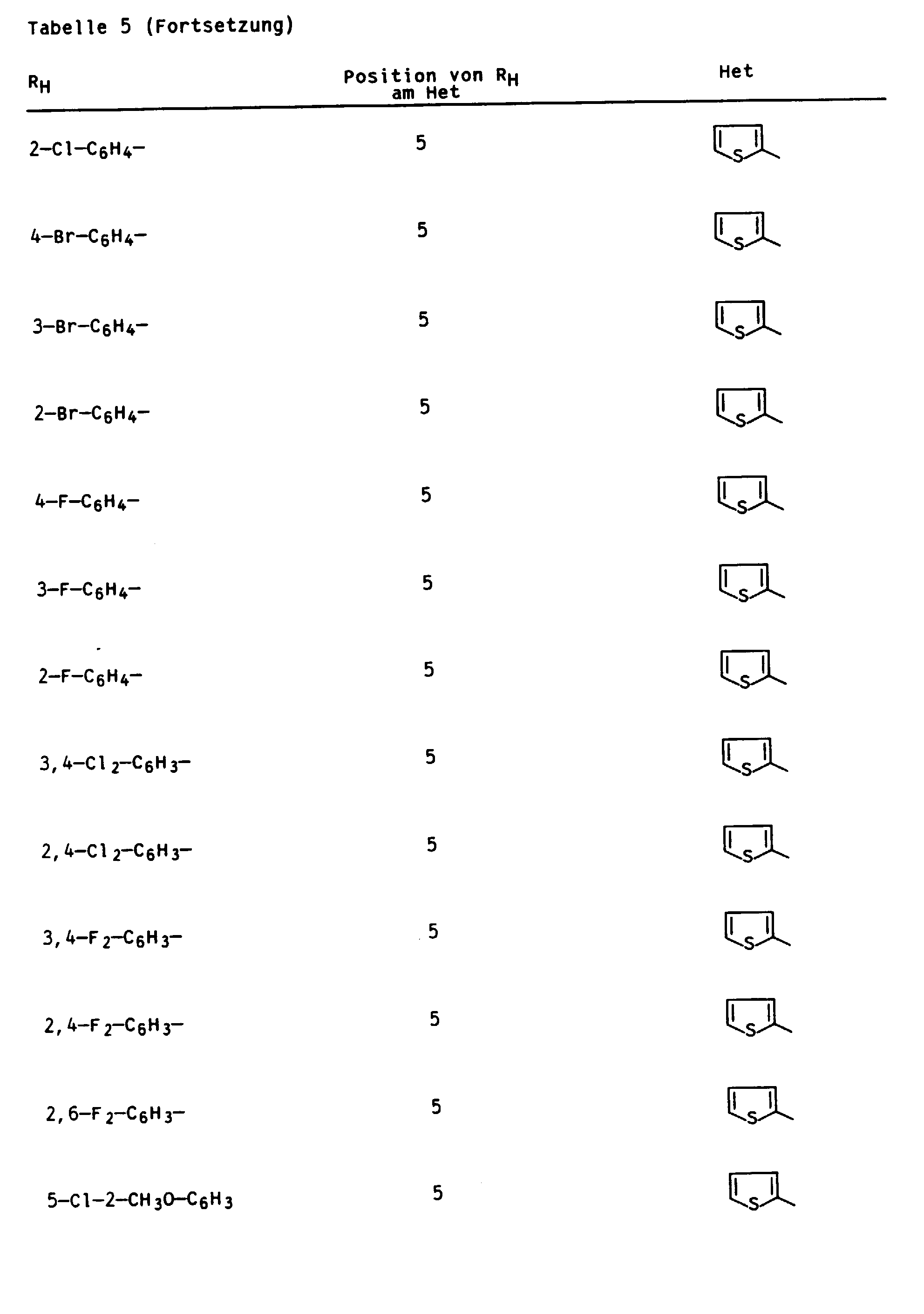

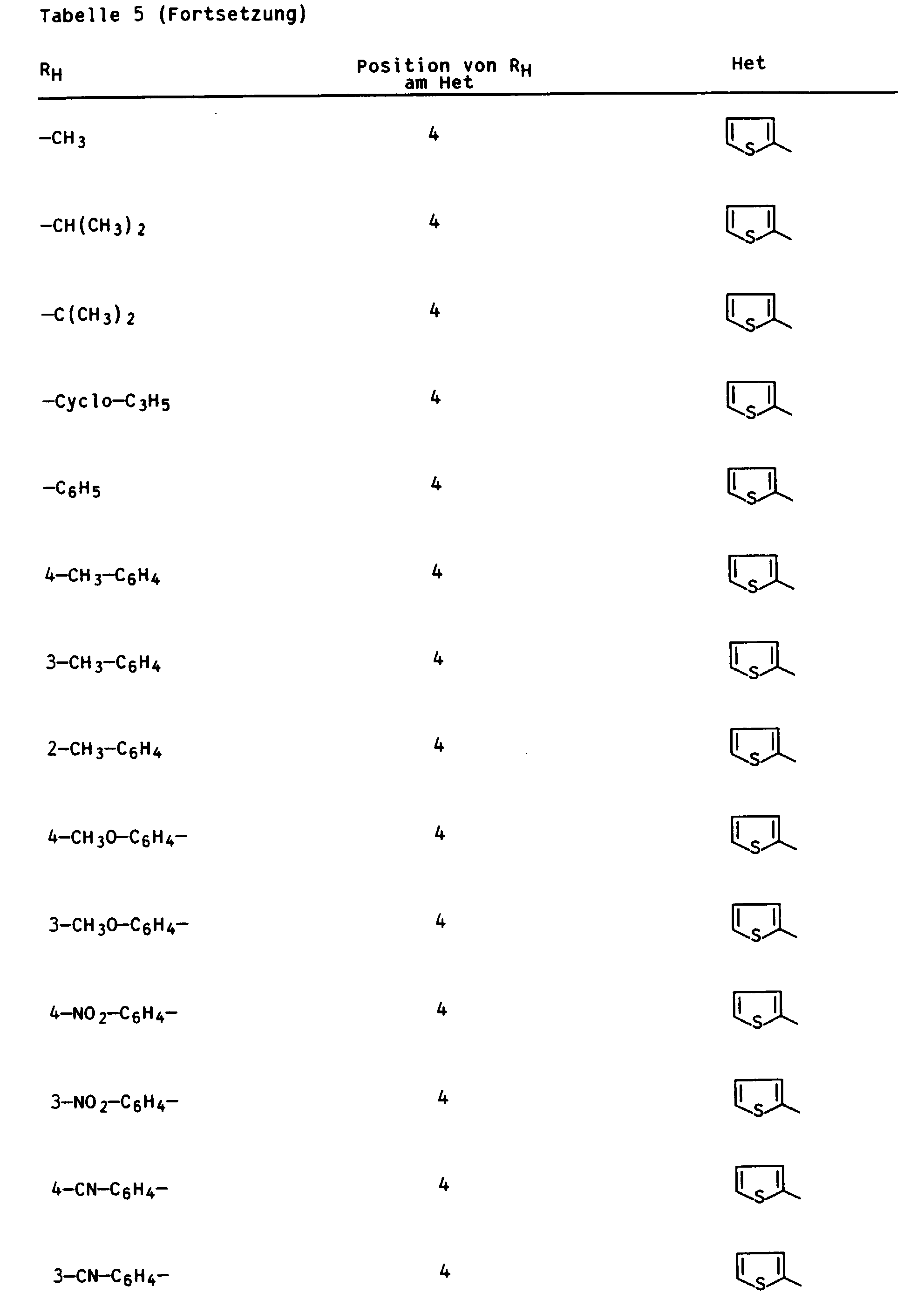

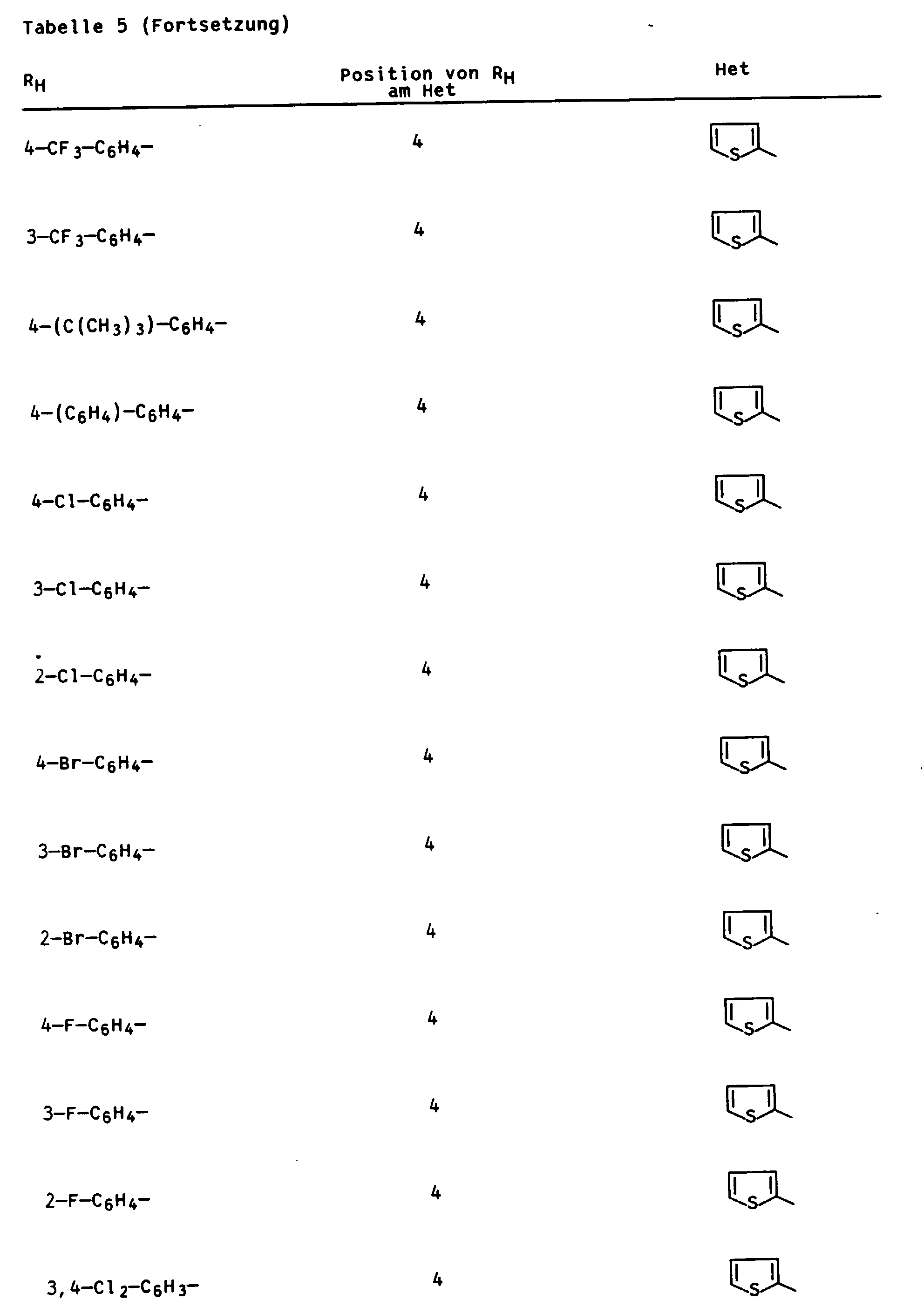

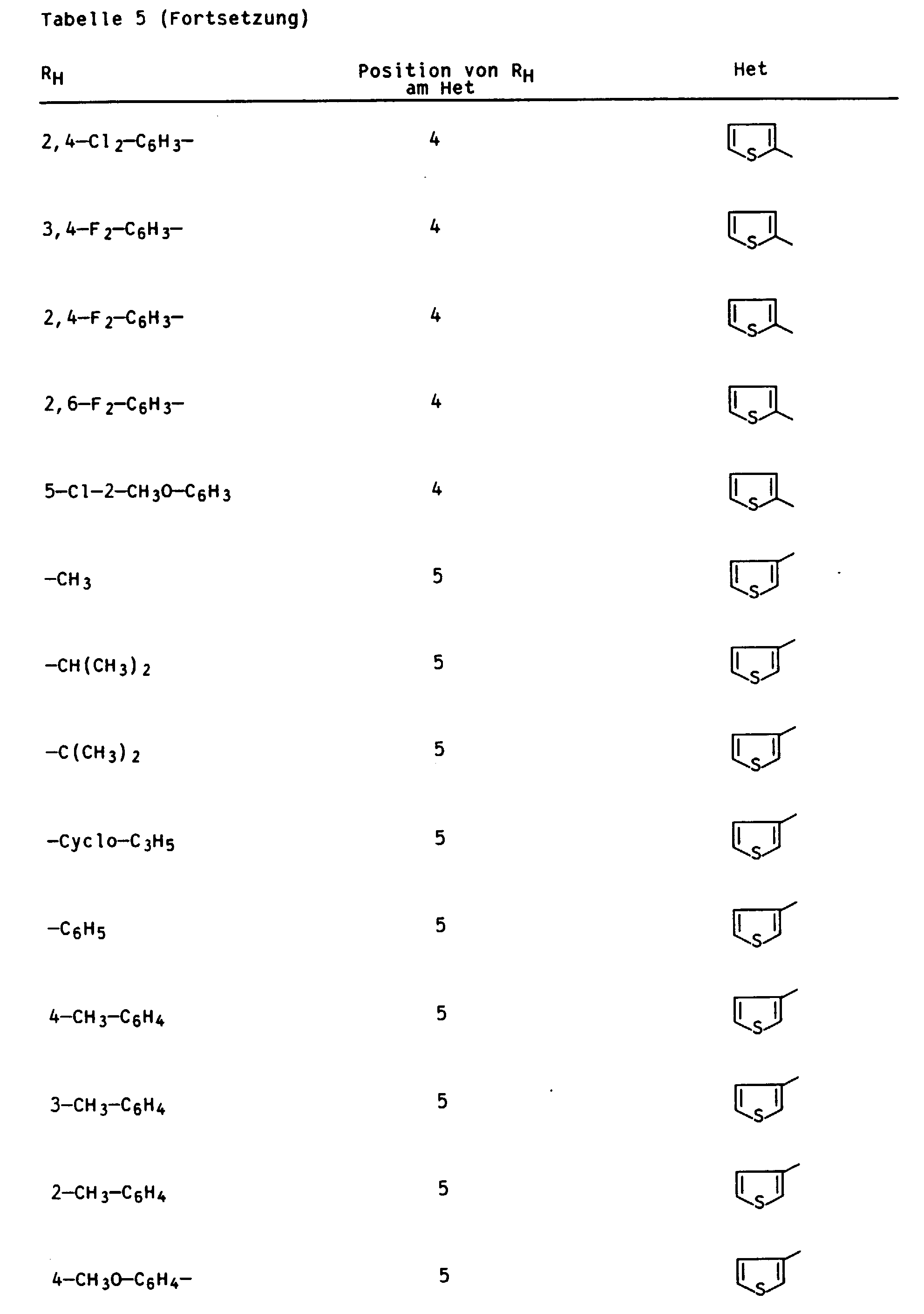

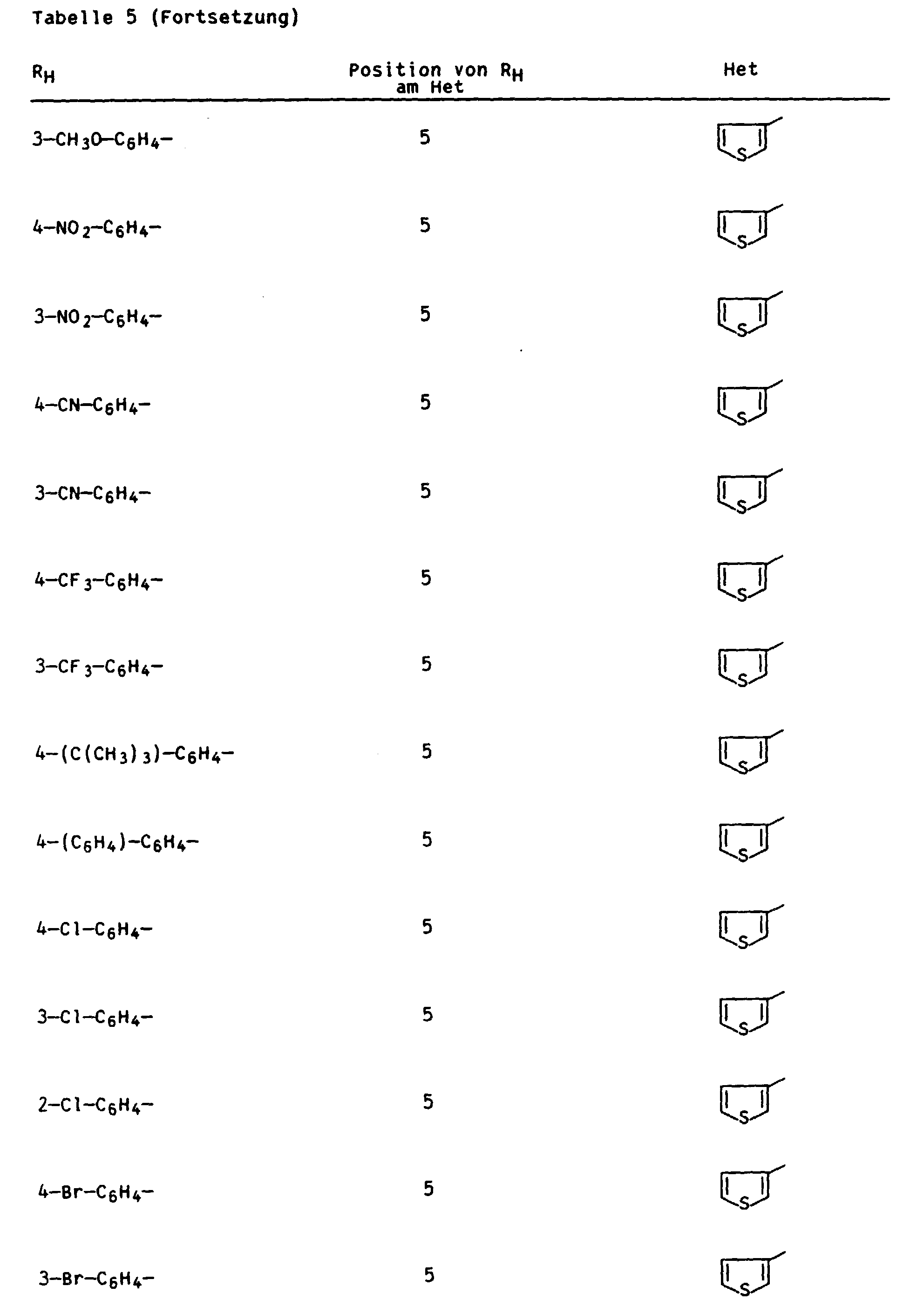

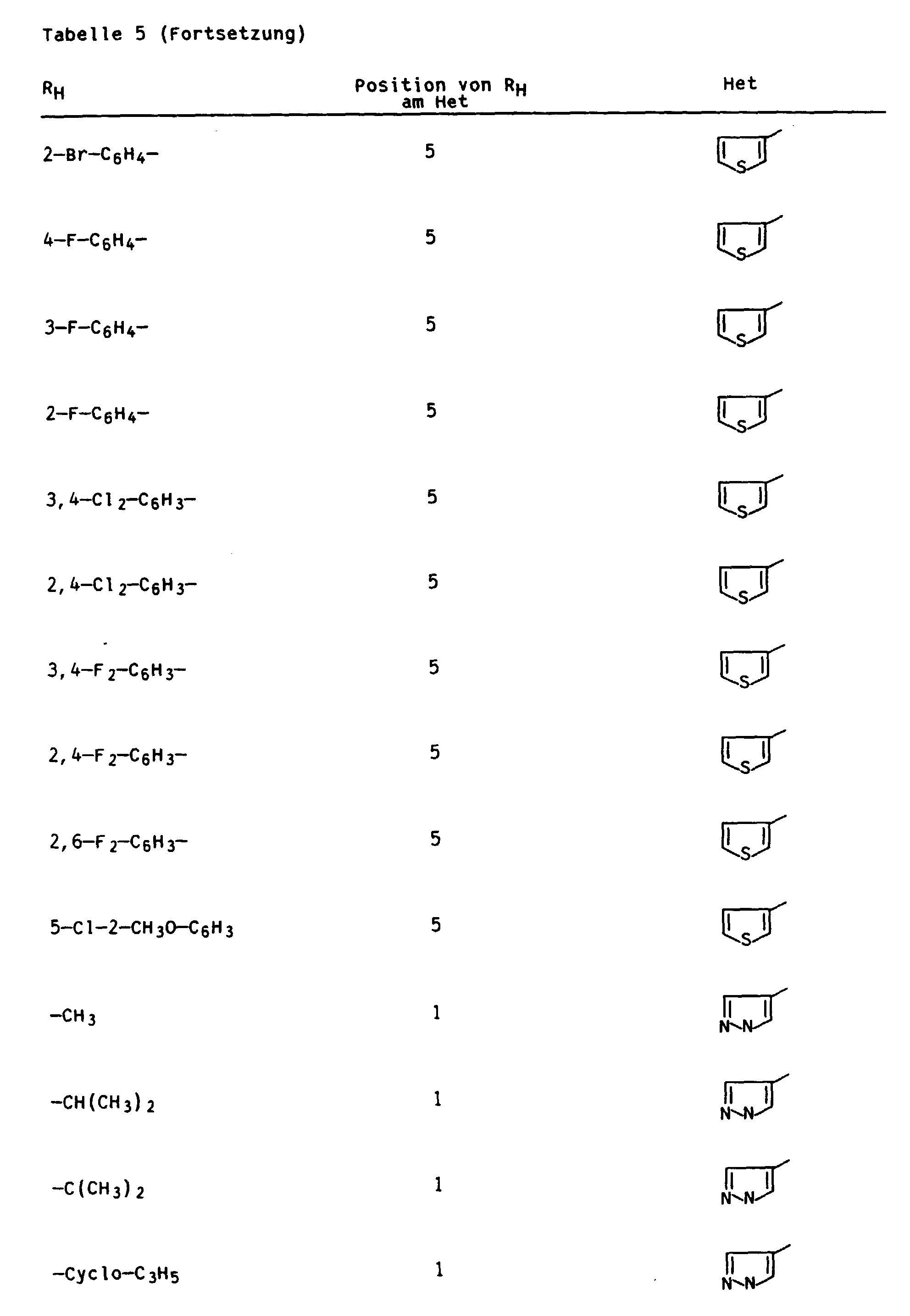

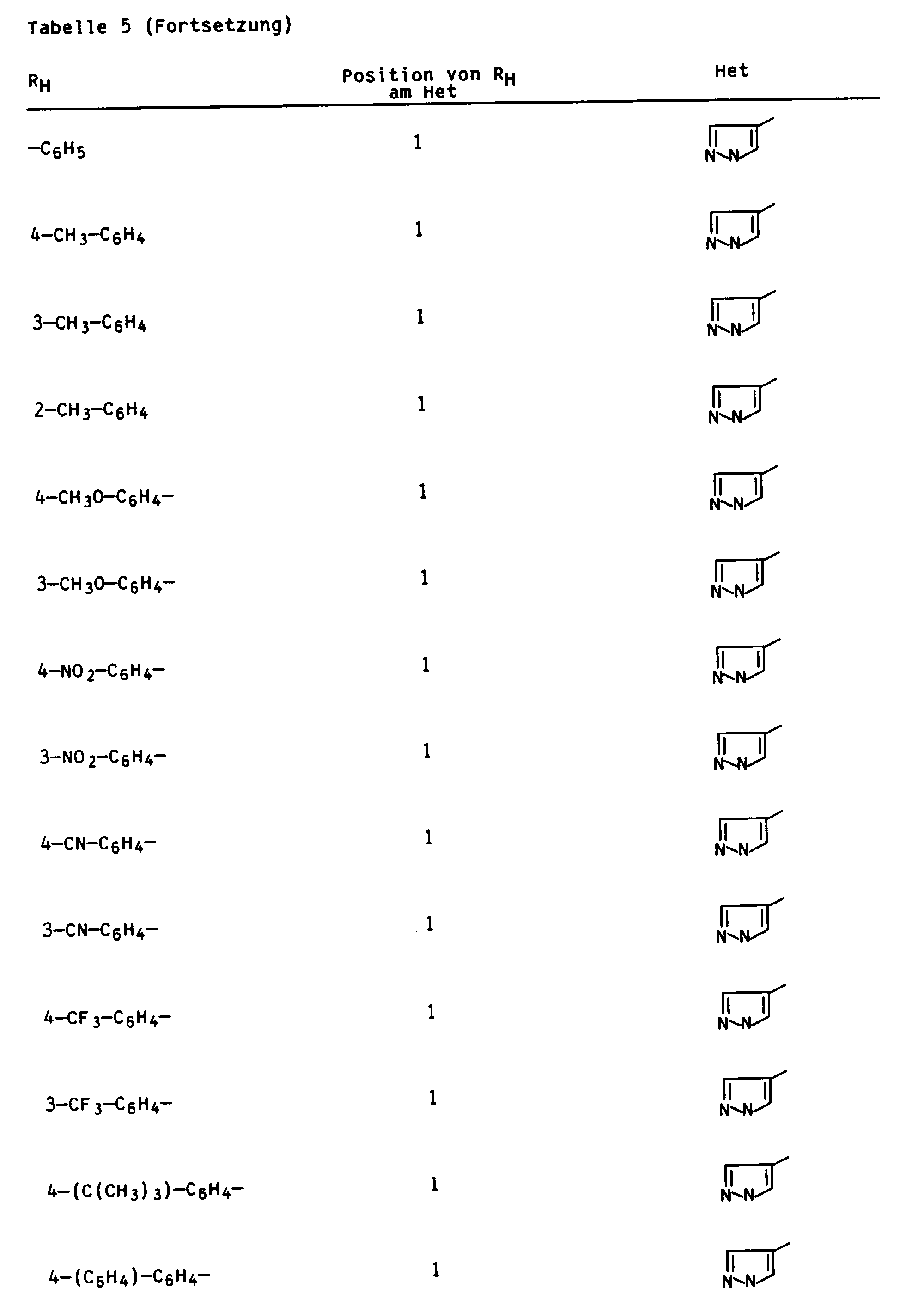

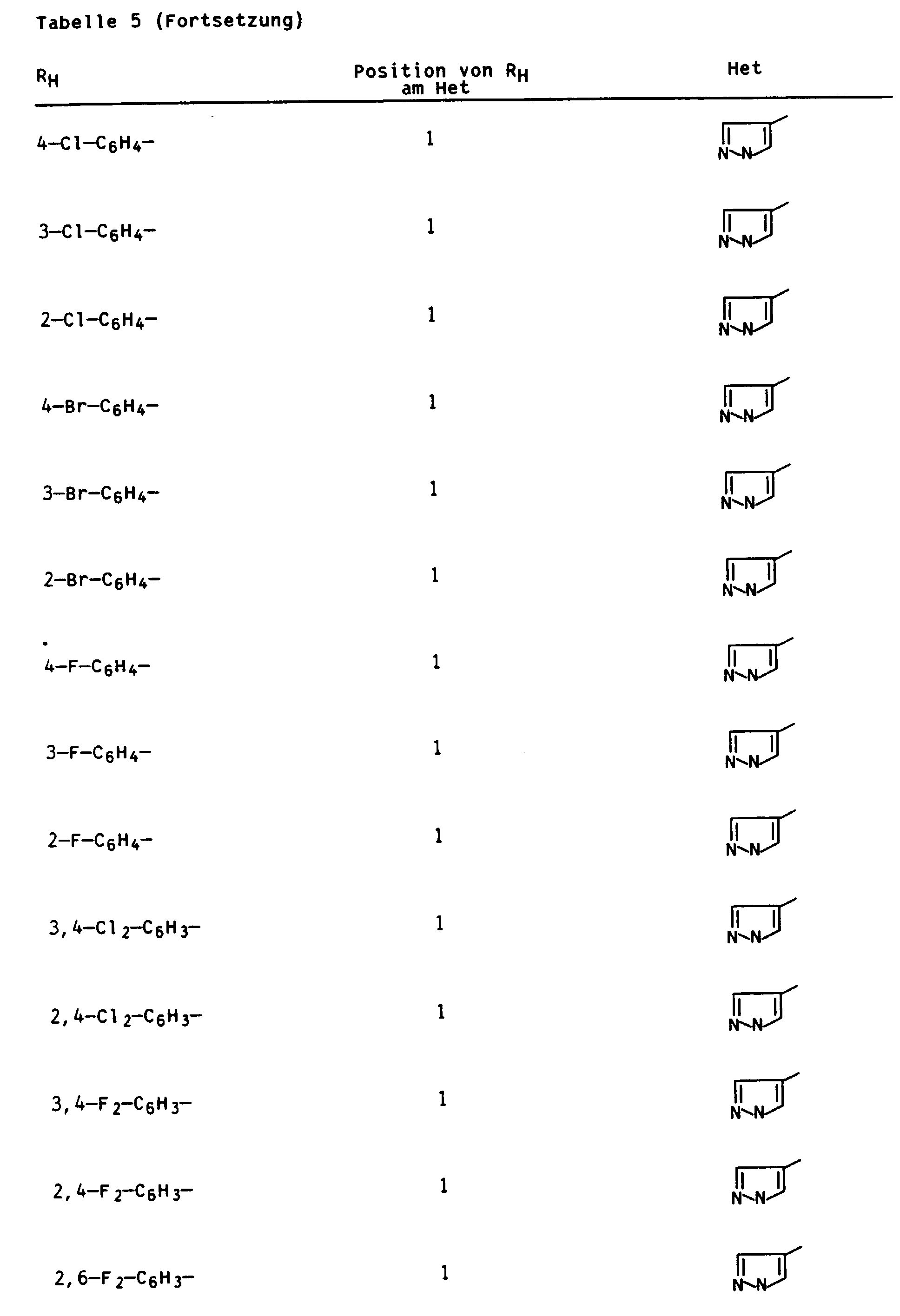

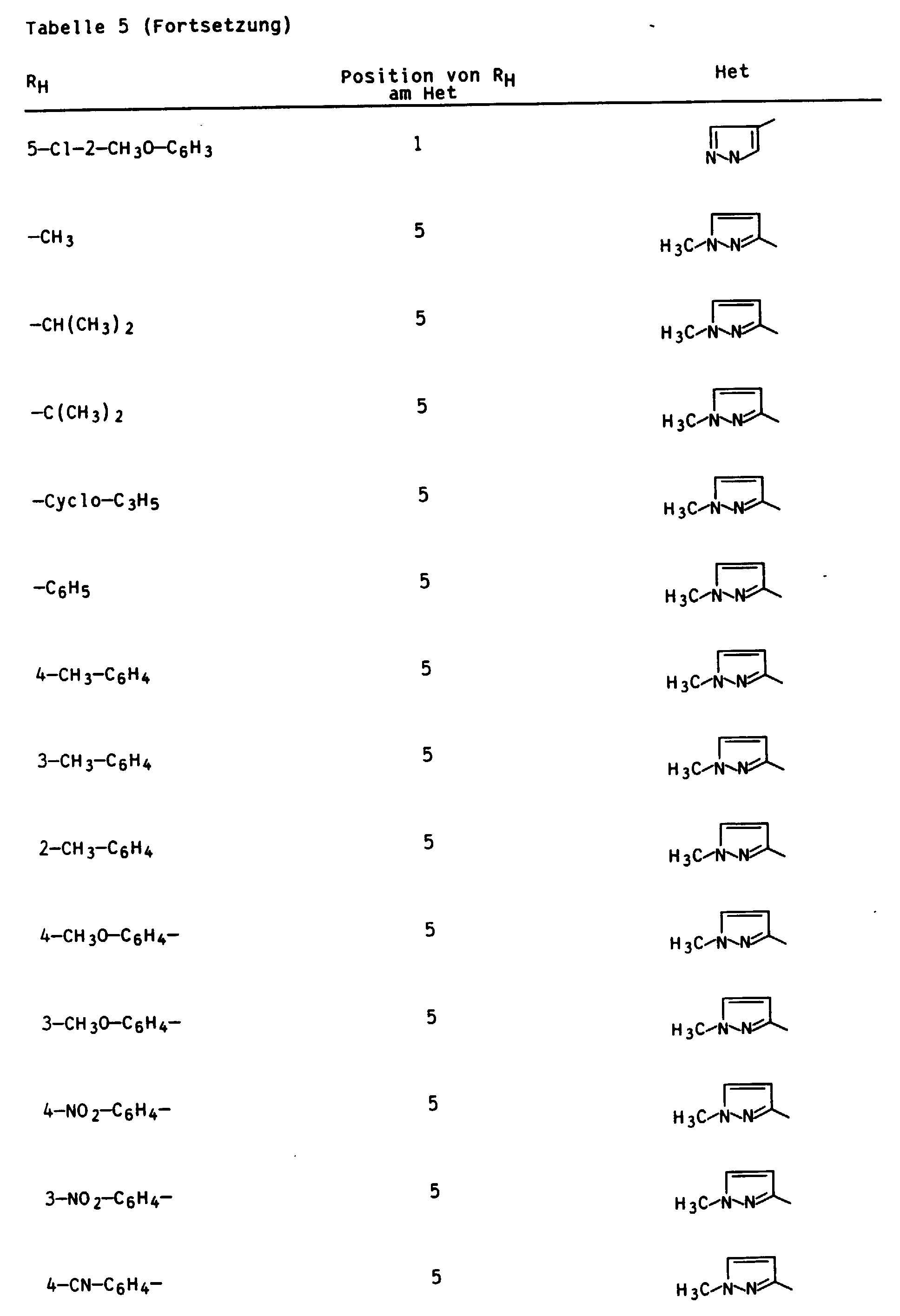

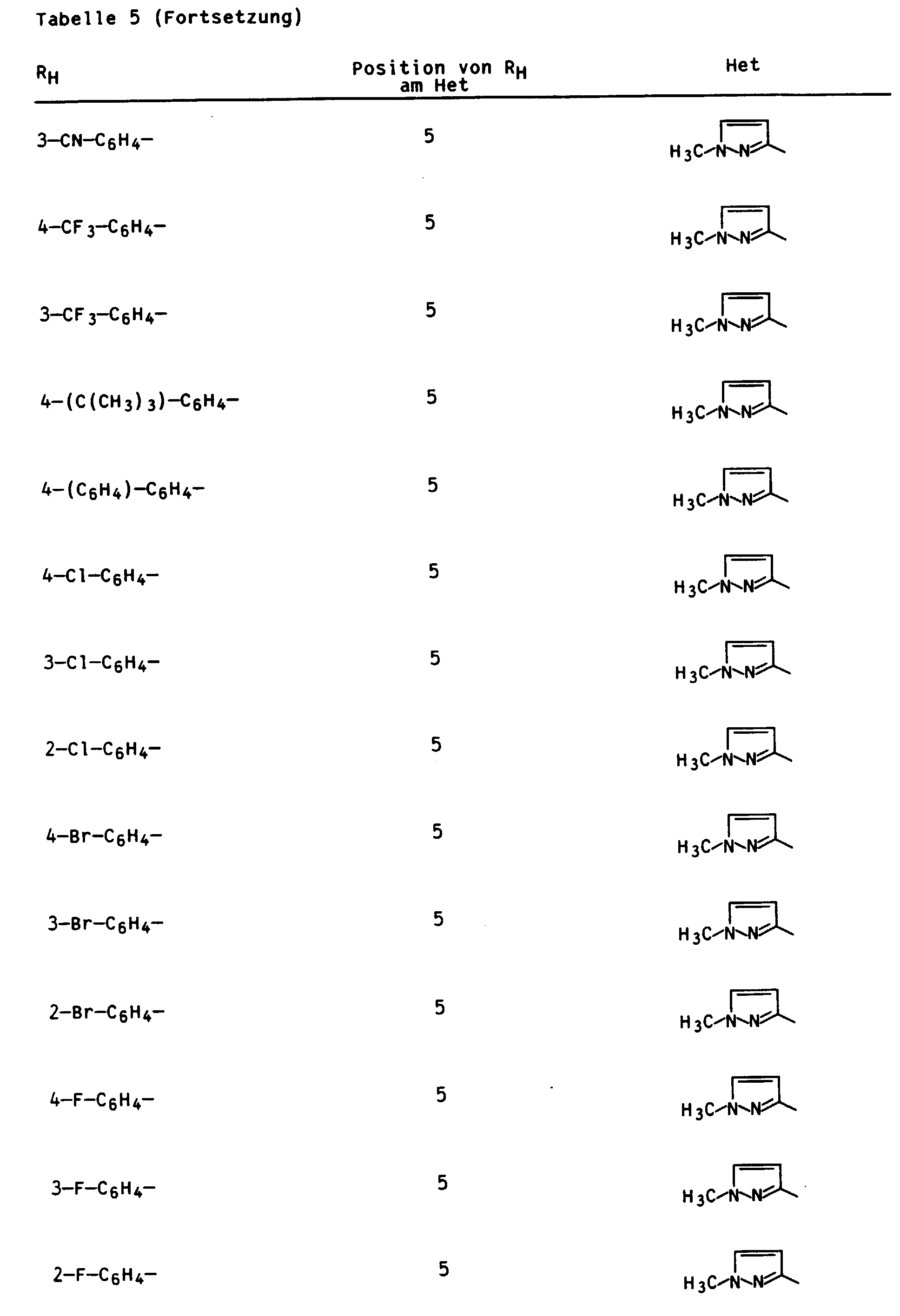

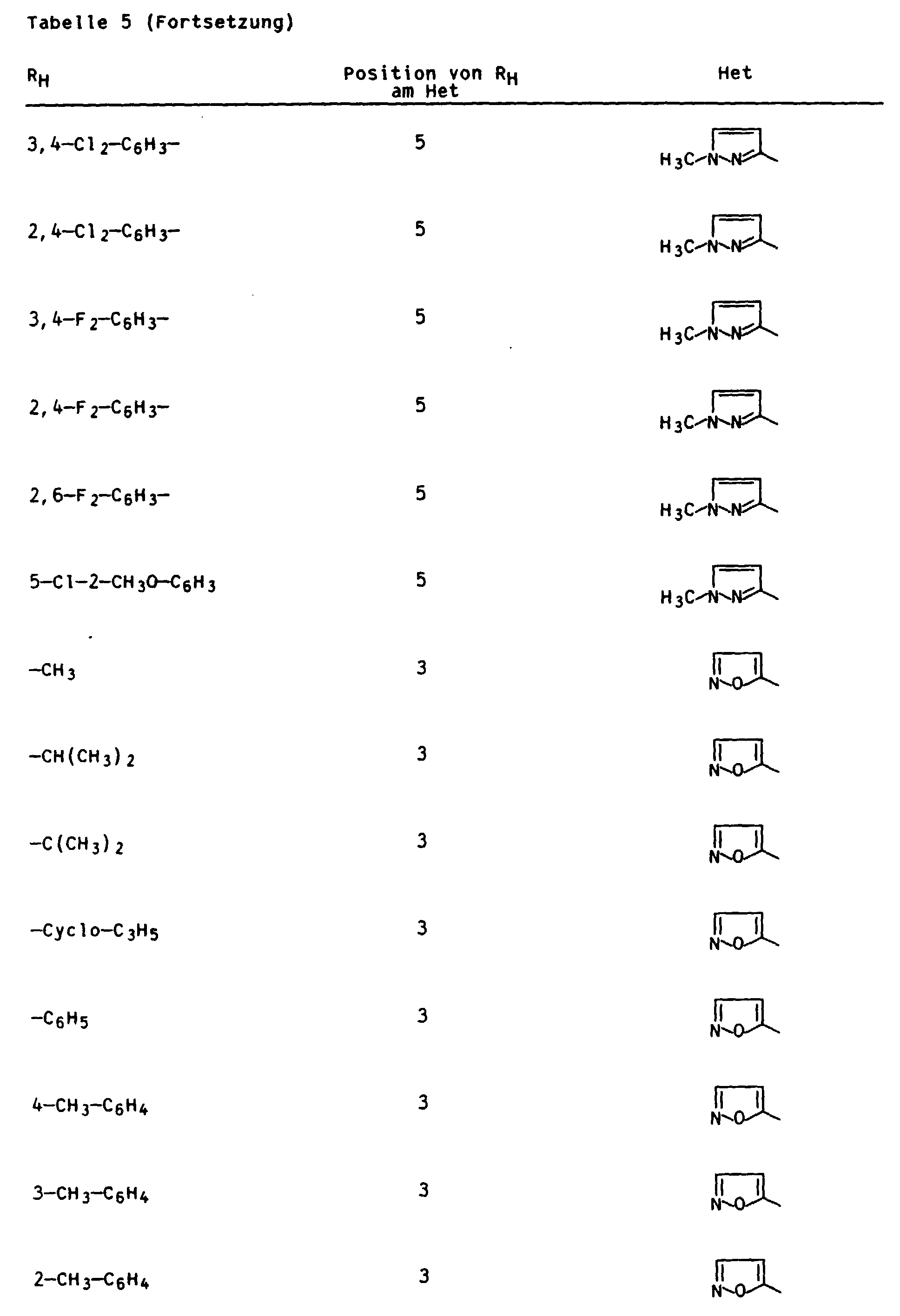

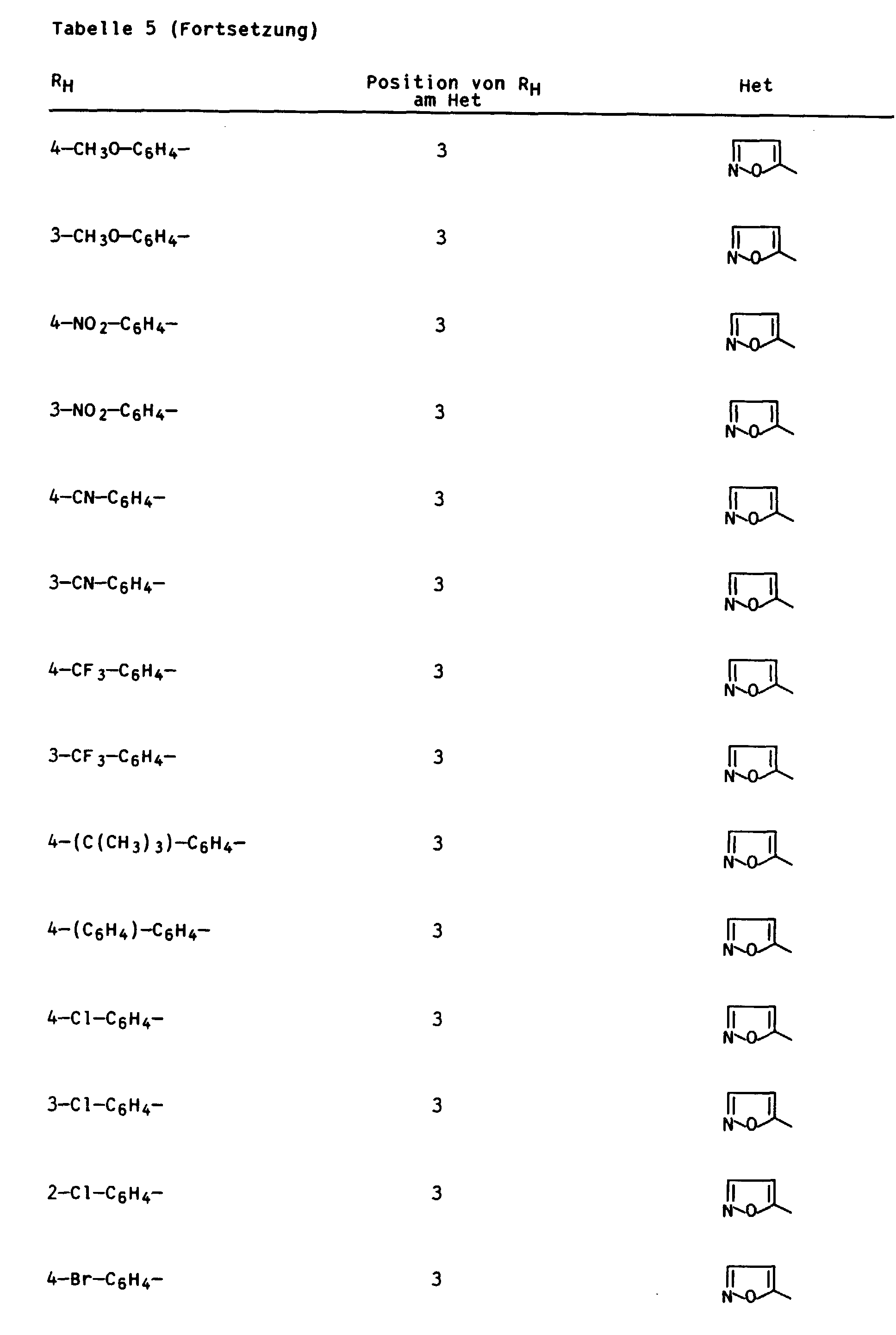

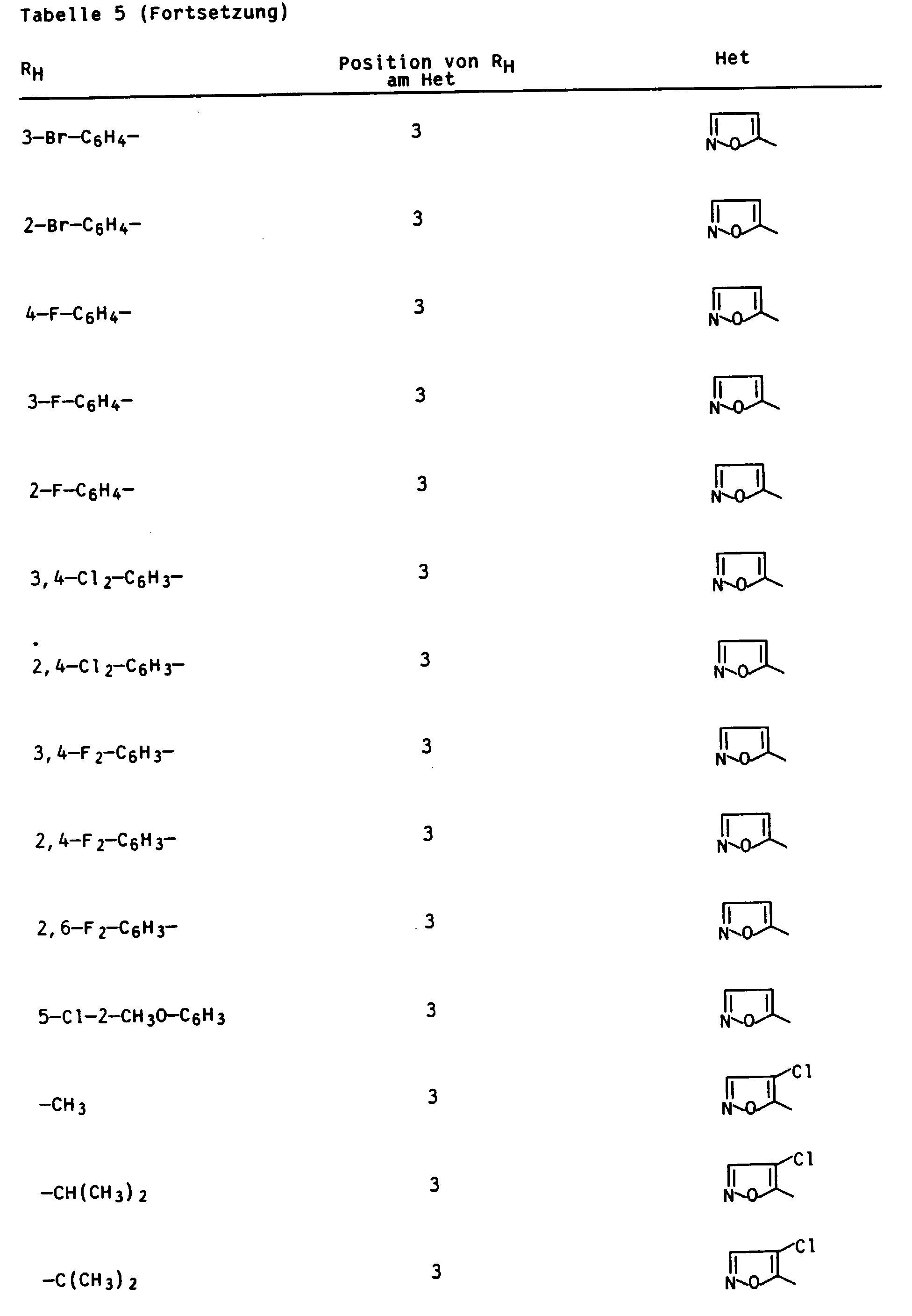

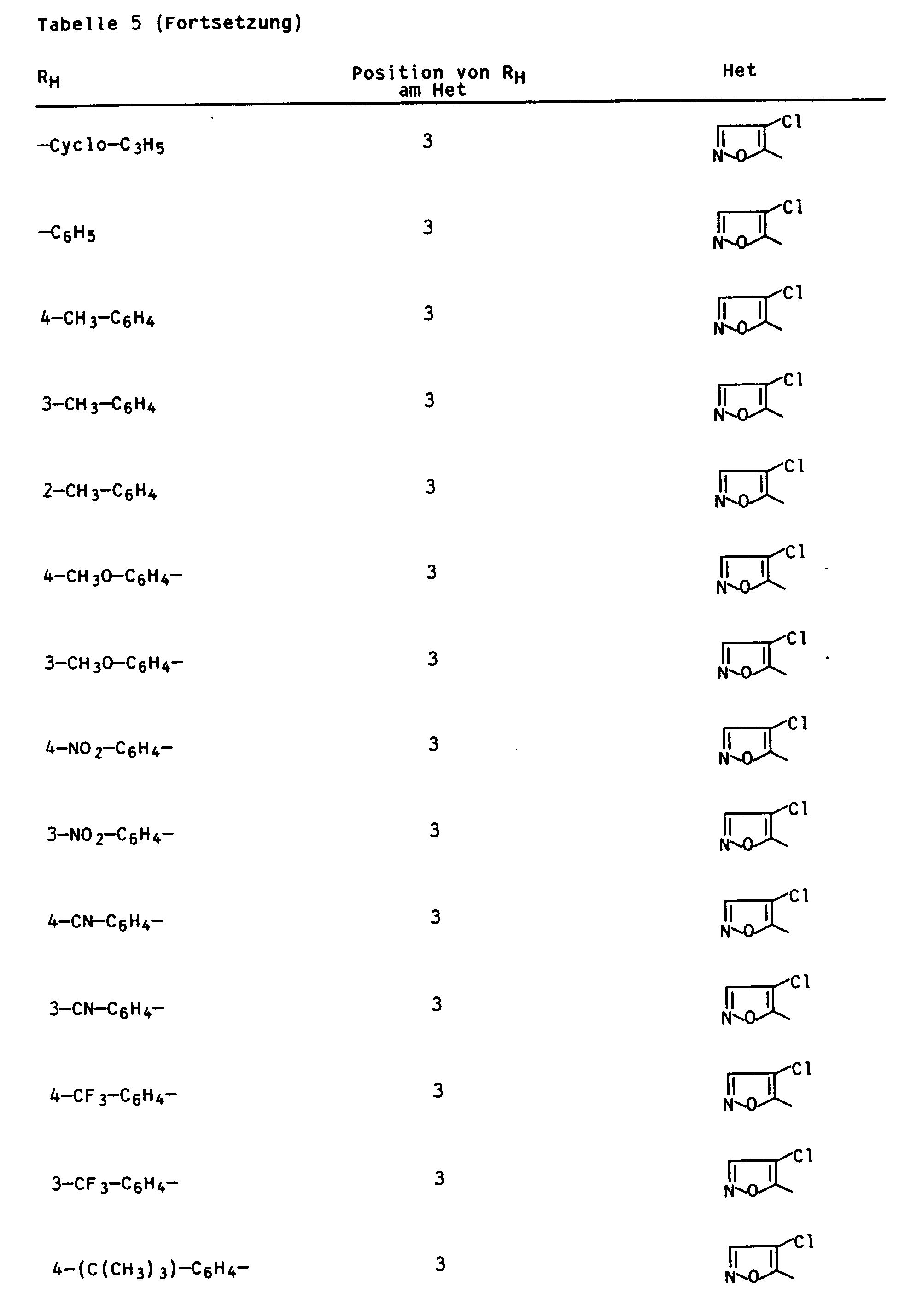

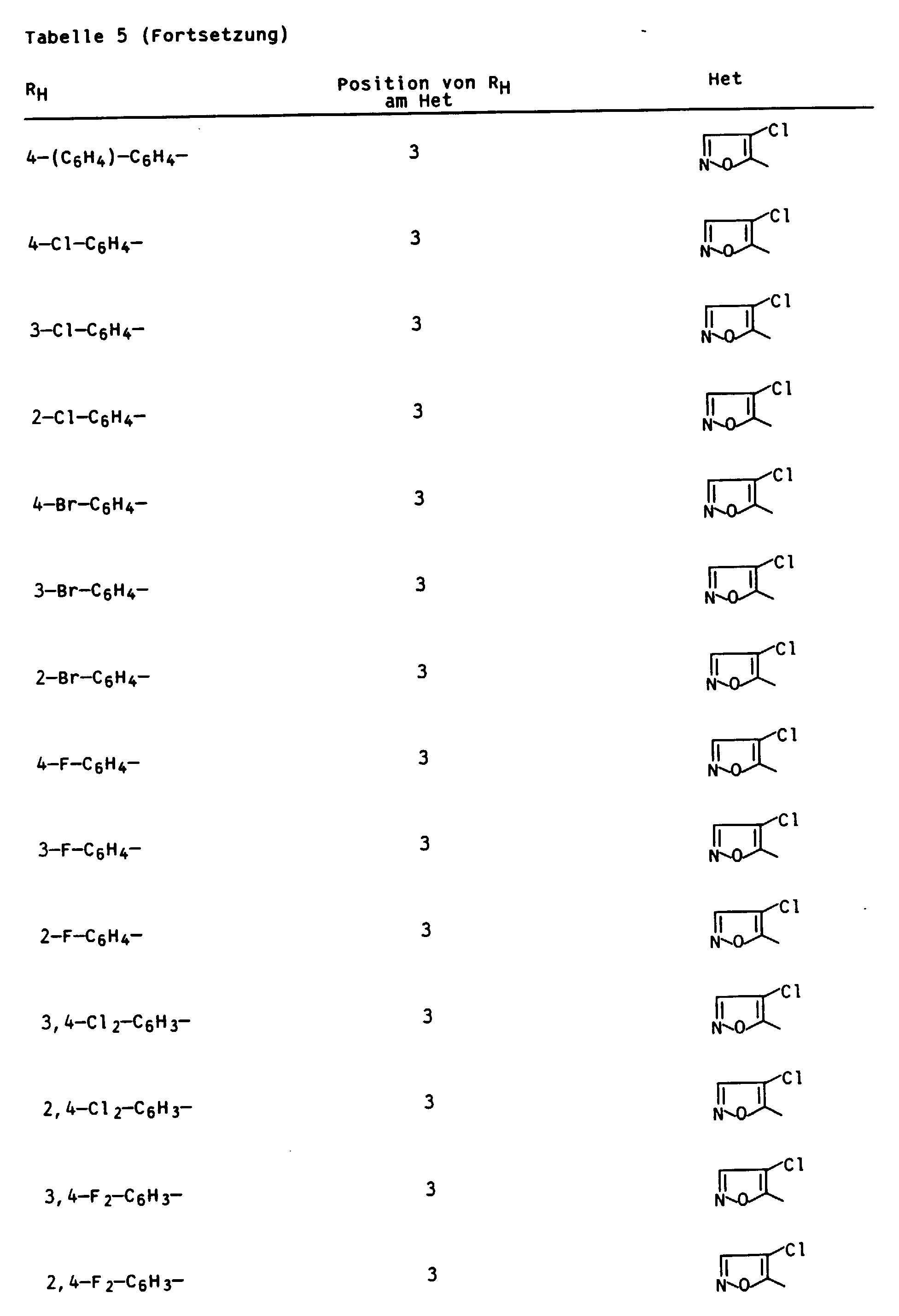

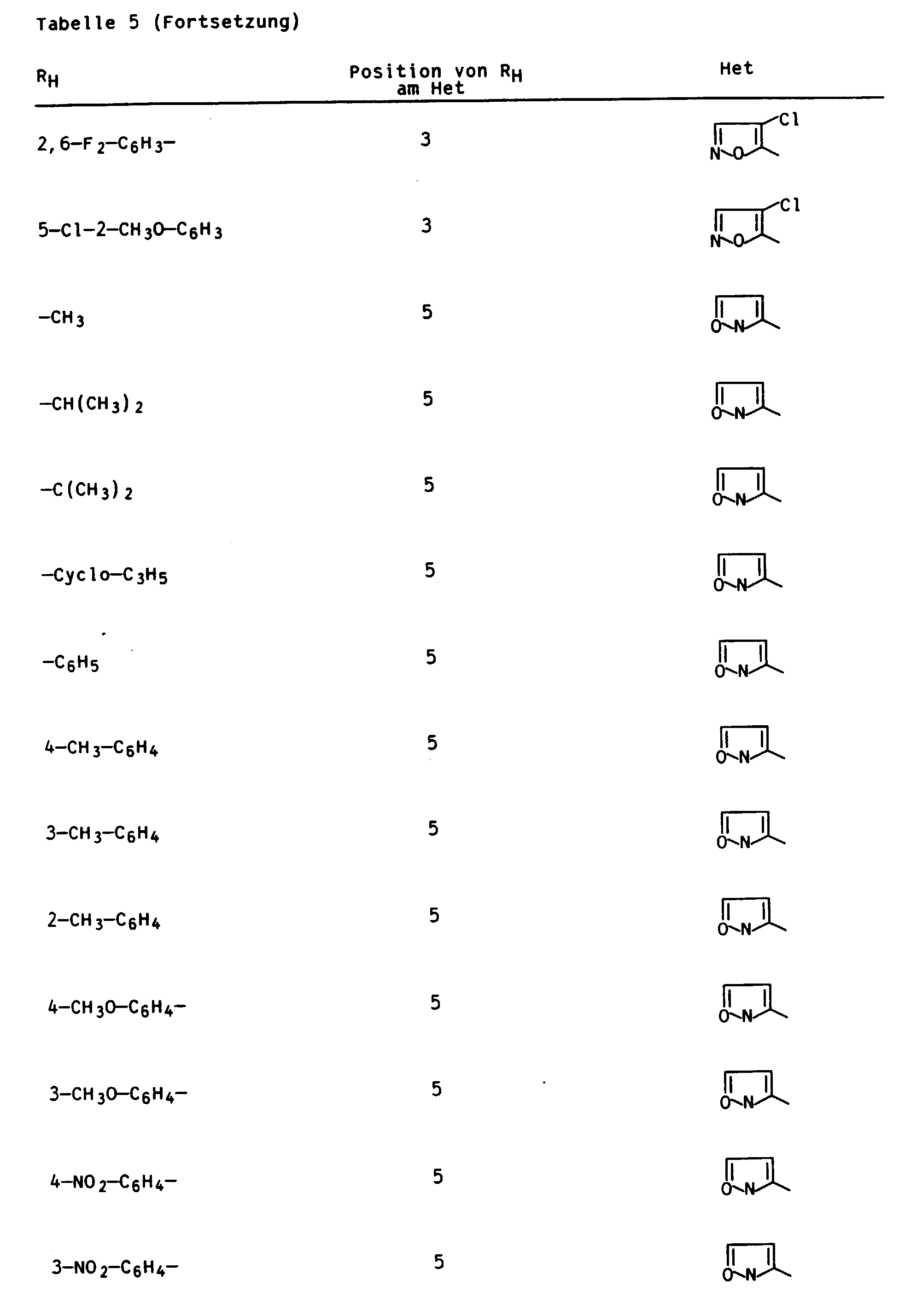

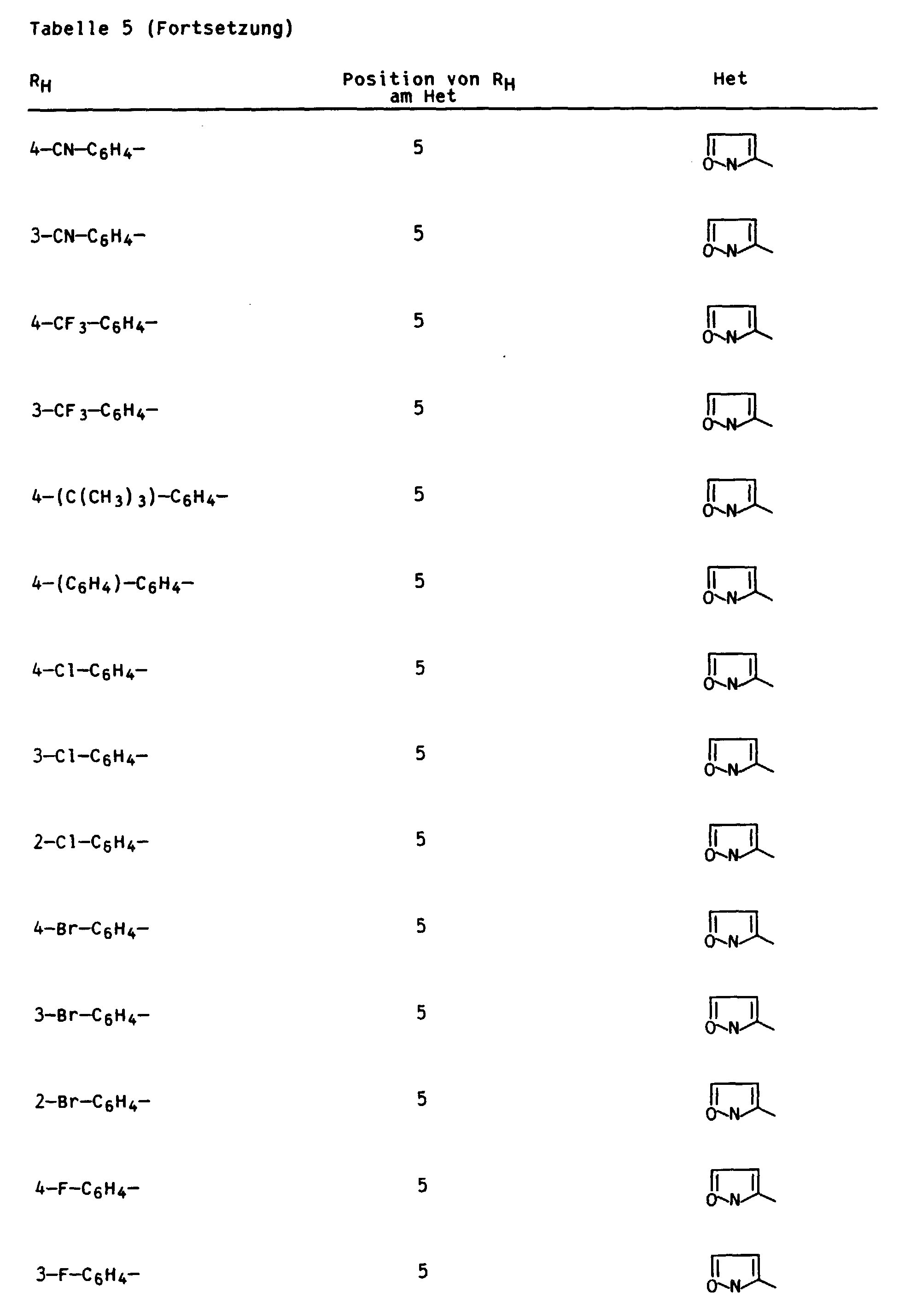

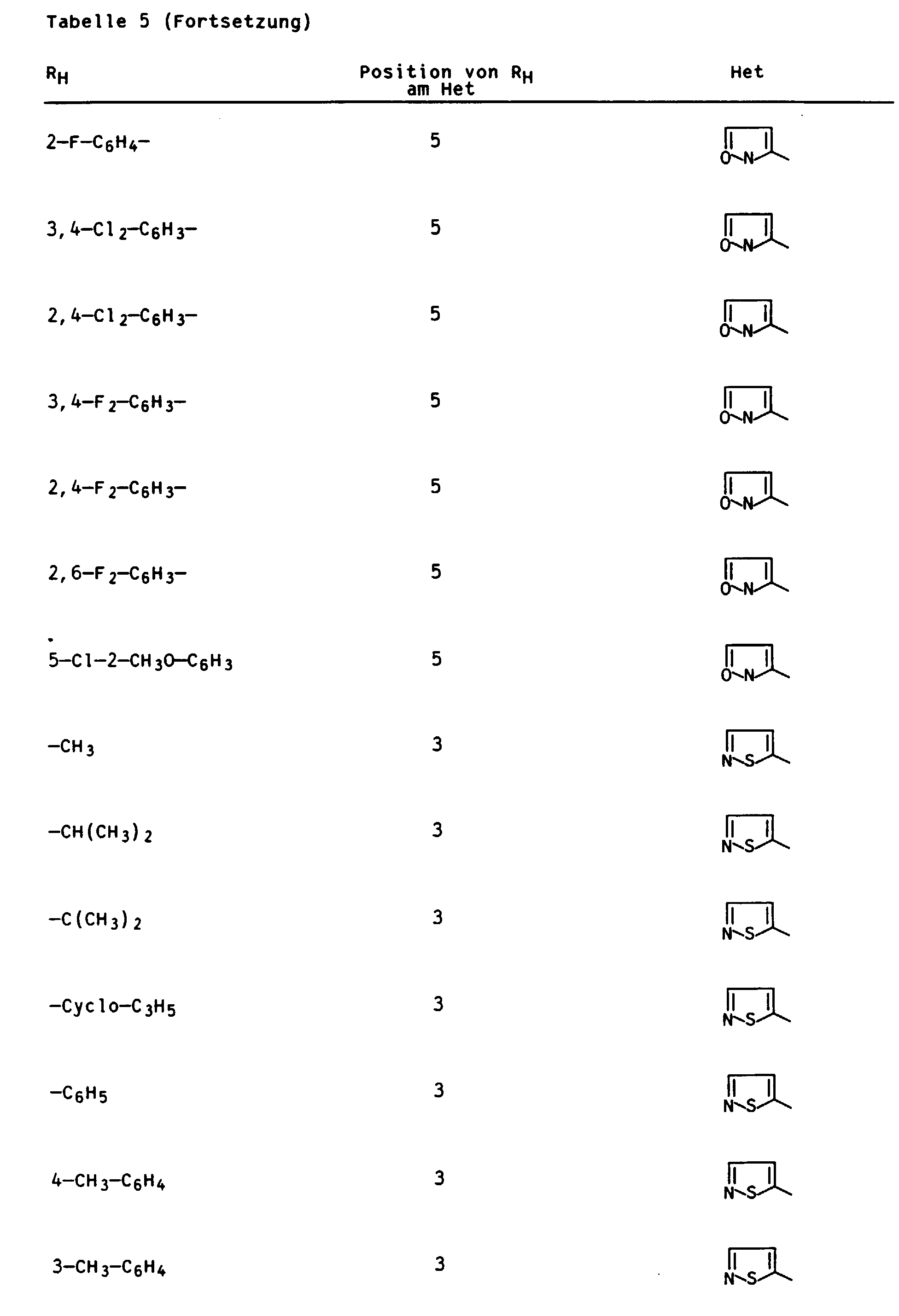

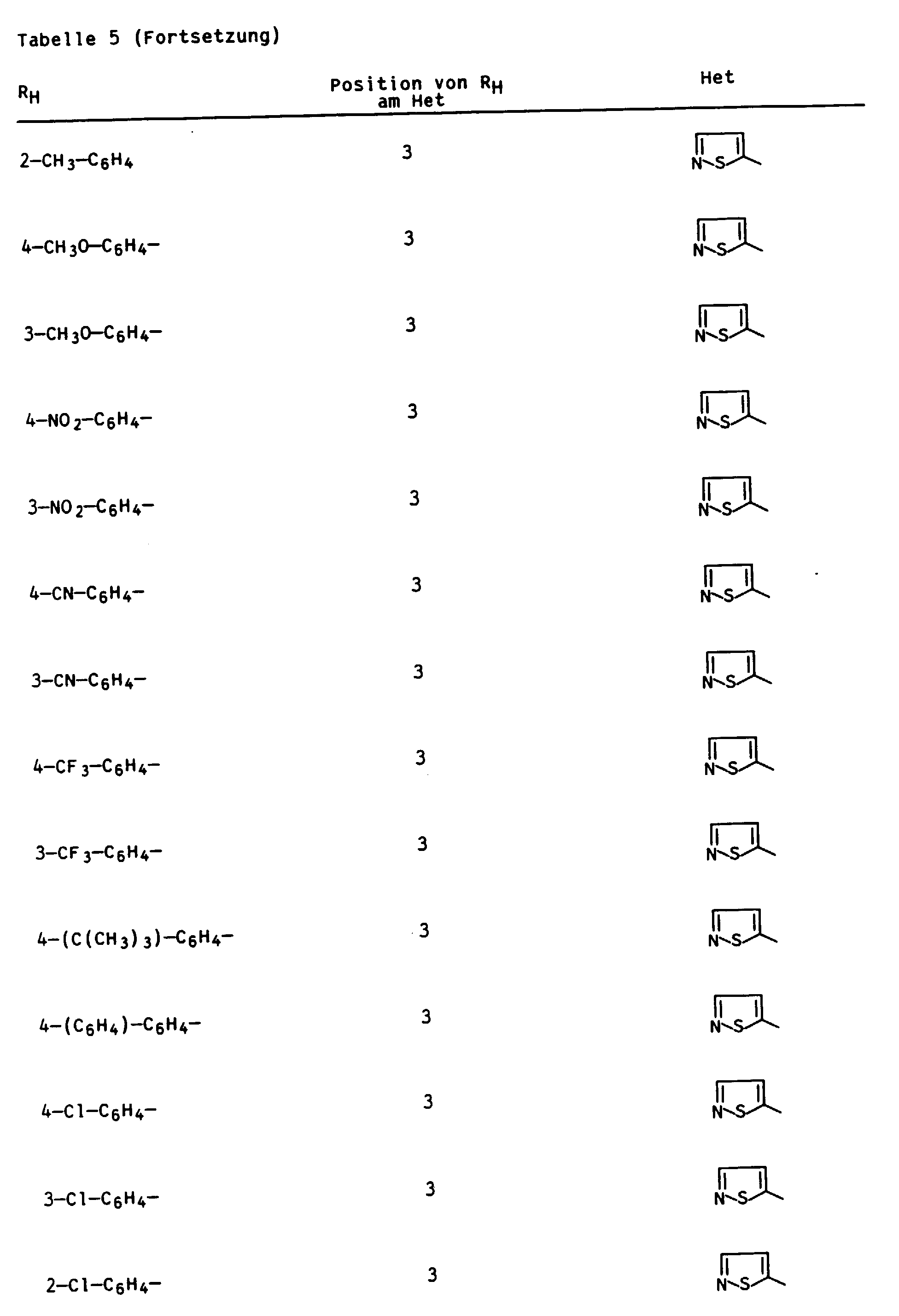

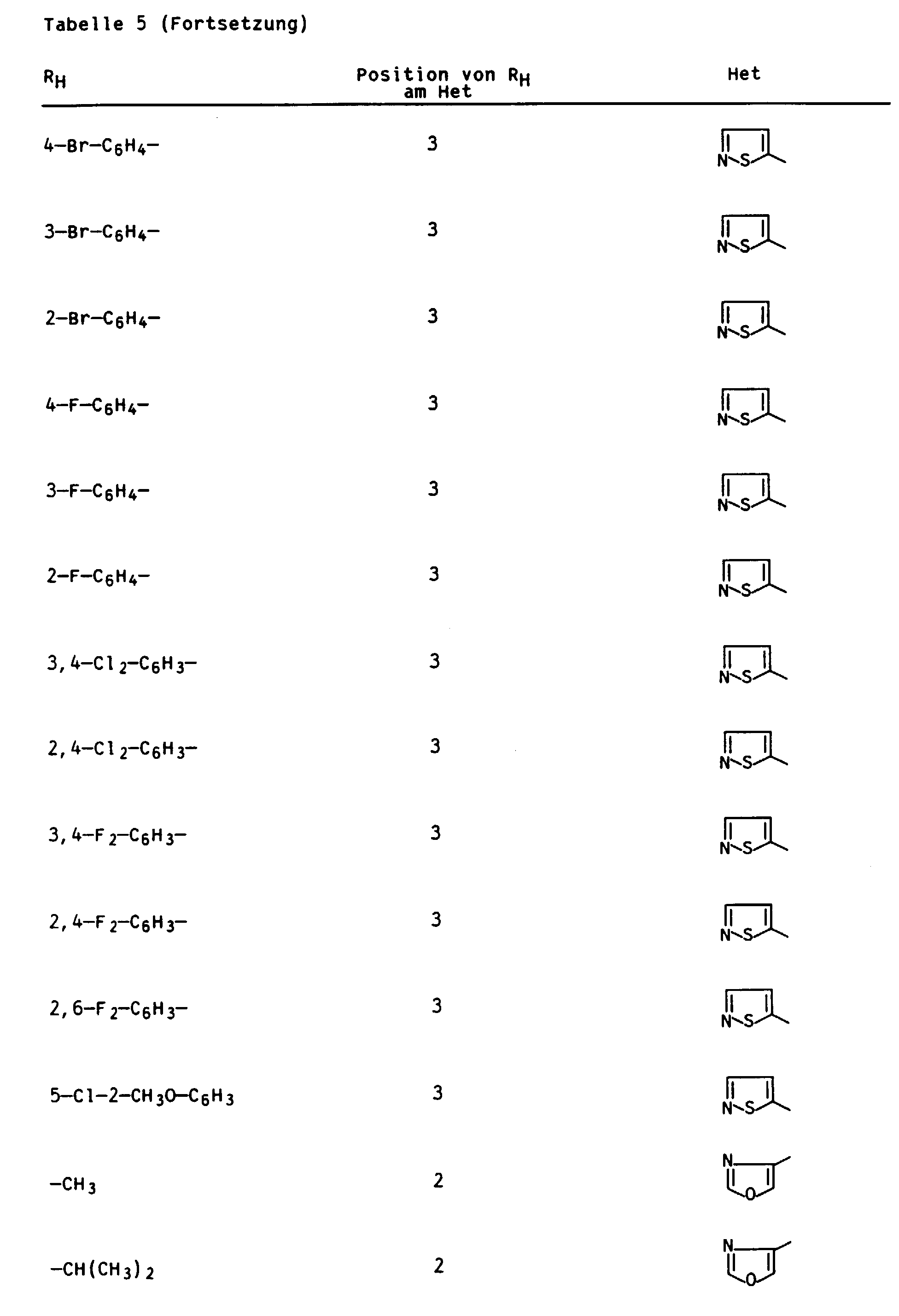

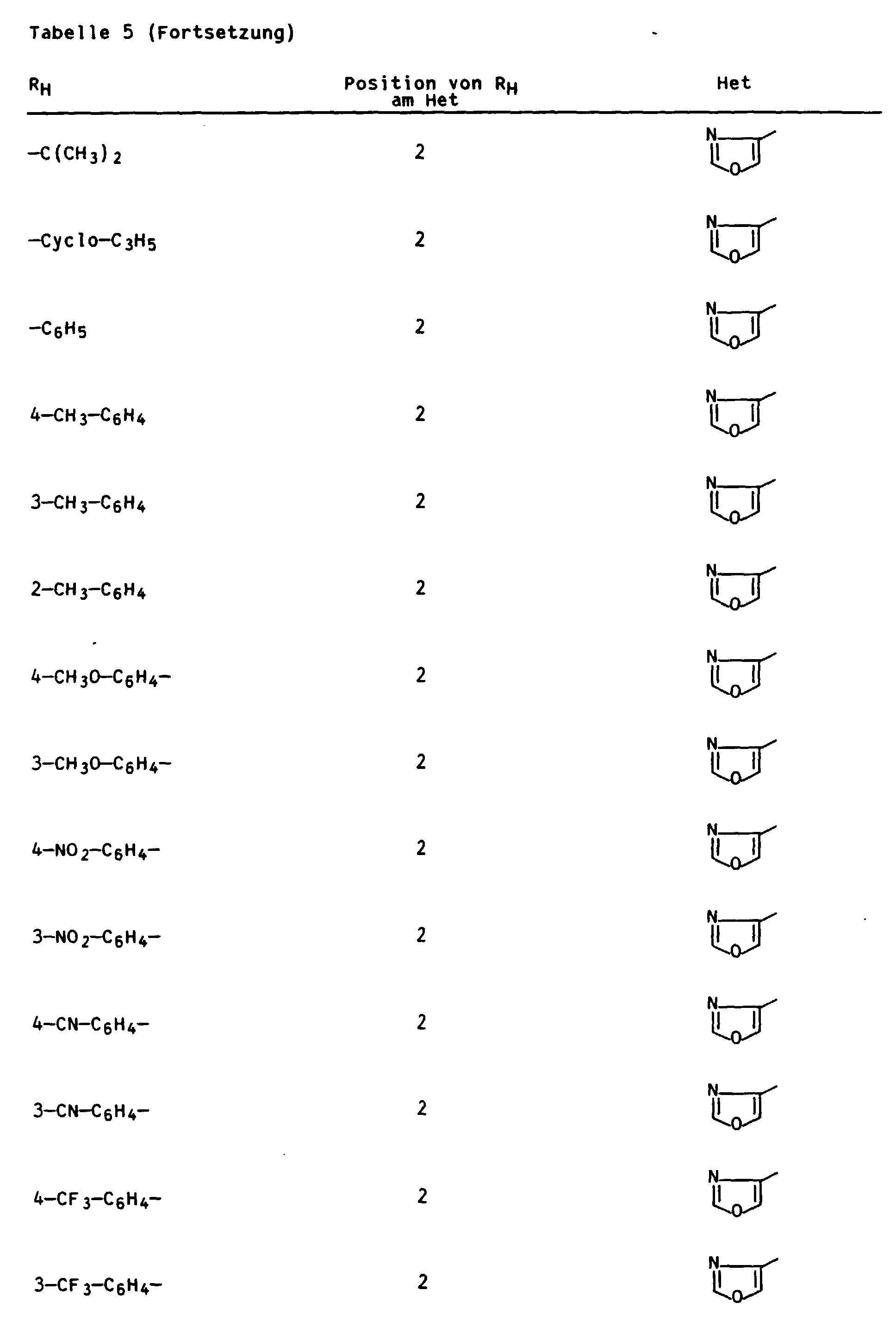

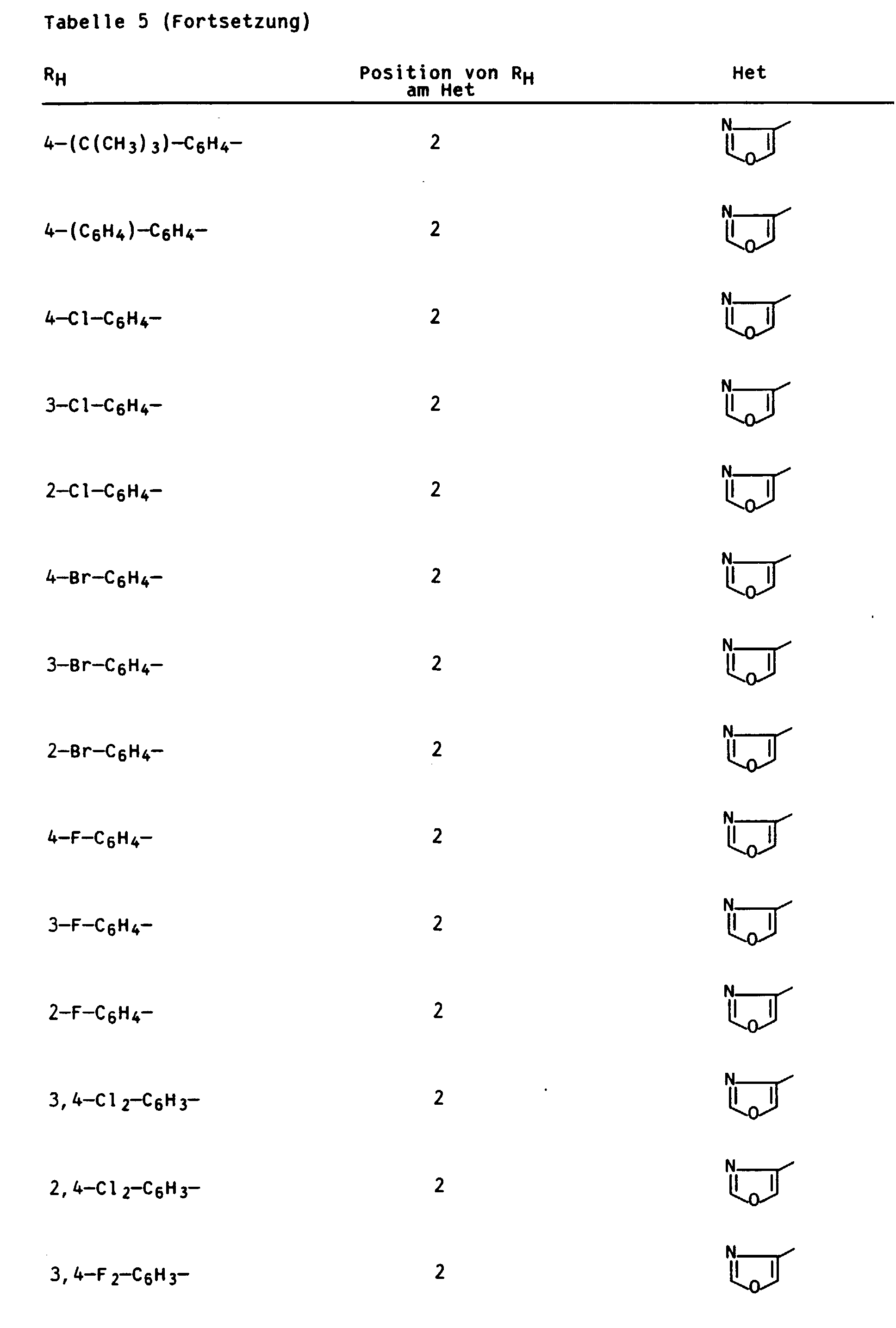

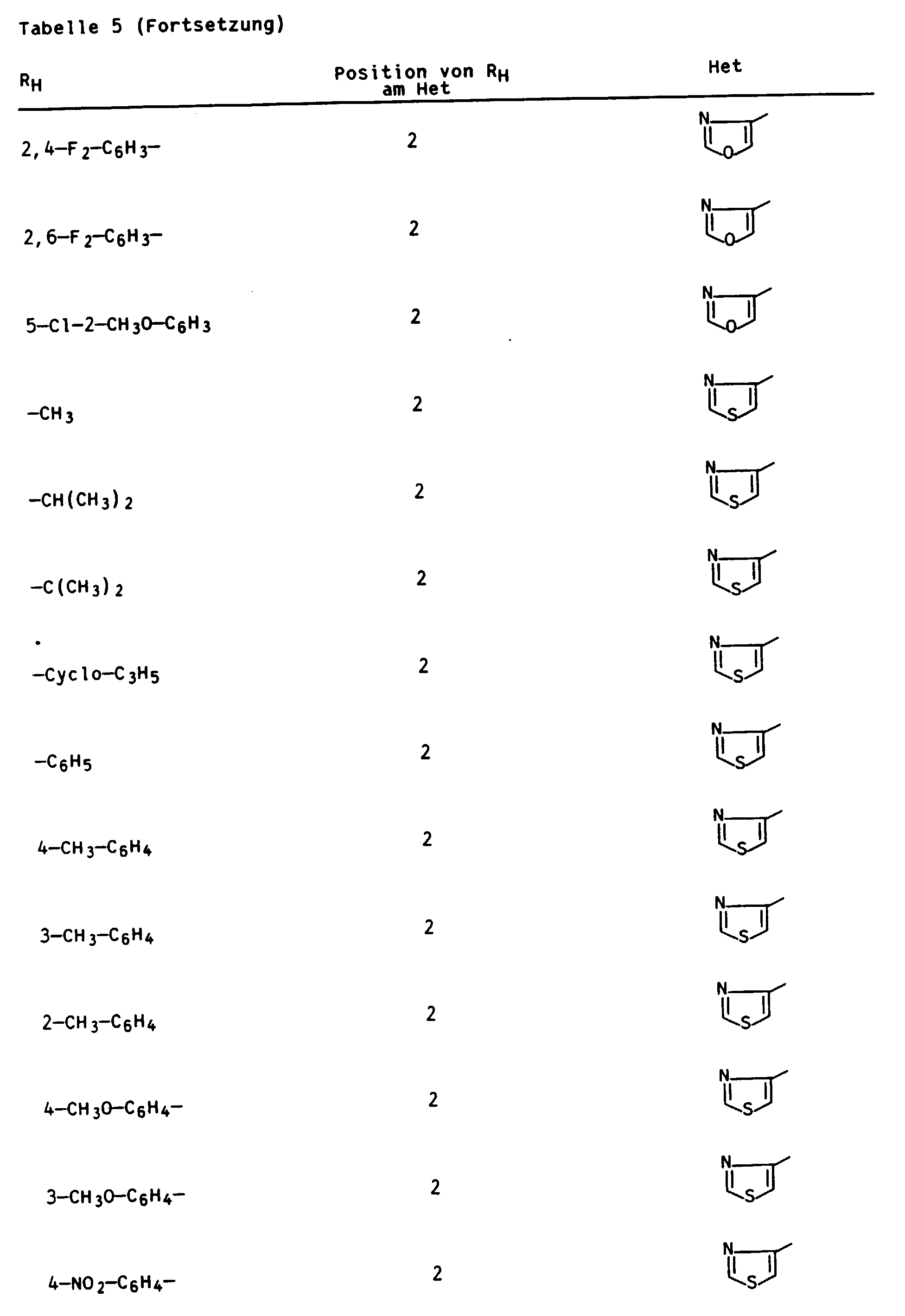

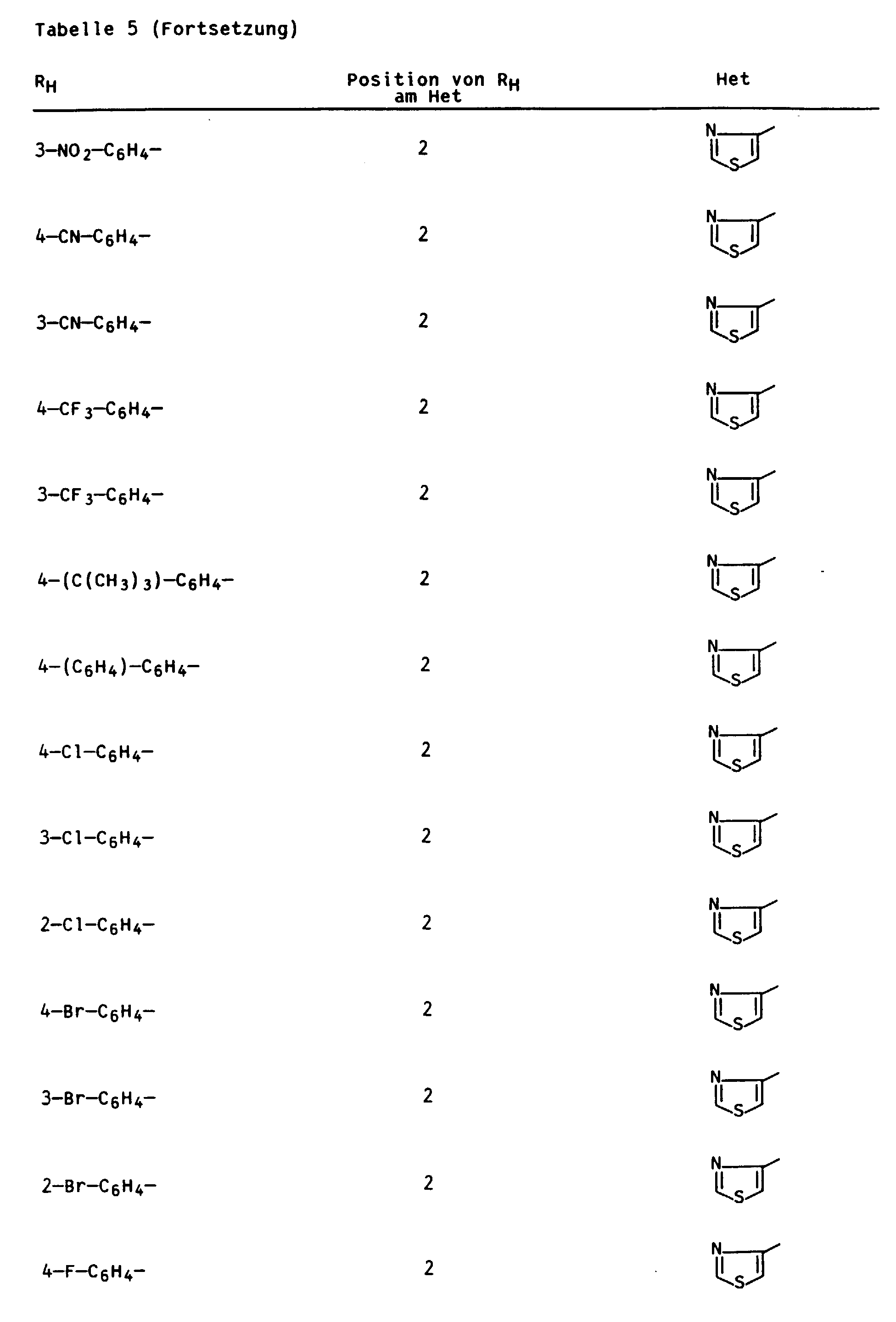

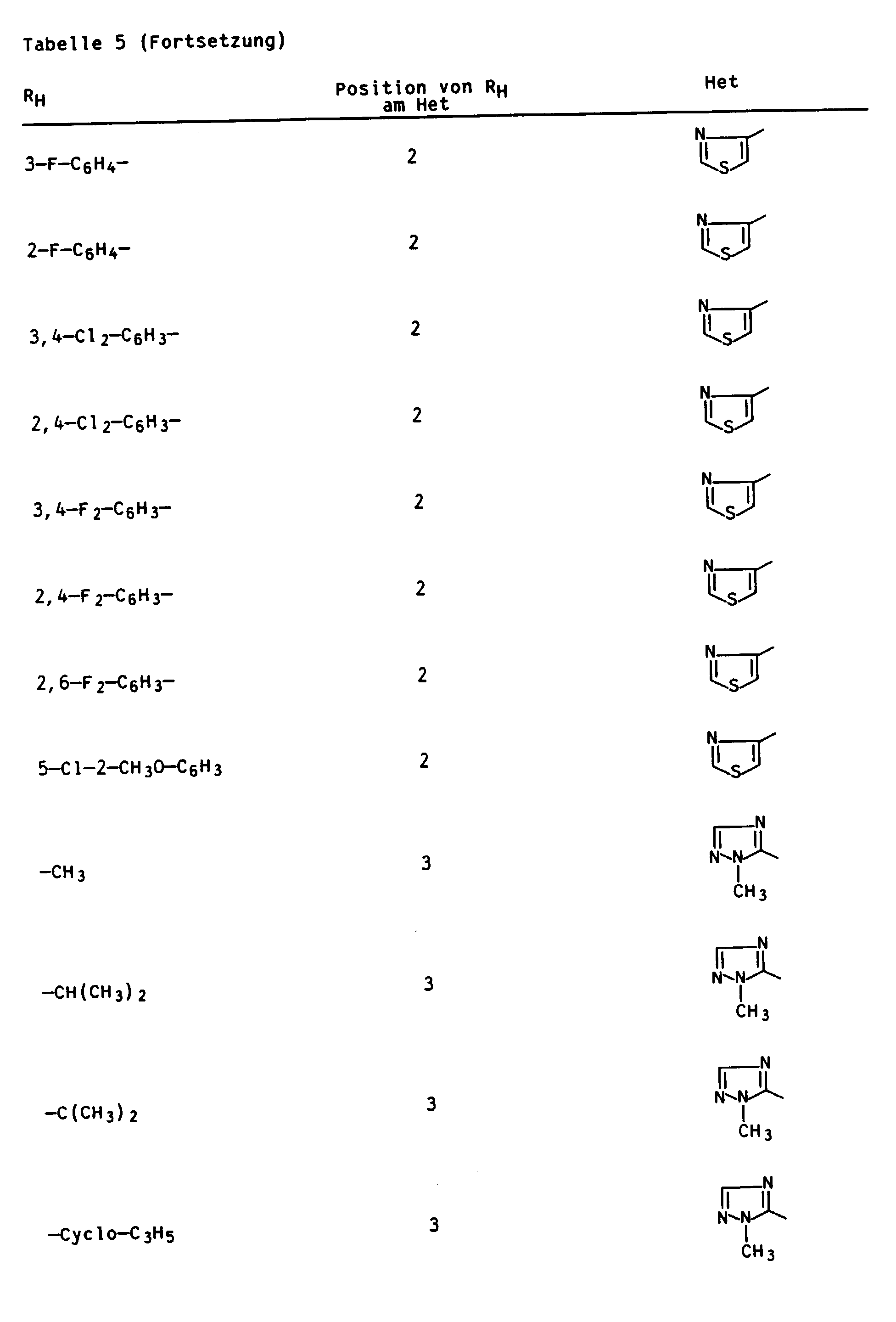

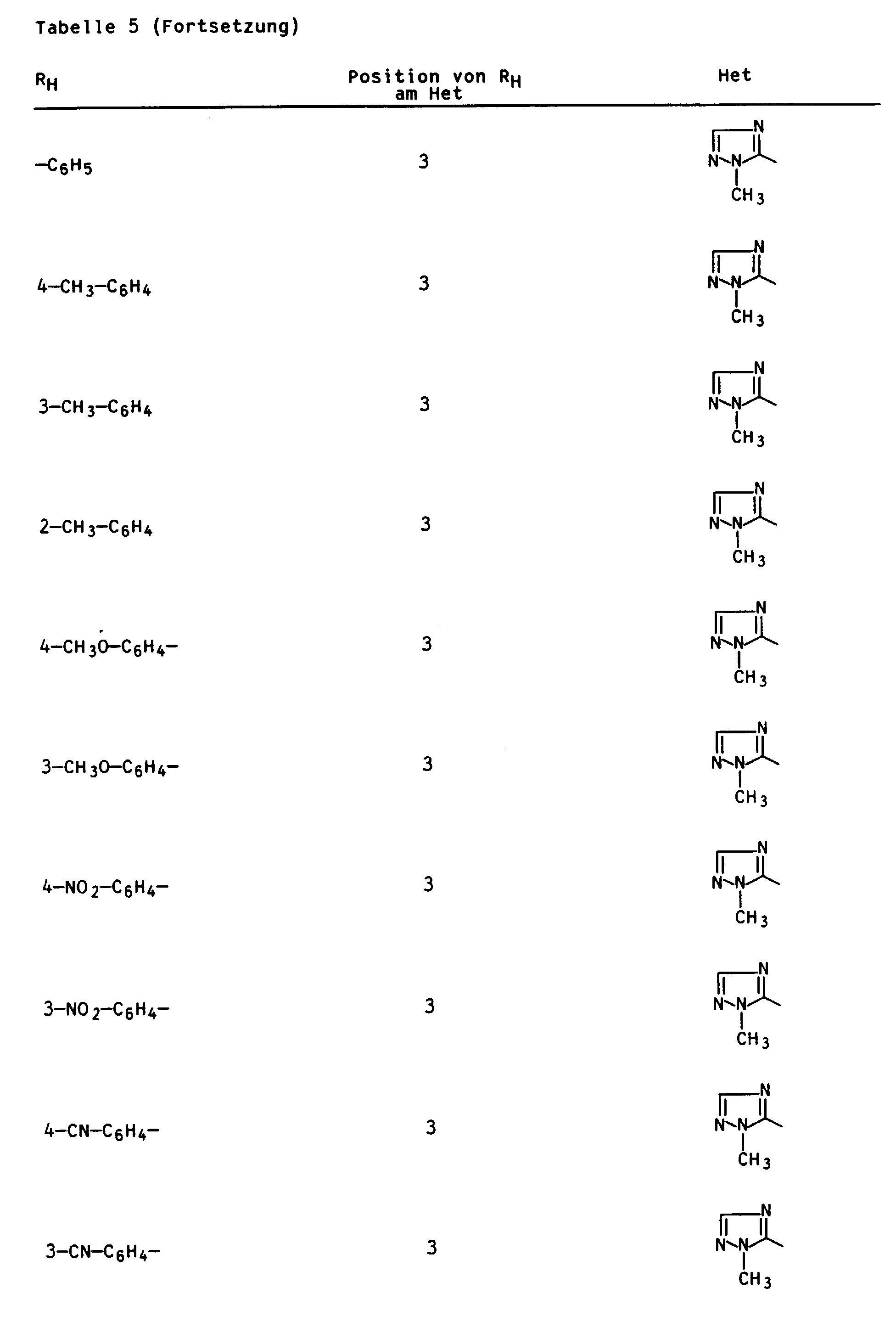

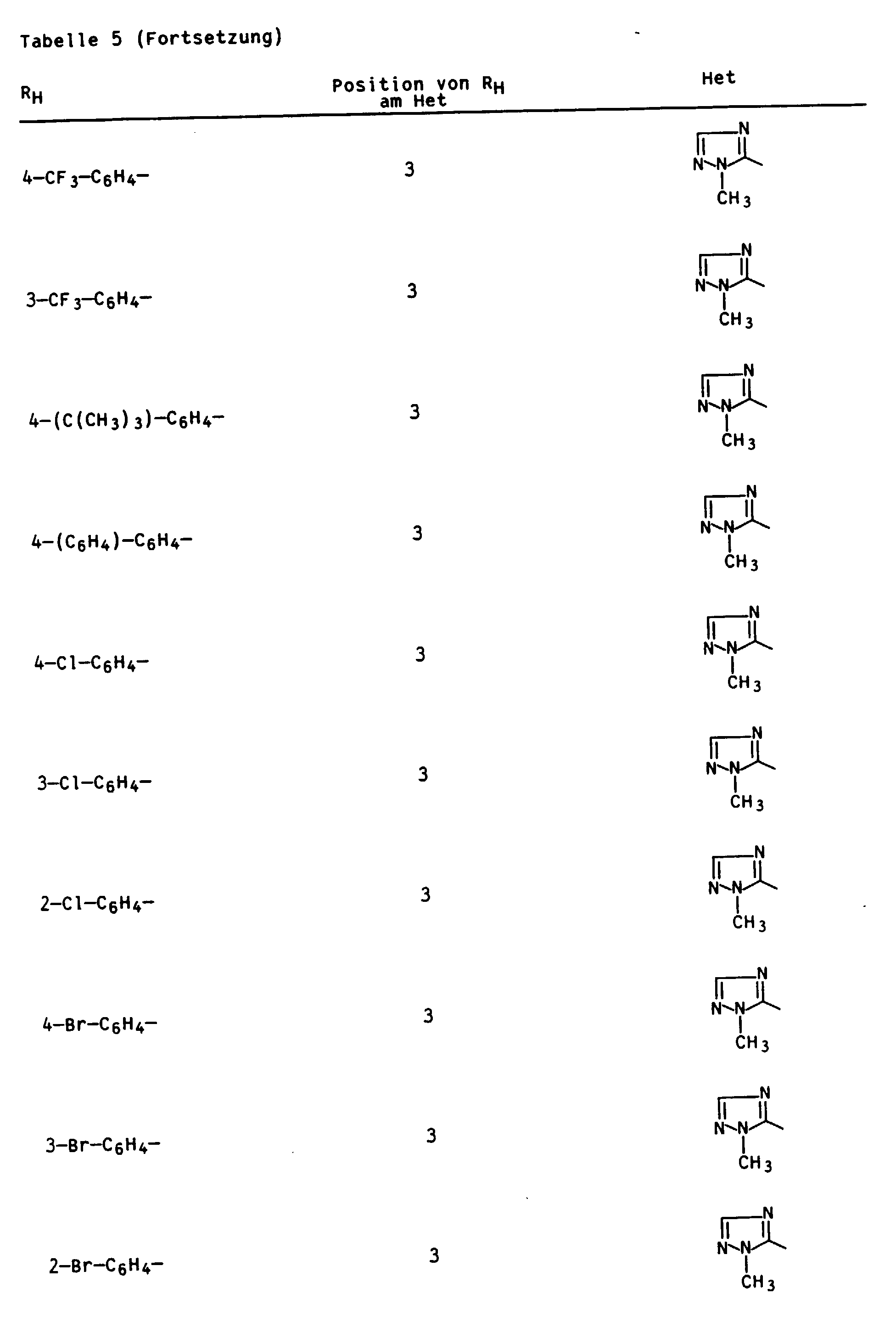

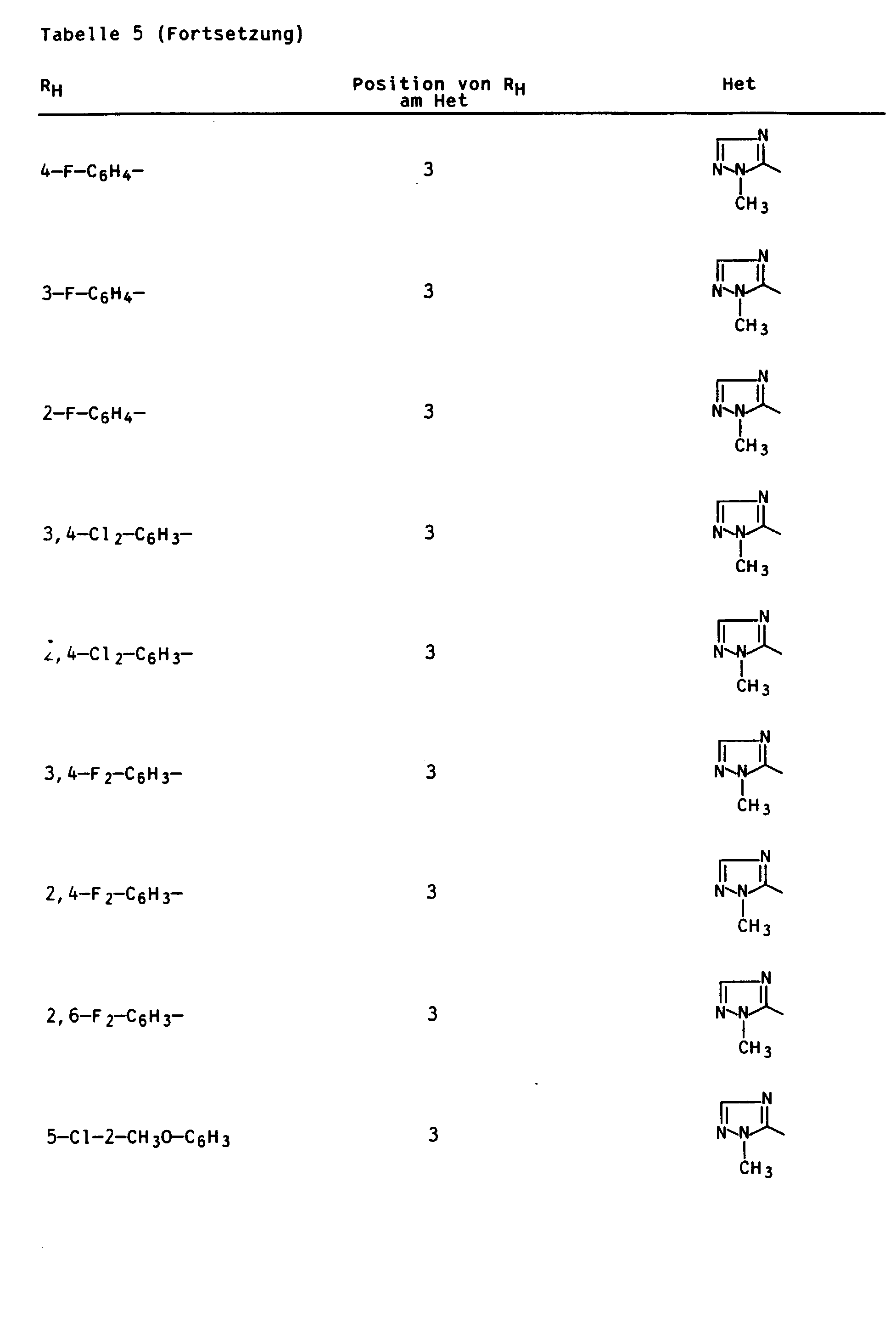

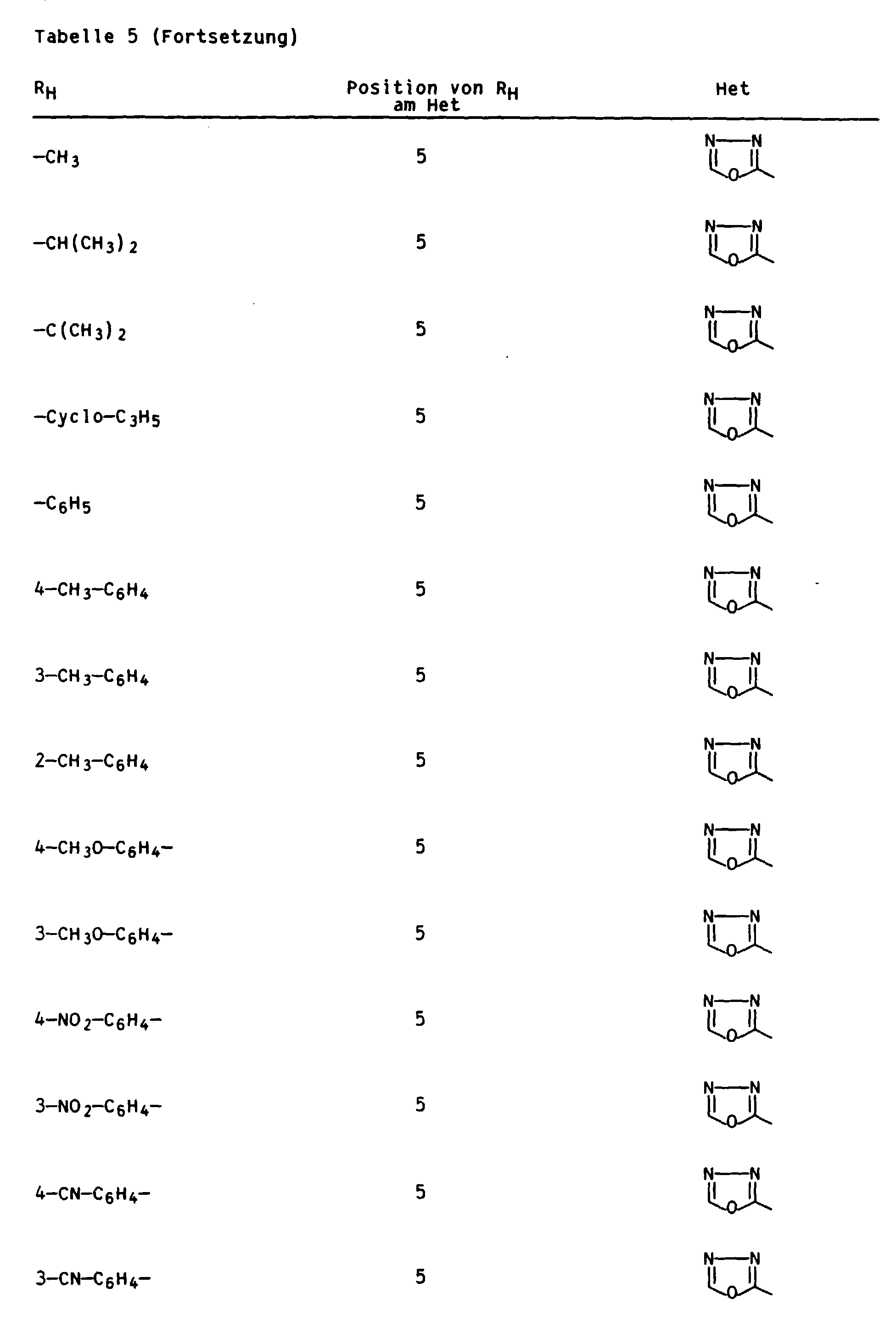

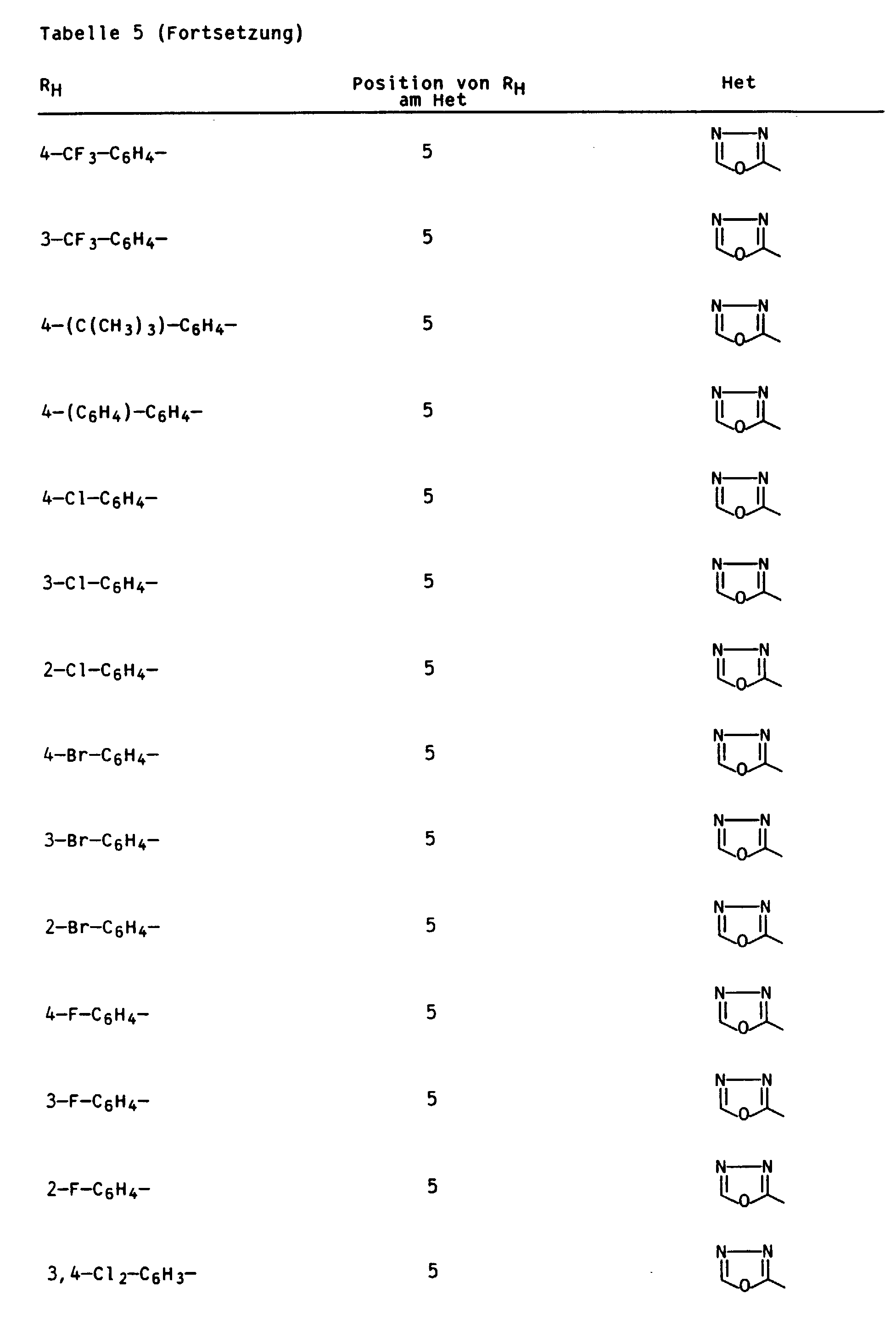

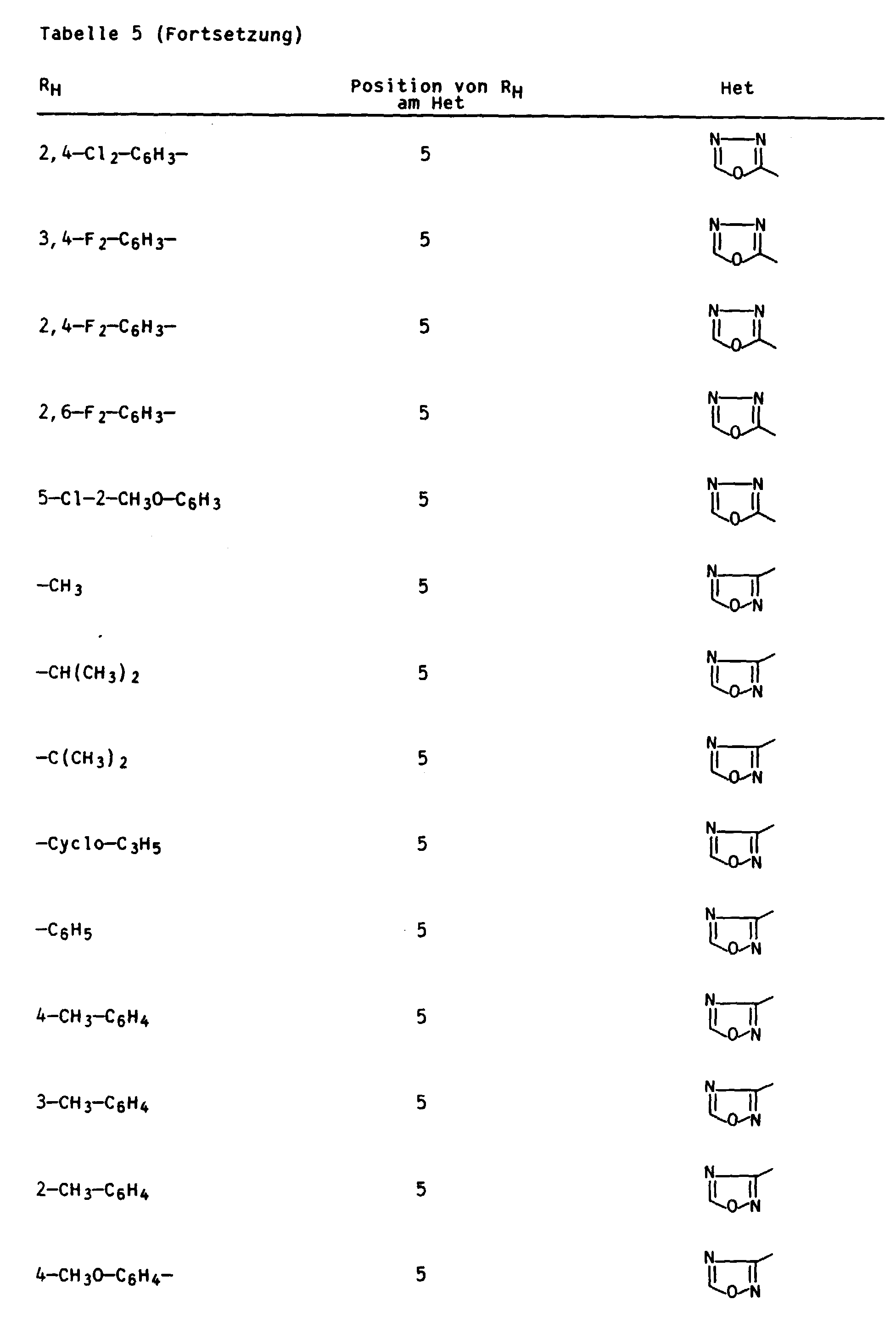

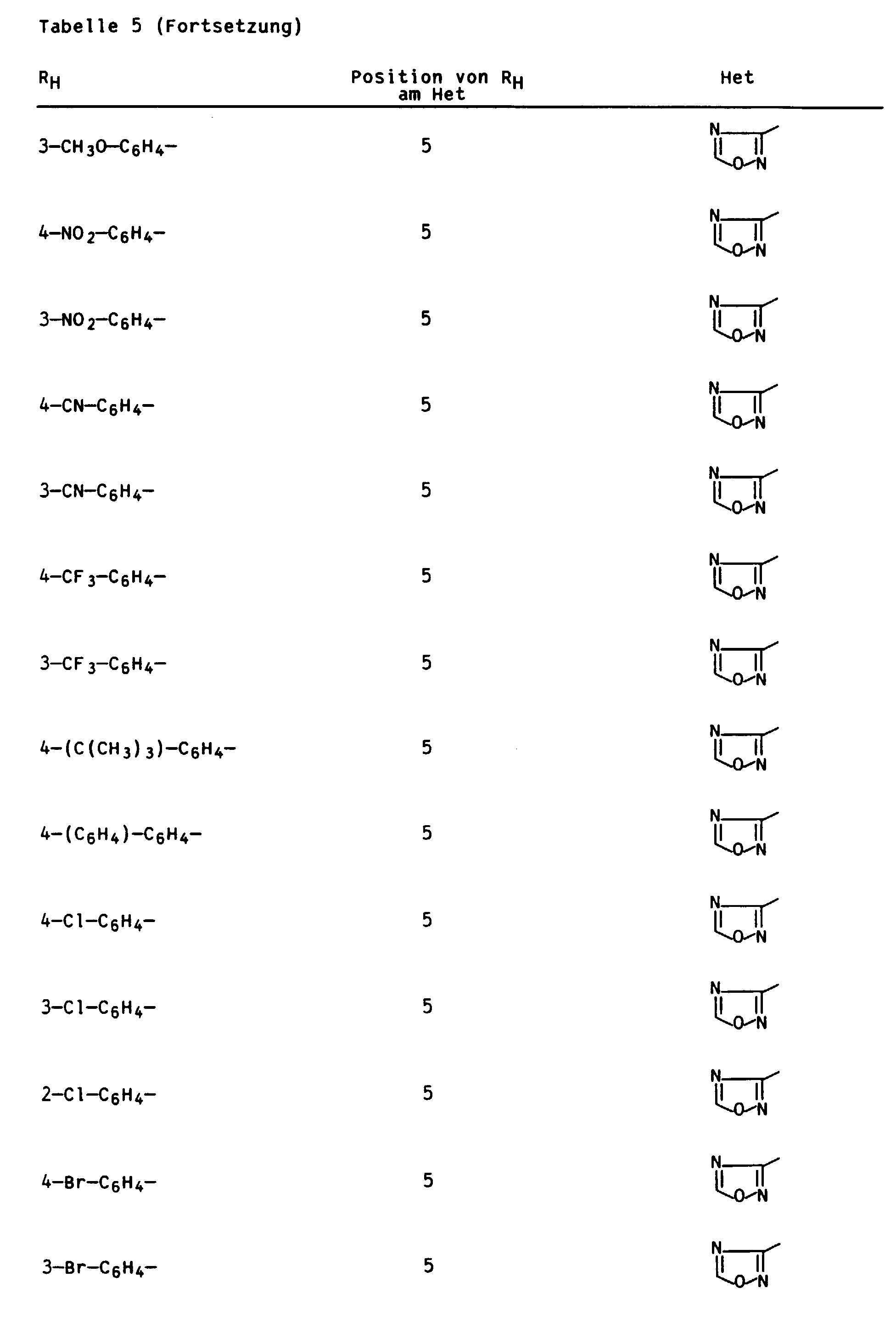

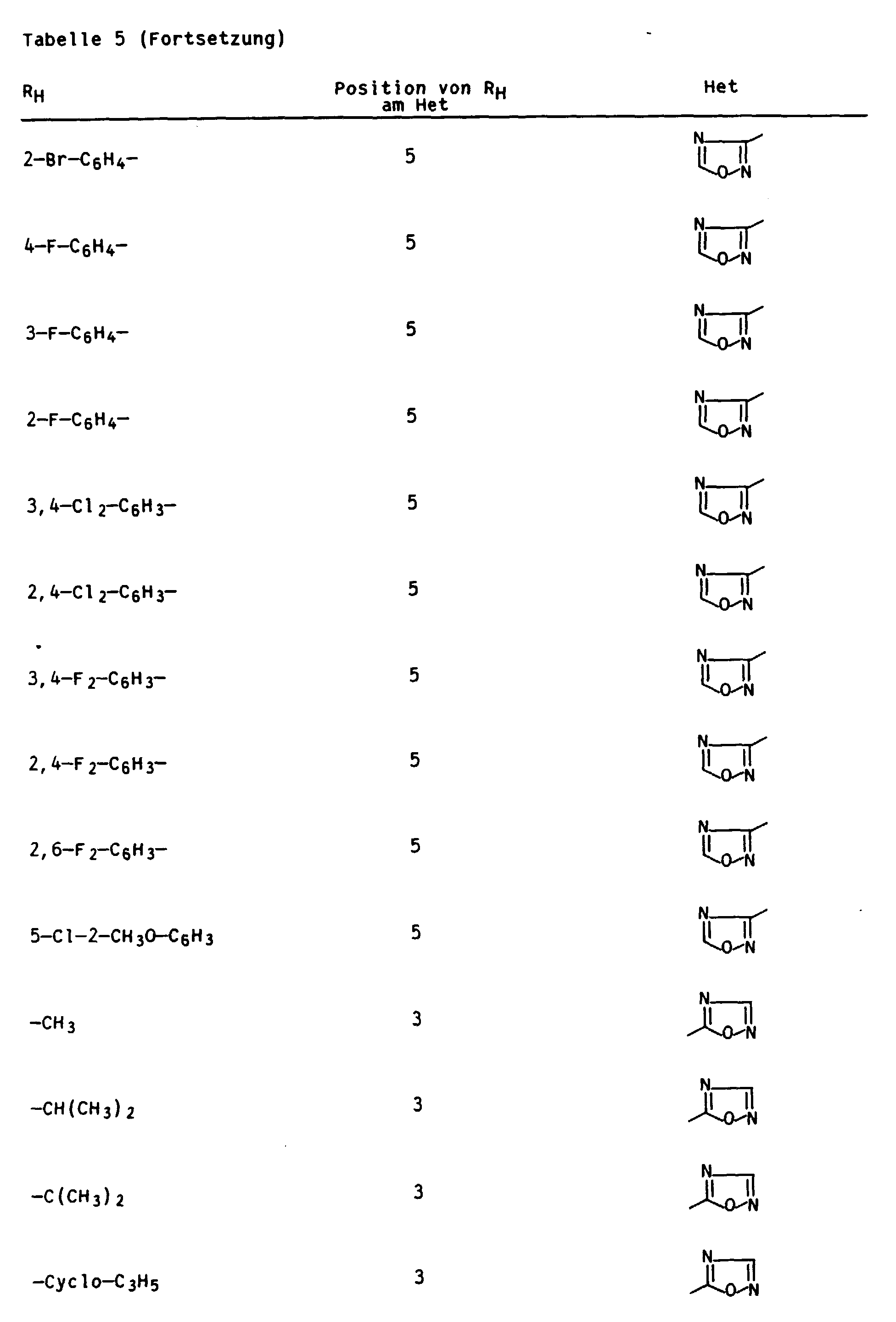

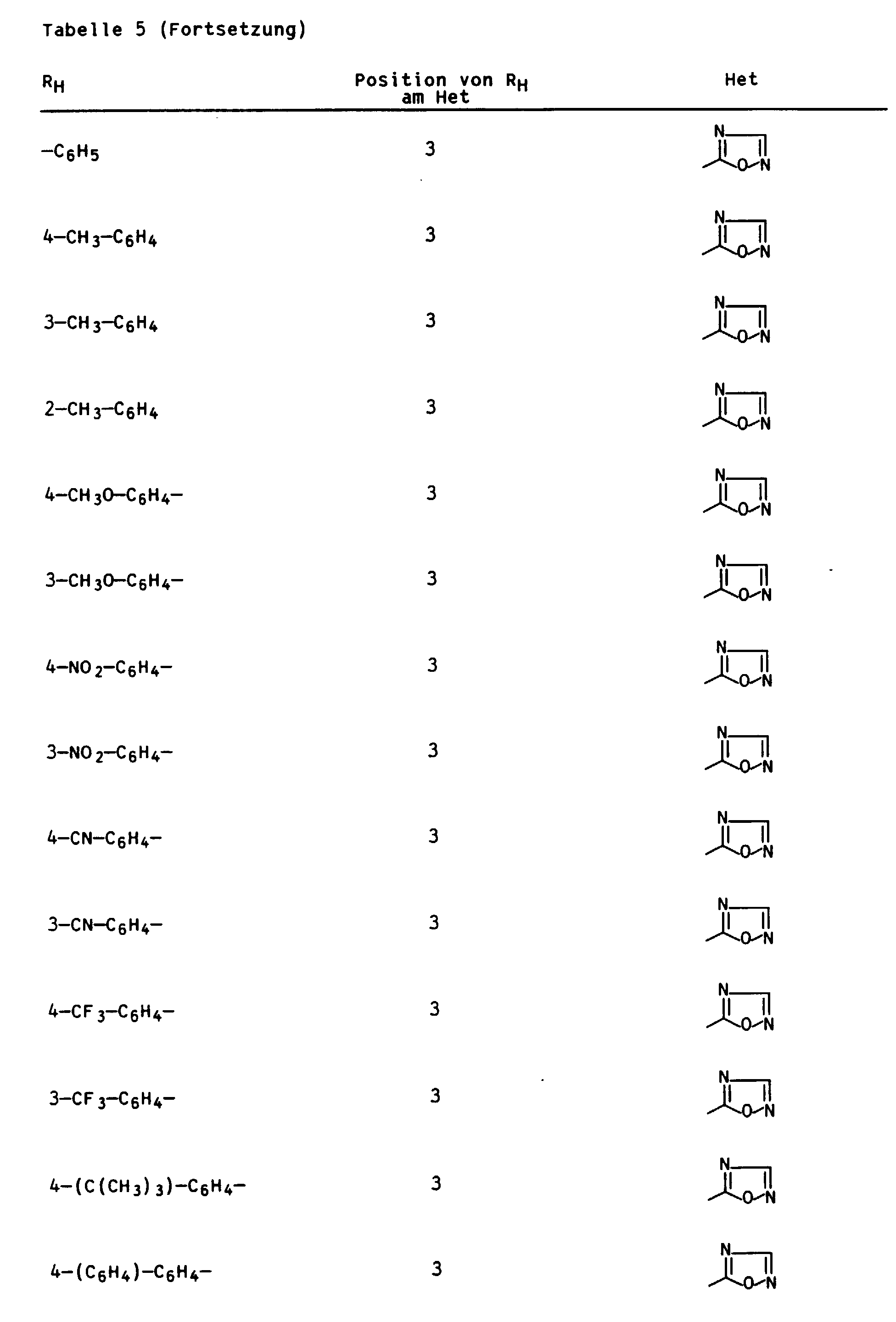

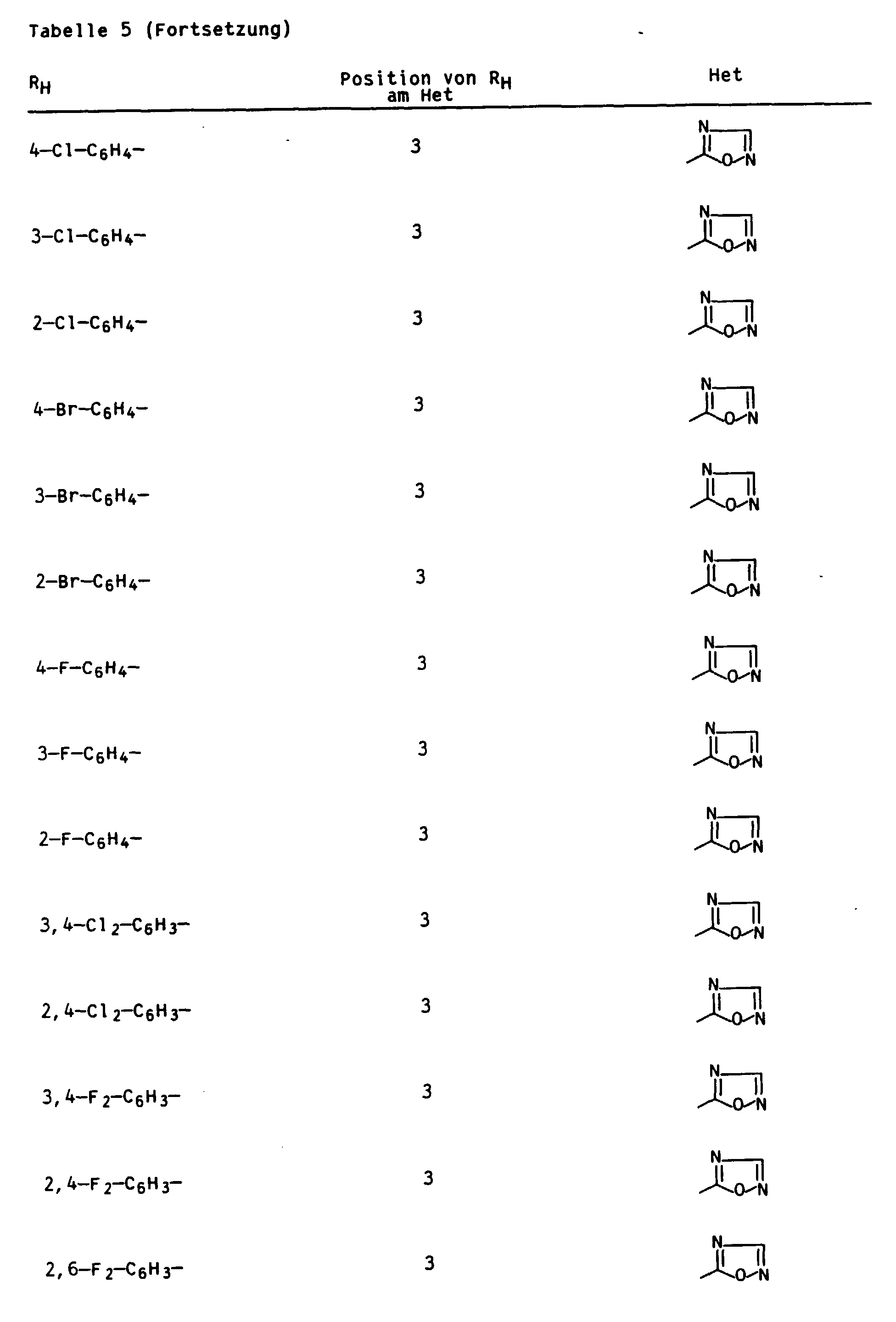

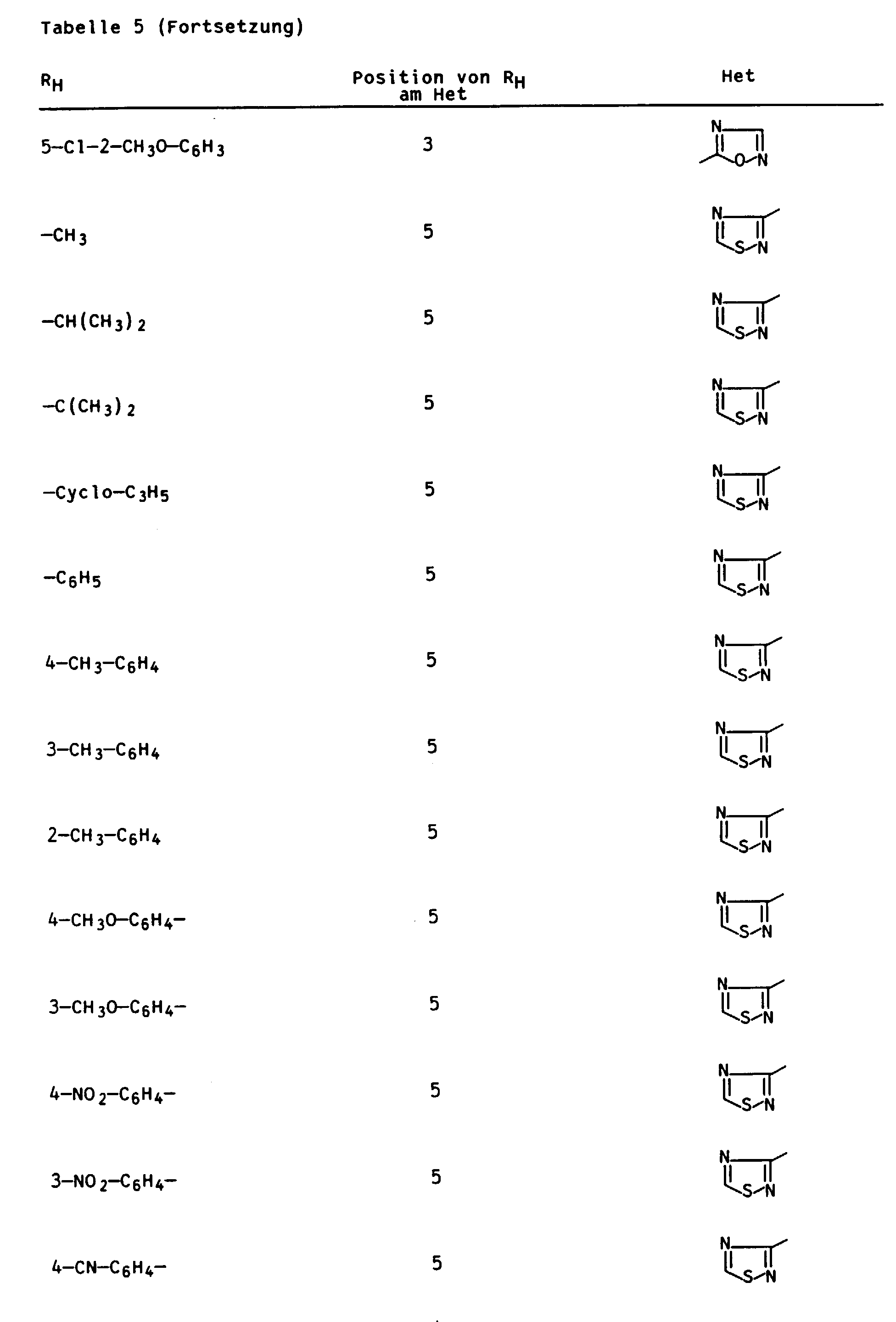

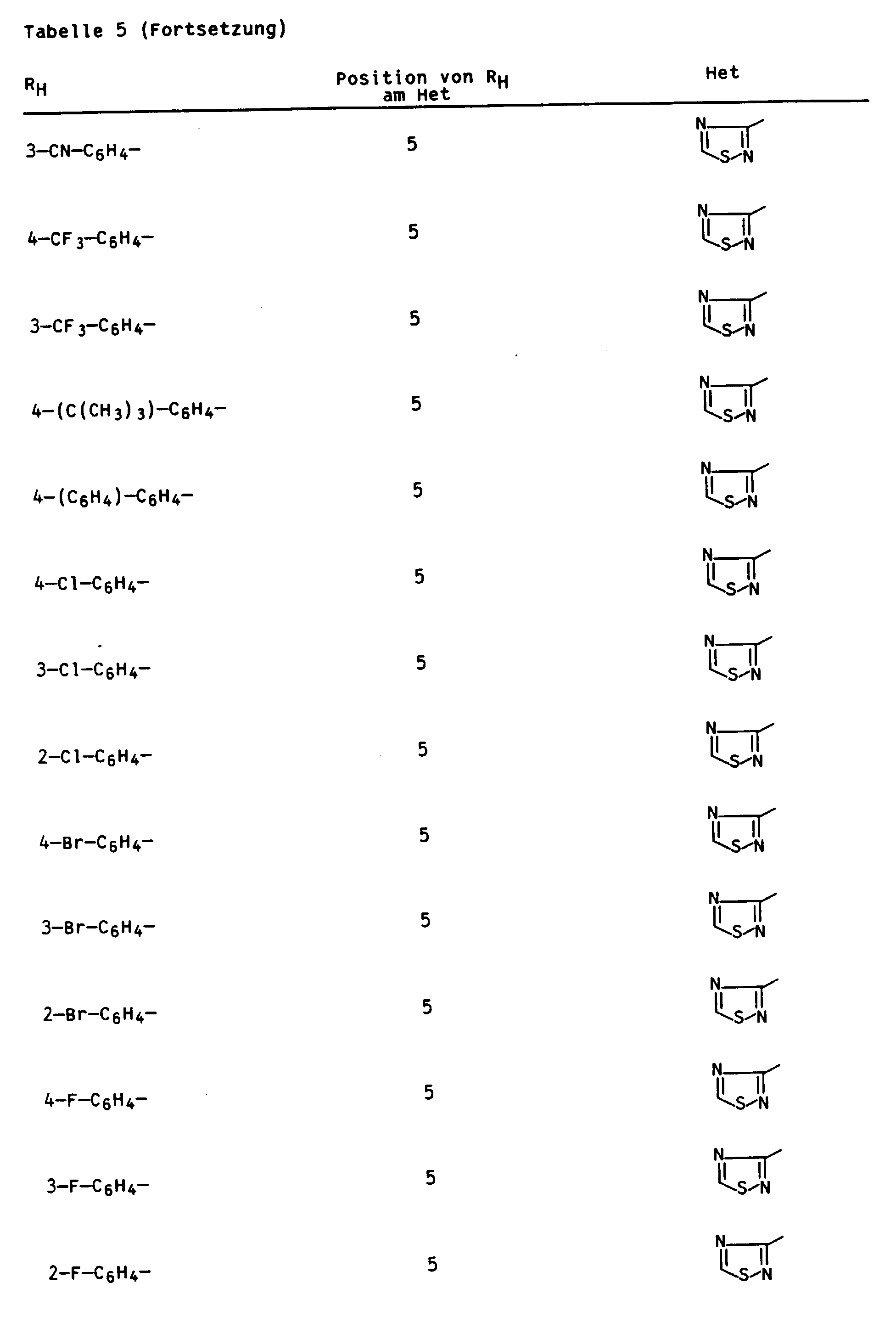

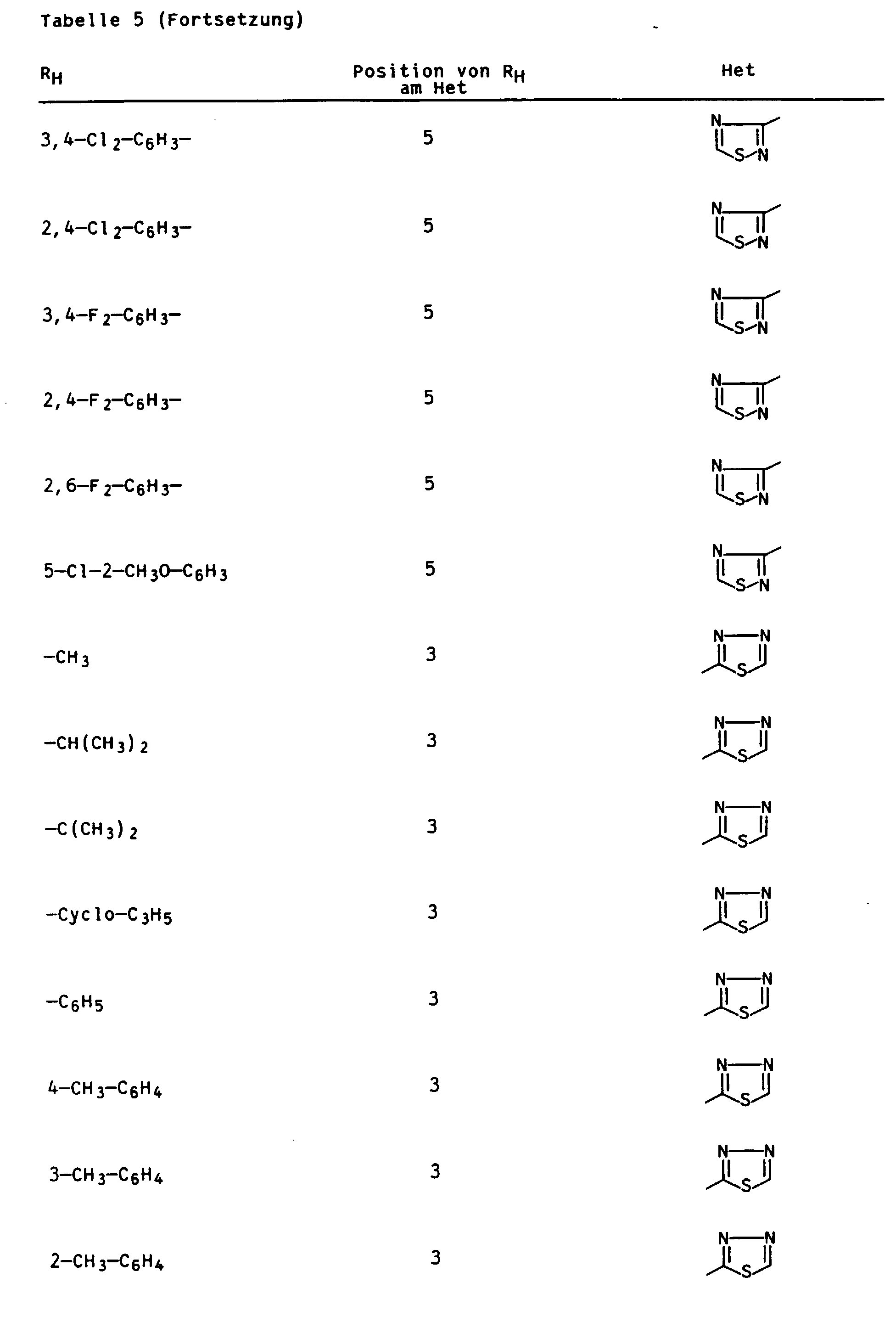

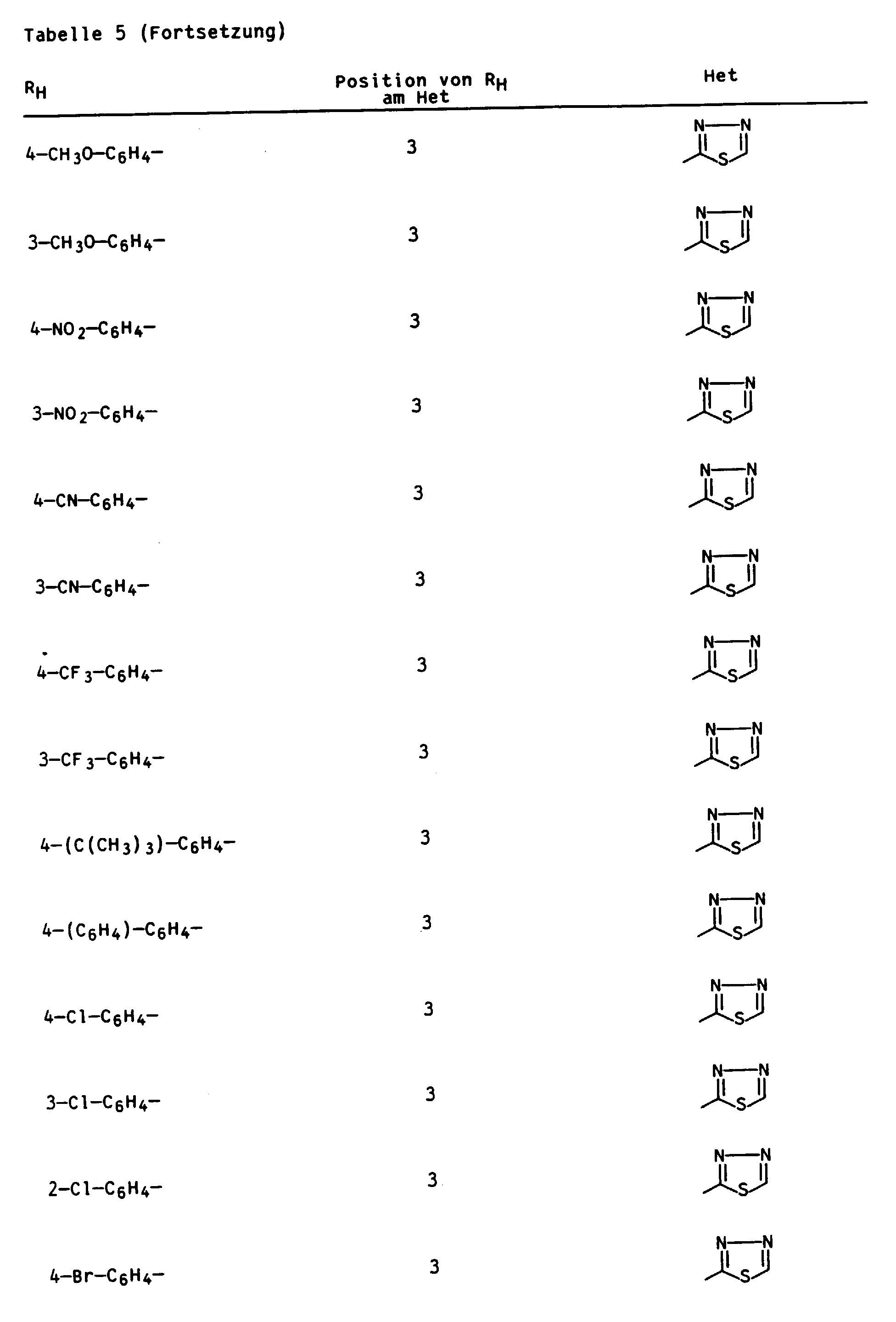

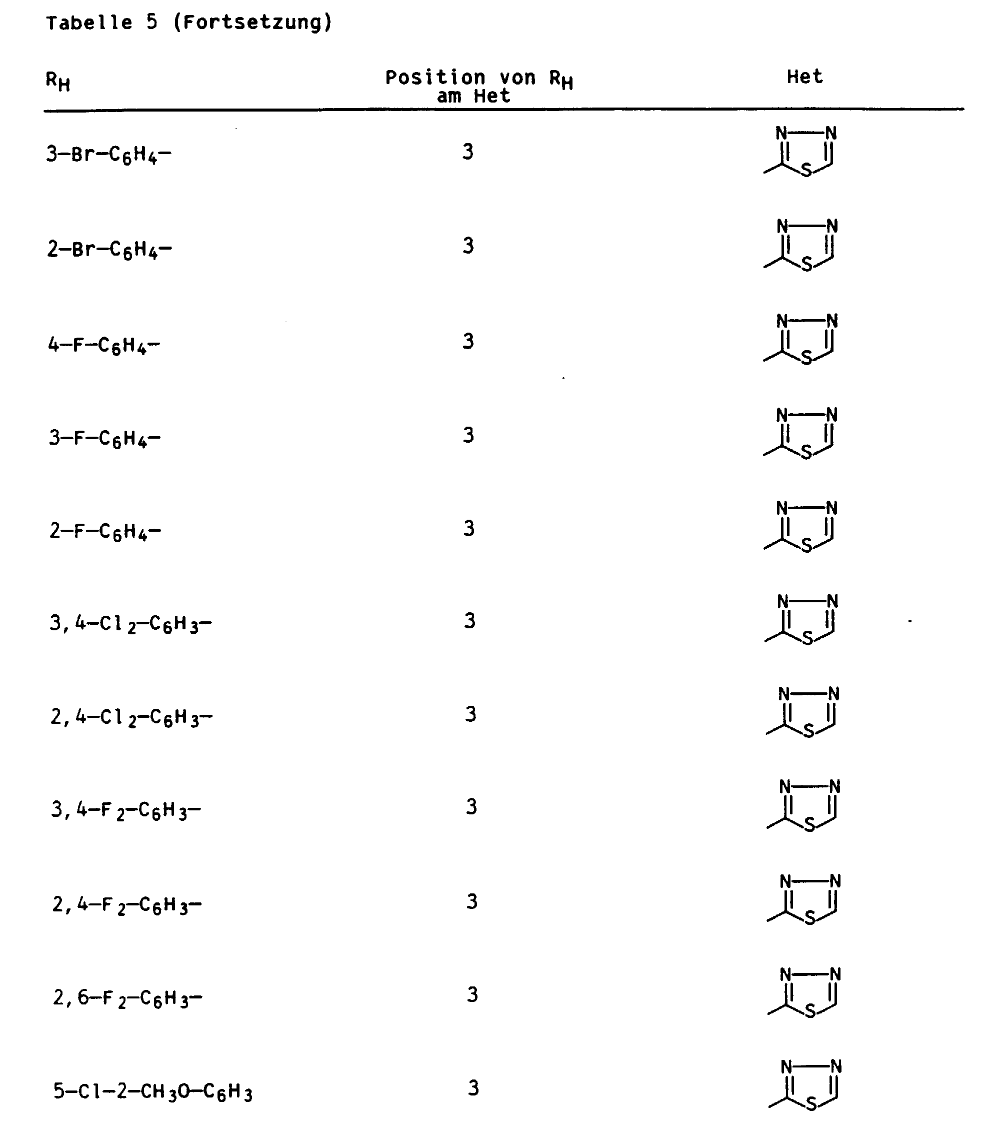

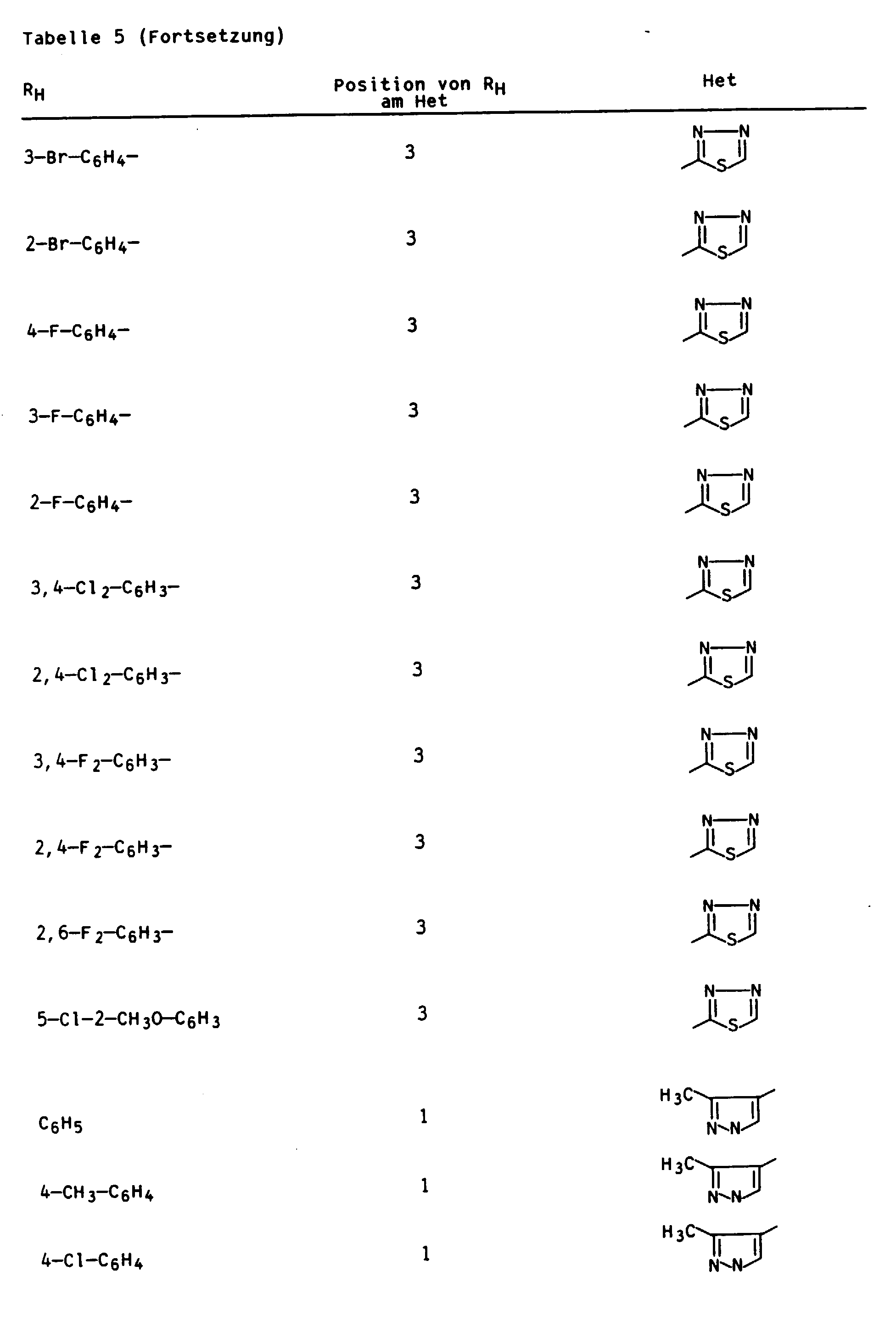

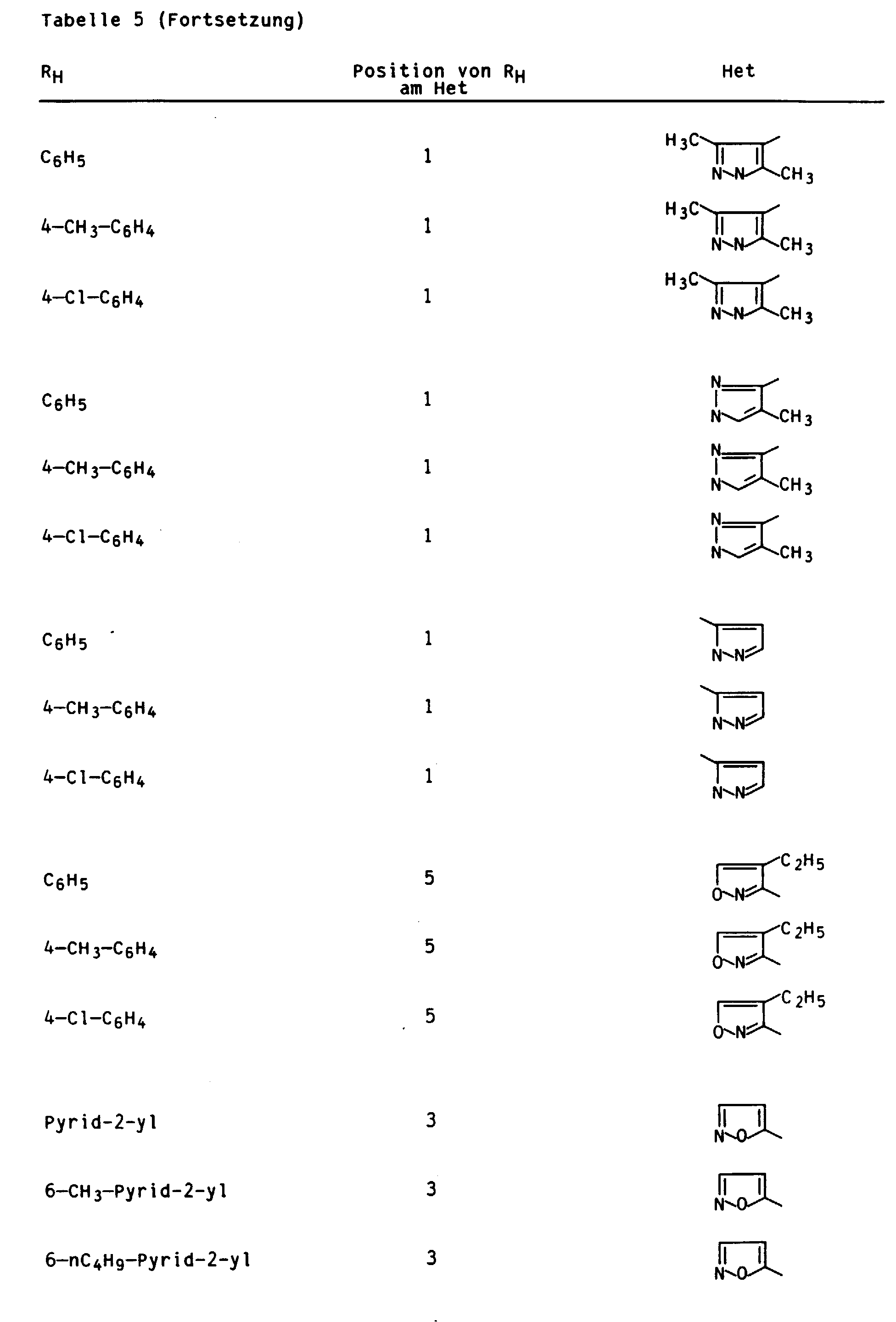

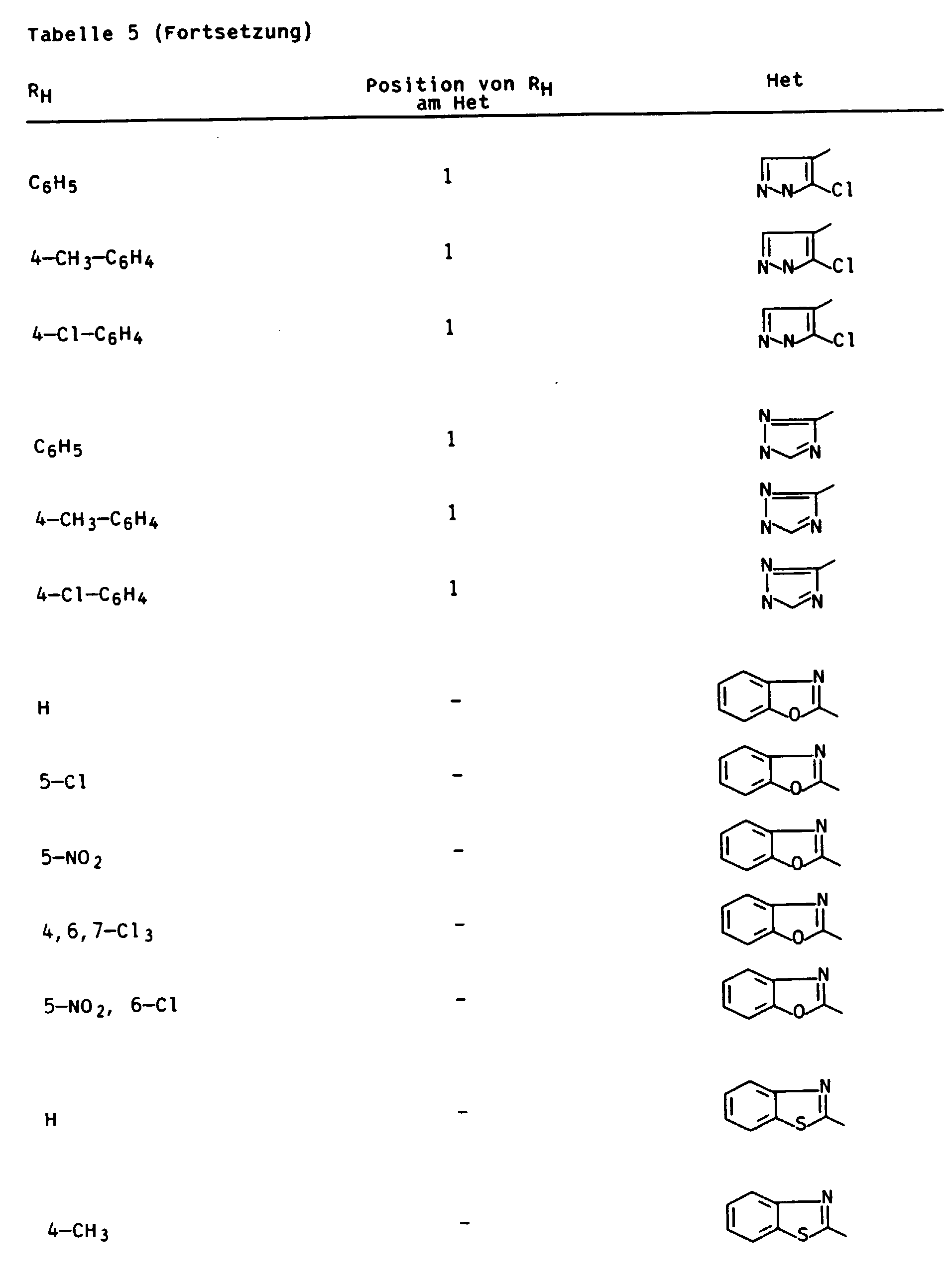

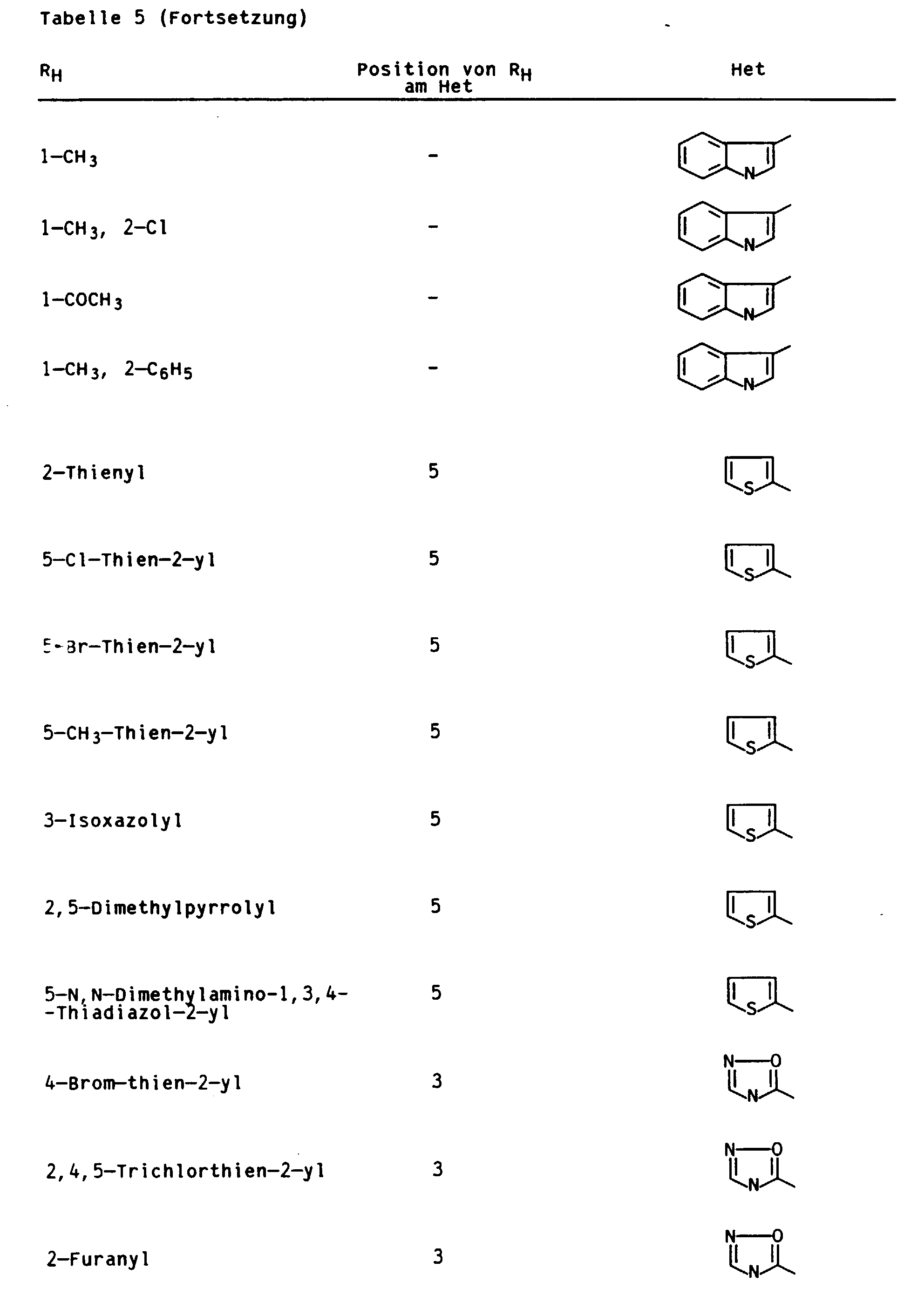

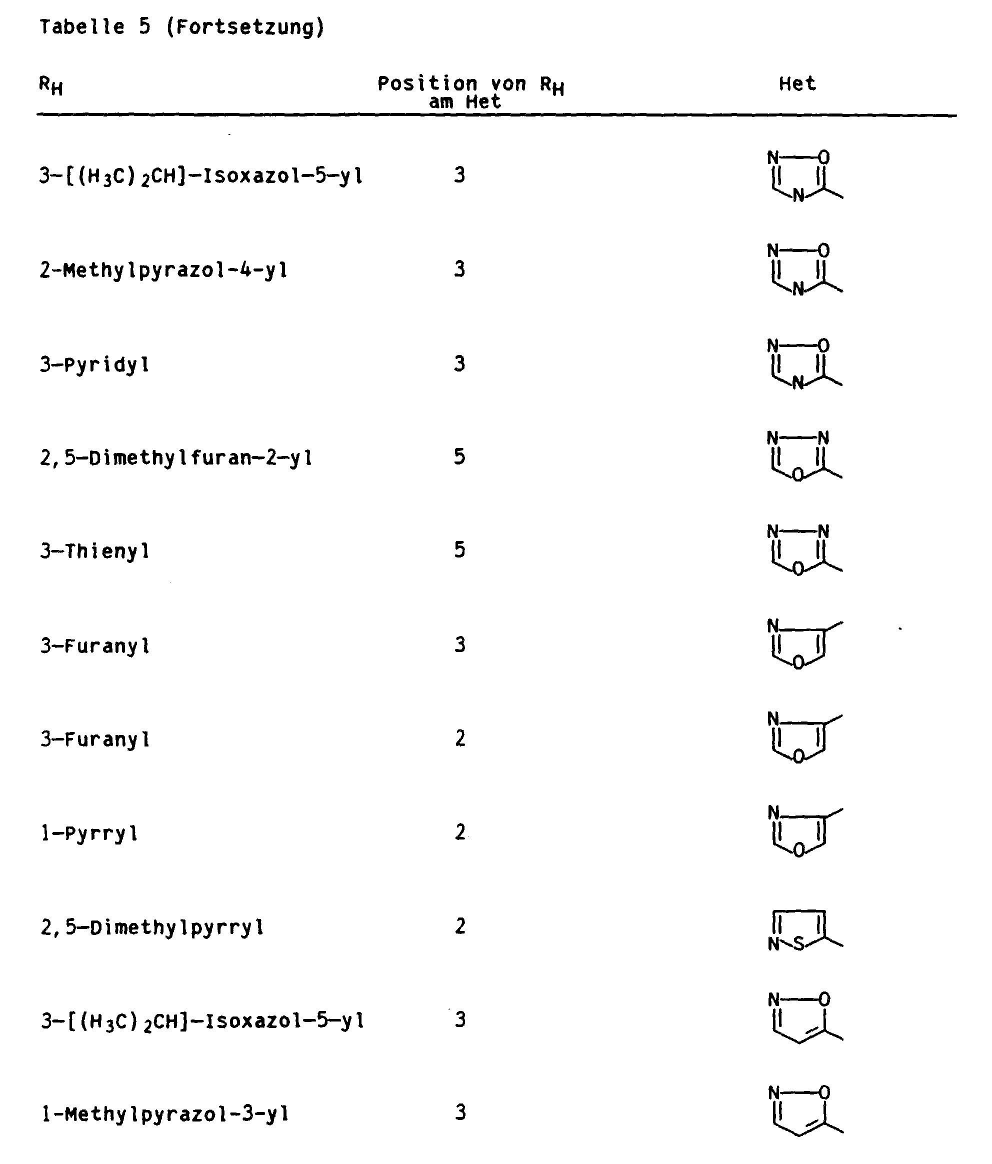

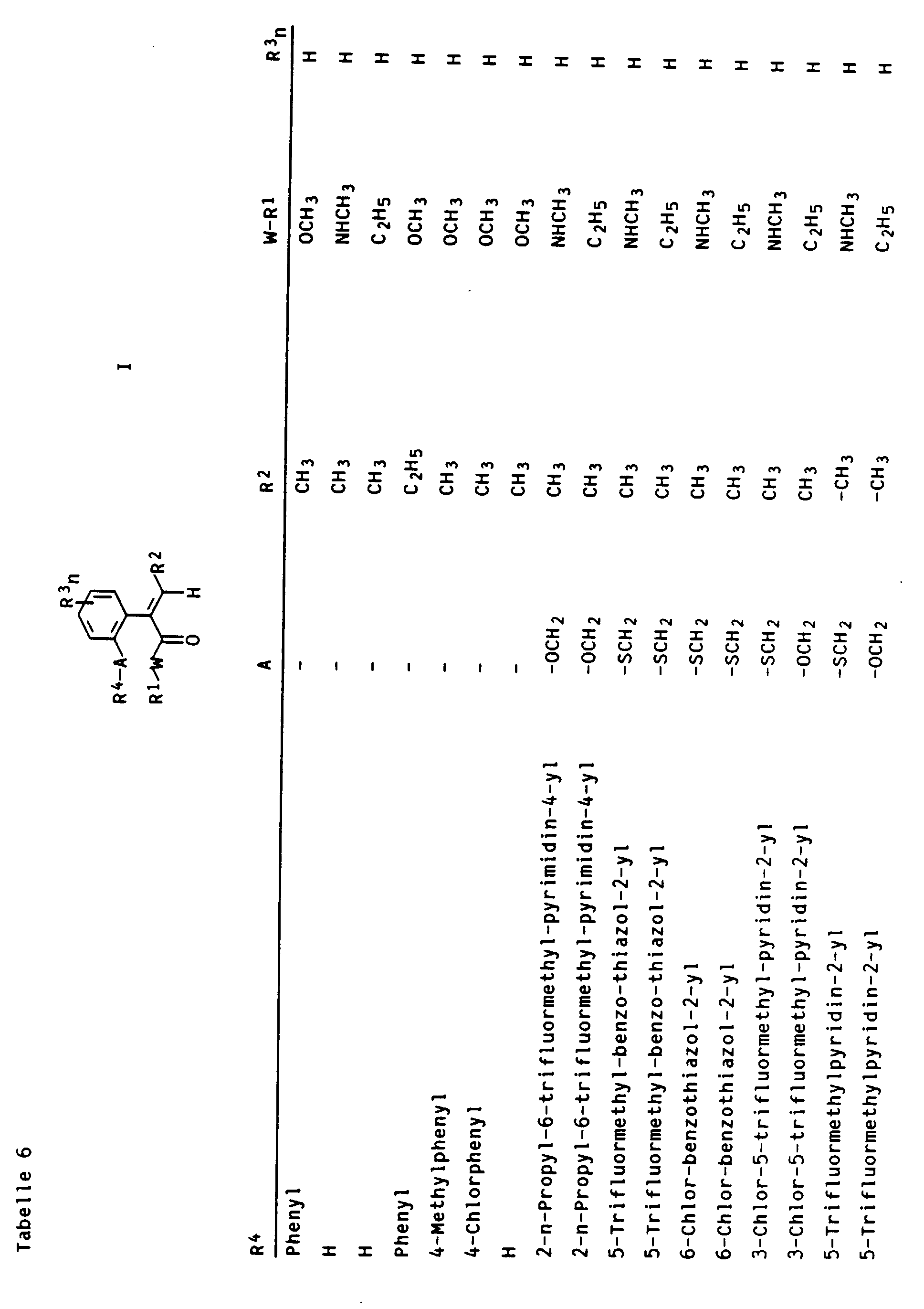

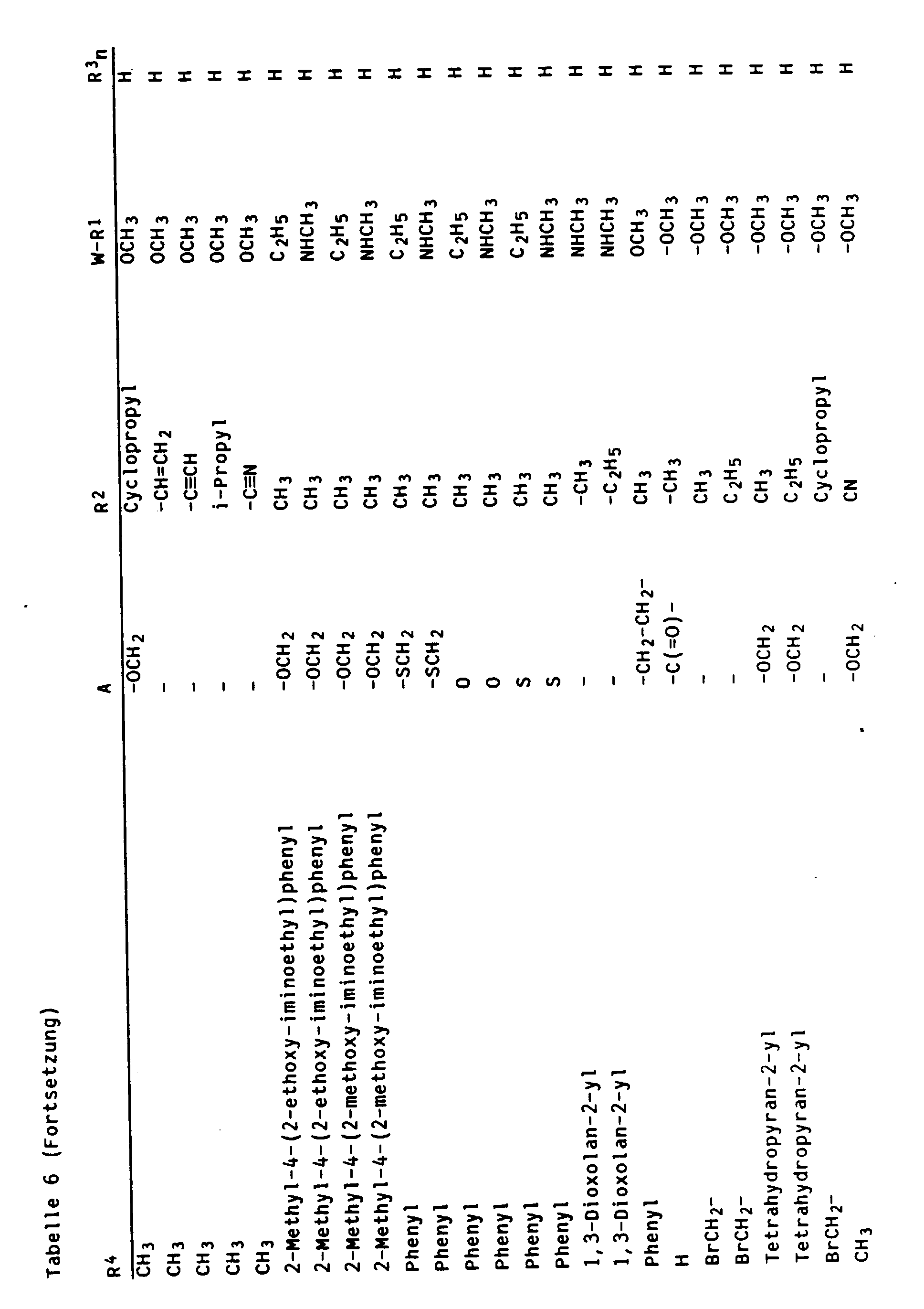

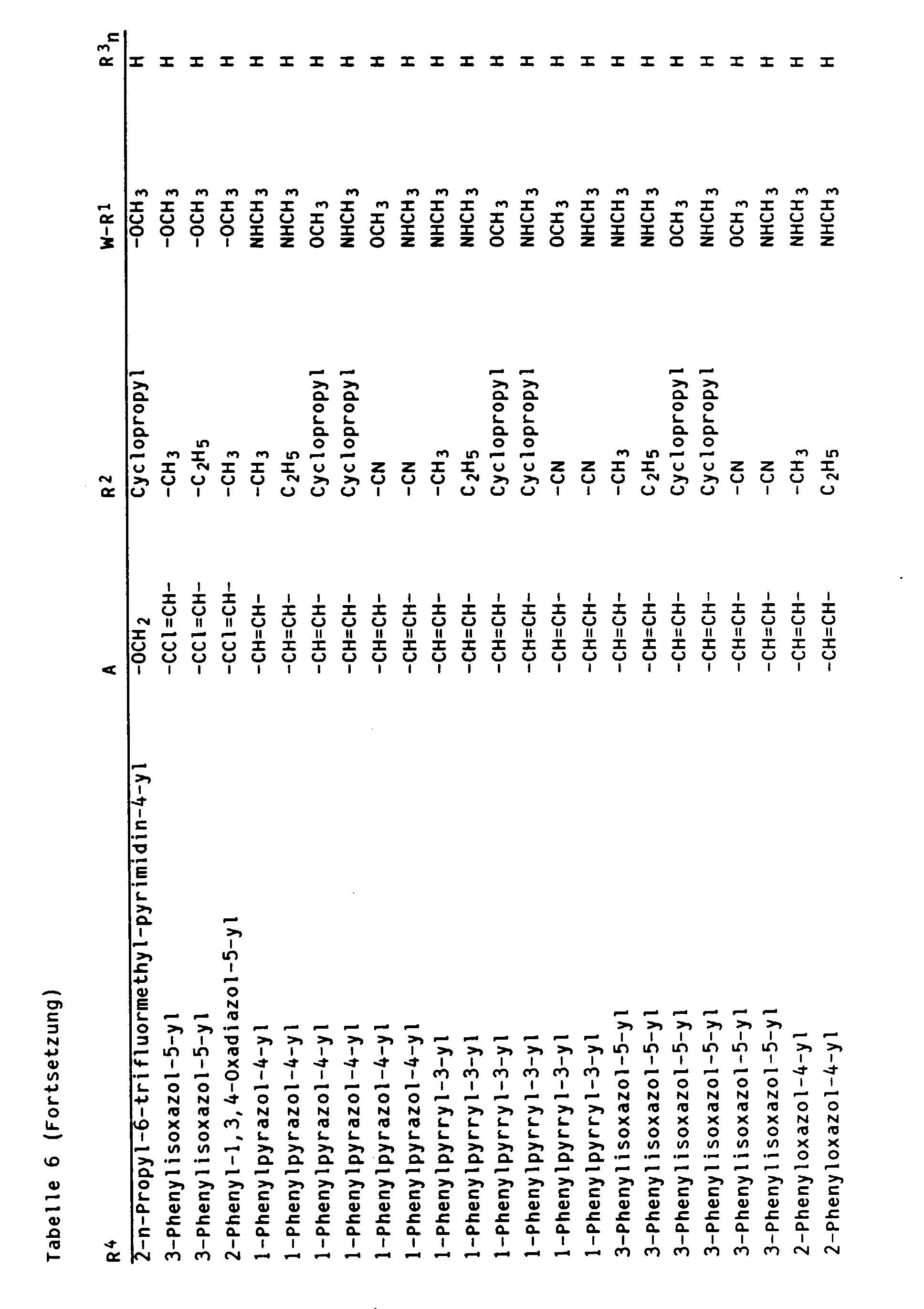

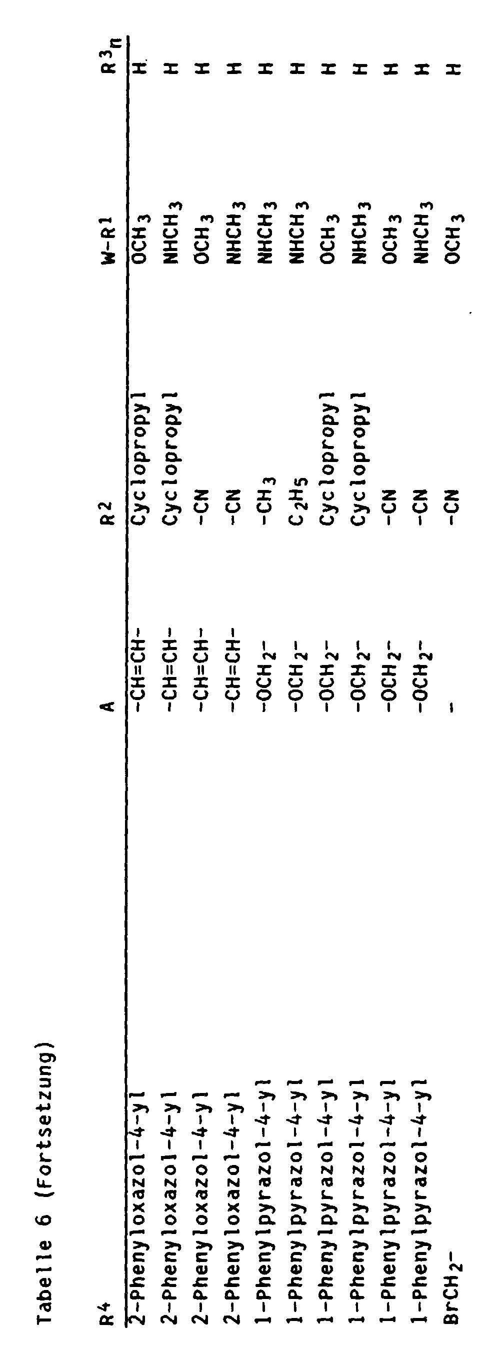

Die ortho-substituierten Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Gercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wriksamen Menge der Wirkstoffe behandelt. Die Anwendung erfolgt vor oder nach der Infektion der Materialien, Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergröperung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
   Ferridimethyldithiocarbamat,
   Zinkdimethyldithlocarbamat,
   Zinkethylenbisdithiocarbamat,
   Manganethylenbisdithiocarbamat,
   Mangan-Zink-ethylendiamin-bis-dithiocarbamat,
   Tetramethylthiuramdisulfide,
   Ammoniak-Komplex von Zink-(N,N-ethylen-bis-dithiocarbamat),
   Ammoniak-Komplex von Zink-(N,N'-propylen-bis-dithiocarbamat),
   Zink-(N,N'-propylen-bis-dithiocarbamat),
   N,N'-Polypropylen-bis-(thiocarbamoyl)-disulfid;
Nitroderivate, wie
   Dinitro-(1-methylheptyl)-phenylcrotonat,
   2-sec-Butyl-4,6-dinitrophenyl-3,3-dimethylacrylat,
   2-sec-Butyl-4,6-dinitrophenyl-isopropylcarbonat,
   5-Nitro-isophthalsäure-di-isopropylester;
heterocyclische Substanzen, wie
   2-Heptadecyl-2-imidazolin-acetat,
   2,4-Dichlor-6-(o-chloranilino)-s-triazin,
   O,O-Diethyl-phthalimidophosphonothioat,
   5-Amino-1-[bis-(dimethylamino)-phosphinyl]-3-phenyl-1,2,4-triazol,
   2,3-Dicyano-1,4-dithioanthrachinon,
   2-Thio-1,3-dithiolo[4,5-b]chinoxalin,
   1-(Butylcarbamoyl)-2-benzimidazol-carbaminsäuremethylester,
   2-Methoxycarbonylamino-benzimidazol,
   2-(Furyl-(2))-benzimidazol,
   2-(Thiazolyl-(4))-benzimidazol,
   N-(1,1,2,2-Tetrachlorethylthio)-tetrahydrophthalimid,
   N-Trichlormethylthio-tetrahydrophthalimid,
   N-Trichlormethylthio-phthalimid,
N-Dichlorfluormethylthio-N',N'-dimethyl-N-phenyl-schwefelsäurediamid,
   5-Ethoxy-3-trichlormethyl-1,2,3-thiadiazol,
   2-Rhodanmethylthiobenzthiazol,
   1,4-Dichlor-2,5-dimethoxybenzol,
   4-(2-Chlorphenylhydrazono)-3-methyl-5-isoxazolon,
   Pyridin-2-thio-1-oxid,
   8-Hydroxychinolin bzw. dessen Kupfersalz,
   2,3-Dihydro-5-carboxanilido-6-methyl-1,4-oxathiin,
   2,3-Dihydro-5-carboxanilido-6-methyl-1,4-oxathlin-4,4-dioxid,
   2-Methyl-5,6-dihydro-4H-pyran-3-carbonsäure-anilid,
   2-Methyl-furan-3-carbonsäureanilid,
   2,5-Dimethyl-furan-3-carbonsäureanilid,
   2,4,5-Trimethyl-furan-3-carbonsäureanilid,
   2,5-Dimethyl-furan-3-carbonsäurecyclohexylamid,
   N-Cyclohexyl-N-methoxy-2,5-dimethyl-furan-3-carbonsäureamid,
   2-Methyl-benzoesäure-anilid,
   2-Iod-benzoesäure-anilid,
   N-Formyl-N-morpholin-2,2,2-trichlorethylacetal,
   Piperazin-1,4-diylbis-(1-(2,2,2-trichlor-ethyl)-formamid,
   1-(3,4-Dichloranilino)-1-formylamino-2,2,2-trichlorethan,
   2,6-Dimethyl-N-tridecyl-morpholin bzw. dessen Salze,
   2,6-Dimethyl-N-cyclododecyl-morpholin bzw. dessen Salze,
   N-[3-(p-tert.-Butylphenyl)-2-methylpropyl]-cis-2,6-dimethylmorpholin, N-[3-(p-tert.-Butylphenyl)-2-methylpropyl]-piperidin,
   1-[2-(2,4-Dichlorphenyl)-4-ethyl-1,3-dioxolan-2-yl-ethyl]-1H-1,2,4-triazol
   1-[2-(2,4-Dichlorphenyl)-4-n-propyl-1,3-dioxolan-2-yl-ethyl]-1H-1, 2,4-triazol,
   N-(n-Propyl)-N-(2,4,6-trichlorphenoxyethyl)-N'-imidazol-yl-harnstoff,
   1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-l-yl)-2-butanol, α-(2-Chlorphenyl)-α-(4-chlorphenyl)-5-pyrimidin-methanol,
   5-Butyl-2-dimethylamino-4-hydroxy-6-methyl-pyrimidin,
   Bis-(p-chlorphenyl)-3-pyridinmethanol,
   1,2-Bis-(3-ethoxycarbonyl-2-thioureido)-benzol,
   1,2-Bis-(3-methoxycarbonyl-2-thioureido)-benzol,
sowie verschiedene Fungizide, wie
   Dodecylguanidinacetat,
   3-[3-(3,5-Dimethyl-2-oxycyclohexyl)-2-hydroxyethyl]-glutarimid,
   Hexachlorbenzol,
   DL-Methyl-N-(2,6-dimethyl-phenyl)-N-furoyl(2)-alaninat,
   DL-N-(2,6-Dimethyl-phenyl)-N-(2'-methoxyacetyl)-alanin-methylester,
   N-(2,6-Dimethylphenyl)-N-chloracetyl-D,L-2-aminobutyrolacton,
   DL-N-(2,6-Dimethylphenyl)-N-(phenylacetyl)-alaninmethylester,
   5-Methyl-5-vinyl-3-(3,5-dichlorphenyl)-2,4-dioxo-1,3-oxazolidin,
   3-[3,5-Dichlorphenyl(-5-methyl-5-methoxymethyl]-1,3-oxazolidin-2,4-dion,
   3-(3,5-Dichlorphenyl)-1-isopropylcarbamoylhydantoin,
   N-(3,5-Dichlorphenyl)-1,2-dimethylcyclopropan-1,2-dicarbonsäureimid,
   2-Cyano-[N-(ethylaminocarbonyl)-2-methoximino]-acetamid,
   1-[2-(2,4-Dichlorphenyl)-pentyl]-1H-1,2,4-triazol,
   2,4-Difluor-α-(1H-1,2,4-triazolyl-1-methyl)-benzhydrylalkohol,
   N-(3-Chlor-2,6-dinitro-4-trifluormethyl-phenyl)-5-trifluormethyl-3--chlor-2-aminopyridin,
   1-((bis-(4-Fluorphenyl)-methylsilyl)-methyl)-1H-1,2,4-triazol.

Die Verbindungen der Formel I' sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile der Verbindung Nr. 11 werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile der Verbindung Nr. 13 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile der Verbindung Nr. 31 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile der Verbindung Nr. 32 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile der Verbindung Nr. 11 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile der Verbindung Nr. 13 mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile der Verbindung Nr. 31 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile des Wirkstoffs Nr. 32 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### Beispiel 1

Herstellung von 1-Brom-2-methoxymethyl-benzol 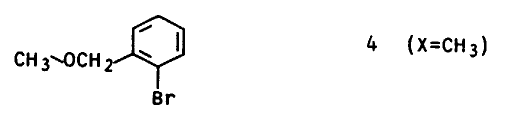 685 g (2,74 mol) o-Brombenzylbromid wurden zusammen mit 2,74 mol einer 30 %igen Natriummethylat-Lösung in Methanol 15 Stunden unter Rückfluß erhitzt. Nach Abkühlen auf Raumtemperatur wurde eingeengt, in Essigester aufgenommen und mit Wasser gewaschen. Nach dem Trocknen der organischen Phase über Natriumsulfat wurde erneut eingeengt. Man erhielt 478,2 g (87 %) Produkt als farblose Flüssigkeit.

¹H-NMR (in CDCl₃): 3,5 ppm (s,3H), 4,58 ppm (s,2H), 7,08-7,6 ppm (m,4H).

### Beispiel 2

Herstellung von 2-Methoxymethyl-phenylglyoxylsäuremethylester

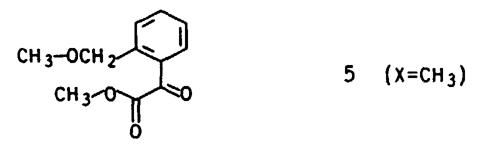

Zu einer aus 12,0 g (0,46 mol) Magnesiumspänen und 99,5 g (0,5 mol) 1-Brom-2-methoxymethyl-benzol in 500 ml THF hergestellten Grignardlösung wurden 500 ml etherische 1 M Zinkchloridlösung getropft. Anschließend wurde 1 Stunde unter Rückfluß erhitzt und eine Lösung von 61,2 g (0,5 mol) Oxalsäuremethylesterchlorid in 100 ml THF bei einer Innentemperatur von (-10)°C zugetropft. Nach Erwärmung auf ca. 20°C rührte man noch 20 Stunden weiter und hydrolysierte dann mit gesättigter Ammoniumchloridlösung. Nach dem Extrahieren mit Ether wurden die vereinigten Etherphasen mit Wasser gewaschen, getrocknet und eingeengt. Man erhielt 60 g Produkt (57,7 %) als eine leichtbewegliche Flüssigkeit.

¹H-NMR (in CDCl₃): 3,38 ppm (s,3H), 3,98 ppm (s,3H), 4,8 ppm (s,2H), 7,3-7,7 ppm (m,4H).

### Beispiel 3

Herstellung von α-(2-Methoxymethyl-phenyl)-β-methylacrylsäuremethylester

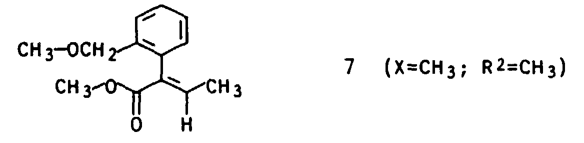

37,1 g (100 mmol) Ethyltriphenylphosphoniumbromid wurden unter Stickstoff in 300 ml absolutem Tetrahydrofuran vorgelegt. Bei 5°C wurden 11,2 g (0,1 mol) Kalium-t-butanolat zugegeben. Nach einer Stunde Rühren bei 5°C wurden 20,8 g (100 mmol) 2-Methoxymethylphenylglyoxylsäuremethylester in 100 ml Tetrahydrofuran zugetropft. Man rührte eine Stunde bei 5°C und eine Stunde bei 25°C, versetzte mit 200 ml Wasser und extrahierte mit Methylenchlorid. Nach Waschen der vereinigten organischen Phasen mit Wasser und Trocknen über Natriumsulfat wurde das Lösungsmittel im Vakuum entfernt. Der Rückstand wurde über eine Kieselgel-Säule (Methyl-tert-butylether/n-Hexan = 1/2) chromatographisch gereinigt. Man erhielt 17,2 g (78 %) des obengenannten Esters als Öl (Z/E-Isomer = 1/9) (Verb.Nr. 25).

¹H-NMR (in CDCl₃): 1,8 ppm (d,3H), 3,3 ppm (s,3H), 3,7 ppm (s,3H), 4,3 ppm (d,2H), 7,05 ppm (m,1H), 7,2-7,6 ppm (m,4H).

### Beispiel 4

Herstellung von α-(2-Brommethyl-phenyl)-β-methylacrylsäuremethylester (Verb.Nr. 24)

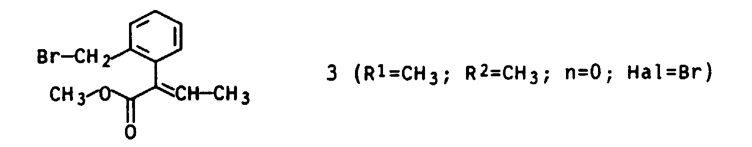

31 g (140 mmol) α-(2-Methoxymethyl-phenyl)-β-methylacryl-säuremethylester in 500 ml CH₂Cl₂ wurden bei 0°C tropfenweise mit 105,3 ml (140 mmol) einer 1,33 M Bortribromid-Lösung in Methylenchlorid versetzt und anschließend 2 Stunden unter Rückfluß erhitzt. Danach versetzte man mit 25 ml Methanol, rührte weitere 90 Minuten bei Raumtemperatur nach, hydrolysierte unter Eiskühlung mit Wasser, wusch mit 3 %iger Natronlauge neutral und extrahierte die wäßrige Phase mit Methylenchlorid. Die vereinigten organischen Phasen wurden dann mit Wasser gewaschen, getrocknet und eingeengt. Die ausgefallenen Kristalle wurden abgetrennt, mit wenig n-Hexan gewaschen und getrocknet. Die Filtrate wurden eingeengt und über eine Kieselgelsäule chromatographisch gereinigt (Cyclohexan/MTBE : 8/1). Man erhielt insgesamt 31 g Produkt in Form hellgelber Kristalle.

¹H-NMR (in CDCl₃): 1,65 ppm (d,3H) 3,7 ppm (s,3H), 4,28 ppm (d,2H), 7,05 ppm (m,1H), 7,2-7,6 ppm (m,4H).

### Beispiel 5

Herstellung von α-(2-Methoxymethyl-phenyl)-β-methylacrylsäure (Verbindung Nr. 1)

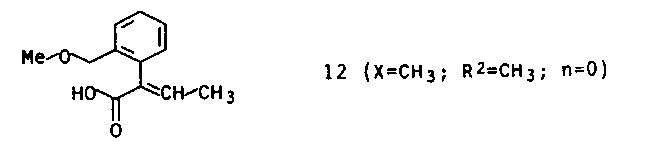

4,4 g (20 mmol) α-(2-Methoxymethyl-phenyl)-β-methylacrylsäuremethylester, wurden in 80 ml 1 N Kaliumhydroxid-Lösung bei 80°C für 30 Minuten gerührt. Es wurde auf Raumtemperatur abgekühlt und zweimal mit je 50 ml Methyl-t.-butylether extrahiert. Die verbleibende wäßrige Phase wurde mit 10 %iger Salzsäure auf pH 1 eingestellt. Anschließend wurde dreimal mit je 50 ml Dichlormethan extrahiert. Die vereinigten Dichlormethanextrakte wurden dreimal mit Wasser gewaschen, über Natriumsulfat getrocknet und eingeengt.

Man erhielt 3,8 g (92 %) der Titelverbindung in Form farbloser Kristalle (Fp.: 67-69°C).

IR (cm⁻¹): 1687, 1631, 1285, 1112, 968, 772, 754.

### Beispiel 6

Herstellung von α-(2-Methoxymethyl-phenyl)-β-methylacrylsäurechlorid

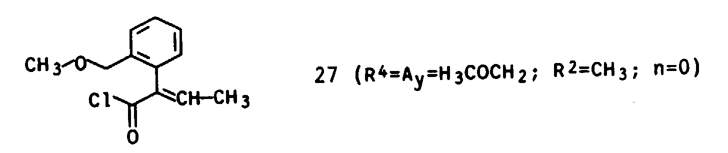

4,9 g (24 mmol) α-(2-Methoxymethyl-phenyl)-β-methylacrylsäure und 2,5 g Pyridin wurden in 40 ml Ether vorgelegt. Bei 0 bis 5°C wurden 3,7 g Thionylchlorid zugetropft. Es wurde 3 Stunden bei ca. 20°C gerührt. Anschließend wurde abfiltriert und der Rückstand mit Diethylether gewaschen. Das Filtrat wurde mit der Ether-Phase vereinigt. Nach dem Entfernen des Lösungsmittels erhielt man 5,2 g (96,5 %) der Titelverbindung als farbloses Öl. Das Rohprodukt kann ohne zusätzliche Reinigung für weitere Umsetzungen eingesetzt werden.

### Beispiel 7

Herstellung von α-(2-Methoxymethyl-phenyl)-β-methylacrylsäure-N-methylamid (Verb.Nr. 22)

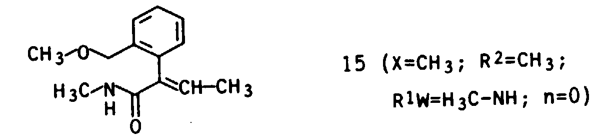

Zu 5,2 g (23 mmol) α-(2-Methoxymethyl-phenyl)-β-methylacrylsäurechlorid in 30 ml Dichlormethan wurden 1,7 g Methylaminhydrochlorid gegeben und anschließend bei 0 bis 5°C 4 g Pyridin zugetropft. Es wurde weitere 15 Stunden bei etwa 20°C gerührt. Das Reaktionsgemisch wurde mit 20 ml Dichlormethan verdünnt und mit Wasser gewaschen. Die organische Phase wurde über Natriumsulfat getrocknet und eingeengt. Das Rohprodukt wurde an Kieselgel mit Methyl-t.-butylether/ Cyclohexan (5/1) chromatographiert. Nach dem Entfernen des Lösungsmittels erhielt man 1,5 g (30 %) der Titelverbindung in Form farbloser Kristalle (Fp.: 58°C).

### Beispiel 8

Herstellung von 2-Methoxymethyl-benzylphosphonsäure-dimethylester

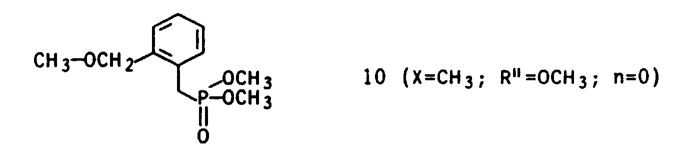

Zu 332 g [1,2-Bis-(brommethyl)]-benzol I in 1400 ml Toluol wurde bei 60°C solange eine 30 % ige Natriummethylat-Lösung in Methanol getropft, bis ein optimales Verhältnis von Edukt, 1-Brommethyl-2-methoxymethylbenzol II und [1,2-Bis-(methoxy-methyl)]-benzol III erreicht war. Nach Abkühlen auf ca. 20°C wurde ausgefallenes Natriumbromid abfiltriert. Das Filtrat wurde eingeengt, wobei überschüssiges Edukt weitgehend auskristallisierte. Der überstand wurde destilliert. Die Fraktion bei 68-71°C/0,4 Torr (156 g) bestand aus einem Produktgemisch von 35 % I, 55 % II und 10 % III. 155 g des Produktgemisches wurden in 500 ml Trimethylphosphit 5 h auf Rückflußtemperatur erhitzt. Nach Abdestillieren vom überschüssigen Phosphit wurde der Rückstand im Hochvakuum fraktioniert. Die Fraktion im Bereich 118-119°C/0,2 Torr (62 g) enthielt zu > 95 % den gewünschten 2-Methoxymethyl-benzylphosphonsäuredimethylester.

¹H-NMR (in CDCl₃): 3,35 ppm (d,2H), 3,4 ppm (s,3H), 3,65 ppm (d,6H), 4,6 ppm (s,3H), 7,2-7,5 ppm (m,4H)

### Beispiel 9

Herstellung von Dimethylphosphono-(2-methoxymethylphenyl)-essigsäure

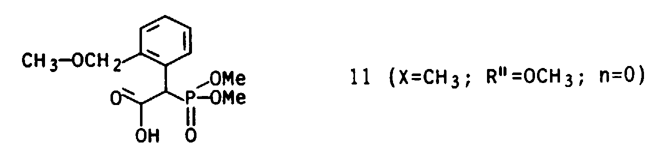

165 ml einer 1,6 M-Lösung von n-Butyllithium im Hexan wurden bei (-65)°C zu 170 ml absolutem Tetrahydrofuran gegeben. Hierzu wurde unter Einhaltung der Temperatur eine Lösung von 61 g 2-Methoxymethyl-benzyl-phosphonsäuredimethylester (aus Beispiel 8) in 100 ml Tetrahydrofuran getropft. Nach 30 min wurde der Ansatz auf eine (-60)°C kalte, gesättigte Trockeneis/Diethylether-Lösung (500 ml) gekippt. Nach 10 min Rühren ließ man den Ansatz innerhalb von 4 h auf ca. 20°C erwärmen. Nach Zugabe von 300 ml Wasser wurde die organische Phase abgetrennt und dreimal mit 100 ml 10 %iger Natriumcarbonatlösung extrahiert. Die vereinigten wasserphasen wurden zweimal mit 150 ml Diethylether extrahiert und mit 2 N Schwefelsäure auf pH 1 eingestellt. Man extrahierte dreimal mit 150 ml Dichlormethan, trocknete die organischen Phasen und entfernte das Lösungsmittel unter reduziertem Druck. Es verblieben 60 g Dimethylphosphono-(2-methoxymethylphenyl)-essigsäure als gelbes Öl.

¹H-NMR (in CDCl₃): 3,3 ppm (s,3H), 3,6 ppm (s,3H), 3,75 ppm (s,3H), 4,45 ppm (s,1H), 4,6 ppm (s,1H), 4,8 ppm (s,1H), 7,2-7,35 ppm (m,3H), 7,8 ppm (d,1H), 10,3 ppm (breit,1H).

### Beispiel 10

Herstellung von α-(2-BromMethylphenyl)-β-ethylacrylsäuremethylester

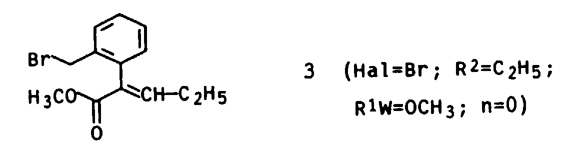

17 g α-(2-Methoxymethylphenyl)-β-ethylacrylsäure wurden analog Beispiel 4 mit Bortribromid und Methanol umgesetzt. Ausbeute: 14,5 g.

¹H-NMR (in CDCl₃): 1,0 ppm (t,3H), 2,0 ppm (m,2H), 3,7 ppm (s,3H), 4,4 ppm (m,2H), 7,0-7,5 ppm (m,5H)

### Beispiel 11

Herstellung von 2-(1,3-Dioxolan-2-yl)-phenylglyoxylsäuremethylester

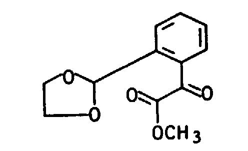

174,5 g 1-Brom-2-(1,3-Dioxolan-2-yl)-benzol wurden analog Beispiel 2 über die Zink-organische Verbindung mit 102 g Oxalsäuremonomethylesterchlorid umgesetzt. Ausbeute: 41,6 g.

¹H-NMR (in CDCl₃): 3,8-4,1 ppm (m,7H), 6,25 ppm (s,lH), 7,4-7,7 ppm (m,4H)

### Beispiel 12

Herstellung von α-[2-(1,3-Dioxolan-2-yl)-phenyl]-β-methylacrylsäuremethylester (Verbindung 33)

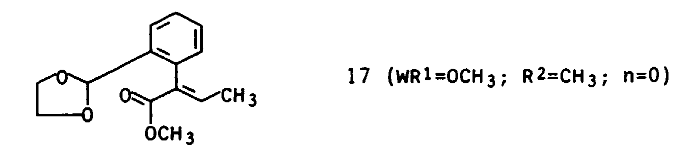

18,0 g 2-(1,3-Dioxolan-2-yl)-phenylglyoxylsäuremethylester wurden analog Beispiel 3 mit 33,9 g Ethyltriphenylphosphoniumbromid umgesetzt.

Ausbeute: 9,0 g.

¹H-NMR (in CDCl₃): 1,6 ppm (d,3H), 3,7 ppm (s,3H), 3,9-4,1 ppm (m,4H), 5,65 ppm (s,1H), 7,0-7,7 ppm (m,5H).

### Beispiel 13

Herstellung von 2-(β-Methyl-α-methoxycarbonylvinyl)-benzaldehyd (Verbindung 42)

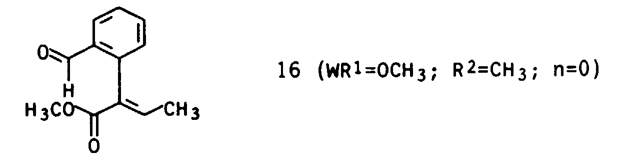

Eine Lösung von 7,0 g α-[2-(1,3-Dioxolan-2-yl)-phenyl]-β-methylacrylsäuremethylester in 30 ml Tetrahydrofuran wurde mit 3 ml 2 N Salzsäure vesetzt. Man ließ über Nacht rühren, entfernte das Lösungsmittel unter reduziertem Druck und nahm den Rückstand in 100 ml 5 %iger wäßriger Natriumhydrogencarbonatlösung/Dichlormethan (1:2, v/v) auf. Die organische Phase wurde abgetrennt, zweimal mit ges. Natriumchloridlösung gewaschen und mit Natriumsulfat getrocknet. Nach Entfernen des Lösungsmittels verblieben 5,3 g des Aldehyds als Öl.

¹H-NMR (in CDCl₃): 1,7 ppm (d,3H), 3,65 ppm (s,3H), 7,2-7,7 ppm (m,4H); 7,95 ppm (d,1H), 10,0 ppm (s,1H)

### Beispiel 14

Herstellung von 2-(β-Methyl-α-methoxycarbonylvinyl)-benzylphosphonsäuredimethylester (Verbindung 36)

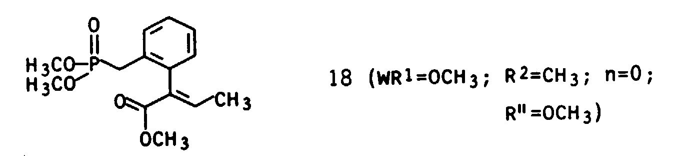

5 g α-(2-Brommethylphenyl)-β-methylacrylsäuremethylester wurden in 40 ml Trimethylphosphit 2 h auf Rückflußtemperatur erhitzt. Überschüssiges Phosphit wurde abdestilliert und der verbleibende Rückstand wurde in Wasser/Dichlormethan (1:1, v/v) aufgenommen. Die wäßrige Phase wurde noch zweimal mit Dichlormethan extrahiert. Die vereinigten organischen Phasen wurden mit Natriumsulfat getrocknet. Nach Entfernen des Lösungsmittels verblieb das Phosphonat als hellgelbes Öl.

¹H-NMR (in CDCl₃): 1,65 ppm (d,3H); 3,1 ppm (m,2H); 3,65 ppm (d,6h); 3,7 ppm (s,3H), 7,1 ppm (d,1H); 7,3 ppm (m,2H); 7,55 ppm (d,1H).

### Beispiel 15

Herstellung von α-[2-(5'Methyl-1',3',4'-thiadiazol-2'-yl)-thiomethylphenyl]-β-methylacrylsäuremethylester (Verbindung 9)

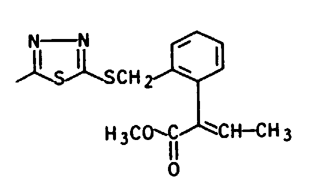

4,04 g (15 mmol) α-(2-Brommethyl-phenyl)-β-methylacrylsäuremethylester, 2,08 g (16 mmol) 5-Methyl-2-mercapto-1,3,4-thiadiazol und 3,1 g (22,4 mol) Kaliumcarbonat wurden 3 Stunden bei 60°C und anschließend 15 Stunden bei 20 bis 25°C gerührt. Das Reaktionsgemisch wurde in Wasser aufgenomen und mit Methyl-t.-butylether extrahiert. Die organische Phase wurde 2 x mit gesättigter Natriumhydrogencarbonat-Lösung und 2 x mit Wasser gewaschen, über Natriumsulfat getrocknet und eingeengt. Das Rohprodukt wurde an Kieselgel mit Methyl-t.-butylether/Cyclohexan (1/1) chromatographiert. Nach dem Entfernen des Lösungsmittels erhielt man 4,6 g (96 %) der Titelverbindung in Form farbloser Kristalle (Fp.: 74-75°C).

### Beispiel 16

Herstellung von α-[2-(2',6'-Dimethyl-4'-pyrimidinyl)-oxymethyl-phenyl]-β-methylacrylsäuremethylester (Verbindung 6)

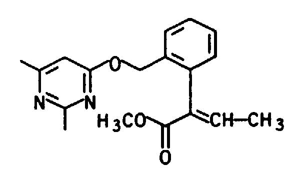

2,05 g (16,5 mmol; 10 % Überschuß) 2,6-Dimethyl-4-hydroxypyrimidin und 3,1 g (22,4 mmol) Kaliumcarbonat wurden in 35 ml Dimethylformamid 30 min bei 60°C gerührt. Dann wurden 4,04 g (15 mmol) α-(2-Brommethyl-phenyl)-β-methylacrylsäuremethylester zugegeben. Die Reaktonsmischung wurde 4 h bei 75°C, dann 15 h bei 20 bis 25°C gerührt. Anschließend wurde das Reaktionsgemisch in Wasser aufgenommen und mit Methyl-t.-Butylether extrahiert. Die organische Phase wurde 2 x mit Natriumhydrogencarbonat-Lösung und 2 x mit Wasser gewaschen, über Natriumsulfat getrocknet und eingeengt. Das Rohprodukt wurde an Kieselgel mit Methyl-t.-butylether/Cyclohexan 8/1) chromatographisch gereinigt. Man erhielt 3,2 g (68 %) der obengenannten Verbindung in Form hellgelber Kristalle (Fp.: 56-58°C).

### Beispiel 17

Herstellung von α-[2-Methyl-4-(2-methoxyiminoethyl)-phenyl-oxymethylphenyl]-β-methylacrylsäuremethylester (Verbindung 32) 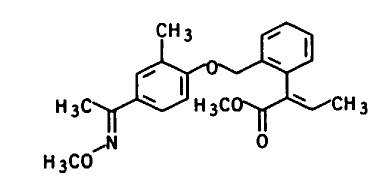
a) 31,8 g (0,21 mol) 3-Methyl-4-hydroxy-acetophenon und 57 g (0,21 mol) α-(2-Brommethyl-phenyl)-β-methylacrylsäuremethylester wurden in 800 ml Dimethylformamid gelöst und mit 58 g (0,42 mol) Kaliumcarbonat versetzt. Man rührte 8 Stunden bei 50°C, hydrolysierte mit Eiswasser und extrahierte mit Essigester.
   Die organische Phase wurde mit Wasser gewaschen, getrocknet und eingeengt. Man erhielt 62 g α-[2-Methyl-4-acetylphenyl-oxymethyl-phenyl]-β-methylacrylsäuremethylester in Form gelblicher Kristalle (Verb. Nr. 30).
   ¹H-NMR (in CDCl₃) [δ in ppm]: 1,62 (d,3H); 2,3 (s,3H); 3,54 (2,3H); 3,75 (s,3H); 4,98 (2,2H); 6,79 (d,2H); 7,0-7,9 (m,7H).
b) 22 g (67,4 mmol) α-[2-Methyl-4-acetylphenyl-oxymethylphenyl]-β-methylacrylsäuremethylester und 11,3 g (0,135 mol) O-Methylhydroxylaminhydrochlorid wurden in 600 ml Methanol 6 Stunden unter Rückfluß erhitzt. Anschließend ließ man auf ca. 25°C abkühlen, hydrolysierte mit Wasser und extrahierte mit Essigester.
   Die organischen Phasen wurden mit Wasser gewaschen, getrocknet und eingeengt. Man erhielt 18 g der Titelverbindung in Form hellgelber Kristalle (Fp.: 55-60°C).
   ¹H-NMR (in CDCl₃) [δ in ppm]: 1,5 (d,3H); 2,1 (s,3H); 2,15 (s,3H); 3,7 (s,3H); 3,98 (s,3H); 4,95 (s,2H); 6,7 (d,1H); 7-7,62 (m,7H).

### Beispiel 18

Herstellung von α-(2-[2-(3-[4-Chlorphenyl]isoxazol-5-yl)-ethenyl])-β-methylacrylsäuremethylester (Verbindung 46)

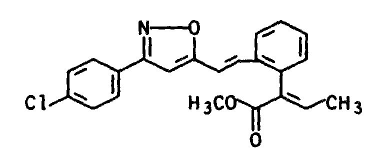

Zu 0,4 g Natriumhydrid in 10 ml wasserfreiem Dimethylformamid wurde bei ca. 20°C eine Lösung von 4,6 g (3-(4-Chlorphenyl)-isoxazol-5-ylmethyl-phosphonsäuredimethylester in 50 ml Dimethylformamid getropft. Man ließ 30 min nachrühren und tropfte dann den Ansatz zu einer Lösung von 2,4 g 2-(β-Methyl-α-methoxycarbonylvinyl)-benzaldehyd in 40 ml Dimethylformamid. Man rührte 4 h bei Raumtemperatur und arbeitete wie folgt auf. Das Reaktionsgemisch wurde auf 200 ml Eiswasser gekippt und dreimal mit Ethylacetat extrahiert. Die vereinigten organischen Phasen wurden mit gesättigter Natriumchloridlösung neutral gewaschen, getrocknet und inertisiert. Aus dem verbleibenden dunkelbraunen Öl kristallisierte das E-Isomer nach Verreiben mit Methanol aus.

### Beispiel 19

Herstellung von α-(2-[-(3-[4-Chlorphenyl]isoxazol-5-yl)-2-chlorethenyl])-β-ethylacrylsäuremethylester (Verbindung 47 und 48)

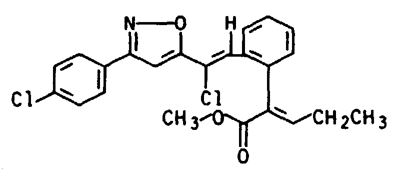

Zu einer auf (-70)°C gekühlten Lösung von 9,0 g 3-(4-Chlorphenyl)-isoxazol-5-ylmethyl-phosphonsäuredimethylester in 50 ml wasserfreiem Tetrahydrofuran wurden 175 ml einer 1,6 M-Lösung von n-Butyllithium in Hexan getropft. Nach 30 min wurden 4,2 g Tetrachlormethan zugegeben und 1 h bei (-70)°C nachgerührt. Man ließ auf -30°C kommen und tropfte bei dieser Temperatur 5,2 g 2-(β-Ethyl-α-methoxy-carbonylvinyl)-benzaldehyd in 30 ml Tetrahydrofuran zu. Nach 1 h ließ man auf ca. 20°C erwärmen und rührte den Ansatz über Nacht. Die Aufarbeitung erfolgte analog zu Beispiel 21. Bei Verreiben des Rohprodukts mit Diisopropylether erhielt man 3 g farblose Kristalle, die zu ca. 85 % das Z-Isomer des gewünschten Produkts enthielten. Aus der Mutterlauge erhielt man 1,9 g Öl, welches zu ca. 95 % das E-Isomer enthielt.

### Beispiel 20

Herstellung von α-(2-(2-Methylphenoxy)methylphenyl)-β-methylacrylsäure-N-methylamid (Verbindung Nr. 76)

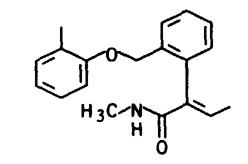

α-(2-(2-Methylphenoxy)methylphenyl)-β-methylacrylsäuremethylester wurde analog der Herstellungsbeispiele 5 und 6 zu α-(2-(2-Methylphenoxy)methylphenyl)-β-methylacrylsäurechlorid umgesetzt. Zu 6,3 g (21 mmol) dieses Säurechlorids in 35 ml Dichlormethan wurde bei 0°C eine Lösung aus 2,6-Methylamin in 35 ml Dichlormethan getropft und anschließend wurde zwei Stunden bei Raumtemperatur nachgerührt. Das Reaktionsgemisch wurde mit Wasser gewaschen, über Natriumsulfat getrocknet und eingeengt. Das Rohprodukt wurde an Kieselgel mit Cyclohexan/Essigsäureethylester (1/1) chromatographiert. Nach dem Entfernen das Lösungsmittels erhielt man 4,8 g (78 %) der Titelverbindung als farbloses Öl.

IR (cm⁻¹): 1664, 1626, 1602, 1517, 1495, 1462, 1241, 1191, 1121, 752.

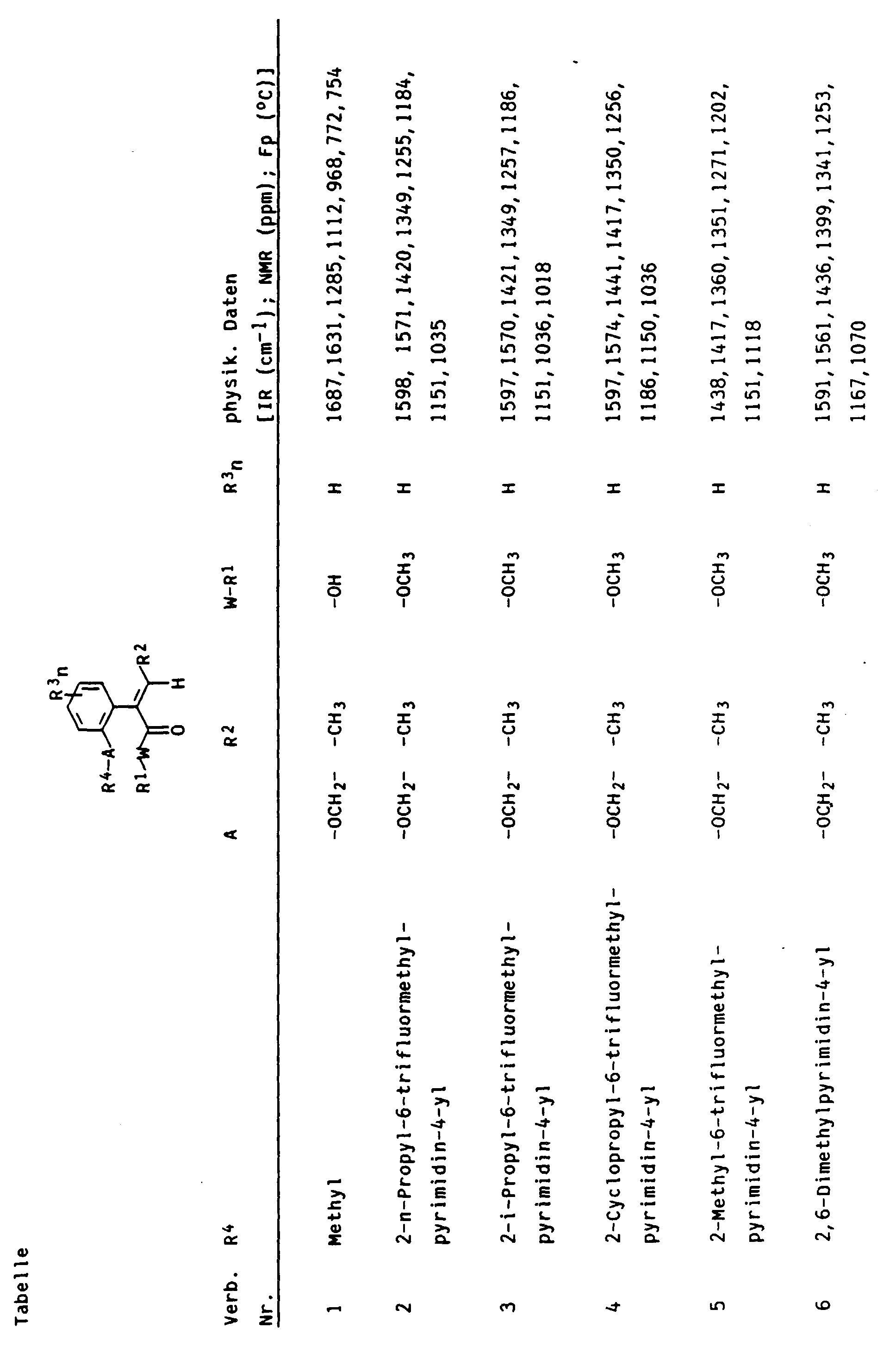

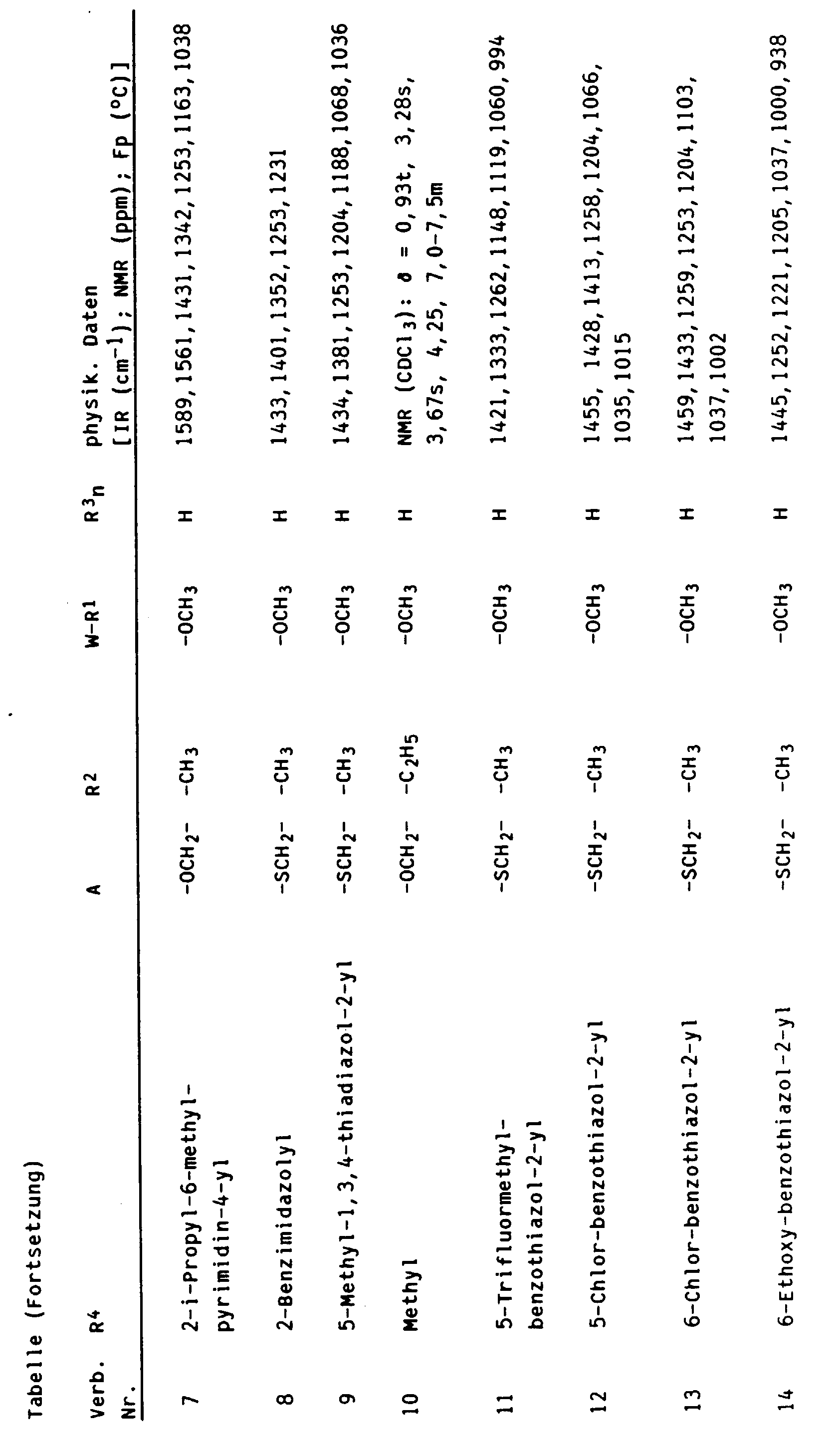

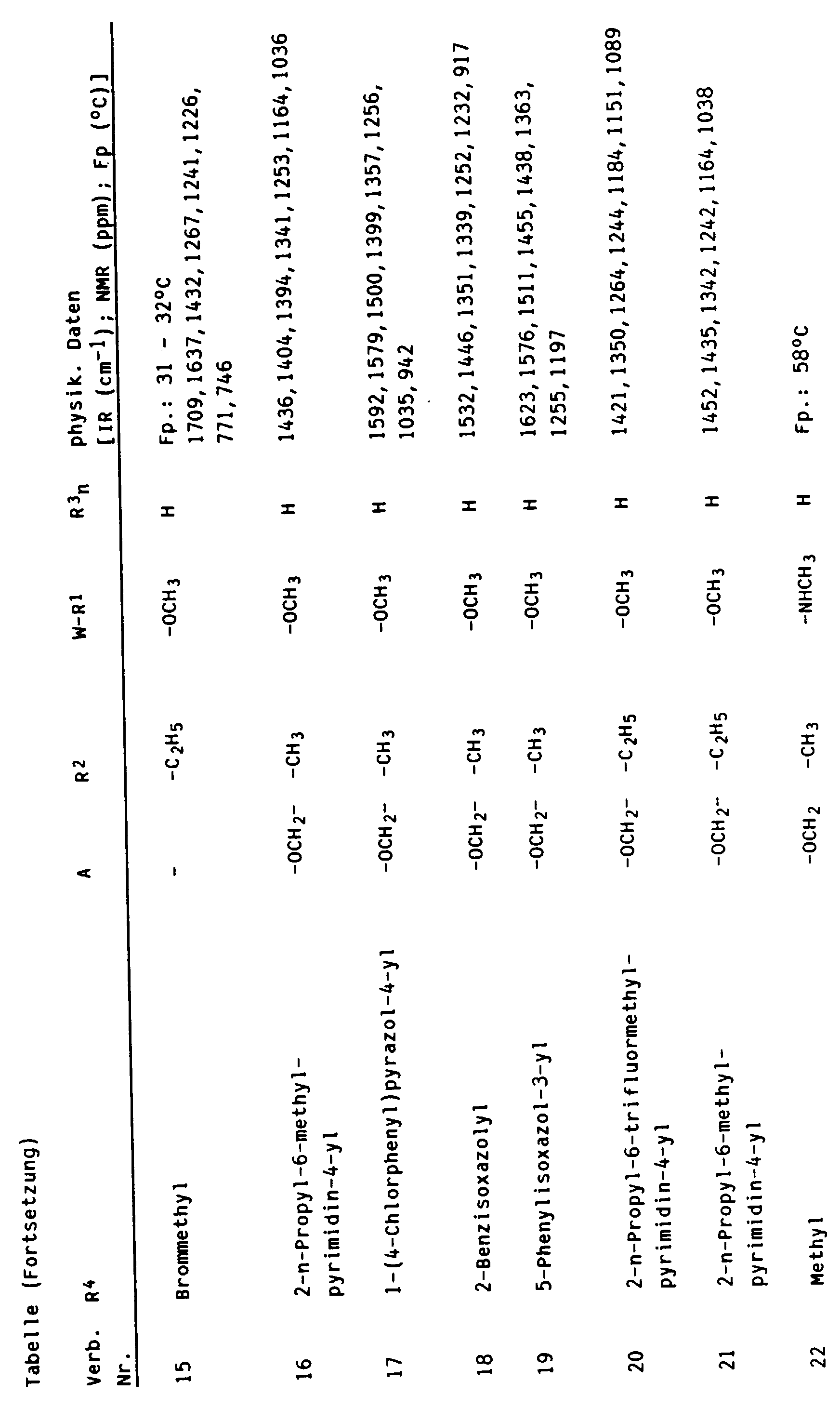

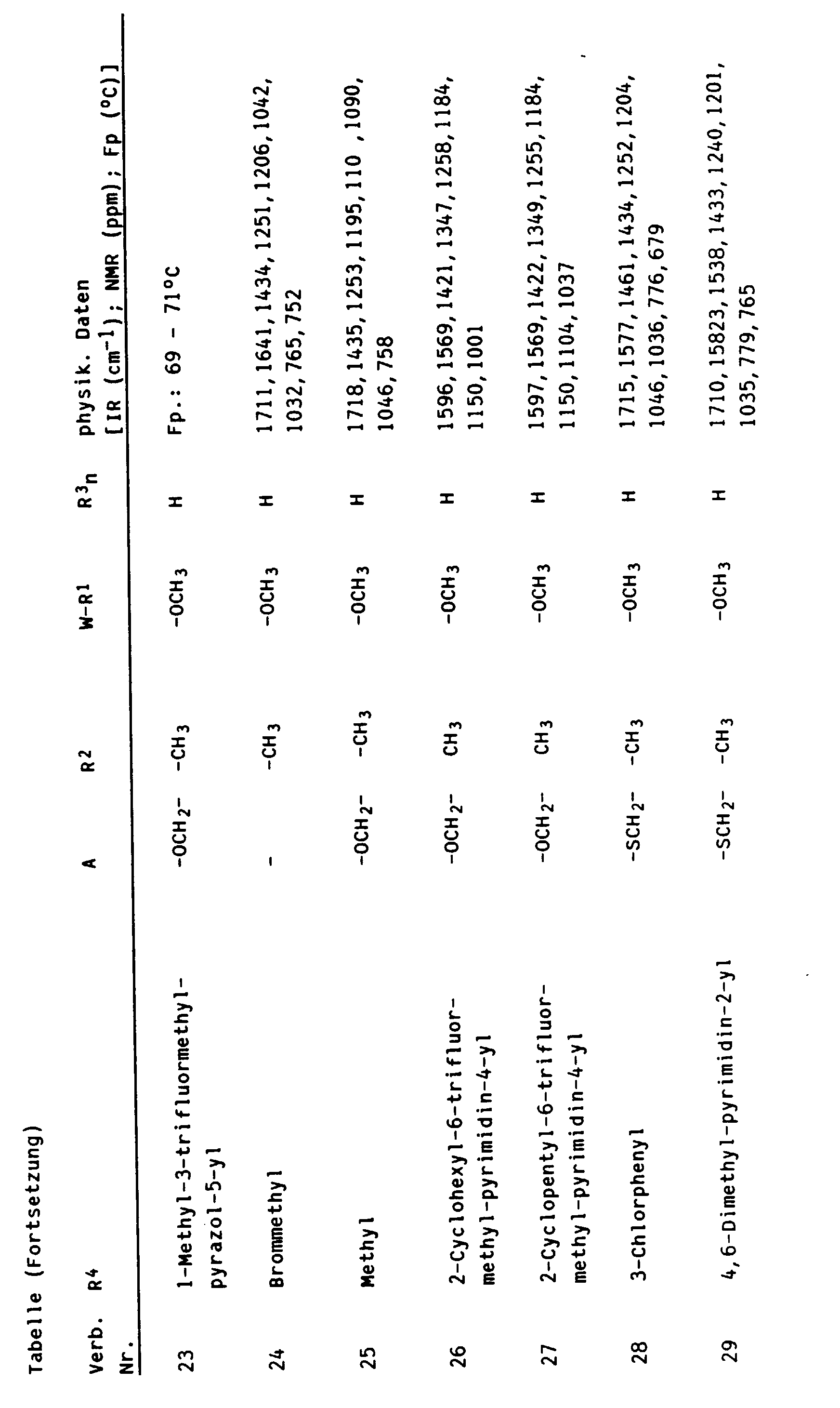

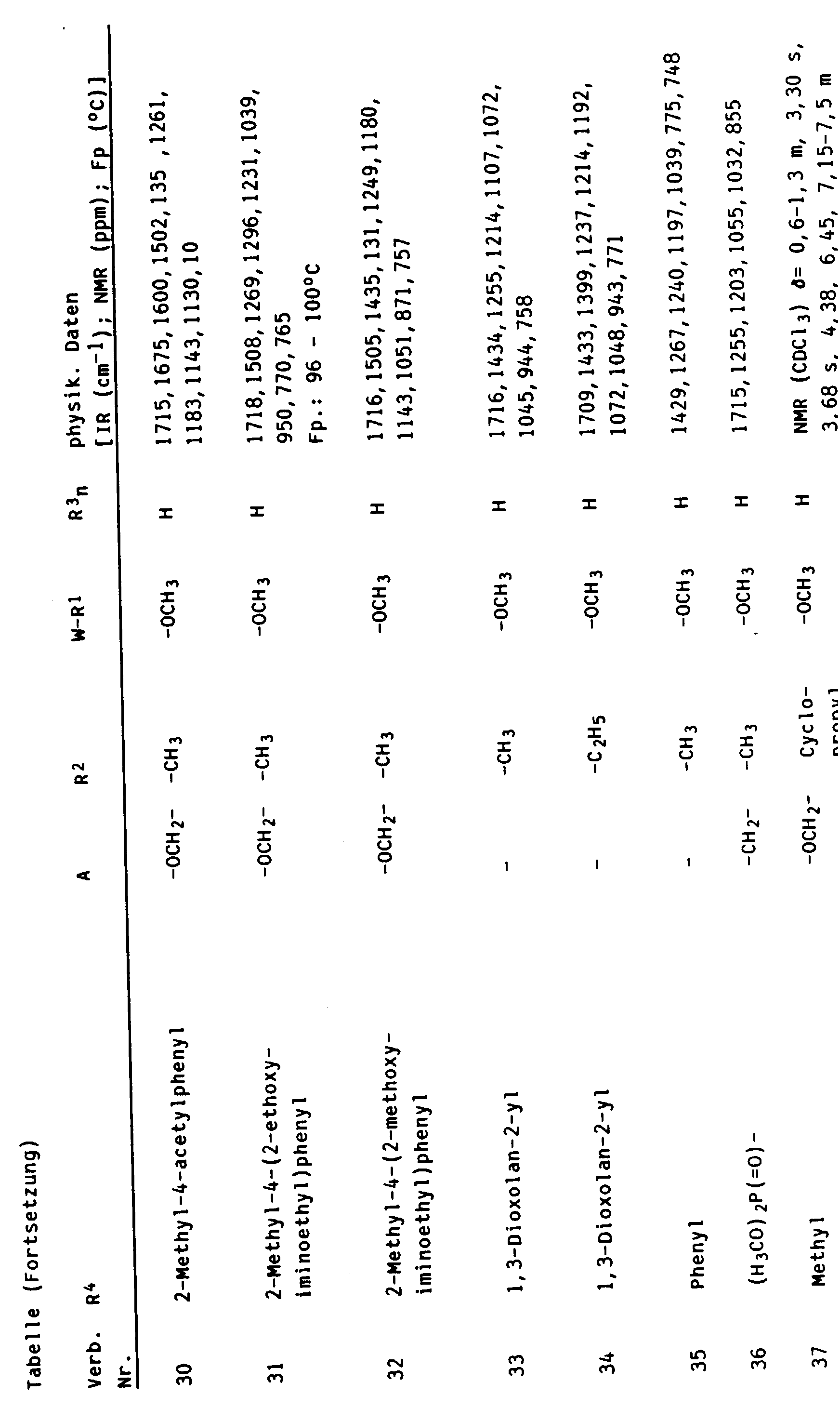

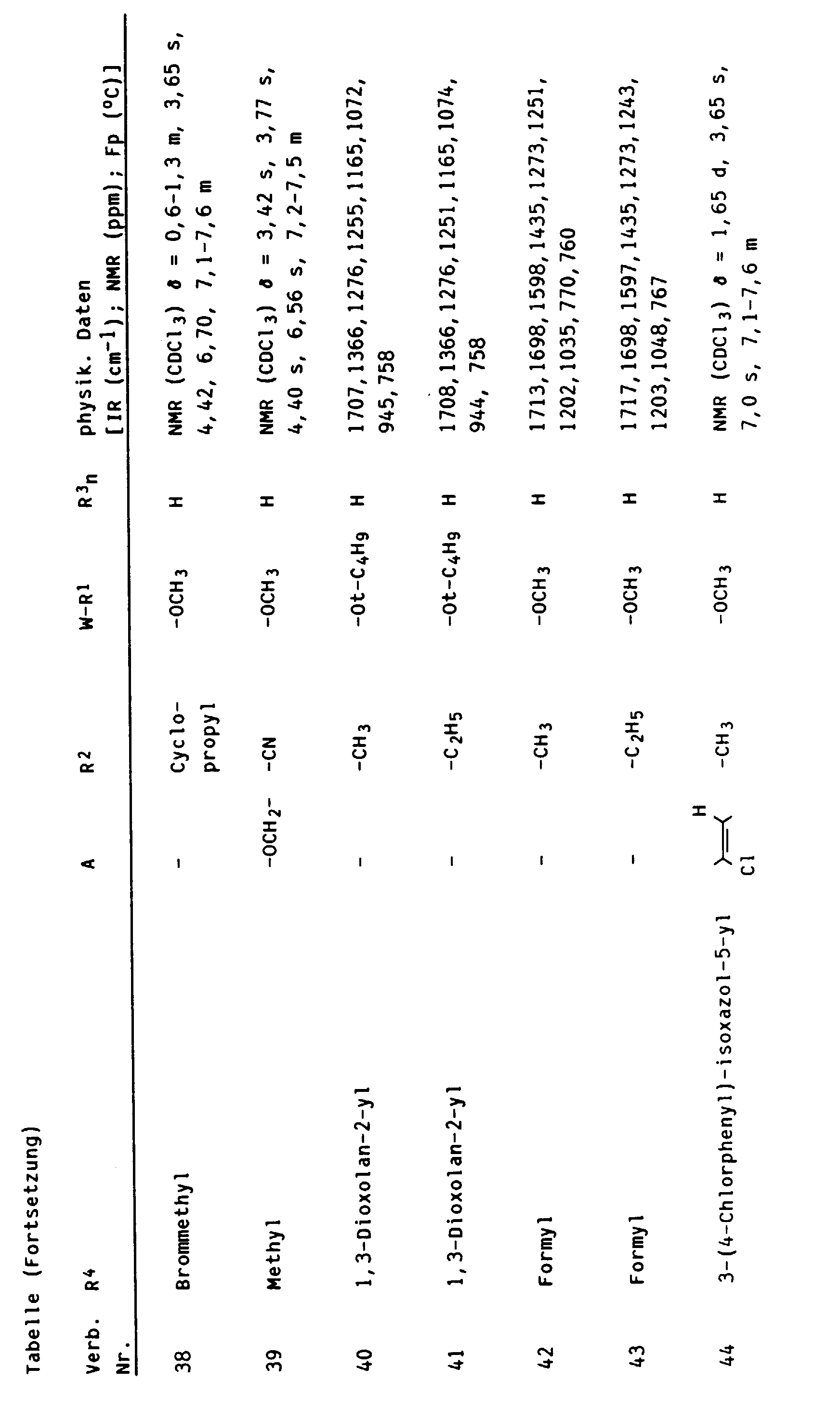

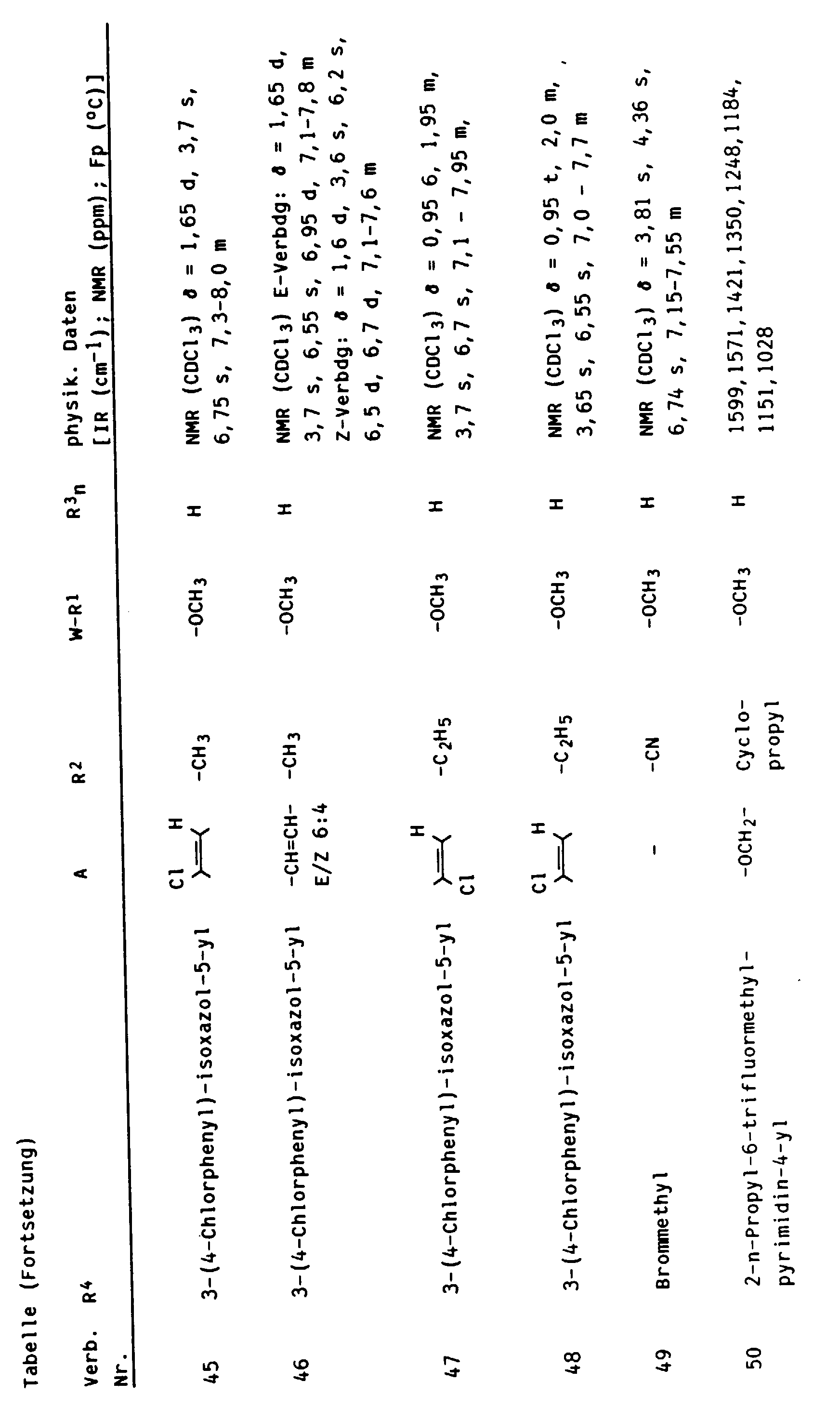

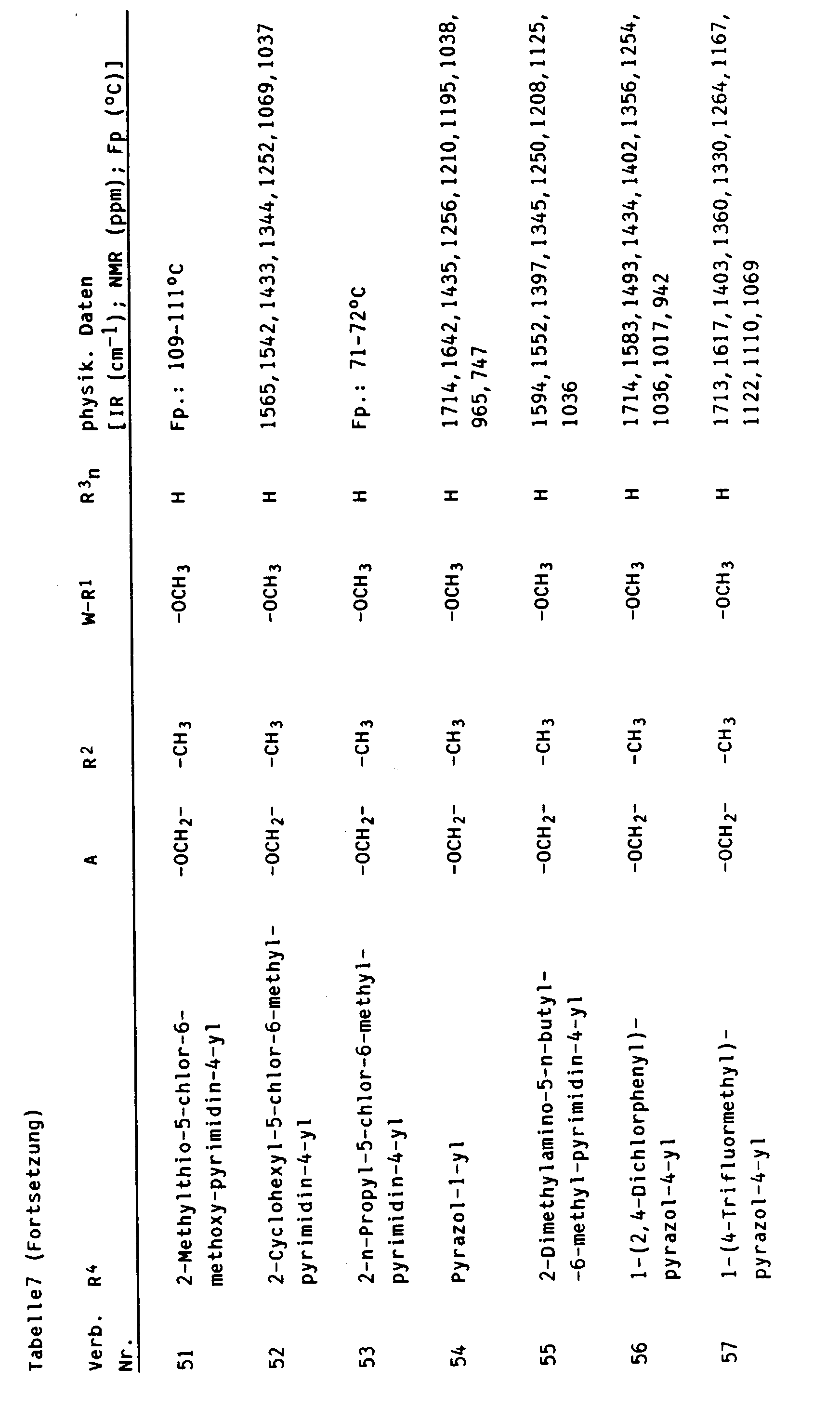

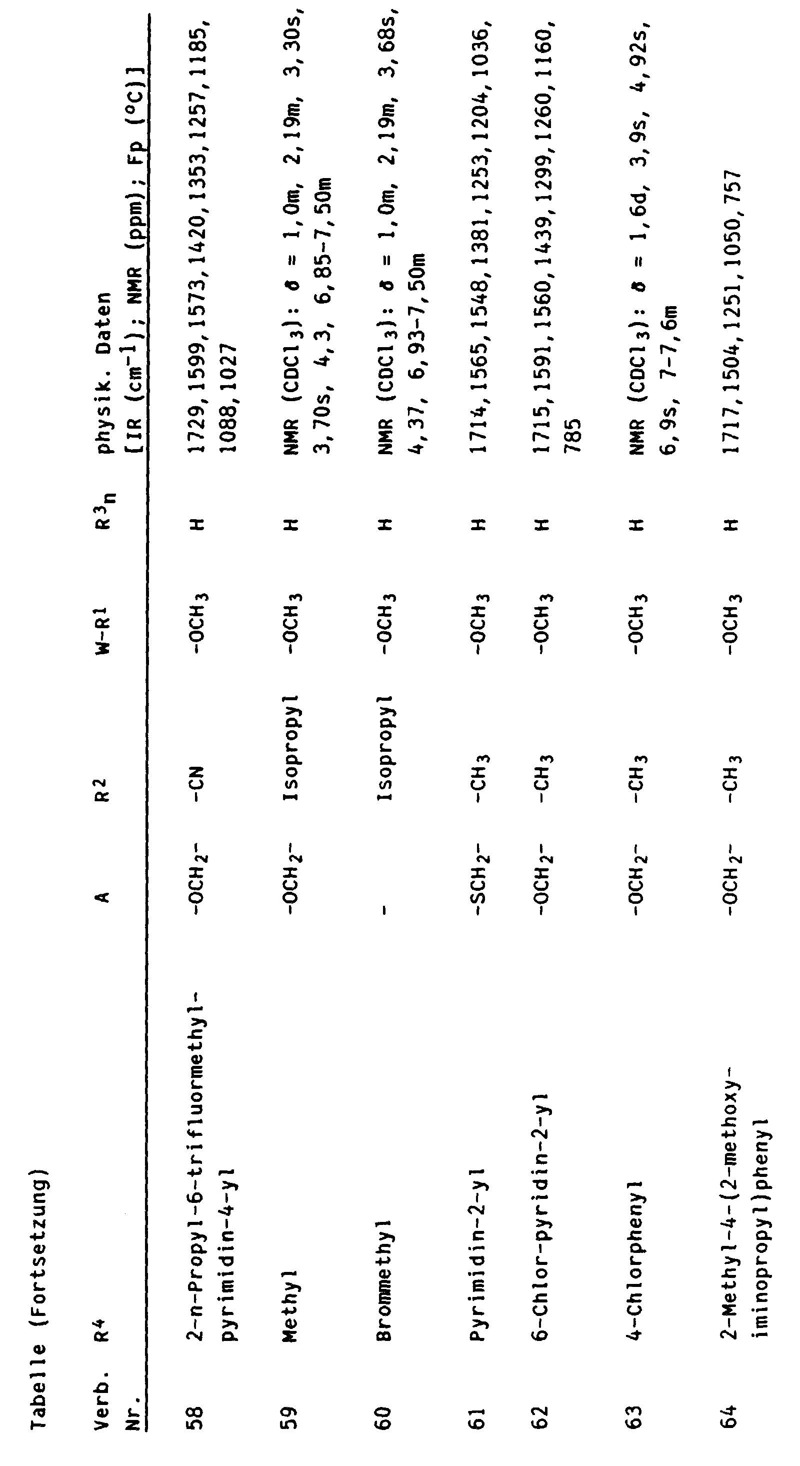

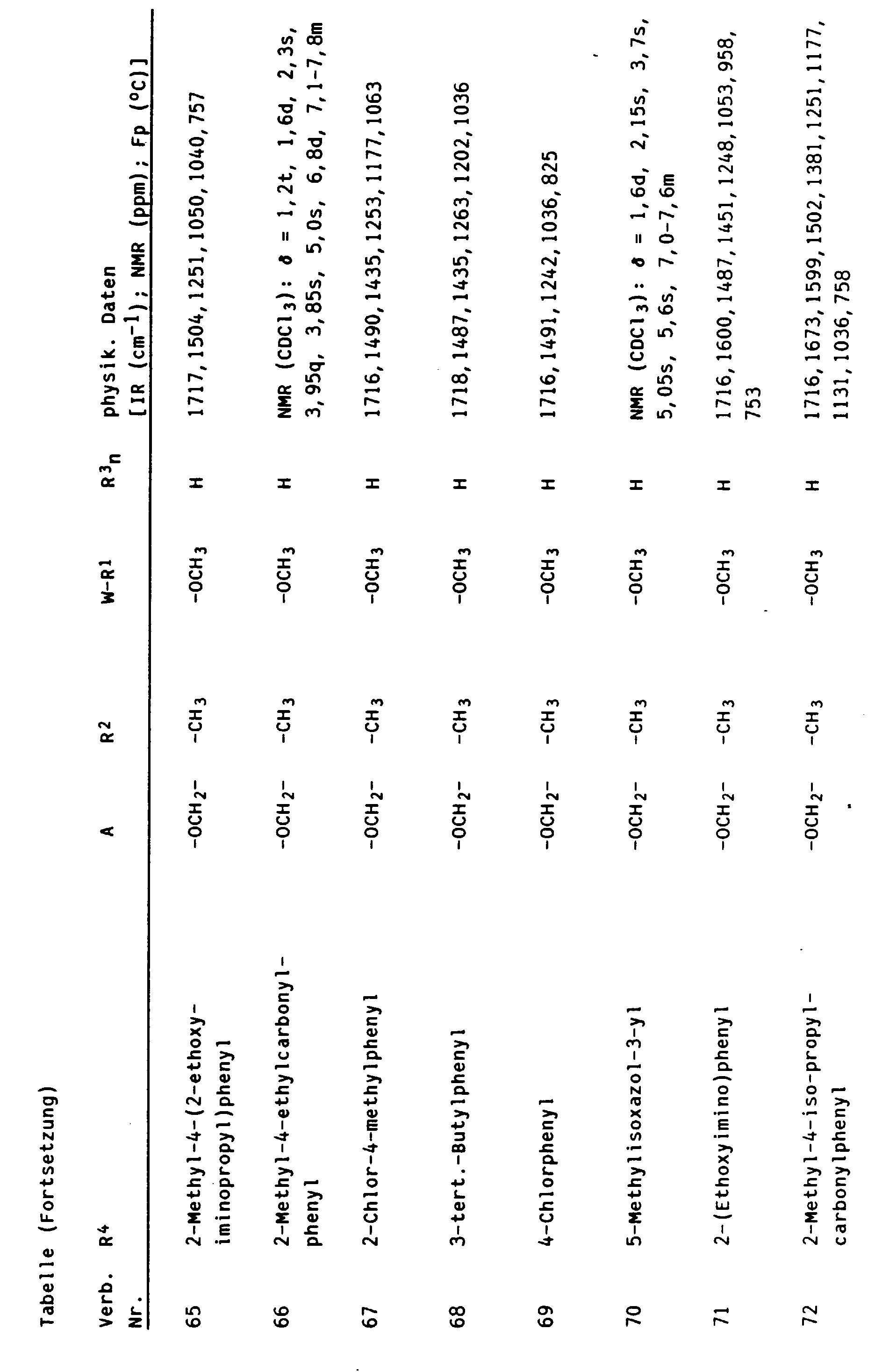

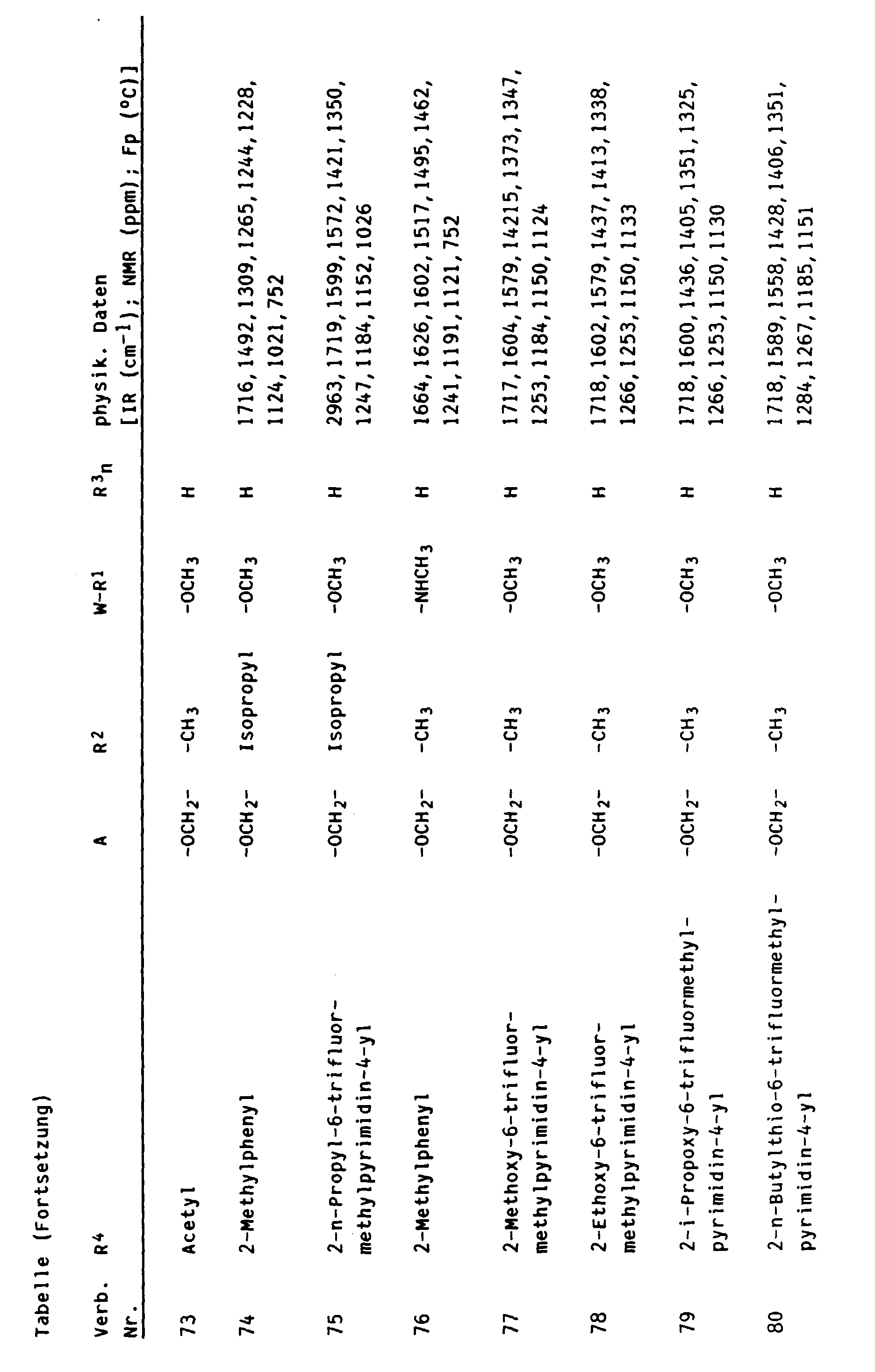

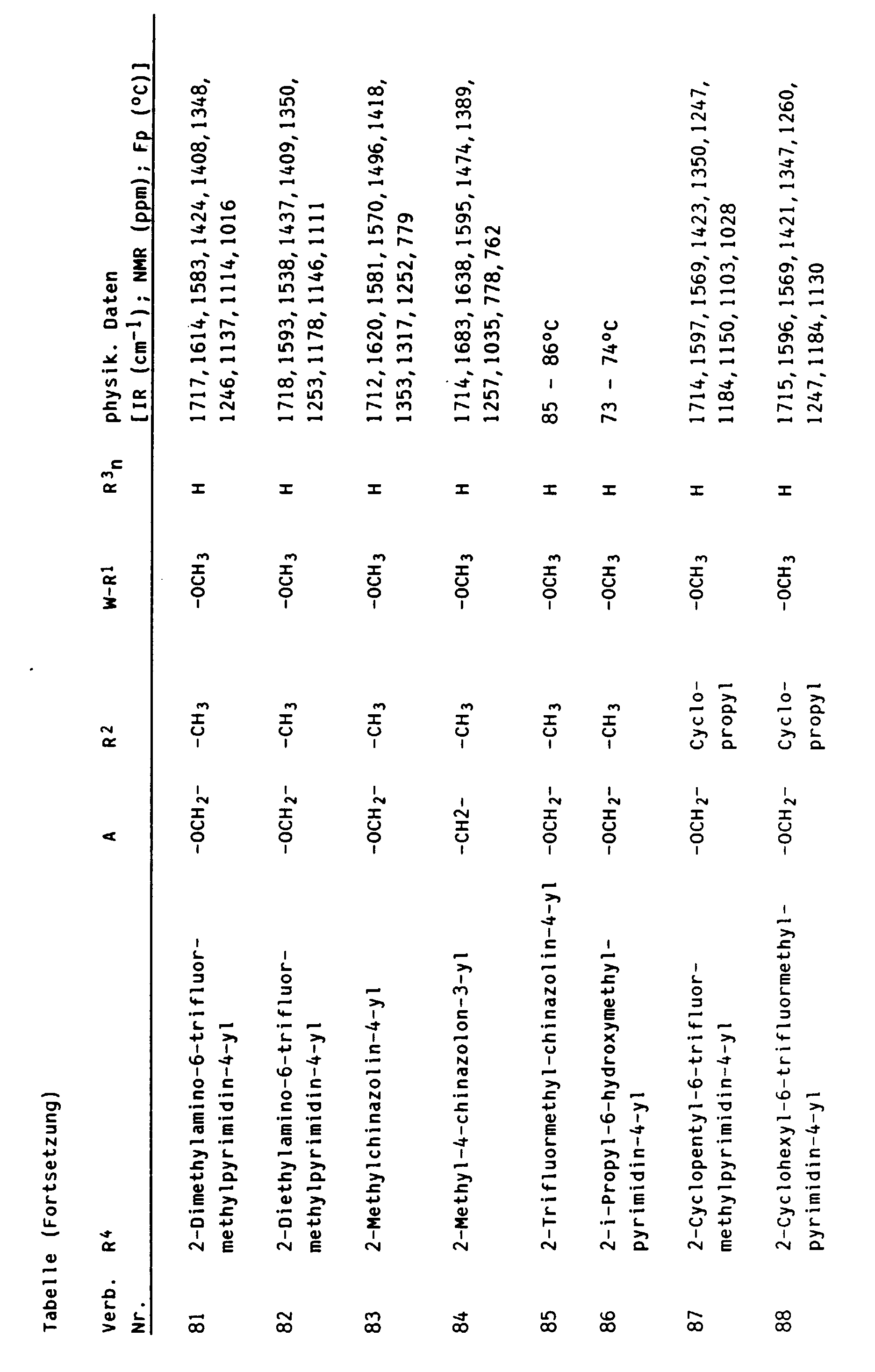

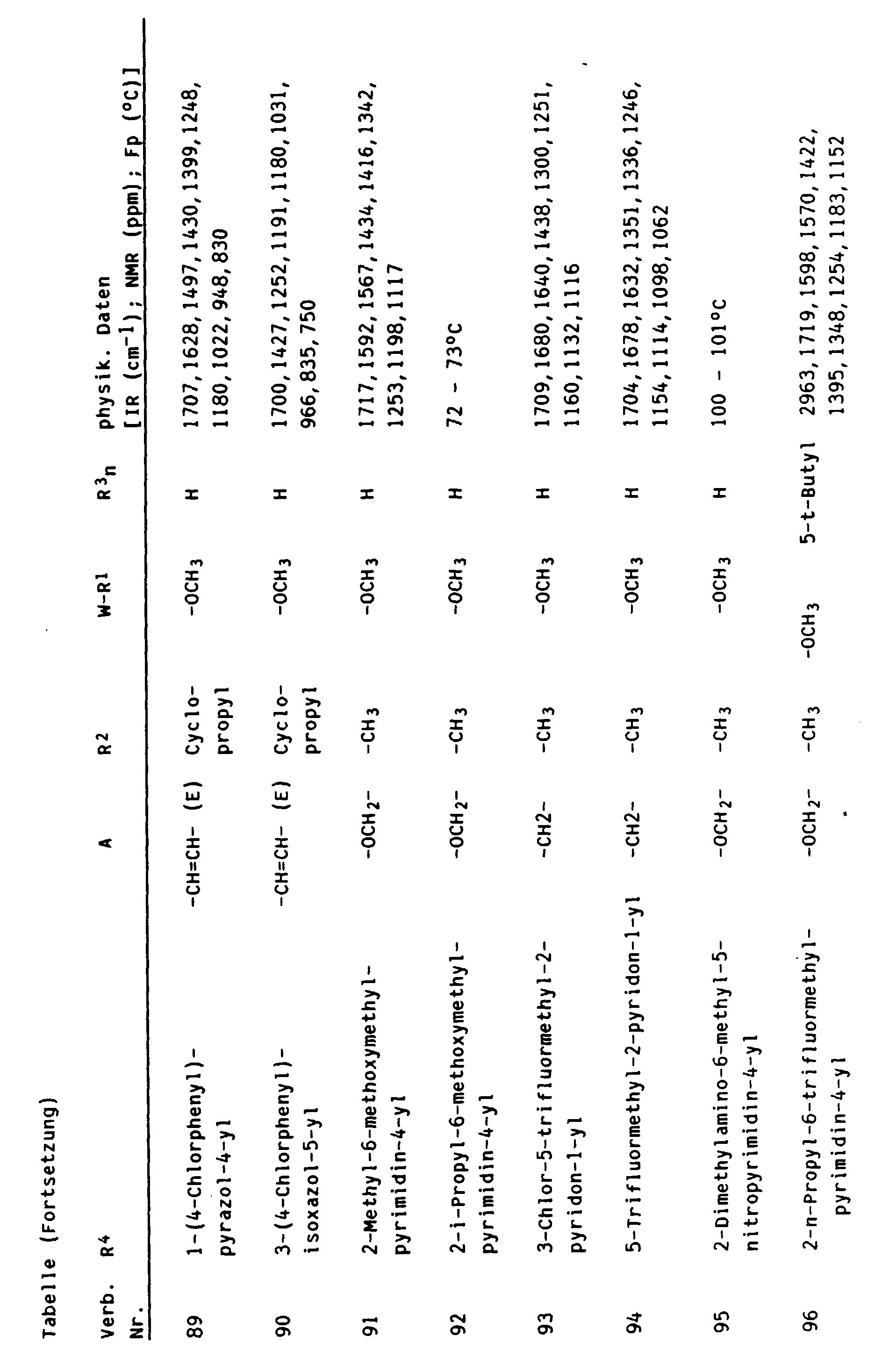

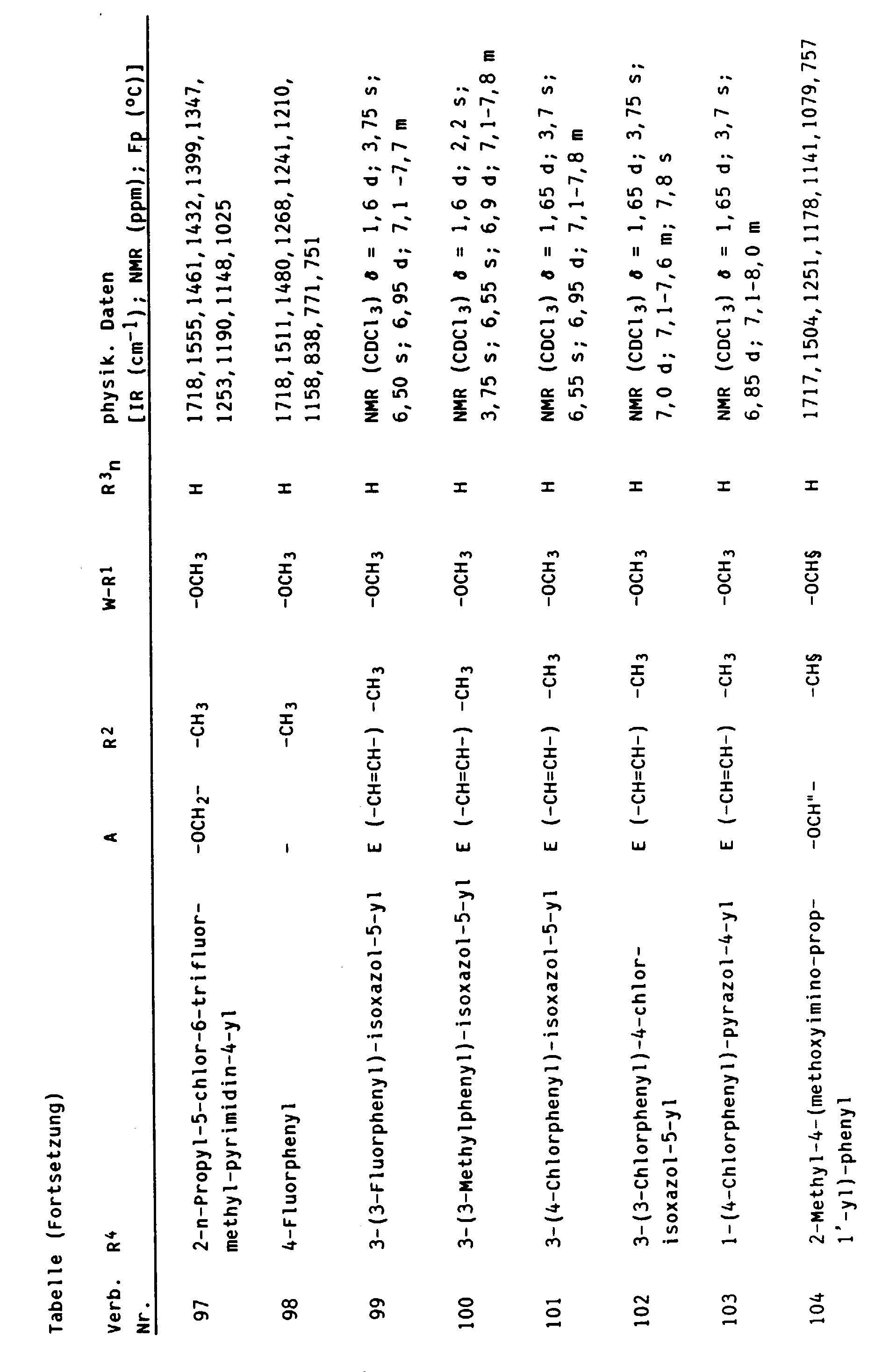

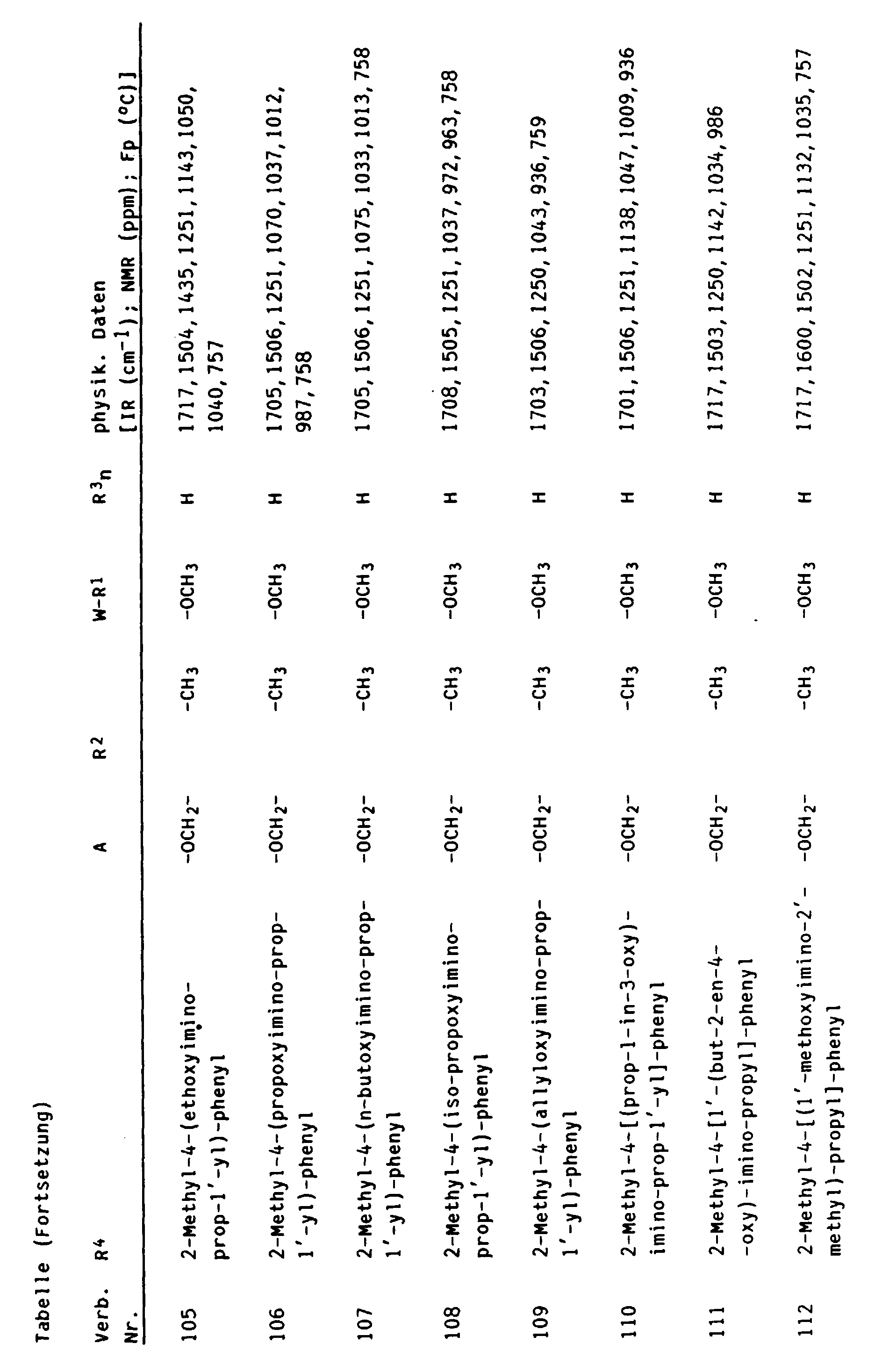

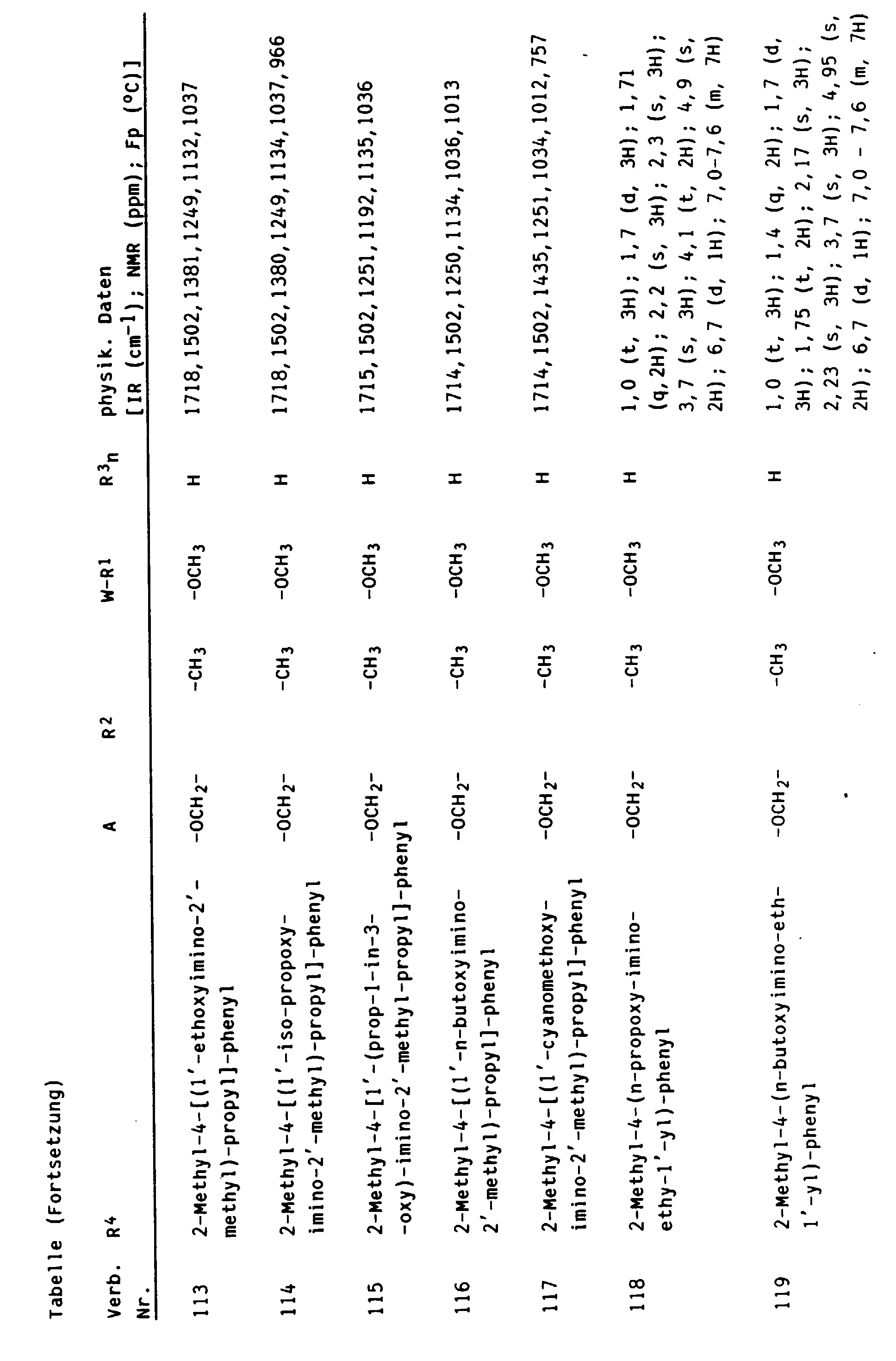

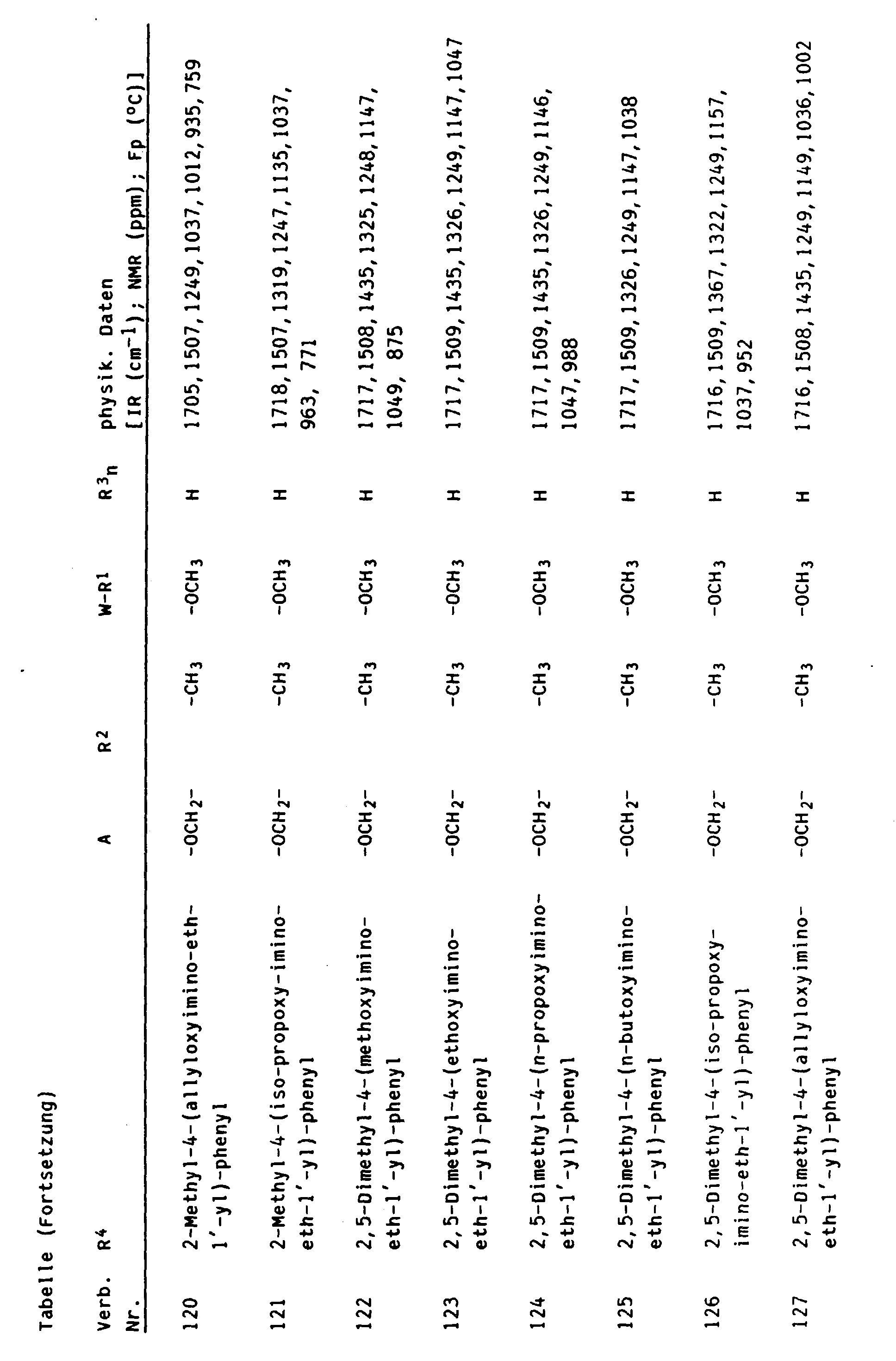

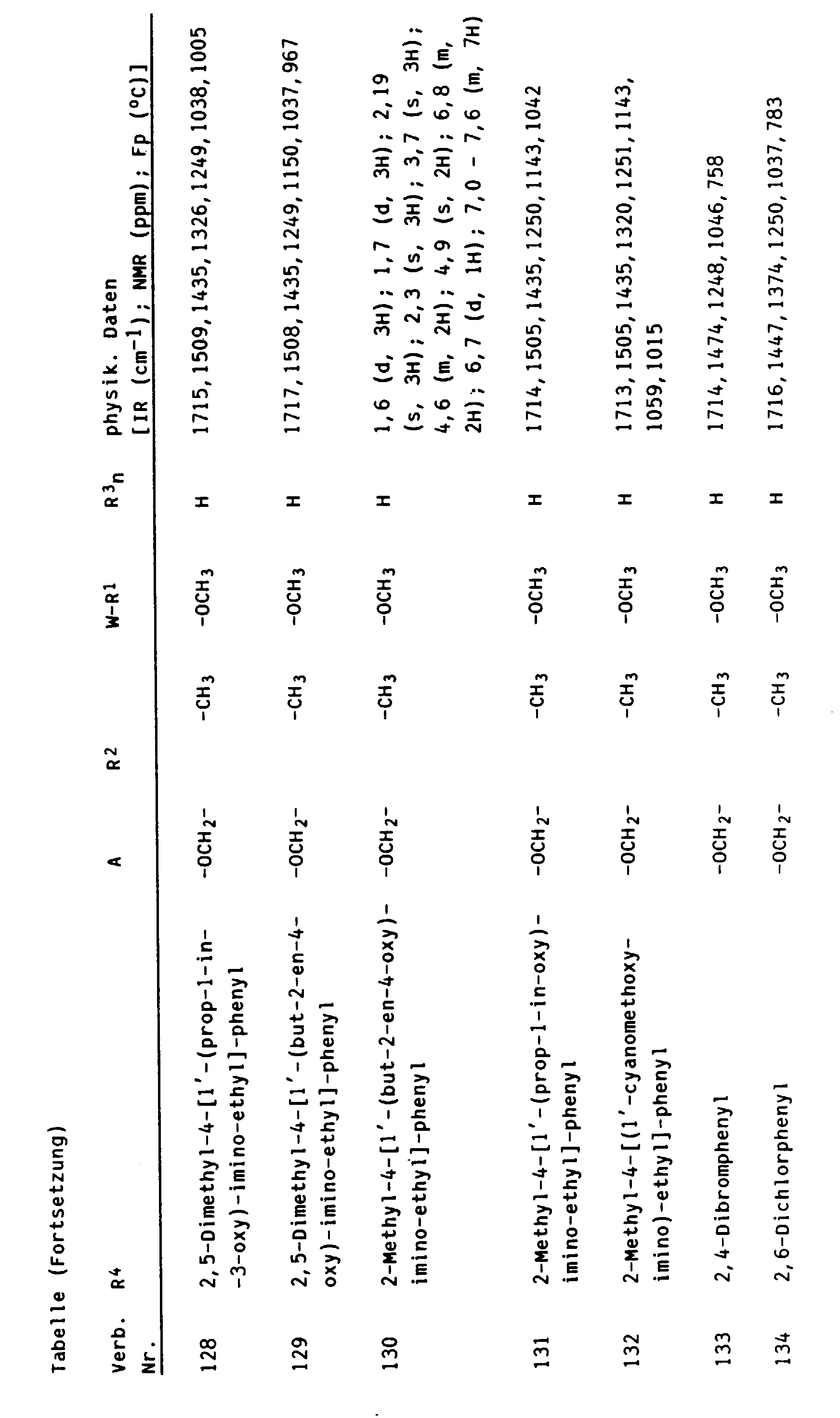

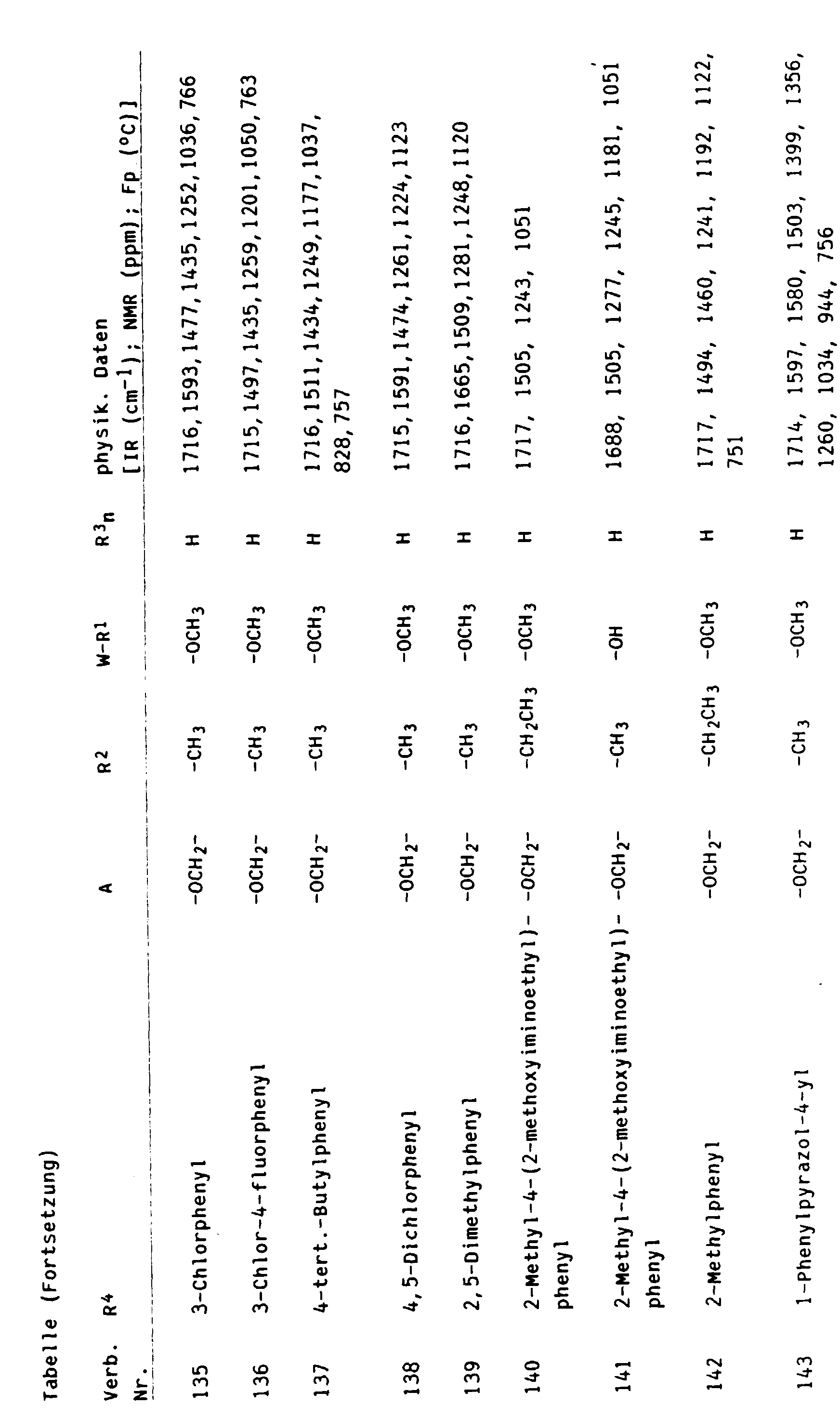

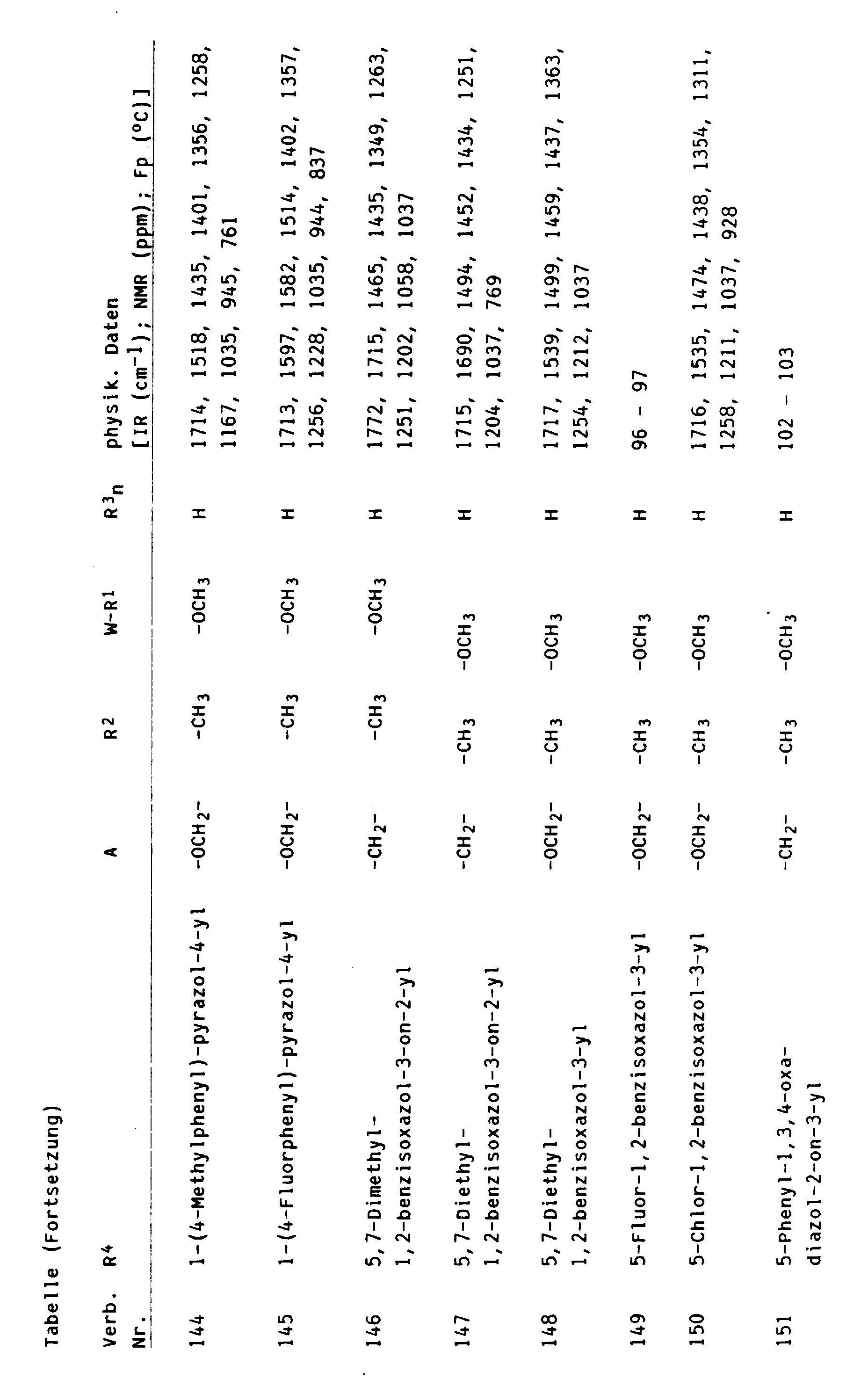

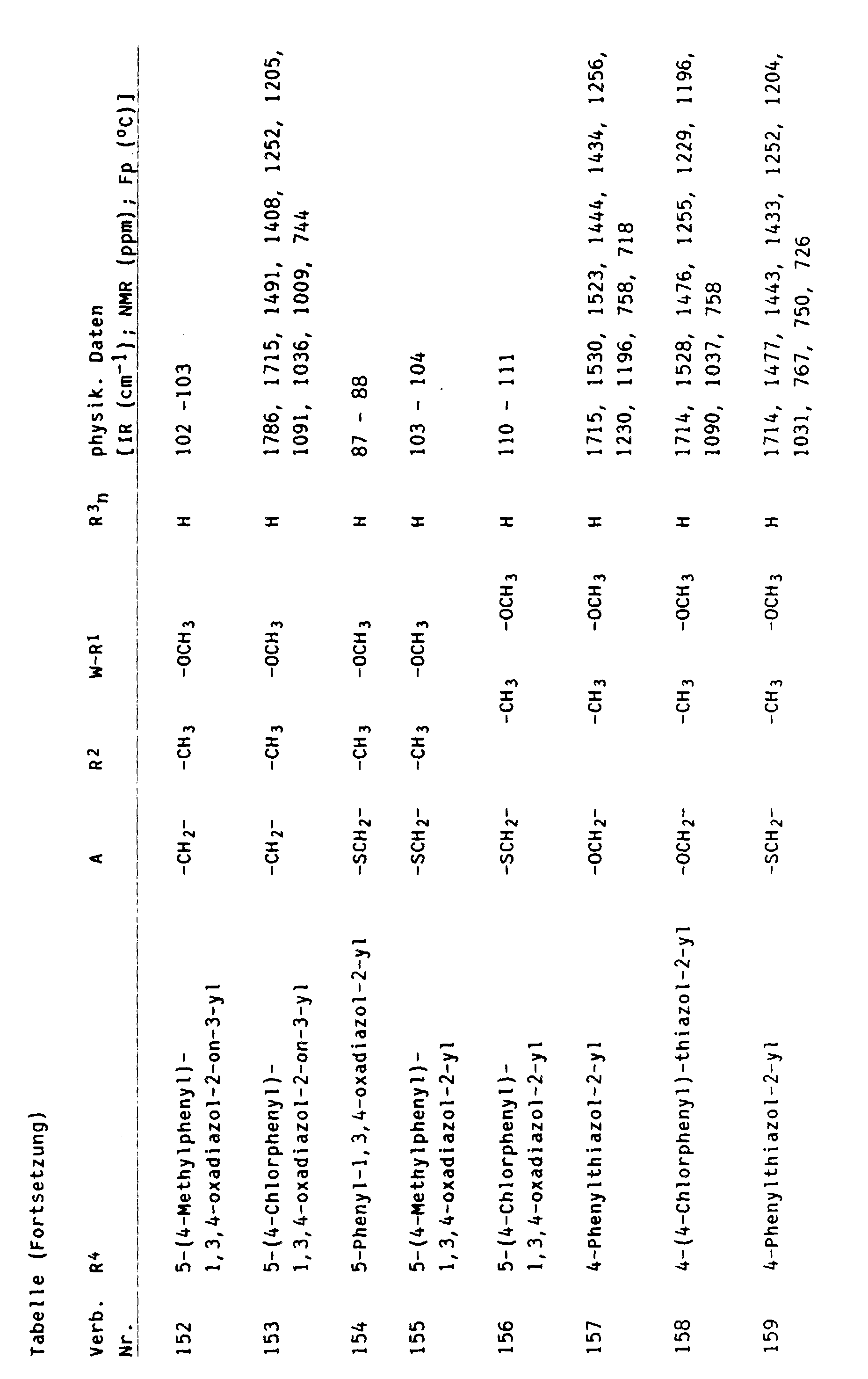

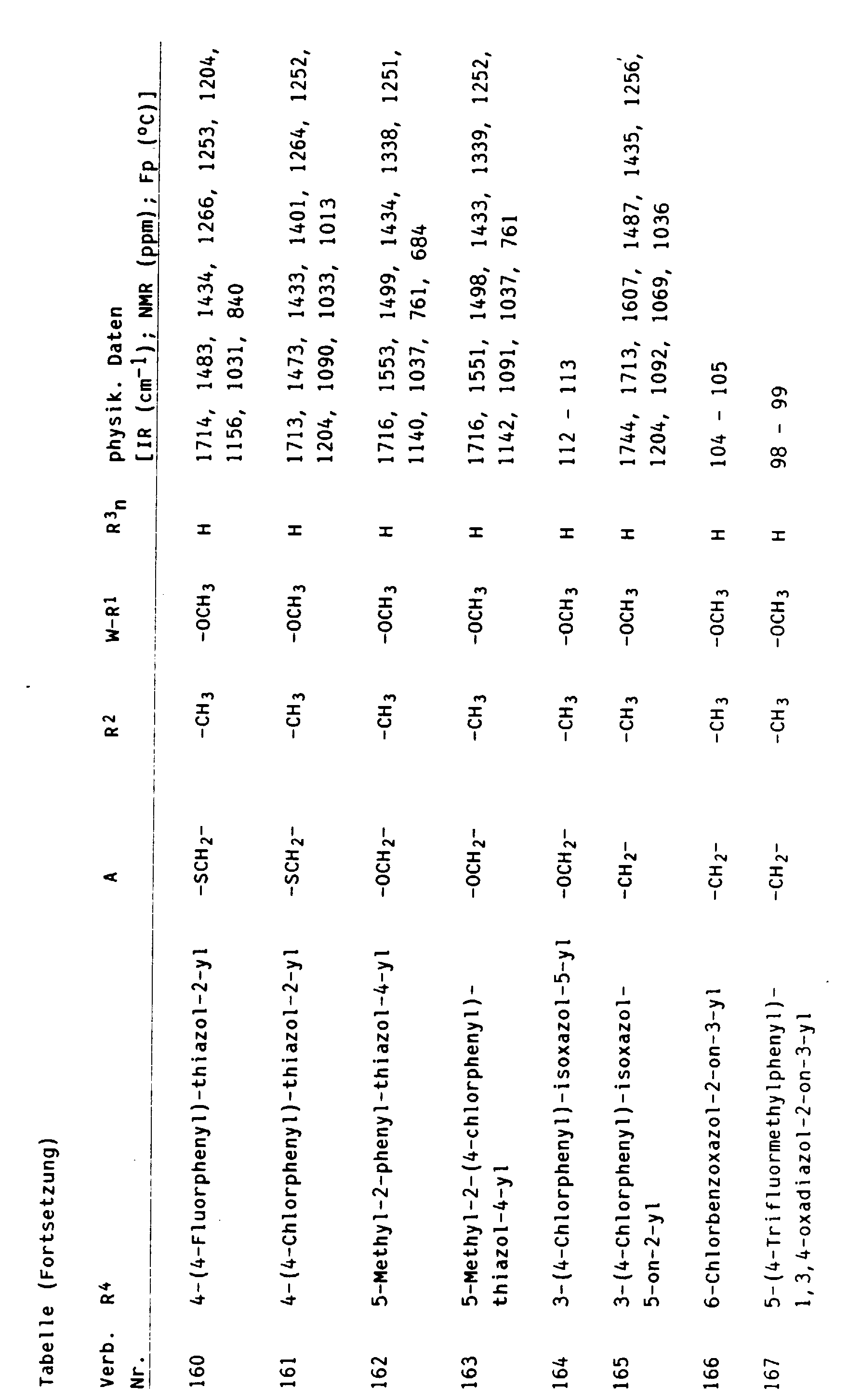

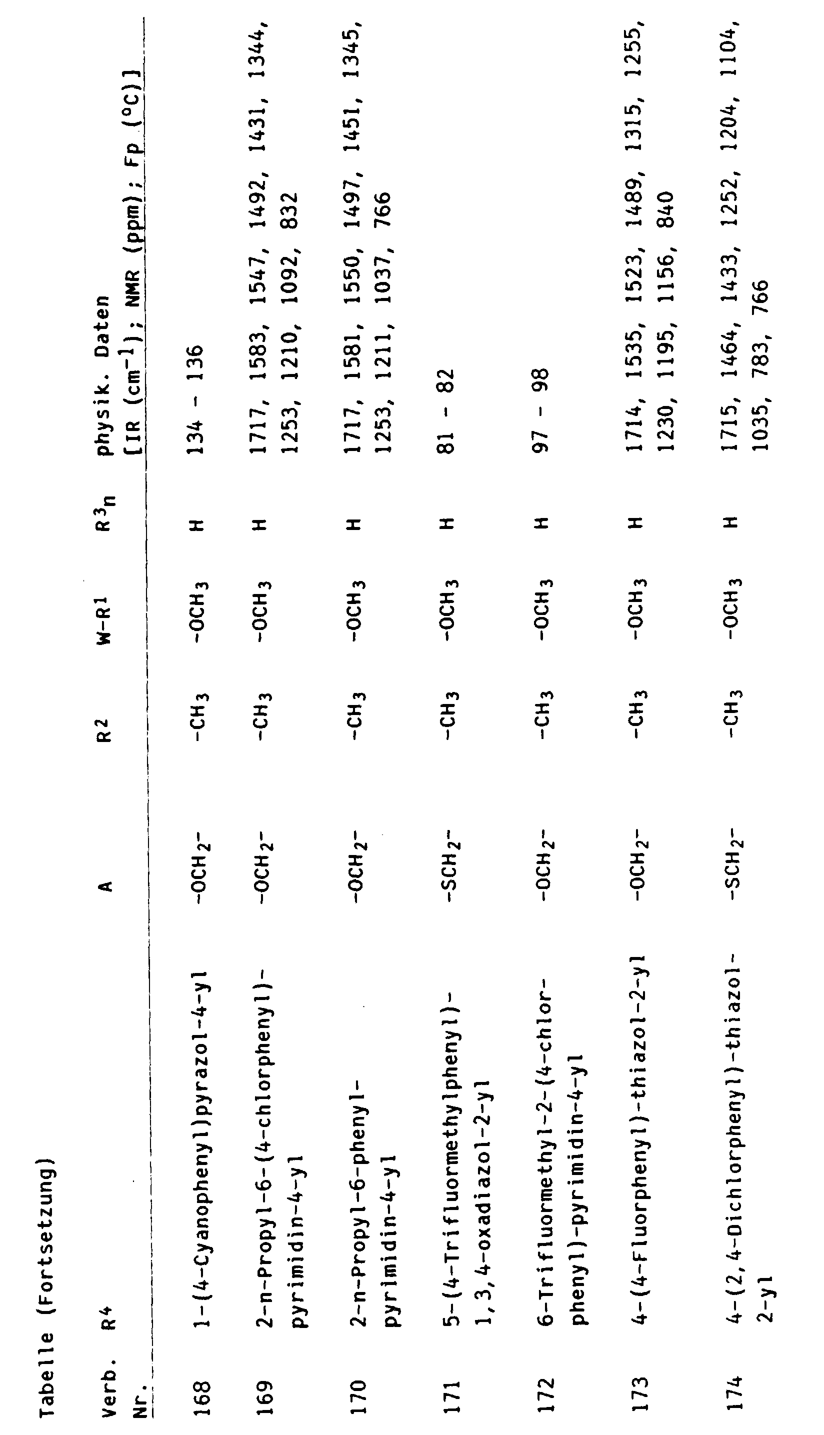

### Anwendungsbeispiele

### Fungizide Wirksamkeit

### Anwendungsbeispiel 1

### Wirksamkeit gegen Rebenperonospora

Blätter von Topfreben der Sorte "Müller Thurgau" wurden mit wäßrigen Aufbereitungen der Wirkstoffe gemäß den Tabellenbeispielen 11, 13, 14, 17, 30, 31, 32, 57, 64, 67, 111, 112, 115, 120, 121, 122, 123, 124, 125, 126, 127, 129, 130, 131, 132, 139, 140, 144, 145, 157, 158 und 160, die jeweils 80 Gew.% Wirkstoff und 20 Gew.% Emulgiermittel in der Trockensubstanz enthielten, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Gegenüber einem Kontrollversuch (keine Behandlung, 70 % Pilzbefall) wiesen die mit wäßrigen, 250 ppm Wirkstoff enthaltenden Spritzbrühen behandelten Pflanzen nur einen Pilzbefall von 0 bis 15 % auf.

### Insektizide Wirksamkeit

Die insektizide Wirkung der Verbindungen der allgemeinen Formel I' ließ sich durch folgende Versuche zeigen:

### Die Wirkstoffe wurden

a) als 0,1 %-ige Lösung in Aceton oder
b) als 10 %ige Emulsion in einem Gemisch aus 70 Gew.% Cyclohexanol, 20 Gew.% Nekanil® LN (Lutensol® AP6, Netzmittel mit Emulgier- und Dispergierwirkung auf der Basis ethoxylierter Alkylphenole) und 10 Gew.% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80-100 %ige Hemmung bzw. Mortalität hervorriefen (Wirkschwelle bzw. Minimalkonzentration).

## Patentansprüche

1. α-Phenylacrylsäurederivate der allgemeinen Formel I, 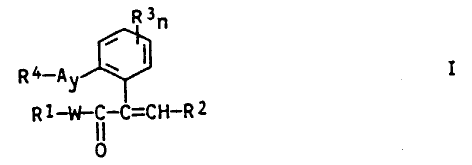 in der die Variablen die folgende Bedeutung haben:
n 0, 1, 2, 3 oder 4, wobei die Reste R³ verschieden sein können, wenn n für 2, 3 oder 4 steht;
y 0 oder 1;
R¹ Wasserstoff;
C₁-C₁₅-Alkyl, C₃-C₁₅-Alkenyl, C₃-C₈-Alkinyl oder C₃-C₈-Cycloalkyl, wobei diese Gruppen ein bis fünf Halogenatome tragen können;
Vinyl oder Ethinyl, wenn W für eine direkte Bindung steht;
R² Cyano, C₂-C₄-Alkenyl, C₂-C₄-Alkinyl,
C₃-C₆-Cycloalkyl, wobei der Ring neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
C₁-C₄-Alkyl, welches unsubstituiert oder partiell oder vollständig halogeniert sein kann;
C₁-C₂-Alkyl, welches eine C₁-C₄-Alkoxygruppe, eine C₁-C₄-Alkylthiogruppe oder einen 3- bis 6-gliedrigen cyclischen Rest trägt, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
R³ Wasserstoff, Nitro, Cyano, Halogen;
C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
oder, für den Fall, daß n 2, 3 oder 4 bedeutet, zwei benachbarte Substituenten zusammen eine 1,3-Butadien-1,4-diylgruppe, welche ein bis vier Halogenatome und/oder eine oder zwei der folgenden Gruppen tragen kann: Nitro, Cyano, C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
R⁴ Wasserstoff; CHO;
ggf. subst. Alkyl; ggf. subst. Alkenyl; ggf. subst. Alkinyl;
ein ggf. subst. gesättigter oder ein- oder zweifach ungesättigter Cyclus mit bis zu acht Ringgliedern, welcher neben Kohlenstoffatomen ein bis drei Heteroatome aus der Gruppe Sauerstoff, Schwefel und Stickstoff als Ringglieder enthalten kann;
R'₃P-, R'₂P(=O) - und R"₂P(=O)-, wobei R' Phenyl und R" C₁-C₄-Alkoxy bedeutet;
oder ein ggf. subst. ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann;
W eine direkte Bindung, Sauerstoff, Schwefel oder Stickstoff, wobei der Stickstoff ein Wasserstoffatom, eine C₁-C₄-Alkylgruppe oder eine C₁-C₄-Alkoxygruppe tragen kann;
A -O-; -C(=O)-; -O-C(=O)-; -C(=O)-O-;
-S-; -S(=O)-; -O-S(=O)-; -S(=O)-O-; -S(=O)₂-; -O-S(=O)₂-; -S (=O)₂-O-;
-NR⁵-; -O-NR⁵-; -NR⁵-C(=O)-; -C(=O)-NR⁵-; -NR⁵-C(=O)-NR⁵-; -S(=O)-NR⁵-; -NR⁵-S(=O)-; -S(=O)₂-NR⁵-; -NR⁵-S(=O)₂-;
-N=N-; -NR⁵-NR⁶-; -NR⁵-NR⁶-C(=O)-; -C(=O)-NR⁵-NR⁶-; -NR⁵-NR⁶-S(=O)₂-; -S(=O)₂-NR⁵-NR⁶-;
-O-(C₂-C₄-alkylen)-O-; -C(=O)-(C₂-C₄-alkylen)-O-; -O-C(=O)-(C₂-C₄-alkylen)-O-; -C(=O)-O-(C₂-C₄-alkylen)-O-;
-S-(C₂-C₄-alkylen)-O-; -S(=O)-(C₂-C₄-alkylen)-O-; -O-S(=O)₂(C₂-C₄-alkylen)-O-; -S(=O)₂-O- (C₂-C₄-alkylen)-O-;
-NR⁵-(C₂-C₄-alkylen)-O-; -ONR⁵-(C₂-C₄-alkylen)-O-; -NR⁵-C(=O)-(C₂-C₄-alkylen)-O-; -C(=O)-NR⁵-(C₂-C₄-alkylen)-O-; -NR⁵-C(=O)-NR⁶-(C₂-C₄-alkylen)-O-;
-NR⁵-NR⁶-(C₂-C₄-alkylen)-O-; -NR⁵-NR⁶-C(=O)-(C₂-C₄-alkylen)-O-; -C(=O)-NR⁵-NR⁶-(C₂-C₄-alkylen)-O-;
C₁-C₆-Alkylen, C₂-C₆-Alkenylen oder C₂-C₆-Alkinylen, wobei diese Kohlenstoffketten ein bis vier Reste, ausgewählt aus einer Gruppe bestehend aus C₁-C₄-Alkylgruppen und Halogenatomen, tragen können und wobei diese Kohlenstoffketten durch eine der folgenden Gruppen unterbrochen sein können oder durch eine der folgenden Gruppen an R⁴ oder an den Phenylring gebunden sein können: -O-, -S-, -NR⁵-, -C(=O)-, -C(=O)-O-, -O-C(=O)-, -S(=O), -S(=O)₂-, -S(=O)₂-O-, -O-S(=O)₂-, -NR⁵-C(=O)-, -C(=O)-NR⁵-, -NR⁵-C(=O)-NR⁶-, -N=N-, -NR⁵-NR⁶-, -NR⁵-NR⁶-C(=O)- und -C(=O)-NR⁵-NR⁶-;
R⁵ und R⁶ unabhängig voneinander
Wasserstoff, C₁-C₄-Alkyl oder C₁-C₄-Alkoxy;
wobei n nicht 0 bedeutet oder R¹ nicht C₁-C₅-Alkyl bedeutet oder W nicht Sauerstoff bedeutet, wenn R⁴ für ggf. subst. Phenyl steht und die Gruppierung A_{y} eine der folgenden Ketten bezeichnet: -O-, -CH₂O-, -OCH₂-, -CH₂CH₂-, -CH=CH-, -C≡C-, Oxy-C₂-C₆-alkylen oder Oxy-C₂-C₆-alenylen;
oder wobei n nicht 0 bedeutet oder R¹ nicht C₁-C₅-Alkyl bedeutet oder W nicht Sauerstoff bedeutet, wenn die Gruppierung A_{y} eine der folgenden Ketten bezeichnet: -C(=O)-OCH₂-, -O-C(=O)-OCH₂-, -C₁-C₁₂-Alkylen-C(=O)-OCH₂-, -Oxy-(C₁-C₁₂)-alkylen-C(=O)-OCH₂-, -C₁-C₁₂-Alkenylen-C(=O)-OCH₂- oder -Oxy-C₁-C₁₂-Alkenylen-C(=O)-OCH₂-, wobei die Alkylen- oder Alkenylengruppen Halogenatome tragen können.

2. Verfahren zur Herstellung der Verbindungen der allgemeinen Formel I gemäß Anspruch 1, in denen W nicht für eine direkte Bindung steht, dadurch gekennzeichnet, daß man eine Phenoxalsäure der allgemeinen Formel II 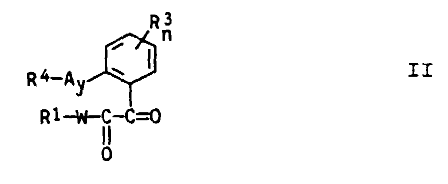 in an sich bekannter Weise in einem inerten organischen Lösungsmittel in Gegenwart einer Base mit einem Wittig- bzw. Wittig-Horner Reagens der allgemeinen Formeln IIIa, IIIb oder IIIc
R'₃P⁺-CH₂-R² Hal⁻ IIIa
R"₂P(=O)-CH₂-R² IIIb
R'₂P(=O)-CH₂-R² IIIc
in denen R' für Phenyl, R" für Alkoxy oder Phenyl und Hal für Halogen steht, umsetzt.

3. Verfahren zur Herstellung der Verbindungen der allgemeinen Formel I gemäß Anspruch 1, dadurch gekennzeichnet, daß man eine Phenylessigsäure der allgemeinen Formel IV 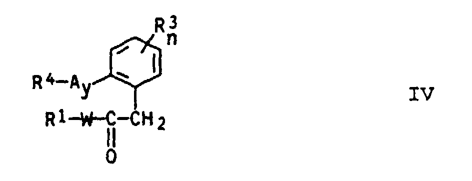 in an sich bekannter Weise in einem inerten organischen Lösungsmittel in Gegenwart einer Base mit einem Aldehyd der allgemeinen Formel V
O=CH-R² V
umsetzt.

4. Verfahren zur Herstellung der Verbindungen der allgemeinen Formel I gemäß Anspruch 1, dadurch gekennzeichnet, daß man eine Phosphonylphenylessigsäure der allgemeinen Formel VI 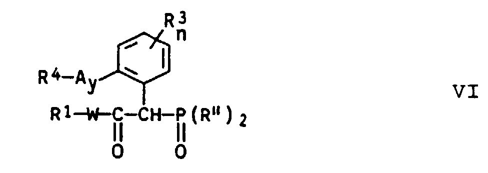 in an sich bekannter Weise in einem inerten organischen Lösungsmittel in Gegenwart einer Base mit einem Aldehyd der allgemeinen Formel V gemäß Anspruch 3 umsetzt.

5. Mittel zur Bekämpfung von Schadpilzen, enthaltend eine fungizid wirksame Menge einer Verbindung der allgemeinen Formel I gemäß Anspruch 1 und inerte Zusatzstoffe.

6. Verfahren zur Bekämpfung von Schadpilzen, dadurch gekennzeichnet, daß man die Schadpilze und/oder ihren Lebensraum mit einer wirksamen Menge einer Verbindung der allgemeinen Formel I gemäß Anspruch 1 behandelt.

7. Mittel zur Bekämpfung von Schädlingen, enthaltend inerte Zusatzstoffe und eine pestizid wirksame Menge einer Verbindung der allgemeinen Formel I gemäß Anspruch 1.

8. Verfahren zur Bekämpfung von Schädlingen aus der Klasse der Insekten, Spinnentiere und Nematoden, dadurch gekennzeichnet, daß man die Schädlingen und/oder ihren Lebensraum mit einer wirksamen Menge einer Verbindung der allgemeinen Formel I' 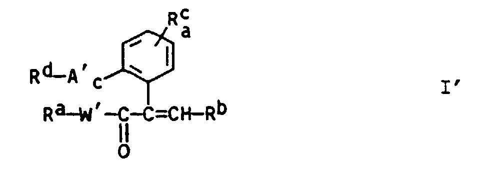 in der die Variablen die folgende Bedeutung haben:
a 0, 1, 2, 3 oder 4, wobei die Reste R^{c} verschieden sein können, wenn a für 2, 3 oder 4 steht;
c 0 oder 1;
R^{a} Wasserstoff;
C₁-C₁₅-Alkyl, C₃-C₁₅-Alkenyl, C₃-C₈-Alkinyl oder C₃-C₈-Cycloalkyl, wobei diese Gruppen ein bis fünf Halogenatome tragen können;
Vinyl oder Ethinyl, wenn W für eine direkte Bindung steht;
R^{b} Cyano, C₂-C₄-Alkenyl, C₂-C₄-Alkinyl,
C₃-C₆-Cycloalkyl, wobei der Ring neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
C₁-C₄-Alkyl, welches unsubstituiert oder partiell oder vollständig halogeniert sein kann;
C₁-C₂-Alkyl, welches eine C₁-C₄-Alkoxygruppe, eine C₁-C₄-Alkylthiogruppe oder einen 3- bis 6-gliedrigen cyclischen Rest trägt, wobei der cyclische Rest neben Kohlenstoffatomen ein oder zwei Heteroatome aus der Gruppe Stickstoff, Sauerstoff und Schwefel, die im Falle von Sauerstoff und/oder Schwefel nicht benachbart sein dürfen, enthalten kann, und wobei der Cyclus zusätzlich noch einen bis drei der folgenden Reste tragen kann: Halogen und C₁-C₄-Alkyl;
R^{c} Wasserstoff, Nitro, Cyano, Halogen;
C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
oder, für den Fall, daß n 2, 3 oder 4 bedeutet, zwei benachbarte Substituenten zusammen eine 1,3-Butadien-1,4-diylgruppe, welche ein bis vier Halogenatome und/oder eine oder zwei der folgenden Gruppen tragen kann: Nitro, Cyano, C₁-C₄-Alkyl, C₁-C₄-Alkoxy, partiell oder vollständig halogeniertes C₁-C₄-Alkyl, partiell oder vollständig halogeniertes C₁-C₄-Alkoxy oder C₁-C₄-Alkylthio;
R^{d} Wasserstoff;
ggf. subst. Alkyl; ggf. subst. Alkenyl; ggf. subst. Alkinyl;
ein ggf. subst. gesättigter oder ein- oder zweifach ungesättigter Cyclus mit bis zu acht Ringgliedern, welcher neben Kohlenstoffatomen ein bis drei Heteroatome aus der Gruppe Sauerstoff, Schwefel und Stickstoff als Ringglieder enthalten kann;
R'₃P-, R'₂P(=O)- und R"₂P(=O)-, wobei R' Phenyl und R" C₁-C₄-Alkoxy bedeutet;
oder ein ggf. subst. ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann;
W' eine direkte Bindung, Sauerstoff, Schwefel oder Stickstoff, wobei der Stickstoff ein Wasserstoffatom, eine C₁-C₄-Alkylgruppe oder eine C₁-C₄-Alkoxygruppe tragen kann;
A' -O-; -C(=O)-; -O-C(=O)-; -C(=O)-O-;
-S-; -S(=O)-; -O-S(=O)-; -S(=O)-O-; -S(=O)₂-; -O-S(=O)₂-; -S(=O)₂-O-;
-NR^{e}-; -O-NR^{e}-; -NR^{e}-C (=O)-; -C(=O)-NR^{e}-; -NR^{e}-C(=O)-NR^{e}-; -S(=O)-NR^{e}-; -NR^{e}-S(=O)-; -S(=O)₂-NR^{e}-; -NR^{e}-S(=O)₂-;
-N=N-; -NR^{e}-NR^{f}-; -NR^{e}-NR^{f}-C(=O)-; -C(=O)-NR^{e}-NR^{f}-; -NR^{e}-NR^{f}-S(=O)₂-; -S(=O)₂-NR^{e}-NR^{f}-;
-O-(C₂-C₄-alkylen)-O-; -C(=O)-(C₂-C₄-alkylen)-O-; -O-C(=O)-(C₂-C₄-alkylen)-O-; -C(=O)-O-(C₂-C₄-alkylen)-O-;
-S- (C₂-C₄-alkylen)-O-; -S(=O)-(C₂-C₄-alkylen)-O-; -O-S(=O)₂-(C₂-C₄-alkylen)-O-; -S(=O)₂-O-(C₂-C₄-alkylen)-O-;
-NR^{e}-(C₂-C₄-alkylen)-O-; -ONR^{e}-(C₂-C₄-alkylen)-O-; -NR^{e}-C(=O)-(C₂-C₄-alkylen)-O-; -C(=O)-NR^{e}-(C₂-C₄-alkylen)-O-; NR^{e}-C(=O)-NR^{f}-(C₂-C₄-alkylen)-O-;
-NR^{e}-NR^{f}-(C₂-C₄-alkylen)-O-; -NR^{e}-NR^{f}-C(=O)-(C₂-C₄-alkylen)-O-; -C(=O)-NR^{e}-NR^{f}-(C₂-C₄-alkylen)-O-;
C₁-C₆-Alkylen, C₂-C₆-Alkenylen oder C₂-C₆-Alkinylen, wobei diese Kohlenstoffketten ein bis vier Reste, ausgewählt aus einer Gruppe bestehend aus C₁-C₄-Alkylgruppen und Halogenatomen, tragen können und wobei diese Kohlenstoffketten durch eine der folgenden Gruppen unterbrochen sein können oder durch eine der folgenden Gruppen an R⁴ oder an den Phenylring gebunden sein können: -O-, -S-, -NR^{e}-, -C(=O)-, -C(=O)-O-, -O-C(=O)-, -S(=O), -S(=O)₂-, -S(=O)₂-O-, -O-S(=O)₂-, -NR^{e}-C(=O)-, -C(=O)-NR^{e}-, -NR^{e}-C(=O)-NR^{f}-, -N=N-, -NR^{e}-NR^{f}-, -NR^{e}-NR^{f}-C(=O)- und -C(=O)-NR^{e}-NR^{f}-;
R^{e} und R^{f} unabhängig voneinander
Wasserstoff, C₁-C₄-Alkyl oder C₁-C₄-Alkoxy;
behandelt.

9. Verbindungen der Formel I' gemäß Anspruch 8, in denen die Substituenten die folgende Bedeutung haben:
A'_{c} -O-, -S-, -OCH₂-, -SCH₂-, -CH=CH-, -CO₂CH₂- oder -N(CH₃)-CH₂;
R^{a} Methyl;
W Sauerstoff;
R^{b} Methyl, Ethyl oder Methoxymethyl;
a 0, und
R^{d} 2,5-Dichlorphenyl, 5-Chlor-2-methylphenyl, 2,4-Dimethylphenyl, 2-Chlor-5-methylphenyl, 2,3, 5-Trimethylphenyl oder 2-Methyl-5-isopropylphenyl,
sowie, für den Fall, daß A' für -OCH₂- und R^{b} für Methyl steht, 2,5-Dimethylphenyl.

10. Verbindungen der Formel I' gemäß Anspruch 9, in denen A' für -OCH₂-steht.

## Claims

1. An α-phenylacrylic acid derivative of the formula I 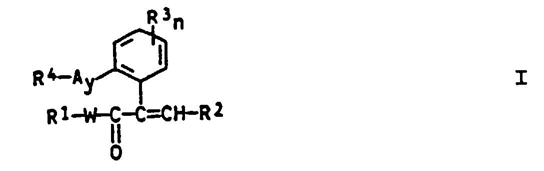 where
n is 0, 1, 2, 3 or 4 and the radicals R³ may be different when n is 2, 3 or 4;
y is 0 or 1;
R¹ is hydrogen;
C₁-C₁₅-alkyl, C₃-C₁₅-alkenyl, C₃-C₈-alkynyl or C₃-C₈-cycloalkyl, where these groups may carry from one to five halogen atoms;
vinyl or ethynyl when W is a direct bond;
R² is cyano, C₂-C₄-alkenyl, C₂-C₄-alkynyl;
C₃-C₆-cycloalkyl where, in addition to carbon atoms, the ring may contain one or two hetero atoms selected from the group consisting of nitrogen, oxygen and sulfur, which in the case of oxygen and/or sulfur must not be adjacent to one another, and where the cyclic structure may additionally carry from one to three of the following radicals: halogen or C₁-C₄-alkyl;
C₁-C₄-alkyl which may be unsubstituted or partially or completely halogenated;
C₁- or C₂-alkyl which carries C₁-C₄-alkoxy, C₁-C₄-alkylthio or a 3-membered to 6-membered cyclic radical which, in addition to carbon atoms, may contain one or two hetero atoms selected from the group consisting of nitrogen, oxygen and sulfur, which in the case of oxygen and/or sulfur must not be adjacent to one another, and where the cyclic structure may additionally carry from one to three of the following radicals: halogen or C₁-C₄-alkyl;
R³ is hydrogen, nitro, cyano, halogen;
C₁-C₄-alkyl, C₁-C₄-alkoxy, partially or completely halogenated C₁-C₄-alkyl, partially or completely halogenated C₁-C₄-alkoxy or C₁-C₄-alkylthio;
or, where n is 2, 3 or 4, two adjacent substituents together form a 1,3-butadiene-1,4-diyl group which may carry from one to four halogen atoms and/or one or two of the following groups: nitro, cyano, C₁-C₄-alkyl, C₁-C₄-alkoxy, partially or completely halogenated C₁-C₄-alkyl, partially or completely halogenated C₁-C₄-alkoxy or C₁-C₄-alkylthio;
R⁴ is hydrogen; CHO;
alkyl, alkenyl or alkynyl, each of which is unsubstituted or substituted;
an unsubstituted or substituted saturated or mono- or diunsaturated cyclic structure having up to eight ring members which, in addition to carbon atoms, may contain from one to three hetero atoms selected from the group consisting of oxygen, sulfur and nitrogen as ring members;
R'₃P-, R'₂P(=O)- or R"₂P(=O)-, where R' is phenyl and R" is C₁-C₄-alkoxy;
or an unsubstituted or substituted mononuclear to trinuclear aromatic system which, in addition to carbon atoms, may contain from one to four nitrogen atoms or from one to three hetero atoms selected from the group consisting of two nitrogen atoms and one oxygen or sulfur atom;
W is a direct bond, oxygen, sulfur or nitrogen, where the nitrogen may carry hydrogen, C₁-C₄-alkyl or C₁-C₄-alkoxy;
A is -O-; -C(=O)-; -O-C(=O)-; -C(=O)-O-;
-S-; -S(=O)-; -O-S-(=O)-; -S(=O)-O-; -S(=O)₂-; -O-S(=O)₂-; -S(=O)₂-O-;
-NR⁵-; -O-NR⁵-; -NR⁵-C(=O)-; -C(=O)-NR⁵-; -NR⁵-C(=O)-NR⁵-; -S(=O)-NR⁵-; -NR⁵-S(=O)-; -S(=O)₂-NR⁵-; -NR⁵-S(=O)₂-; -N=N-; -NR⁵-NR⁶-; -NR⁵-NR⁶-C (=O) -; -C(=O)-NR⁵-NR⁶-; -NR⁵-NR⁶-S(=O)₂-; -S(=O)₂-NR⁵-NR⁶-;
-O-(C₂-C₄-alkylene)-O-; -C(=O)-(C₂-C₄-alkylene)-O-; -O-C(=O) -(C₂-C₄-alkylene)-O-; -C(=O)-O-(C₂-C₄-alkylene)-O-; -S-(C₂-C₄-alkylene)-O-; -S(=O)-(C₂-C₄-alkylene)-O-; -O-S(=O)₂-(C₂-C₄-alkylene)-O-;-S-(=O)₂-O-(C₂-C₄-alkylene)-O-; -NR⁵-(C₂-C₄-alkylene)-O-; -ONR⁵-(C₂-C₄-alkylene)-O-; -NR⁵-C(=O)-(C₂-C₄-alkylene)-O-; -C(=O)-NR⁵-(C₂-C₄-alkylene)-O-; -NR⁵-C(=O)-NR⁶-(C₂-C₄-alkylene)-O-; -NR⁵-NR⁶-(C₂-C₄-alkylene)-O-; -NR⁵-NR⁶-C(=O)-(C₂-C₄-alkylene)-O-; -C(=O)-NR⁵-NR⁶-(C₂-C₄-alkylene)-O-;
C₁-C₆-alkylene, C₂-C₆-alkenylene or C₂-C₆-alkynylene, where these carbon chains may carry from one to four radicals selected from the group consisting of C₁-C₄-alkyl and halogen, and may be interrupted by one of the following groups or bonded to R⁴ or to the phenyl ring by one of the following groups: -O-, -S-, -NR⁵-, -C(=O)-, -C(=O)-O-, -O-C(=O)-, -S(=O)-, -S(=O)₂-, -S(=O)₂-O-, -O-S (=O)₂-, -NR⁵-C(=O)-, -C(=O)-NR⁵-, -NR⁵-C(=O)-NR⁶-, -N=N-, -NR⁵-NR⁶-, -NR⁵-NR⁶-C(=O)- or -C(=O)-NR⁵-NR⁶-; R⁵ and R⁶ independently of one another are each hydrogen, C₁-C₄-alkyl or C₁-C₄-alkoxy;
where n is not 0 or R¹ is not C₁-C₅-alkyl or W is not oxygen when R⁴ is unsubstituted or substituted phenyl and A_{y} is one of the following chains: -O-, -CH₂O-, -OCH₂-, -CH₂CH₂-, -CH=CH-, -C≡C-, oxy-C₂-C₆-alkylene or oxy-C₂-C₆-alkenylene;
or where n is not 0 or R¹ is not C₁-C₅-alkyl or W is not oxygen when A_{y} is one of the following chains: -C(=O)-O-CH₂-, -O-C(=O)-OCH₂-, -C₁-C₁₂-alkylene-C(=O)-OCH₂-, -oxy-(C₁-C₁₂)-alkylene-C(=O)-OCH₂-,-C₁-C₁₂-alkenylene-C(=O)-O-CH₂- or -oxy-(C₁-C₁₂) -alkenylene-C(=O)-OCH₂-, where the alkylene or alkenylene groups may carry halogen atoms.

2. A process for the preparation of a compound of the formula I as claimed in claim 1, in which W is not a direct bond, wherein a phenoxalic acid of the formula II 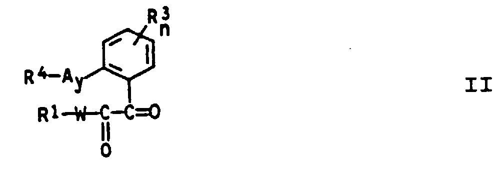 is reacted in a conventional manner with a Wittig or Wittig-Horner reagent of the formulae IIIa, IIIb or IIIc
R'₃P⁺-CH₂-R² Hal⁻ IIIa
R"₂P (=O)-CH₂-R² IIIb
R'₂P(=O)-CH₂-R² IIIc
where R' is phenyl, R" is alkoxy or phenyl and Hal is halogen, in an inert organic solvent in the presence of a base.

3. A process for the preparation of a compound of the formula I as claimed in claim 1, wherein a phenylacetic acid of the formula IV 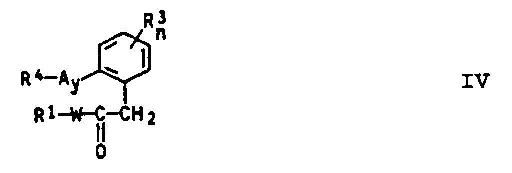 is reacted in a conventional manner with an aldehyde of the formula V
O=CH-R² V
in an inert organic solvent in the presence of a base.

4. A process for the preparation of a compound of the formula I as claimed in claim 1, wherein a phosphonylphenylacetic acid of the formula VI 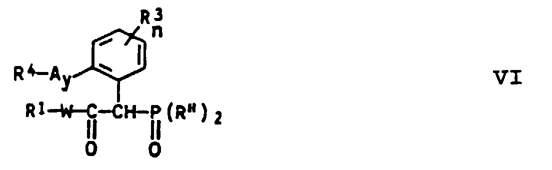 is reacted in a conventional manner with an aldehyde of the formula V as in claim 3 in an inert organic solvent in the presence of a base.

5. A fungicide containing a fungicidal amount of a compound of the formula I as claimed in claim 1 and inert additives.

6. A method for controlling harmful fungi, wherein the harmful fungi and/or their habitat is or are treated with an effective amount of a compound of the formula I as claimed in claim 1.

7. A pesticide containing inert additives and a pesticidal amount of a compound of the formula I as claimed in claim 1.

8. A method for controlling pests from the class of the insects, arachnids and nematodes, wherein the pests and/or their habitat is or are treated with an effective amount of a compound of the formula I' 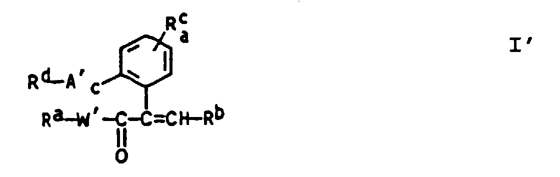 where
a is 0, 1, 2, 3 or 4 and the radicals R^{c} may be different when a is 2, 3 or 4;
c is 0 or 1;
R^{a} is hydrogen;
C₁-C₁₅-alkyl, C₃-C₁₅-alkenyl, C₃-C₈-alkynyl or C₃-C₈-cycloalkyl, where these groups may carry from one to five halogen atoms;
vinyl or ethynyl when W is a direct bond;
R^{b} is cyano, C₂-C₄-alkenyl, C₂-C₄-alkynyl;
C₃-C₆-cycloalkyl where the ring, in addition to carbon atoms, may contain one or two hetero atoms selected from the group consisting of nitrogen, oxygen and sulfur, which in the case of oxygen and/or sulfur must not be adjacent to one another, and where the cyclic structure may additionally carry from one to three of the following radicals: halogen or C₁-C₄-alkyl;
C₁-C₄-alkyl which may be unsubstituted or partially halogenated;
C₁- or C₂-alkyl which carries C₁-C₄-alkoxy, C₁-C₄-alkylthio or a 3-membered to 6-membered cyclic radical which, in addition to carbon atoms, may contain one or two hetero atoms selected from the group consisting of nitrogen, oxygen and sulfur, which in the case of oxygen and/or sulfur must not be adjacent to one another, and where the cyclic structure may additionally carry from one to three of the following radicals: halogen and C₁-C₄-alkyl;
R^{c} is hydrogen, nitro, cyano, halogen;
C₁-C₄-alkyl, C₁-C₄-alkoxy, partially or completely halogenated C₁-C₄-alkyl, partially or completely halogenated C₁-C₄-alkoxy or C₁-C₄-alkylthio;
or, where n is 2, 3 or 4, two adjacent substituents together form a 1,3-butadiene-1,4-diyl group which may carry from one to four halogen atoms and/or one or two of the following groups: nitro, cyano, C₁-C₄-alkyl, C₁-C₄-alkoxy, partially or completely halogenated C₁-C₄-alkyl, partially or completely halogenated C₁-C₄-alkoxy or C₁-C₄-alkylthio;
R^{d} is hydrogen;
alkyl, alkenyl or alkynyl, each of which is unsubstituted or substituted;
an unsubstituted or substituted, saturated or mono- or diunsaturated cyclic structure having up to eight ring members which, in addition to carbon atoms, may contain from one to three hetero atoms, selected from the group consisting of oxygen, sulfur and nitrogen, as ring members;
R'₃P-, R'₂P(=O)- or R"₂P(=O)-, where R' is phenyl and R" is C₁-C₄-alkoxy;
or an unsubstituted or substituted mono- to trinuclear aromatic system which, in addition to carbon atoms, may contain from one to four nitrogen atoms or from one to three hetero atoms selected from the group consisting of two nitrogen atoms and one oxygen or sulfur atom;
W' is a direct bond, oxygen, sulfur or nitrogen, where the nitrogen may carry hydrogen, C₁-C₄-alkyl or C₁-C₄-alkoxy;
A' is -O-; -C(=O)-; -O-C(=O)-; -C(=O)-O-;
- S-; -S (=O)-; -O-S-(=O)-; -S(=O)-O-; -S(=O)₂-; -O-S(=O)₂-; -S(=O)₂-O-;
-NR^{e}-; -O-NR^{e}-; -NR^{e}-C(=O)-; -C(=O)-NR^{e}-; -NR^{e}-C(=O)-NR^{e}-; -S(=O)-NR^{e}-; -NR^{e}-S(=O)-; -S(=O)₂-NR^{e}-; -NR^{e}-S(=O)₂-; -N=N-; -NR^{e}-NR^{f}-; -NR^{e}-NR^{f}-C(=O)-; -C(=O)-NR^{e}-NR^{f}-; -NR^{e}-NR^{f}-S(=O)₂-; -S(=O)₂-NR^{e}-NR^{f}-;
-O-(C₂-C₄-alkylene)-O-; -C(=O)-(C₂-C₄-alkylene)-O-; -O-C(=O)-(C₂-C₄-alkylene)-O-; -C(=O)-O-(C₂-C₄-alkylene)-O-; -S-(C₂-C₄-alkylene)-O-; -S(=O)-(C₂-C₄-alkylene)-O-; -O-S(=O)₂-(C₂-C₄-alkylene)-O-; -S(=O)₂-O-(C₂-C₄-alkylene)-O-; -NR^{e}-(C₂-C₄-alkylene)-O-; -ONR^{e}-(C₂-C₄-alkylene)-O-; -NR^{e}-C(=O)-(C₂-C₄-alkylene)-O-; -C(=O)-NR^{e}-(C₂-C₄-alkylene)-O-; -NR^{e}-C(=O)-NR^{f}-(C₂-C₄-alkylene)-O-;
-NR^{e}-NR^{f}-(C₂-C₄-alkylene)-O-; -NR^{e}-NR^{f}-C(=O)-(C₂-C₄-alkylene)-O-; -C(=O)-NR^{e}-NR^{f}-(C₂-C₄-alkylene)-O-;
C₁-C₆-alkylene, C₂-C₆-alkenylene or C₂-C₆-alkynylene, where these carbon chains may carry from one to four radicals selected from the group consisting of C₁-C₄-alkyl and halogen, and may be interrupted by one of the following groups or may be bonded to R^{d} or to the phenyl ring by one of the following groups: -O-, -S-, -NR^{e}-, -C(=O)-, -C(=O)-O-, -O-C(=O)-, -S(=O)-, -S(=O)₂-, -S(=O)₂-O-, -O-S(=O)₂-, -NR^{e}-C(=O)-, -C(=O)-NR^{e}-, -NR^{e}-C(=O)-NR^{f}-, -N=N-, -NR^{e}-NR^{f}-, -NR^{e}-NR^{f}-C(=O)- or -C(=O). NR^{e}-NR^{f}-;
and R^{e} and R^{f} independently of one another are each hydrogen, C₁-C₄-alkyl or C₁-C₄-alkoxy.

9. A compound of the formula I'as claimed in claim 8, wherein A'_{c} is -O-, -S-, -OCH₂-, -SCH₂-, -CH=CH-, -CO₂CH₂- or -N(CH₃)-CH₂;
R^{a} is methyl;
W is oxygen;
R^{b} is methyl, ethyl or methoxymethyl;
a is 0, and
R^{d} is 2,5-dichlorophenyl, 5-chloro-2-methylphenyl, 2,4-dimethylphenyl, 2-chloro-5-methylphenyl, 2,3,5-trimethylphenyl or 2-methyl-5-isopropylphenyl, or, where A' is -OCH₂- and R^{b} is methyl, 2,5-dimethylphenyl.

10. A compound of the formula I' as claimed in claim 9, wherein A' is -OCH₂-.

## Revendications

1. Dérivés de l'acide α-phénylacrylique de formule générale I 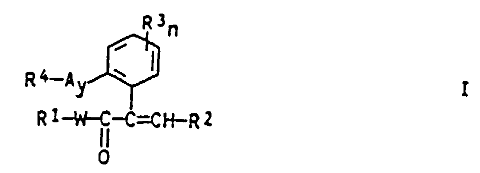 dans laquelle les symboles ont les significations suivantes :
n est un nombre égal à 0, 1, 2, 3 ou 4, les substituants R³ pouvant être différents lorsque n est égal à 2, 3 ou 4 ;
y est égal à 0 ou 1 ;
R¹ représente l'hydrogène ;
un groupe alkyle en C1-C15, alcényle en C3-C15, alcynyle en C3-C8 ou cycloalkyle en C3-C8, ces groupes pouvant porter un à cinq atomes d'halogènes ;
un groupe vinyle ou éthynyle lorsque W représente une liaison directe ;
R² représente un groupe cyano, alcényle en C2-C4, alcynyle en C2-C4,
un groupe cycloalkyle en C3-C6 dont le cycle, en plus des atomes de carbone, peut contenir un ou deux hétéroatomes choisis parmi l'azote, l'oxygène et le soufre, ces hétéroatomes ne pouvant être voisins dans le cas de l'oxygène et/ou du soufre, et le cycle peut encore porter un à trois des substituants suivants : les halogènes et les groupes alkyle en C1-C4 ;
un groupe alkyle en C1-C4 qui peut être non substitué ou halogéné en totalité ou en partie ;
un groupe alkyle en C1-C2 qui porte un groupe alcoxy en C1-C4, un groupe alkylthio en C1-C4 ou un groupe cyclique de trois à six chaînons, ce dernier pouvant contenir, en plus des atomes de carbone, un ou deux hétéroatomes choisis parmi l'azote, l'oxygène et le soufre, ces hétéroatomes ne pouvant être voisins dans le cas de l'oxygène et/ou du soufre, le cycle pouvant encore porter un à trois des substituants suivants : les halogènes et les groupes alkyle en C1-C4 ;
R³ représente l'hydrogène, un groupe nitro, cyano, un halogène ;
un groupe alkyle en C1-C4, alcoxy en C1-C4 partiellement ou totalement halogéné, alcoxy en C1-C4 partiellement ou totalement halogéné ou alkylthio en C1-C4 ;
ou bien, lorsque n est égal à 2, 3 ou 4, deux substituants voisins forment ensemble un groupe 1,3-butadiène-1,4-diyle qui peut porter un à quatre atomes d'halogènes et/ou un ou deux groupes suivants : nitro, cyano, alkyle en C1-C4, alcoxy en C1-C4, alkyle en C1-C4 partiellement ou totalement halogéné, alcoxy en C1-C4 partiellement ou totalement halogéné, ou alkylthio en C1-C4 ;
R⁴ représente l'hydrogène ; un groupe CHO ;
un groupe alkyle éventuellement substitué, alcényle éventuellement substitué ;
alcynyle éventuellement substitué ;
un cycle saturé ou mono- ou di-insaturé, éventuellement substitué, contenant jusqu'à huit chaînons cycliques et qui, en plus des atomes de carbone, peut contenir un à trois hétéroatomes choisis parmi l'oxygène, le soufre et l'azote en tant que chaînons cycliques ;
un groupe R'₃P-, R'₂P(=O)- ou R"₂P(=O)-, R' représentant un groupe phényle et R" un groupe alcoxy en C1-C4 ;
ou bien un système aromatique mono- à tri-cyclique éventuellement substitué qui, en plus des atomes de carbone, peut contenir un à quatre atomes d'azote ou un à trois hétéroatomes choisis dans un groupe consistant en deux atomes d'azote et un atome d'oxygène ou de soufre ;
W représente une liaison directe, l'oxygène, le soufre ou l'azote, l'azote pouvant porter un atome d'hydrogène, un groupe alkyle en C1-C4 ou alcoxy en C1-C4 ;
A représente -O-; -C(=O)-; -O-C(=O)- ; -C(=O)-O- ;
-S- ; -S(=O)- ; -O-S(=O)- ; -S(=O)-O- ; -S(=O)₂- ; -O-S(=O)₂- ; S(=O)₂-O- ;
-NR⁵-; -O-NR⁵-, -NR⁵-C(=O)-; -C(=O)-NR⁵- ; -NR⁵-C(=O)-NR⁵-;-S(=O)-NR⁵-; -NR⁵-S(=O)-; -S(=O)₂-NR⁵-; -NR⁵-S(=O)₂- ;
-N=N- ; -NR⁵-NR⁶-; -NR⁵-NR⁶-C(=O)-; -C(=O)-NR⁵-NR⁶- ; -NR⁵-NR⁶-S(=O)₂- ; -S(=O)₂-NR⁵-NR⁶- ;
-O-(alkylène en C2-C4)-O- ; -C(=O)-(alkylène en C2-C4)-O- ; -O-C(=O)-(alkylène en C2-C4)-O- ; -C(=O)-O-(alkylène en C2-C4)-O- ;
-S-(alkylène en C2-C4)-O- ; -S(=O)-(alkylène en C2-C4)-O- ; -O-S(=O)₂-(alkylène en C2-C4)-O- ; S(=O)₂-O-(alkylène en C2-C4)-O- ;
-NR⁵-(alkylène en C2-C4)-O- ; -ONR⁵-(alkylène en C2-C4)-O- ; -NR⁵-C(=O)-(alkylène en C2-C4)-O- ; -C(=O)-NR⁵-(alkylène en C2-C4)-O- ; -NR⁵-C(=O)-NR⁶-(alkylène en C2-C4)-O- ;
-NR⁵-NR⁶-(alkylène en C2-C4)-O- ; -NR⁵-NR⁶-C(=O)-(alkylène en C2-C4)-O- ; -C(=O)-NR⁵-NR⁶-(alkylène en C2-C4)-O- ;
un groupe alkylène en C1-C6, alcénylène en C2-C6 ou alcynylène en C2-C6, ces chaînes carbonées pouvant porter un à quatre substituants choisis dans un groupe consistant en les groupes alkyle en C1-C4 et les atomes d'halogènes, ces chaînes carbonées pouvant être interrompues par l'un des groupes suivants ou reliées à R⁴ ou au cycle phényle par l'un des groupes suivants : -O-, -S-, -NR⁵-, -C(=O)-, -C(=O)-O-, -O-C(=O)-, -S(=O), -S(=O)₂-, -S(=O)₂-O-, -O-S(=O)₂-, -NR₅-C(=O)-, -C(=O)-NR⁵-, -NR⁵-C(=O)-NR⁶-, -N=N-, -NR⁵-NR⁶-, -NR⁵-NR⁶-C(=O)- et -C(=O)-NR⁵-R⁶- ;
R⁵ et R⁶ représentent chacun, indépendamment l'un de l'autre :
l'hydrogène, un groupe alkyle en C1-C4 ou alcoxy en C1-C4 ;
sous réserve que
n ne peut être égal à 0 ou bien R¹ ne peut représenter un groupe alkyle en C1-C5 ou bien
W ne peut représenter l'oxygène lorsque R⁴ représente un groupe phényle éventuellement substitué et que le groupement A_{y} consiste en l'une des chaînes suivantes : -O-, -CH₂O-, -OCH₂-, -CH₂CH₂-,-CH=CH-, -C≡C-, oxyalkylène en C2-C6 ou oxyalcénylène en C2-C6 ;
ou bien n ne peut être égal à 0 ou bien R¹ ne peut représenter un groupe alkyle en C1-C5 ou bien W ne peut représenter l'oxygène lorsque le groupement Ay consiste en l'une des chaînes suivantes :-C(=O)-OCH₂-, -O-C-=O)-OCH₂-, -alkylène en C1-C12-C-=O)-OCH₂-, -oxy-alkylène en C1-C12-C(=O)-OCH₂, -alcénylène en C1-C12-C(=O)-OCH₂ ou -oxy-alcénylène en C1-C12-C(=O)-OCH₂-, les groupes alkylène et alcénylène pouvant porter des atomes d'halogènes.

2. Procédé de préparation des composés de formule générale I de la revendication 1 dans laquelle W ne représente pas une liaison directe, caractérisé par le fait que l'on fait réagir un acide phénoxalique de formule générale II 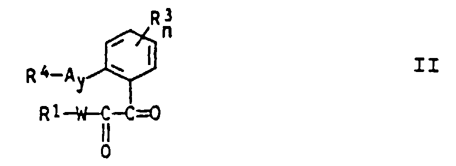 de manière connue en soi, dans un solvant organique inerte, en présence d'une base, avec un réactif de Wittig ou de Wittig-Horner de formule générale IIIa, IIIb ou IIIc
R'₃P⁺-CH₂-R² Hal⁻ IIIa
R"₂P(=O)-CH₂-R² IIIb
R'₂P(=O)-CH₂-R² IIIc
dans lesquelles R' représente un groupe phényle, R" un groupe alcoxy ou phényle et Hal un halogène.

3. Procédé de préparation des composés de formule générale I de la revendication 1, caractérisé par le fait que l'on fait réagir un acide phénylacétique de formule générale IV 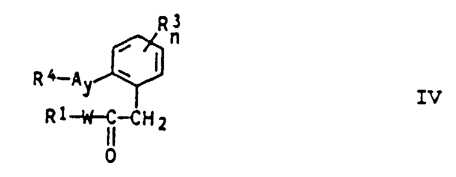 de manière connue en soi, dans un solvant organique inerte, en présence d'une base, avec un aldéhyde de formule générale V
O=CH-R² V

4. Procédé de préparation des composés de formule générale I de la revendication 1, caractérisé par le fait que l'on fait réagir un acide phosphonylphénylacétique de formule générale VI 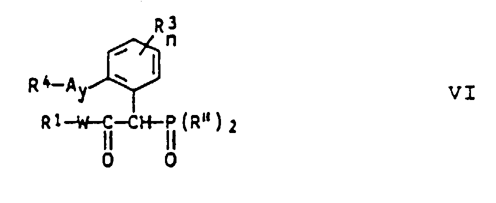 de manière connue en soi, dans un solvant organique inerte, en présence d'une base, avec un aldéhyde de formule générale V de la revendication 3.

5. Produit pour combattre les mycètes nuisibles, contenant une quantité fongicide efficace d'un composé de formule générale I de la revendication 1 et des additifs inertes.

6. Procédé pour combattre les mycètes nuisibles, caractérisé par le fait que l'on traite ces mycètes et/ou leur habitat par une quantité efficace d'un composé de formule générale I de la revendication 1.

7. Produit pour combattre les parasites, contenant des additifs inertes et une quantité parasiticide efficace d'un composé de formule générale I de la revendication 1.

8. Procédé pour combattre les parasites de la classe des insectes, des arachnides et des nématodes, caractérisé par le fait que l'on traite les parasites et/ou leur habitat par une quantité efficace d'un composé de formule générale I' 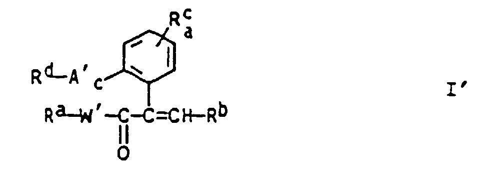 dans laquelle les symboles ont les significations suivantes :
a est égal à 0, 1, 2, 3 ou 4, les substituants R^{c} pouvant être différents lorsque a est égal à 2, 3 ou 4 ;
c est égal à 0 ou 1 ;
R^{a} représente l'hydrogène ;
un groupe alkyle en C1-C15, alcényle en C3-C15, alcynyle en C3-C8 ou cycloalkyle en C3-C8, ces groupes pouvant porter un à cinq atomes d'halogènes ;
un groupe vinyle ou éthynyle lorsque W représente une liaison directe ;
R^{b} représente
un groupe cyano, alcényle en C2-C4, alcynyle en C2-C4 , un groupe cycloalkyle en C3-C6 dont le cycle peut contenir, en plus des atomes de carbone, un ou deux hétéroatomes choisis parmi l'azote, l'oxygène et le soufre, mais qui ne peuvent être voisins dans le cas de l'oxygène et/ou du soufre, ce cycle pouvant en outre porter un à trois des substituants suivants : les halogènes et les groupes alkyle en C1-C4 ;
un groupe alkyle en C1-C4 non substitué ou qui peut être partiellement ou totalement halogéné ;
un groupe alkyle en C1-C2 qui porte un groupe alcoxy en C1-C4, un groupe alkylthio en C1-C4 ou un groupe cyclique de trois à six chaînons, ce groupe cyclique pouvant contenir, en plus des atomes de carbone, un ou deux hétéroatomes choisis parmi l'azote, l'oxygène et le soufre mais qui, dans le cas de l'oxygène et/ou du soufre, ne peuvent être voisins, et ce cycle pouvant en outre porter un à trois des substituants suivants : les halogènes et les groupes alkyle en C1-C4 ;
R^{c} représente l'hydrogène, un groupe nitro, cyano, un halogène ;
un groupe alkyle en C1-C4, alcoxy en C1-C4, alkyle en C1-C4 partiellement ou totalement halogéné, un groupe alcoxy en C1-C4 partiellement ou totalement halogéné, ou un groupe alkylthio en C1-C4 ;
ou bien, lorsque n est égal à 2, 3 ou 4, deux substituants voisins peuvent former ensemble un groupe 1,3-butandiène-1,4-diyle qui peut porter un à quatre atomes d'halogènes et/ou un ou deux des substituants suivants : nitro, cyano, alkyle en C1-C4, alcoxy en C1-C4, alkyle en C1-C4 partiellement ou totalement halogéné, alcoxy en C1-C4 partiellement ou totalement halogéné ou alkylthio en C1-C4 ;
R^{d} représente
l'hydrogène ;
un groupe alkyle éventuellement substitué ; alcényle éventuellement substitué ; alcynyle éventuellement substitué ;
un cycle saturé ou mono- ou di-insaturé, éventuellement substitué, contenant jusqu'à huit chaînons cycliques et qui, en plus des atomes de carbone, peut contenir un à trois hétéroatomes choisis parmi l'oxygène, le soufre et l'azote, en tant que chaînons cycliques ;
un groupe R'3P-, R'₂(=O)- ou R"₂P(=O)-, R' représentant un groupe phényle et R" un groupe alcoxy en C1-C4 ;
ou bien un système aromatique mono- à tri-cyclique éventuellement substitué qui, en plus des atomes de carbone, peut contenir un à quatre atomes d'azote ou un à trois hétéroatomes choisis dans un groupe consistant en deux atomes d'azote et un atome d'oxygène et de soufre ;
W' représente une liaison directe, l'oxygène, le soufre ou l'azote, l'azote pouvant porter un atome d'hydrogène, un groupe alkyle en C1-C4 ou un groupe alcoxy en C1-C4 ;
A' représente -O-; -C(=O)- ; -O-C(=O)- ; -C(=O)-O- ;
-S- ; -S(=O)- ; -O-S-=O)- ;-S(=O)-O- ; -S(=O)₂- ; -O-S(=O)₂- ;-S(=O)₂-O- ;
-NR^{e}- ; -O-NR^{e}- ; -NR^{e}-C(=O)- ; -C(=O)-NR^{e}- ; -NR^{e}-C(=O)-NR^{e}- ; -S(=O)-NR^{e} ; -NR^{e}-S(=O)- ; -S(=O)₂-NR^{e}- ; -NR^{e}-S(=O)₂- ;
-N=N- ; -NR^{e}-NR^{f}- ; -NR^{e}-NR^{f}-C(=O)- ; -C(=O)-NR^{e}-NR^{f}- ; -NR^{e}-NR^{f}-S(=O)2- ; -S(=O)₂-NR^{e}-NR^{f}- ;
-O-(alkylène en C2-C4)-O- ; -C(=O)-(alkylène en C2-C4)-O- ; -O-C(=O)-(alkylène en C2-C4)-O- ; -C(=O)-O-(alkylène en C2-C4)-O- ;
-S-(alkylène en C2-C4)-O- ; -S(=O)-(alkylène en C2-C4)-O- ; -O-S(=O)-(alkylène en C2-C4)-O- ; -S(=O)₂-O-(alkylène en C2-C4)-O- ;
-NR^{e}-(alkylène en C2-C4)-O- ; -ONR^{e-}(alkylène en C2-C4)-O- ; -NR^{e}-C(=O)-(alkylène en C2-C4)-O- ; -C(=O)-NR^{e}-(alkylène en C2-C4)-O- ; -NR^{e}-C(=O)-NR^{f}-(alkylène en C2-C4)-O- ;
-NR^{e}-NR^{f}-(alkylène en C2-C4)-O- ; -NR^{e}-NR^{f}-C(=O)-(alkylène en C2-C4)-O- ; -C(=O)-NR^{e}-NR^{f}-(alkylène en C2-C4)-O- ;
un groupe alkylène en C1-C6, alcénylène en C2-C6 ou alcynylène en C2-C6, ces chaînes carbonées pouvant porter un à quatre substituants choisis dans un groupe consistant en les groupes alkyle en C1-C4 et les atomes d'halogènes, et ces chaînes carbonées pouvant être interrompues par l'un des groupes suivants ou reliées à R⁴ ou au cycle phényle par l'un des groupes suivants : -O-, -S-, -NR^{e}-, -C(=O)-, -C(=O)-O-, -O-C(=O)-, -S(=O), -S(=O)₂-, -S(=O)₂-O-, -O-S(=O)₂-, -NR^{e}-C(=O)-,-C(=O)-NR^{e}-, -NR^{e}-C(=O)-NR^{f}-, -N=N-, -NR^{e}-NR^{f}-, -NR^{e}-NR^{f}-C(=O)-et -C(=O)-NR^{e}-NR^{f}- ;
R^{e} et R^{f} représentent chacun, indépendamment l'un de l'autre, l'hydrogène, un groupe alkyle en C1-C4 ou alcoxy en C1-C4

9. Composés de formule I' de la revendication 8, dans laquelle les symboles ont les significations suivantes :
A'c représente -O-, -S-, -OCH₂-, -SCH₂-, -CH-CH-, -CO₂CH₂- ou-N(CH₃)-CH₂ ;
R^{a} représente un groupe méthyle ;
W représente l'oxygène ;
R^{b} représente un groupe méthyle, éthyle ou méthoxyméthyle ;
a est égal à 0 et
R^{d} représente un groupe 2,5-dichlorophényle, 5-chloro-2-méthylphényle, 2,4-diméthylphényle, 2-chloro-5-méthylphényle, 2,3,5-triméthylphényle ou 2-méthyl-5-isopropylphényle, ou bien encore, lorsque A' représente -OCH₂- et R^{b} un groupe méthyle, un groupe 2,5-diméthylphényle.

10. Composés de formule I' de la revendication 9 dans laquelle A' représente -OCH₂-.
